(19) Europäisches Patentamt / European Patent Office / Office européen des brevets

(11) **EP 3 589 742 B1**

(12) # EUROPEAN PATENT SPECIFICATION

(45) Date of publication and mention of the grant of the patent: **04.08.2021 Bulletin 2021/31**

(21) Application number: **18710124.1**

(22) Date of filing: **28.02.2018**

(51) Int Cl.: ***C12Q 1/02*** *(2006.01)* ***C12Q 1/04*** *(2006.01)* ***C12Q 1/10*** *(2006.01)*

(86) International application number: **PCT/GB2018/050522**

(87) International publication number: **WO 2018/158573 (07.09.2018 Gazette 2018/36)**

(54) **METHOD OF DETECTION**

VERFAHREN ZUR DETEKTION

PROCÉDÉ DE DÉTECTION

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2017 GB 201703261**

(43) Date of publication of application: **08.01.2020 Bulletin 2020/02**

(73) Proprietor: **Imperial College Innovations Limited London SW7 2AZ (GB)**

(72) Inventors:
- **LARROUY-MAUMUS, Gerald London SW7 2AZ (GB)**
- **DORTET, Laurent London SW7 2AZ (GB)**
- **FILLOUX, Alain London SW7 2AZ (GB)**

(74) Representative: **MacLean, Martin Robert et al**
**Mathys & Squire**
**The Shard**
**32 London Bridge Street**
**London SE1 9SG (GB)**

(56) References cited:
**US-A1- 2012 197 535**

- **LUIS A. ARROYO ET AL: "The pmrCAB operon mediates polymyxin resistance in Acinetobacter baumannii ATCC 17978 and clinical isolates through phosphoethanolamine modification of Lipid A.", ANTIMICROBIAL AGENTS AND CHEMOTHERAPY, vol. 55, no. 8, 6 June 2011 (2011-06-06), pages 3743-3751, XP055458878, ISSN: 0066-4804, DOI: 10.1128/AAC.00256-11**
- **AN X. TRAN ET AL: "Periplasmic Cleavage and Modification of the 1-Phosphate Group of Helicobacter pylori Lipid A", JOURNAL OF BIOLOGICAL CHEMISTRY, vol. 279, no. 53, 31 December 2004 (2004-12-31), pages 55780-55791, XP055466978, US ISSN: 0021-9258, DOI: 10.1074/jbc.M406480200**
- **Y.K. PARK ET AL: "Transcriptomic analysis of colistin-susceptible and colistin-resistant isolates identifies genes associated with colistin resistance in Acinetobacter baumannii", CLINICAL MICROBIOLOGY AND INFECTION., vol. 21, no. 8, 1 August 2015 (2015-08-01) , pages 765.e1-765.e7, XP055467007, United Kingdom, Switzerland ISSN: 1198-743X, DOI: 10.1016/j.cmi.2015.04.009**
- **ZUBAIR A. QURESHI ET AL: "Colistin-Resistant Acinetobacter baumannii: Beyond Carbapenem Resistance", CLINICAL INFECTIOUS DISEASES, vol. 60, no. 9, 28 January 2015 (2015-01-28), pages 1295-1303, XP055467009, US ISSN: 1058-4838, DOI: 10.1093/cid/civ048**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 589 742 B1

- MARK R. PELLETIER ET AL: "Unique Structural Modifications Are Present in the Lipopolysaccharide from Colistin-Resistant Strains of Acinetobacter baumannii", ANTIMICROBIAL AGENTS AND CHEMOTHERAPY, vol. 57, no. 10, 1 October 2013 (2013-10-01), pages 4831-4840, XP055467017, ISSN: 0066-4804, DOI: 10.1128/AAC.00865-13
- MURALI PENDELA ET AL: "Characterization of dihydrostreptomycin-related substances by liquid chromatography coupled to ion trap mass spectrometry", RAPID COMMUNICATIONS IN MASS SPECTROMETRY., vol. 23, no. 12, 30 June 2009 (2009-06-30) , pages 1856-1862, XP055467042, GB ISSN: 0951-4198, DOI: 10.1002/rcm.4080

SEQUENCE LISTING

<110> Imperial Innovations Limited

<120> P48551WO - Sequence Listing

<130> P48551WO

<160> 16

<170> PatentIn version 3.5

<210> 1
<211> 1626
<212> DNA
<213> Escherichia coli

<400> 1

```
atgatgcagc atacttctgt gtggtaccga cgctcggtca gtccgtttgt tcttgtggcg      60
agtgttgccg ttttcttgac cgcgaccgcc aatcttacct tttttgataa aatcagccaa     120
acctatccca tcgcggacaa tctcggcttt gtgctgacga tcgctgtcgt gctctttggc     180
gcgatgctac tgatcaccac gctgttatca tcgtatcgct atgtgctaaa gcctgtgttg     240
attttgctat taatcatggg cgcggtgacc agttatttta ctgacactta tggcacggtc     300
tatgatacga ccatgctcca aaatgcccta cagaccgacc aagccgagac caaggatcta     360
ttaaacgcag cgtttatcat gcgtatcatt ggtttgggtg tgctaccaag tttgcttgtg     420
gcttttgtta aggtggatta tccgacttgg ggcaagggtt tgatgcgccg attgggcttg     480
atcgtggcaa gtcttgcgct gattttactg cctgtggtgg cgttcagcag tcattatgcc     540
agtttctttc gcgtgcataa gccgctgcgt agctatgtca atccgatcat gccaatctac     600
tcggtgggta agcttgccag tattgagtat aaaaaagcca gtgcgccaaa agataccatt     660
tatcacgcca aagacgcggt acaagcaacc aagcctgata tgcgtaagcc acgcctagtg     720
gtgttcgtcg tcggtgagac ggcacgcgcc gatcatgtca gcttcaatgg ctatgagcgc     780
gatactttcc cacagcttgc caagatcgat ggcgtgacca attttagcaa tgtcacatcg     840
tgcggcacat cgacggcgta ttctgtgccg tgtatgttca gctatctggg cgcggatgag     900
tatgatgtcg ataccgccaa ataccaagaa aatgtgctgg atacgctgga tcgcttgggc     960
gtaagtatct tgtggcgtga taataattcg gactcaaaag gcgtgatgga taagctgcca    1020
aaagcgcaat ttgccgatta taaatccgcg accaacaacg ccatctgcaa caccaatcct    1080
tataacgaat gccgcgatgt cggtatgctc gttggcttag atgactttgt cgctgccaat    1140
aacggcaaag atatgctgat catgctgcac caaatgggca atcacgggcc tgcgtatttt    1200
aagcgatatg atgaaaagtt tgccaaattc acgccagtgt gtgaaggtaa tgagcttgcc    1260
aagtgcgaac atcagtcctt gatcaatgct tatgacaatg ccttgcttgc caccgatgat    1320
ttcatcgctc aaagtatcca gtggctgcag acgcacagca atgcctatga tgtctcaatg    1380
ctgtatgtca gcgatcatgg cgaaagtctg ggtgagaacg gtgtctatct acatggtatg    1440
ccaaatgcct ttgcaccaaa agaacagcgc agtgtgcctg catttttctg gacggataag    1500
caaactggca tcacgccaat ggcaaccgat accgtcctga cccatgacgc gatcacgccg    1560
acattattaa agctgtttga tgtcaccgcg gacaaagtca aagaccgcac cgcattcatc    1620
cgctga                                                                1626
```

<210> 2
<211> 1626
<212> DNA
<213> Escherichia coli

<400> 2

atgatgctgc atacttctgt gtggtaccga cgctcggtca gtccgtttgt tcttgtggcg 60
agtgttgccg ttttcttgac cgcgaccgcc aatcttacct tttttgataa aatcagccaa 120
acctatccca tcgcggacaa tctcggcttt gtgctgacga tcgctgtcgt gctctttggc 180
gcgatgctac tgatcaccac gctgttatca tcgtatcgct atgtgctaaa gcctgtgttg 240
attttgctat taatcatggg cgcggtgacc agttatttta ctgacactta tggcacggtc 300
tatgatacga ccatgctcca aaatgcccta cagaccgacc aagccgagac caaggatcta 360
ttaaacgcag cgtttatcat gcgtatcatt ggtttgggtg tgctaccaag tttgcttgtg 420
gcttttgtta aggtggatta tccgacttgg ggcaagggtt tgatgcgccg attgggcttg 480
atcgtggcaa gtcttgcgct gattttactg cctgtggtgg cgttcagcag tcattatgcc 540
agtttctttc gcgtgcataa gccgctgcgt agctatgtca atccgatcat gccaatctac 600
tcggtgggta agcttgccag tattgagtat aaaaaagcca gtgcgccaaa agataccatt 660
tatcacgcca aagacgcggt acaagcaacc aagcctgata tgcgtaagcc acgcctagtg 720
gtgttcgtcg tcggtgagac ggcacgcgcc gatcatgtca gcttcaatgg ctatgagcgc 780
gatactttcc cacagcttgc caagatcgat ggcgtgacca attttagcaa tgtcacatcg 840
tgcggcacat cgacggcgta ttctgtgccg tgtatgttca gctatctggg cgcggatgag 900
tatgatgtcg ataccgccaa ataccaagaa aatgtgctgg atacgctgga tcgcttgggc 960
gtaagtatct tgtggcgtga taataattcg gactcaaaag gcgtgatgga taagctgcca 1020
aaagcgcaat ttgccgatta taaatccgcg accaacaacg ccatctgcaa caccaatcct 1080
tataacgaat gccgcgatgt cggtatgctc gttggcttag atgactttgt cgctgccaat 1140
aacggcaaag atatgctgat catgctgcac caaatgggca atcacgggcc tgcgtatttt 1200
aagcgatatg atgaaaagtt tgccaaattc acgccagtgt gtgaaggtaa tgagcttgcc 1260
aagtgcgaac atcagtcctt gatcaatgct tatgacaatg ccttgcttgc caccgatgat 1320
ttcatcgctc aaagtatcca gtggctgcag acgcacagca atgcctatga tgtctcaatg 1380

ctgtatgtca gcgatcatgg cgaaagtctg ggtgagaacg gtgtctatct acatggtatg 1440
ccaaatgcct ttgcaccaaa agaacagcgc agtgtgcctg cattttctg gacggataag 1500
caaactggca tcacgccaat ggcaaccgat accgtcctga cccatgacgc gatcacgccg 1560
acattattaa agctgtttga tgtcaccgcg gacaaagtca aagaccgcac cgcattcatc 1620
cgctga 1626

<210> 3
<211> 1626

<212> DNA
<213> Escherichia coli

<400> 3

```
atgatgcagc atacttctgt gtggtaccga cgctcggtca gtccgtttgt tcttgtggcg      60
agtgttgccg ttttcttgac cgcgaccgcc aatcttacct tttttgataa ggtcagccaa     120
acctatccca tcgcggacaa tctcggcttt gtgctgacga tcgctgtcgt gctctttggc     180
gcgatgctac tgatcaccac gctgttatca tcgtatcgct atgtgctaaa gcctgtgttg     240
attttgctat taatcatggg cgcggtgacc agttatttta ctgacactta tggcacggtc     300
tatgatacga ccatgctcca aaatgcccta cagaccgacc aagccgagac caaggatcta     360
ttaaacgcag cgtttatcat gcgtatcatt ggtttgggtg tgctaccaag tttgcttgtg     420
gcttttgtta aggtggatta tccgacttgg ggcaagggtt tgatgcgccg attgggcttg     480
atcgtggcaa gtcttgcgct gattttactg cctgtggtgg cgttcagcag tcattatgcc     540
agtttctttc gcgtgcataa gccgctgcgt agctatgtca atccgatcat gccaatctac     600
tcggtgggta agcttgccag tattgagtat aaaaaagcca gtgcgccaaa agataccatt     660
tatcacgcca aagacgcggt acaagcaacc aagcctgata tgcgtaagcc acgcctagtg     720
gtgttcgtcg tcggtgagac ggcacgcgcc gatcatgtca gcttcaatgg ctatgagcgc     780
gatactttcc cacagcttgc caagatcgat ggcgtgacca attttagcaa tgtcacatcg     840
tgcggcacat cgacggcgta ttctgtgccg tgtatgttca gctatctggg cgcggatgag     900
tatgatgtcg ataccgccaa ataccaagaa aatgtgctgg atacgctgga tcgcttgggc     960
gtaagtatct tgtggcgtga taataattcg gactcaaaag gcgtgatgga taagctgcca    1020
aaagcgcaat ttgccgatta taaatccgcg accaacaacg ccatctgcaa caccaatcct    1080
tataacgaat gccgcgatgt cggtatgctc gttggcttag atgactttgt cgctgccaat    1140
aacggcaaag atatgctgat catgctgcac caaatgggca atcacgggcc tgcgtatttt    1200
aagcgatatg atgaaaagtt tgccaaattc acgccagtgt gtgaaggtaa tgagcttgcc    1260
aagtgcgaac atcagtcctt gatcaatgct tatgacaatg ccttgcttgc caccgatgat    1320
ttcatcgctc aaagtatcca gtggctgcag acgcacagca atgcctatga tgtctcaatg    1380
ctgtatgtca gcgatcatgg cgaaagtctg ggtgagaacg gtgtctatct acatggtatg    1440
ccaaatgcct ttgcaccaaa agaacagcgc agtgtgcctg cattttctg gacggataag    1500
caaactggca tcacgccaat ggcaaccgat accgtcctga cccatgacgc gatcacgccg    1560
acattattaa agctgtttga tgtcaccgcg gacaaagtca aagaccgcac cgcattcatc    1620
cgctga                                                               1626
```

<210> 4
<211> 1626
<212> DNA
<213> Escherichia coli

<400> 4

```
atgatgcagc atacttctgt gtggtaccga cgctcggtca gtccgtttgt tcttgtggcg      60
agtgttgccg ttttcttgac cgcgaccgcc aatcttacct tttttgataa aatcagccaa     120
acctatccca tcgcggacaa tctcggcttt gtgctgacga tcgctgtcgt gctctttggc     180
gcgatgctac tgatcaccac gctgttatca tcgtatcgct atgtgctaaa gcctgtgttg     240
attttgctat taatcatggg cgcggtgacc agttatttta ctgacactta tggcacggtc     300
tatgatacga ccatgctcca aaatgcccta cagaccgacc aagccgagac caaggatcta     360
ttaaacgcag cgtttatcat gcgtatcatt ggtttgggtg tgctaccaag tttgcttgtg     420
gcttttgtta aggtggatta tccgacttgg ggcaagggtt tgatgcgccg attgggcttg     480
atcgtggcaa gtcttgcgct gattttactg cctgtggtgg cgttcagcag tcattatgcc     540
agtttctttc gcgtgcataa gccgctgcgt agctatgtca atccgatcat gccaatctac     600
tcggtgggta agcttgccag tattgagtat aaaaaagcca gtgcgccaaa agataccatt     660
tatcacgcca aagacgcggt acaagcaacc aagcctgata tgcgtaagcc acgcctagtg     720
gtgttcgtcg tcggtgagac ggcacgcgcc gatcatgtca gcttcaatgg ctatgagcgc     780
gatactttcc cacagcttgc caagatcgat ggcgtgacca attttagcaa tgtcacatcg     840
tgcggcacat cgacggcgta ttctgtgccg tgtatgttca gctatctggg cgcggatgag     900
tatgatgtcg ataccgccaa ataccaagaa aatgtgctgg atacgctgga tcgcttgggc     960
gtaagtatct tgtggcgtga taataattcg gactcaaaag gcgtgatgga taagctgcca    1020
aaagcgcaat ttgccgatta taaatccgcg accaacaacg ccatctgcaa caccaatcct    1080
tataacgaat gccgcgatgt cggtatgctc gttggcttag atgactttgt cgctgccaat    1140
aacggcaaag atatgctgat catgctgcac caaatgggca atcacgggcc tgcgtatttt    1200
aagcgatatg atgaaaagtt tgccaaattc acgccagtgt gtgaaggtaa tgagcttgcc    1260
aagtgcgaac atcagtcctt gatcaatgct tatgacaatg ccttgcttgc caccgataat    1320
ttcatcgctc aaagtatcca gtggctgcag acgcacagca atgcctatga tgtctcaatg    1380
```

ctgtatgtca gcgatcatgg cgaaagtctg ggtgagaacg gtgtctatct acatggtatg 1440

ccaaatgcct ttgcaccaaa agaacagcgc agtgtgcctg catttttctg gacggataag 1500

caaactggca tcacgccaat ggcaaccgat accgtcctga cccatgacgc gatcacgccg 1560

acattattaa agctgtttga tgtcaccgcg gacaaagtca aagaccgcac cgcattcatc 1620

cgctga 1626

<210> 5
<211> 1626
<212> DNA
<213> Escherichia coli

<400> 5

```
atgatgcagc atacttctgt gtggtaccga cgctcggtca gtccgtttgt tcttgtggcg      60
agtgttgccg ttttcttgac cgcgaccgcc aatcttacct tttttgataa aatcagccaa     120
acctatccca tcgcggacaa tctcggcttt gtgctgacga tcgctgtcgt gctctttggc     180
gcgatgctac tgatcaccac gctgttatca tcgtatcgct atgtgctaaa gcctgtgttg     240
attttgctat taatcatggg cgcggtgacc agttatttta ctgacactta tggcacggtc     300
tatgatacga ccatgctcca aaatgcccta cagaccgacc aagccgagac caaggatcta     360
ttaaacgcag cgtttatcat gcgtatcatt ggtttgggtg tgctaccaag tttgcttgtg     420
gcttttgtta aggtggatta tccgacttgg ggcaagggtt tgatgcgccg attgggcttg     480
atcgtggcaa gtcttgcgct gattttactg cctgtggtgg cgttcagcag tcattatgcc     540
agtttctttc gcgtgcataa gccgctgcgt agctatgtca atccgatcat gccaatctac     600
tcggtgggta agcttgccag tattgagtat aaaaaagcca gtgcgccaaa agataccatt     660
tatcacgcca aagacgcggt acaagcaacc aagcctgata tgcgtaagcc acgcctagtg     720
gtgttcgtcg tcggtgagac ggcacgcgcc gatcatgtca gcttcaatgg ctatgagcgc     780
gatactttcc cacagcttgc caagatcgat ggcgtgacca attttagcaa tgtcacatcg     840
tgcggcacat cgacggcgta ttctgtgccg tgtatgttca gctatctggg cgcggatgag     900
tatgatgtcg ataccgccaa ataccaagaa aatgtgctgg atacgctgga tcgcttgggc     960
gtaagtatct tgtggcgtga taataattcg gactcaaaag gcgtgatgga taagctgcca    1020
aaagcgcaat ttgccgatta taaatccgcg accaacaacg ccatctgcaa caccaatcct    1080
tataacgaat gccgcgatgt cggtatgctc gttggcttag atgactttgt cgctgccaat    1140
aacggcaaag atatgctgat catgctgcac caaatgggca atcacgggcc tgcgtatttt    1200
aagcgatatg atgaaaagtt tgccaaattc acgccagtgt gtgaaggtaa tgagcttgcc    1260
aagtgcgaac atcagtcctt gatcaatgct tatgacaatg ccttgcttgc caccgatgat    1320
ttcatcgctc aaagtatcca gtggctgcag acgtacagca atgcctatga tgtctcaatg    1380
ctgtatgtca gcgatcatgg cgaaagtctg ggtgagaacg gtgtctatct acatggtatg    1440
ccaaatgcct ttgcaccaaa agaacagcgc agtgtgcctg catttttctg gacggataag    1500
caaactggca tcacgccaat ggcaaccgat accgtcctga cccatgacgc gatcacgccg    1560
acattattaa agctgtttga tgtcaccgcg gacaaagtca aagaccgcac cgcattcatc    1620
cgctga                                                                1626
```

<210> 6
<211> 1626
<212> DNA
<213> Escherichia coli

<400> 6

```
atgatgcagc atacttctgt gtggtaccga cgctcggtca gtccgtttgt tcttgtggcg      60
agtgttgccg ttttcttgac cgcgaccgcc aatcttacct tttttgataa aatcagccaa     120
acctatccca tcgcggacaa tctcggcttt gtgctgacga tcgctgtcgt gctctttggc     180
gcgatgctac tgatcaccac gctgttatca tcgtatcgct atgtgctaaa gcctgtgttg     240
attttgctat taatcatggg cgcggtgacc agttatttta ctgacactta tggcacggtc     300
tatgatacga ccatgctcca aaatgcccta cagaccgacc aagccgagac caaggatcta     360
ttaaacgcag cgtttatcat gcgtatcatt ggtttgggtg tgctaccaag tttgcttgtg     420
gcttttgtta aggtggatta tccgacttgg ggcaagggtt tgatgcgccg attgggcttg     480
atcgtggcaa gtcttgcgct gattttactg cctgtggtgg cgttcagcag tcattatgcc     540
agtttctttc gcgtgcataa gccgctgcgt agctatgtca atccgatcat gccaatctac     600
tcggtgggta agcttgccag tattgagtat aaaaaagcca gtgcgccaaa agataccatt     660
tatcacgcca aagacgcggt acaagcaacc aagcctgata tgcgtaagcc acgcctagtg     720
gtgttcgtcg tcggtgagac ggcacgcgcc gatcatgtca gcttcaatgg ctatgagcgc     780
gatactttcc cacagcttgc caagatcgat ggcgtgacca attttagcaa tgtcacatcg     840
tgcggcacat cgacggcgta ttctgtgccg tgtatgttca gctatctggg cgcggatgag     900
tatgatgtcg ataccgccaa ataccaagaa aatgtgctgg atacgctgga tcgcttgggc     960
gtaagtatct tgtggcgtga taataattcg gactcaaaag gcgtgatgga taagctgcca    1020
aaagcgcaat ttgccgatta taaatccgcg accaacaacg ccatctgcaa caccaatcct    1080
tataacgaat gccgcgatgt cggtatgctc gttggcttag atgactttgt cgctgccaat    1140
aacggcaaag atatgctgat catgctgcac caaatgggca atcacgggcc tgcgtatttt    1200
aagcgatatg atgaaaagtt tgccaaattc acgccagtgt gtgaaggtaa tgagcttgcc    1260
aaatgcgaac atcagtcctt gatcaatgct tatgacaatg ccttgcttgc caccgatgat    1320
ttcatcgctc aaagtatcca gtggctgcag acgcacagca atgcctatga tgtctcaatg    1380
ctgtatgtca gcgatcatgg cgaaagtctg ggtgagaacg gtgtctatct acatggtatg    1440
ccaaatgcct ttgcaccaaa agaacagcgc agtgtgcctg catttttctg gacggataag    1500
caaactggca tcacgccaat ggcaaccgat accgtcctga cccatgacgc gatcacgccg    1560
acattattaa agctgtttga tgtcaccgcg gacaaagtca aagaccacac cgcattcatc    1620
cgctga                                                               1626
```

<210> 7
<211> 1626

<212> DNA
<213> Escherichia coli

<400> 7

```
atgatgcagc atacttctgt gtggtaccga cgctcggtca gtccgtttgt tcttgtggcg      60
agtgttgccg ttttcttgac cgcgaccgcc aatcttacct tttttgataa aatcagccaa     120
acctatccca tcgcggacaa tctcggcttt gtgctgacga tcgctgtcgt gctctttggc     180
gcgatgctac tgatcaccac gctgttatca tcgtatcgct atgtgctaaa gcctgtgttg     240
attttgctat taatcatggg cgcggtgacc agttatttta ctgacactta tggcacggtc     300
tatgatacga ccatgctcca aaatgcccta cagaccgacc aagccgagac caaggatcta     360
ttaaacgcag cgtttatcat gcgtatcatt ggtttgggtg tgctaccaag tttgcttgtg     420
gcttttgtta aggtggatta tccgacttgg ggcaagggtt tgatgcgccg attgggcttg     480
atcgtggcaa gtcttgcgct gattttactg cctgtggtgg cgttcagcag tcattatgcc     540
agtttctttc gcgtgcataa gccgctgcgt agctatgtca atccgatcat gccaatctac     600
tcggtgggta agcttgccag tattgagtat aaaaaagcca gtacgccaaa agataccatt     660
tatcacgcca aagacgcggt acaagcaacc aagcctgata tgcgtaagcc acgcctagtg     720
gtgttcgtcg tcggtgagac ggcacgcgcc gatcatgtca gcttcaatgg ctatgagcgc     780
gatactttcc cacagcttgc caagatcgat ggcgtgacca attttagcaa tgtcacatcg     840
tgcggcacat cgacggcgta ttctgtgccg tgtatgttca gctatctggg cgcggatgag     900
tatgatgtcg ataccgccaa ataccaagaa aatgtgctgg atacgctgga tcgcttgggc     960
gtaagtatct tgtggcgtga taataattcg gactcaaaag gcgtgatgga taagctgcca    1020
aaagcgcaat ttgccgatta taaatccgcg accaacaacg ccatctgcaa caccaatcct    1080
tataacgaat gccgcgatgt cggtatgctc gttggcttag atgactttgt cgctgccaat    1140
aacggcaaag atatgctgat catgctgcac caaatgggca atcacgggcc tgcgtatttt    1200
aagcgatatg atgaaaagtt tgccaaattc acgccagtgt gtgaaggtaa tgagcttgcc    1260
aagtgcgaac atcagtcctt gatcaatgct tatgacaatg ccttgcttgc caccgatgat    1320
ttcatcgctc aaagtatcca gtggctgcag acgcacagca atgcctatga tgtctcaatg    1380
ctgtatgtca gcgatcatgg cgaaagtctg ggtgagaacg gtgtctatct acatggtatg    1440
ccaaatgcct ttgcaccaaa agaacagcgc agtgtgcctg catttttctg gacggataag    1500
caaactggca tcacgccaat ggcaaccgat accgtcctga cccatgacgc gatcacgccg    1560
acattattaa agctgtttga tgtcaccgcg gacaaagtca aagaccgcac cgcattcatc    1620
cgctga                                                               1626
```

<210> 8
<211> 1626
<212> DNA
<213> Escherichia coli

<400> 8

```
atgatgcggc atacttctgt gtggtaccga cgctcggtca gtccgtttgt tcttgtggcg      60
agtgttgccg ttttcttgac cgcgaccgcc aatcttacct tttttgataa aatcagccaa     120
acctatccca tcgcggacaa tctcggcttt gtgctgacga tcgctgtcgt gctctttggc     180
gcgatgctac tgatcaccac gctgttatca tcgtatcgct atgtgctaaa gcctgtgttg     240
attttgctat taatcatggg cgcggtgacc agttatttta ctgacactta tggcacggtc     300
tatgatacga ccatgctcca aaatgcccta cagaccgacc aagccgagac caaggatcta     360
ttaaacgcag cgtttatcat gcgtatcatt ggtttgggtg tgctaccaag tttgcttgtg     420
gcttttgtta aggtggatta tccgacttgg ggcaagggtt tgatgcgccg attgggcttg     480
atcgtggcaa gtcttgcgct gattttactg cctgtggtgg cgttcagcag tcattatgcc     540
agtttctttc gcgtgcataa gccgctgcgt agctatgtca atccgatcat gccaatctac     600
tcggtgggta agcttgccag tattgagtat aaaaaagcca gtgcgccaaa agataccatt     660
tatcacgcca aagacgcggt acaagcaacc aagcctgata tgcgtaagcc acgcctagtg     720
gtgttcgtcg tcggtgagac ggcacgcgcc gatcatgtca gcttcaatgg ctatgagcgc     780
gatactttcc cacagcttgc caagatcgat ggcgtgacca attttagcaa tgtcacatcg     840
tgcggcacat cgacggcgta ttctgtgccg tgtatgttca gctatctggg cgcggatgag     900
tatgatgtcg ataccgccaa ataccaagaa aatgtgctgg atacgctgga tcgcttgggc     960
gtaagtatct tgtggcgtga taataattcg gactcaaaag gcgtgatgga taagctgcca    1020
aaagcgcaat ttgccgatta taaatccgcg accaacaacg ccatctgcaa caccaatcct    1080
tataacgaat gccgcgatgt cggtatgctc gttggcttag atgactttgt cgctgccaat    1140
aacggcaaag atatgctgat catgctgcac caaatgggca atcacgggcc tgcgtatttt    1200
aagcgatatg atgaaaagtt tgccaaattc acgccagtgt gtgaaggtaa tgagcttgcc    1260
aagtgcgaac atcagtcctt gatcaatgct tatgacaatg ccttgcttgc caccgatgat    1320
ttcatcgctc aaagtatcca gtggctgcag acgcacagca atgcctatga tgtctcaatg    1380
```

ctgtatgtca gcgatcatgg cgaaagtctg ggtgagaacg gtgtctatct acatggtatg 1440

ccaaatgcct ttgcaccaaa agaacagcgc agtgtgcctg catttttctg gacggataag 1500

caaactggca tcacgccaat ggcaaccgat accgtcctga cccatgacgc gatcacgccg 1560

acattattaa agctgtttga tgtcaccgcg gacaaagtca aagaccgcac cgcattcatc 1620

cgctga 1626

<210> 9
<211> 1608
<212> DNA
<213> Escherichia coli

<400> 9

```
gtgtggtacc gacgctcggt cagtccgttt gttcttgtgg cgagtgttgc cgttttcttg      60
accgcgaccg ccaatcttac ctttttgat aaaatcagcc aaacctatcc catcgcggac      120
aatctcggct ttgtgctgac gatcgctgtc gtgctctttg gcgcgatgct actgatcacc     180
acgctgttat catcgtatcg ctatgtgcta aagcctgtgt tgattttgct attaatcatg     240
ggcgcggtga ccagttattt tactgacact tatggcacgg tctatgatac gaccatgctc     300
caaaatgccc tacagaccga ccaagccgag accaaggatc tattaaacgc agcgtttatc     360
atgcgtatca ttggtttggg tgtgctacca agtttgcttg tggcttttgt taaggtggat     420
tatccgactt ggggcaaggg tttgatgcgc cgattgggct tgatcgtggc aagtcttgcg     480
ctgattttac tgcctgtggt ggcgttcagc agtcattatg ccagtttctt tcgcgtgcat     540
aagccgctgc gtagctatgt caatccgatc atgccaatct actcggtggg taagcttgcc     600
agtattgagt ataaaaaagc cagtgcgcca aaagatacca tttatcacgc caaagacgcg     660
gtacaagcaa ccaagcctga tatgcgtaag ccacgcctag tggtgttcgt cgtcggtgag     720
acggcacgcg ccgatcatgt cagcttcaat ggctatgagc gcgatacttt cccacagctt     780
gccaagatcg atggcgtgac caattttagc aatgtcacat cgtgcggcac atcgacggcg     840
tattctgtgc cgtgtatgtt cagctatctg ggcgcggatg agtatgatgt cgataccgcc     900
aaataccaag aaaatgtgct ggatacgctg gatcgcttgg gcgtaagtat cttgtggcgt     960
gataataatt cggactcaaa aggcgtgatg gataagctgc caaaagcgca atttgccgat    1020
tataaatccg cgaccaacaa cgccatctgc aacaccaatc cttataacga atgccgcgat    1080
gtcggtatgc tcgttggctt agatgacttt gtcgctgcca ataacggcaa agatatgctg    1140
atcatgctgc accaaatggg caatcacggg cctgcgtatt ttaagcgata tgatgaaaag    1200
tttgccaaat tcacgccagc gtgtgaaggt aatgagcttg ccaagtgcga acatcagtcc    1260
ttgatcaatg cttatgacaa tgccttgctt gccaccgatg atttcatcgc tcaaagtatc    1320
cagtggctgc agacgcacag caatgcctat gatgtctcaa tgctgtatgt cagcgatcat    1380
ggcgaaagtc tgggtgagaa cggtgtctat ctacatggta tgccaaatgc ctttgcacca    1440
aaagaacagc gcagtgtgcc tgcatttttc tggacggata agcaaactgg catcacgcca    1500
atggcaaccg ataccgtcct gacccatgac gcgatcacgc cgacattatt aaagctgttt    1560
gatgtcaccg cggacaaagt caaagaccgc accgcattca tccgctga                 1608
```

<210> 10
<211> 1608
<212> DNA
<213> Escherichia coli

<400> 10

gtgtggtacc gacgctcggt cagtccgttt gttcttgtgg cgagtgttgc cgttttcttg 60

accgcgaccg ccaatcttac ctttttgat aaaatcagcc aaacctatcc catcgcggac 120

aatctcggct ttgtgctgac gatcgctgtc gtgctctttg gcgcgatgct actgatcacc 180

acgctgttat catcgtatcg ctatgtgcta aagcctgtgt tgattttgct attaatcatg 240

ggcgcggtga ccagttattt tactgacact tatggcacgg tctatgatac gaccatgctc 300

caaaatgccc tacagaccga ccaagccgag accaaggatc tattaaacgc agcgtttatc 360

atgcgtatca ttggtttggg tgtgctacca agtttgcttg tggcttttgt taaggtggat 420

tatccgactt ggggcaaggg tttgatgcgc cgattgggct tgatcgtggc aagtcttgcg 480

ctgattttac tgcctgtggt ggcgttcagc agtcattatg ccagtttctt tcgcgtgcat 540

aagccgctgc gtagctatgt caatccgatc atgccaatct actcggtggg taagcttgcc 600

agtattgagt ataaaaaagc cagtgcgcca aaagatacca tttatcacgc caaagacgcg 660

gtacaagcaa ccaagcctga tatgcgtaag ccacgcctag tggtgttcgt cgtcggtgag 720

acggcacgcg ccgatcatgt cagcttcaat ggctatgagc gcgatacttt cccacagctt 780

gccaagatcg atggcgtgac caattttagc aatgtcacat cgtgcggcac atcgacggcg 840

tattctgtgc cgtgtatgtt cagctatctg ggcgcggatg agtatgatgt cgataccgcc 900

aaataccaag aaaatgtgct ggatacgctg gatcgcttgg gcgtaagtat cttgtggcgt 960

gataataatt cggactcaaa aggcgtgatg gataagctgc caaaagcgca atttgccgat 1020

tataaatccg cgaccaacaa cgccatctgc aacaccaatc cttataacga atgccgcgat 1080

gtcggtatgc tcgttggctt agatgacttt gtcgctgcca ataacggcaa agatatgctg 1140

atcatgctgc accaaatggg caatcacggg cctgcgtatt ttaagcgata tgatgaaaag 1200

tttgccaaat tcacgccagc gtgtgaaggt aatgagcttg ccaagtgcga acatcagtcc 1260

ttgatcaatg cttatgacaa tgccttgctt gccaccgatg atttcatcgc tcaaagtatc 1320

cagtggctgc agacgcacag caatgcctat gatgtctcaa tgctgtatgt cagcgatcat 1380

ggcgaaagtc tgggtgagaa cggtgtctat ctacatggta tgccaaatgc ctttgcacca 1440

aaagaacagc gcagtgtgcc tgcatttttc tggacggata agcaaactgg catcacgcca 1500

atggcaaccg ataccgtcct gacccatgac gcgatcacgc cgacattatt aaagctgttt 1560

gatgtcaccg cggacaaagt caaagaccgc accgcattca tccgctga 1608

<210> 11
<211> 1617
<212> DNA
<213> Escherichia coli

<400> 11

```
atgacatcac atcactcttg gtatcgctat tctatcaatc cttttgtgct gatgggtttg       60
gtggcgttat ttttggcagc gacagcgaac ctgacatttt ttgaaaaagc gatggcggtc      120
tatcctgtat cggataactt aggctttatc atctcaatgg cggtggcggt gatgggtgct      180
atgctactga ttgtcgtgct gttatcctat cgctatgtgc taaagcctgt cctgattttg      240
ctactgatta tgggtgcggt gacgagctat tttaccgata cttatggcac ggtctatgac      300
accaccatgc tccaaaatgc catgcaaacc gaccaagccg agtctaagga cttgatgaat      360
ttggcgtttt ttgtgcgaat tatcgggctt ggcgtgttgc caagtgtgtt ggtcgcagtt      420
gccaaagtca attatccaac atggggcaaa ggtctgattc agcgtgcgat gacatggggt      480
gtcagccttg tgctgttgct tgtgccgatt ggactattta gcagtcagta tgcgagtttc      540
tttcgggtgc ataagccagt gcgtttttat atcaacccga ttacgccgat ttattcggtg      600
ggtaagcttg ccagtatcga gtacaaaaaa gccactgcgc caacagacac catctatcat      660
gccaaagacg ccgtgcagac caccaagccg agcgagcgta agccacgcct agtggtgttc      720
gtcgtcggtg agacggcgcg tgctgaccat gtgcagttca atggctatgg ccgtgagact      780
ttcccgcagc ttgccaaagt tgatggcttg gcgaatttta gccaagtgac atcgtgtggc      840
acatcgacgg cgtattctgt gccgtgtatg ttcagctatt tgggtcaaga tgactatgat      900
gtcgataccg ccaaatacca agaaaatgtg ctagatacgc ttgaccgctt gggtgtgggt      960
atcttgtggc gtgataataa ttcagactca aaaggcgtga tggataagct acctgccacg     1020
cagtattttg attataaatc agcaaccaac aataccatct gtaacaccaa tccctataac     1080
gaatgccgtg atgtcggtat gcttgtcggg ctagatgact atgtcagcgc caataatggc     1140
aaagatatgc tcatcatgct acaccaaatg ggcaatcatg ggccggcgta ctttaagcgt     1200
tatgatgagc aatttgccaa attcaccccc gtgtgcgaag gcaacgagct tgccaaatgc     1260
gaacaccaat cactcatcaa tgcctatgac aatgcgctac ttgcgactga tgattttatc     1320
gccaaaagca tcgattggct aaaaacgcat gaagcgaact acgatgtcgc catgctctat     1380
gtcagtgacc acggcgagag cttgggcgaa aatggtgtct atctgcatgg tatgccaaat     1440
gcctttgcac caaaagaaca gcgagctgtg cctgcgtttt tttggtcaaa taatacgaca     1500
ttcaagccaa ctgccagcga tactgtgctg acgcatgatg cgattacgcc aacactgctt     1560
aagctgtttg atgtcacagc gggcaaggtc aaagaccgcg cggcatttat ccagtaa        1617
```

<210> 12
<211> 1608
<212> DNA
<213> Escherichia coli

<400> 12

```
gtgtggtacc gacgctcggt cagtccgttt gttcttgtgg cgagtgttgc cgttttcttg      60
accgcgaccg ccaatcttac ctttttgat aaaatcagcc aaacctatcc catcgcggac      120
aatctcggct ttgtgctgac gatcgctgtc gtgctctttg gcgcgatgct actgatcacc      180
acgctgttat catcgtatcg ctatgtgcta aagcctgtgt tgattttgct attaatcatg      240
ggcgcggtga ccagttattt tactgacact tatggcacgg tctatgatac gaccatgctc      300
caaaatgccc tacagaccga ccaagccgag accaaggatc tattaaacgc agcgtttatc      360
atgcgtatca ttggtttggg tgtgctacca agtttgcttg tggcttttgt taaggtggat      420
tatccgactt ggggcaaggg tttgatgcgc cgattgggct tgatcgtggc aagtcttgcg      480
ctgattttac tgcctgtggt ggcgttcagc agtcattatg ccagtttctt tcgcgtgcat      540
aagccgctgc gtagctatgt caatccgatc atgccaatct actcggtggg taagcttgcc      600
agtattgagt ataaaaaagc cagtgcgcca aaagatacca tttatcacgc caaagacgcg      660
gtacaagcaa ccaagcctga tatgcgtaag ccacgcctag tggtgttcgt cgtcggtgag      720
acggcacgcg ccgatcatgt cagcttcaat ggctatgagc gcgatacttt cccacagctt      780
gccaagatcg atggcgtgac caattttagc aatgtcacat cgtgcggcac atcgacggcg      840
tattctgtgc cgtgtatgtt cagctatctg ggcgcggatg agtatgatgt cgataccgcc      900
aaataccaag aaaatgtgct ggatacgctg gatcgcttgg gcgtaagtat cttgtggcgt      960
gataataatt cggactcaaa aggcgtgatg gataagctgc caaaagcgca atttgccgat     1020
tataaatccg cgaccaacaa cgccatctgc aacaccaatc cttataacga atgccgcgat     1080
gtcggtatgc tcgttggctt agatgacttt gtcgctgcca ataacggcaa agatatgctg     1140
atcatgctgc accaaatggg caatcacggg cctgcgtatt ttaagcgata tgatgaaaag     1200
tttgccaaat tcacgccagc gtgtgaaggt aatgagcttg ccaagtgcga acatcagtcc     1260
ttgatcaatg cttatgacaa tgccttgctt gccaccgatg atttcatcgc tcaaagtatc     1320
cagtggctgc agacgcacag caatgcctat gatgtctcaa tgctgtatgt cagcgatcat     1380
ggcgaaagtc tgggtgagaa cggtgtctat ctacatggta tgccaaatgc ctttgcacca     1440
aaagaacagc gcagtgtgcc tgcatttttc tggacggata agcaaactgg catcacgcca     1500
atggcaaccg ataccgtcct gacccatgac gcgatcacgc cgacattatt aaagctgttt     1560
gatgtcaccg cggacaaagt caaagaccgc accgcattca tccgctga                  1608
```

<210> 13
<211> 1626
<212> DNA
<213> Escherichia coli

<400> 13

atgccttccc ttataaaaat aaaaattgtt ccgcttatgt tctttttggc actgtatttt 60
gcatttatgc tgaactggcg tggagttctc catttttacg aaatccttta caaattagaa 120
gattttaagt ttggtttcgc catttcatta ccaatattgc ttgttgcagc gcttaacttt 180
gtatttgttc cattttcgat acggtattta ataaagcctt tttttgcact tcttatcgca 240
cttagtgcaa tcgttagtta cacaatgatg aagtatagag tcttgtttga tcaaaacatg 300
attcagaata tttttgaaac caatcaaaat gaggcgttag catatttaag cttaccaatt 360
atagtatggg ttactattgc tggttttatc cctgccattt tacttttctt tgttgaaatt 420
gaatatgagg aaaaatggtt caaagggatt ctaactcgtg ccctatcgat gtttgcatca 480
cttatagtga ttgcggttat tgcagcacta tactatcaag attatgtgtc agtggggcgc 540
aacaattcaa acctccagcg tgagattgtt ccagccaatt tcgttaatag taccgttaaa 600
tacgtttaca atcgttatct tgctgaacca atcccattta caactttagg tgatgatgca 660
aaacgggata ctaatcaaag taagcccacg ttgatgtttc tggtcgttgg tgaaaccgct 720
cgtggtaaaa atttctcgat gaatggctat gagaaagaca ccaatccatt taccagtaaa 780
tctggtggcg tgatctcctt taatgatgtt cgttcgtgtg ggactgcaac cgctgtatcc 840
gtcccctgca tgttctccaa tatggggaga aaggagtttg atgataatcg cgctcgcaat 900
agcgagggcc tgctagatgt gttgcaaaaa acggggatct ccattttttg gaaggagaac 960
gatggaggct gcaaaggcgt ctgcgaccga gtacctaaca tcgaaatcga accaaaggat 1020
caccctaagt tctgcgataa aaacacatgc tatgacgagg ttgtccttca agacctcgat 1080
agtgaaattg ctcaaatgaa aggggataag ctggttggct tccacctgat aggtagccat 1140
ggcccaacct actacaagcg ctaccctgat gctcatcgtc agttcacccc tgactgtcca 1200
cgcagtgata ttgaaaactg cacagatgaa gagctcacca acacctatga caacaccatc 1260
cgctacaccg atttcgtgat tggagagatg attgccaagt tgaaaaccta cgaagataag 1320
tacaacaccg cgttgctcta cgtctccgat catggtgaat cactgggagc attagggctt 1380
tacctacacg gtacaccgta ccagtttgca ccggatgatc agacccgtgt tcctatgcag 1440
gtgtggatgt cacctggatt taccaaagag aaaggcgttg atatggcgtg tttgcagcag 1500
aaagccgctg atactcgtta ctcacacgat aatattttct catctgtatt gggtatctgg 1560
gacgtcaaaa catcagttta cgaaaagggt ctagatattt tcagtcaatg tcgtaatgtt 1620

caataa 1626

<210> 14
<211> 1626
<212> DNA
<213> Escherichia coli

<400> 14

atgccttccc ttataaaaat aaaaattgtt ccgcttatgt tctttttggc actgtatttt 60
gcatttatgc tgaactggcg tggagttctc catttttacg aaatccttta caaattagaa 120
gattttaagt ttggtttcgc catttcatta ccaatattgc ttgttgcagc gcttaacttt 180
gtatttgttc cattttcgat acggtattta ataaagcctt tttttgcact tcttatcgca 240
cttagtgcaa tcgttagtta cacaatgatg aagtatagag tcttgtttga tcaaaacatg 300
attcagaata tttttgaaac caatcaaaat gaggcgttag catatttaag cttaccaatt 360
atagtatggg ttactattgc tggttttatc cctgccattt tacttttctt tgttgaaatt 420
gaatatgagg aaaaatggtt caaagggatt ctaactcgtg ccctatcgat gtttgcatca 480
cttatagtga ttgcggttat tgcagcacta tactatcaag attatgtgtc agtggggcgc 540
aacaattcaa acctccagcg tgagattgtt ccagccaatt tcgttaatag taccgttaaa 600
tacgtttaca atcgttatct tgctgaacca atcccattta caactttagg tgatgatgca 660
aaacgggata ctaatcaaag taagcccacg ttgatgtttc tggtcgttgg tgaaaccgct 720
cgtggtaaaa atttctcgat gaatggctat gagaaagaca ccaatccatt taccagtaaa 780
tctggtggcg tgatctcctt taatgatgtt cgttcgtgtg ggactgcaac cgctgtatcc 840
gtcccctgca tgttctccaa tatggggaga aaggagtttg atgaaaatcg cgctcgcaat 900
agcgagggcc tgctagatgt gttgcaaaaa acggggatct ccatttttttg gaaggagaac 960
gatggaggct gcaaaggcgt ctgcgaccga gtacctaaca tcgaaatcga accaaaggat 1020
caccctaagt tctgcgataa aaacacatgc tatgacgagg ttgtccttca agacctcgat 1080
agtgaaattg ctcaaatgaa aggggataag ctggttggct tccacctgat aggtagccat 1140
ggcccaacct actacaagcg ctaccctgat gctcatcgtc agttcacccc tgactgtcca 1200
cgcagtgata ttgaaaactg cacagatgaa gagctcacca acacctatga caacaccatc 1260
cgctacaccg atttcgtgat tggagagatg attgccaagt tgaaaaccta cgaagataag 1320
tacaacaccg cgttgctcta cgtctccgat catggtgaat cactgggagc attagggctt 1380
tacctacacg gtacaccgta ccagtttgca ccggatgatc agacccgtgt tcctatgcag 1440
gtgtggatgt cacctggatt taccaaagag aaaggcgttg atatggcgtg tttgcagcag 1500
aaagccgctg atactcgtta ctcacacgat aatattttct catctgtatt gggtatctgg 1560
gacgtcaaaa catcagttta cgaaaagggt ctagatattt tcagtcaatg tcgtaatgtt 1620

caataa 1626

<210> 15
<211> 1626

<212> DNA
<213> Escherichia coli

<400> 15

gtgatttcta gatttaagac gttatcggtt aaccaattca ctttcatcac tgcgttgttt 60
tatgttgcca ttttcaatct accgctcttt ggtatagtgc gaaaaggaat tgaaaaacaa 120
ccagaagttg atcccctttt catcgcatct atgccgctat ttttaacatt tgcgctgagt 180
tttttgtttt caatttttac cgtcaaatac ctgctgaagc ccttttttat cgtattgacg 240
ttactttcct caagtgtatt ttttgcagcc tatcaataca atgtcgtgtt tgactacggc 300
atgatagaaa acacgtttca aacacatcct gctgaagcat tgatgtatgt aaatcttgca 360
tcaattacca atctactgct gactgggcta ttaccgtcat atcttattta taaggccgat 420
attcattatc agcccttttt taaggagtta ttgcataaat tagcctttat gctgctaatg 480
ttcgttggca ttgggatagt cgcctttttt tactatcaag attatgctgc atttgttcga 540
aacaacagtg agttaaggcg ttacattgtc cctacctatt ttgtcagtag tgcatctaaa 600
tatctcaatg agcactattt gcagacgccc atggaatacc aacaacttgg cctagatgcg 660
aagaatgcca gtcgtaaccc gaacactaaa cctaacttat tagtggttgt tgtgggtgaa 720
actgcgcgct caatgagcta tcaatattat ggatataaca agccaaccaa tgctcatacc 780
caaaatcagg ggctgattgc gtttaacgat actagctcat gcggcacggc cacggcggtg 840
tctctaccct gtatgttttc acgaatgggg cgggcagact atgatcctcg ccgtgctaat 900
gctcaagaca cagtgattga tgtgttaagt catagtggta taaaagtaca gtggtttgat 960
aatgattctg gctgtaaagg tgtgtgtgat caggttgaaa atctcacgat agatttgaag 1020
agtgatccga agctgtgttc tggccaatat tgttttgacc aagtattgct caacaaatta 1080
gataaaattc tggcagtagc accaagtcaa gatacagtaa tttttttgca tatcattggt 1140
agtcatggac caacttatta tcttagatac ccgccagagc atcgtaaatt tataccggat 1200
tgtccgcgca gtgatattca aaattgcagt caagaagaac tgattaacac ctacgacaac 1260
actattctat atacggattt tattctcagt gaagtggtga ataaattaaa aggtaagcag 1320
gatatgttcg atactgcaat gctgtatctc tctgaccatg gtgagtcttt gggtgaaaag 1380
ggcatgtatt tacatggtgc gccctatagt attgcaccga aagaacaaac tagcgtacca 1440
atgctggctt gggtatctaa tgactttagc caagataatc agttgaacat gacttgtgtt 1500
gcacagcgag cagaacaggg cggcttttcc cacgacaatt tgttcgacag tttgctagga 1560
cttatgaatg taaaaaccac cgtctatcag agccaactcg atatttttgc accttgcagg 1620

tattag 1626

<210> 16
<211> 1644
<212> DNA
<213> Escherichia coli

<400> 16

```
atgcggttgt ctgcatttat cactttcttg aaaatgcgcc cgcaagtgcg cactgaattt      60
ttgactctgt tcatcagcct tgtgttcacc ctgctgtgca atggcgtgtt ttggaatgcc     120
cttcttgctg gacgcgactc cctaacttct ggaacatggc taatgctcct ttgcactggg     180
ttgctgatca ccgggctgca atggttgttg ctccttctgg tggccacgcg ctggagtgtc     240
aagccactac tgattctgct tgctgtcatg acgcccgccg ccgtttattt catgcgcaac     300
tacggggttt atctcgacaa ggccatgctg cggaatctga tggagacgga cgtcagggaa     360
gccagtgagc tgttgcaatg gagaatgctg ccctacttgt tggttgcagc cgtatccgtg     420
tggtggattg cgagagtcag ggttttacga acgggctgga aacaagcggt aatgatgcgc     480
agcgcttgtc tggctggcgc tctcgccatg atttccatgg gtctgtggcc agtcatggat     540
gtgctgatac ccacgcttcg tgaaaacaag ccgcttcgct atttgatcac tcctgcaaac     600
tacgtcatct cgggcattcg ggttttgact gaacaggcgt catcgtcagc agacgaagca     660
agggaagtcg ttgcagccga tgcgcatcga gggcctcaag aacaaggccg ccgtcctcgt     720
gctctcgtac tggttgtcgg ggaaaccgtc agggcggcta attgggggtt gagcggctat     780
gaacgacaaa ccacccctga gttggccgca cgcgacgtga tcaatttttc cgatgtcacc     840
agttgcggga cggatacggc tacatccctt ccctgcatgt tttccctcaa tggtcggcgc     900
gactacgacg aacgccagat tcgtcggcgc gagtccgtgc tgcacgtttt aaaccgtagt     960
gacgtcaaca ttctctggcg cgataaccag tcgggctgta aaggcgtctg tgatggactg    1020
ccctttgaaa acctgtcttc ggcaggccat cccacactgt gccatggcga gcgctgcctg    1080
gatgaaattc tgctcgaagg gttggccgag aagataacaa caagccgcag cgatatgctg    1140
atcgttctgc atatgctggg caatcacggc ccagcgtatt tccagcgcta tcccgcaagc    1200
taccgacgct ggtcgccaac ctgcgacacc accgatctgg ccagctgttc gcatgaagcc    1260
ttggtgaaca cctacgacaa cgccgtgctt tacaccgatc atgtgcttgc ccgtaccatt    1320
gacctgctgt ccggcatccg ctcacacgac acggcgctgc tgtacgtttc cgatcatggg    1380
gaatcgctcg gcgagaaagg cctgtatctc catggcatac cttacgtcat cgcgccggat    1440
gagcagatca aggtgccgat gatctggtgg cagtcgagtc aggtttatgc cgaccaagcc    1500
tgtatgcaaa ctcatgcctc tcgggcaccg gtaagtcacg atcacctgtt tcacaccttg    1560
ctcgggatgt tcgacgtgaa aaccgctgcc tacacgccag agttggacct tctggcaaca    1620
```

```
tgcagaaaag gacaaccaca atga                                            1644
```

## Description

The present invention relates to detection of antibiotic resistant bacteria, in particular bacteria resistant to cyclic cationic polypeptide antibiotics.

The increasing trend in antibiotic resistance continues to threaten global health due to the limited pipeline of new antibiotics. Multidrug resistance in Gram-negative bacteria is of special concern since it may be associated, in single isolates, with resistance to the three main classes of antibiotics that are (i) the β-lactams with plasmid-encoded extended-spectrum β-lactamases (ESBLs) hydrolyzing cephalosporins and with carbapenemases hydrolyzing additionally carbapenems, (ii) the aminoglycosides with 16S rRNA methylases modifying their cellular target and conferring pan-aminoglycoside resistance, (iii) the fluoroquinolones mostly with topoisomerases mutations. Due to the paucity of remaining antibiotics for treating infections, cyclic cationic polypeptide antibiotics (e.g. polymyxins such as colistin and polymyxin B) have become the last resort in particular for treating infections due to carbapenem-resistant *Enterobacteriaceae.* In addition, these carbapenem-resistant *Enterobacteriaceae* are more and more prevalent worldwide leading to increased use of polymyxins as first line treatment for highly invasive infections (e.g. bacteriaemia) in "endemic" countries. For example, according to European Antimicrobial Resistance Surveillance Network (EARS-Net), the prevalence of carbapenem-resistant *Klebsiella pneumoniae* isolated from blood culture reached 35% in Italy and 63% in Greece in 2015 compared to 1.3% and 43.5% in 2009, respectively. Unfortunately, this increased resistance to carbapenems in *Enterobacteriaceae,* that led to an increased use of polymyxins, is strongly correlated with increased resistance to polymyxins.

Acquired resistance to colistin in *Enterobacteriaceae* results mostly from modification of the polymyxin target, the lipopolysaccharide. Lipopolysaccharide (LPS), composed of the O-antigen, the oligosaccharide core and the Lipid A, is the major surface glycolipid located in the outer membrane of Gram-negative bacteria. Polymyxins (e.g. Colistin, Polymyxin B and Polymyxin M) are antibiotics, with a general structure having a cyclic peptide with a hydrophobic tail. They disrupt the structure of the bacterial cell membrane by interacting with its phospholipids. After binding to lipopolysaccharide (LPS) in the outer membrane of Gram-negative bacteria, polymyxins disrupt both the outer and inner membranes. The hydrophobic tail is important in causing membrane damage, suggesting a detergent-like mode of action.

Addition of phosphoethanolamine, 4-amino-L-arabinose cationic groups, or both to lipopolysaccharide (e.g. to the Lipid A) decreases cyclic cationic peptide antibiotic (e.g. polymyxin) binding to the bacterial outer membrane. Addition of these groups may be due to chromosome-encoded mechanisms (e.g. mutations in the PmrAB or PhoPQ two-component systems or alterations of the mgrB gene). Such alteration leads to a lower negative charge of the outer membrane of the bacterium, leading to reduced interaction of this membrane with cyclic cationic peptide antibiotic (e.g. polymyxin). A recent report revealed that addition of phosphoethanolamine may also be plasmid mediated through the *mcr-1* gene, which confers the first known plasmid-encoded resistance to colistin in isolates from humans and animals. More recently, the *mcr-1* gene was identified in several plasmid backbones, mostly in *Escherichia coli.* There is therefore an urgent need for a test that enables rapid detection of polymyxin resistance in bacteria (e.g. *Enterobacteriaceae*) and that may contribute to its containment.

The standard reference technique for determining susceptibility to polymyxins is broth microdilution, which requires fastidious attention and a long time (24 h) to perform. Other techniques for determining susceptibility to polymyxins (disk diffusion and Etest) have been proposed but require 18-24 hours to yield results. Because of poor diffusion of polymyxin molecules in agar, rates of false susceptibility are high (up to 32%).

A biochemical test (the rapid polymyxin NP test) that detects bacterial growth in the presence of a defined concentration of a polymyxin has also been used. In this colorimetric test, bacterial growth detection (or absence) is based on carbohydrate metabolism. Acid formation associated with carbohydrate metabolism in *Enterobacteriaceae* can be observed through the color change of a pH indicator. The interpretation of results of this test is subjective, leading to issues with reproducibility, and requiring more vigilance from laboratory technicians leading to reading errors. Whilst an improvement on other conventional methods, this biochemical test can take as long as 2 hours to yield a result.

Due to the variety of genes that can be involved in cyclic cationic peptide antibiotic (e.g. polymyxin) resistance (e.g. plasmid-encoded mcr-like genes, such as *mcr-1* and *mcr-2,* share only 79% identity; and the chromosome encoded genes related to polymyxin resistance are numerous), the use of molecular biology tools (e.g. amplification and sequencing of target genes) for the detection of polymyxin resistant bacteria is unreliable. For chromosome-encoded genes associated with polymyxin resistance, the gene modifications (disruptions, deletions mutations) involved are also not systematically described nor characterized. As for plasmid-encoded resistance, five families of *mcr* genes are known in *Enterobacteriaceae.* MCR-2, MCR-3, MCR-4 and MCR-5 share only 81%, 34%, 33% and 31% amino acid identity with MCR-1, respectively. This diversity would inevitably lead to failure in systematic detection of polymyxin resistance, when relying on using available molecular biology tools dedicated to *mcr-1* and/or *mcr-2* detection.

Other conventional approaches have employed the use of mass-spectrometry (MS). In fact, MS has emerged as a standard technique available in most clinical laboratories. The "Bioyper" systems from Bruker and the "VITEK-MS" systems from bioMerieux are examples of standard mass spectrometry systems and have been used for bacteria

identification based on the mass spectrum profile obtained using Matrix-Assisted Laser Desorption Ionization Time Of Flight Mass Spectrometry (MALDI-TOF MS). The system allows discrimination of bacteria mainly based on proteins. Indeed, all current diagnostic MS applications rely on the profiling of proteins. This is because proteins can be more easily ionized than hydrophobic and strongly hydrophilic molecules such as lipids and polysaccharides respectively. Therefore, the analysis of LPS and the modification of LPS composition on bacteria, which occurs during cyclic cationic peptide antibiotic (e.g. polymyxin) resistance, through conventional MALDI-TOF MS is challenging.

Recently, this technology has also been developed to detect antimicrobial resistance mechanisms (e.g. to the β-lactam antibiotic) by detecting the presence or absence of some β-lactamases (e.g. ESBL or carbapenemase). Here, the degradation (meaning hydrolysis) of the β-lactam is followed by MS analysis to generate a mass spectrum - the absence of the peak corresponding to the native β-lactam indicates antimicrobial resistance to β-lactam antibiotics. However, in the case of polymyxin, resistance is not due to the modifications or destruction of the antibiotic itself - therefore the strategy to detecting polymyxin modification or destruction using MALDI-TOF MS to detect polymyxin resistant bacteria is not applicable.

To-date, the use of mass spectrometry (e.g. MALDI-TOF MS) based methods for the detection of Lipid A and modified Lipid A has required the purification of the actual molecule (Lipid A) from large amounts of culture (e.g. from 500 ml up to several litres). The global procedure takes approximately 2 to 3 weeks which is not compatible with clinic analysis. Thus, the conventional methods are costly and/or time-consuming and/or inefficient for the detection of cyclic cationic polypeptide antibiotic (e.g. polymyxin) resistant bacteria in a sample and require a significant amount of skilled labour and costly equipment. No current MS based method is adequate for detecting cyclic cationic polypeptide antibiotic (e.g. polymyxin) resistant bacteria in a crude sample comprising intact bacteria.

Arroyo et al. (Antimicrobial agents and chemotherapy, 2011, 55, 8, p. 3743-3751) describes the pmrCAB operon mediates polymyxin resistance in *Acinetobacter baumannii* ATCC 17978 and clinical isolates through phosphoethanolamine modification of Lipid A. Tran et al. (Journal of Biological Chemistry, 2004, 279, 53, p. 55780-55791) describes periplasmic cleavage and codification of the 1-phosphate group of *Helicobacter pylori* Lipid A. Pendela et al. (Rapid Communications in Mass Spectrometry, 2009, 23, 12, p. 1856-1862) describes characterization of dihydrostreptomycin-related substances by liquid chromatography coupled to ion trap mass spectrometry. Pelletier et al. (Antimicrobial Agents and Chemotherapy, 2013, 57, 10, p. 4831-4840) describes structural modifications in the lipopolysaccharide from colistin-resistant strains of *Acinetobacter baumannii.* Qureshi et al. (Clinical Infectious Diseases, 2015, 60, 9, p. 1295-1303) describes colistin-resistant *Acinetobacter baumannii.* Park et al. (Clinical Microbiology and Infection, 2015, 21, 8, p. 765.e1-7) describes that transcriptomic analysis of colistin-susceptible and colistin-resistant isolates identifies genes associated with colistin resistance in *Acinetobacter baumannii.* US 2012/197535A1 describes methods for identifying bacteria.

The present invention solves at least one (e.g. more than one) of the above mentioned problems, by allowing cyclic cationic polypeptide antibiotic resistant bacteria detection in a sample comprising an intact bacterium, or bacterial membranes. The present invention is therefore uniquely compatible with the clinic (e.g. clinical analysis) and may allow detection of a cyclic cationic polypeptide antibiotic (e.g. polymyxin) resistant bacterium in less than 15 minutes.

Advantageously, said method does not require the purification of a Lipid A molecule, and indeed allows the identification of any Lipid A and any modified Lipid A directly on intact bacteria or in an unpurified sample containing bacterial membranes or fragments thereof. Furthermore, the present method is can be used with a small bacterial sample (e.g. comprising fewer than $10^7$ bacterial cells). This allows the time and materials required to detect the presence or absence of a bacterium resistant to a cyclic cationic polypeptide antibiotic in a sample to be greatly reduced.

Advantageously, the method allows for the detection of both plasmid encoded and chromosome encoded cyclic cationic polypeptide antibiotic resistant bacteria in a single analysis. For example, wherein the bacteria have been isolated from an infected patient, this allows patients infected with such bacteria to be separated, for example for the quarantine of patients infected with plasmid encoded polymyxin resistant bacteria. Further, the detection of chromosome encoded cyclic cationic polypeptide antibiotic resistant bacteria through a method of the present invention, advantageously providing information on the physiological state of Lipid A, circumvents the need for molecular analysis of samples requiring the amplification and sequencing of many genes whose mutation may give rise to cyclic cationic polypeptide antibiotic resistance.

The present invention provides rapid (15-minute) and accurate methods (e.g. diagnostic methods), providing major advances for the detection of polymyxin resistance by directly assessing Lipid A modifications, the cellular target of the polymyxins, on intact bacteria. The combination of excellent performance, cost-effectiveness and high-throughput scalability are all desirable attributes distinguishing the present methods from the prior art. Finally, the methods of the present invention use technology that is already available in many clinical microbiology laboratories, thus allowing no-cost and hassle free implementation.

The present invention is defined by the claims. There is described herein a method for detecting the presence or absence of a bacterium resistant to a cyclic cationic polypeptide antibiotic, comprising:

a. subjecting a test sample to mass spectrometry analysis and generating a mass spectrum output;
b. identifying in said mass spectrum output a first defined peak indicative of the presence of Lipid A modified by phosphoethanolamine, wherein said first defined peak is a peak present in a mass spectrum output for Lipid A modified by phosphoethanolamine and wherein said first defined peak is absent from a corresponding mass spectrum output for native Lipid A; and
c. wherein the presence of said first defined peak indicates the presence of a bacterium resistant to a cationic polypeptide antibiotic, and wherein the absence of said first defined peak indicates the absence of a bacterium resistant to a cyclic cationic polypeptide antibiotic.

In one aspect of the invention, there is provided a method for detecting the presence or absence of a bacterium resistant to a cyclic cationic polypeptide antibiotic, comprising:

a. subjecting a test sample to mass spectrometry analysis and generating a mass spectrum output;
wherein said test sample comprises a bacterial membrane or a fragment thereof, wherein the fragment comprises a non-Lipid A component;
b. identifying in said mass spectrum output a first defined peak indicative of the presence of Lipid A modified by phosphoethanolamine, wherein said first defined peak is a peak present in a mass spectrum output for Lipid A modified by phosphoethanolamine and wherein said first defined peak is absent from a corresponding mass spectrum output for native Lipid A; and
c. wherein the presence of said first defined peak indicates the presence of a bacterium resistant to a cationic polypeptide antibiotic, and wherein the absence of said first defined peak indicates the absence of a bacterium resistant to a cyclic cationic polypeptide antibiotic.

The term "bacterium" as used herein refers to a Gram-negative bacterium. A bacterium may be an intact bacterium or a fragmented bacterium. The bacterium may have been subjected to any chemical (e.g. treatment with alkaline agents) or physical treatment (e.g. heating, vortexing or pipetting).
The term "test sample" as used herein is not a purified sample of native Lipid A or modified Lipid A. The "test sample" comprises a bacterial membrane or a fragment thereof. The bacterial membrane comprises a native Lipid A or a modified Lipid A. Suitably the bacterial membrane may comprise a modified Lipid A. Suitably, a native Lipid A or a modified Lipid A is present as an integral part of the bacterial membrane or a fragment thereof in the test sample.
Advantageously, the utilisation of a test sample comprising a bacterial membrane or a fragment thereof (e.g. a crude sample) in a method of the present invention avoids the need to purify Lipid A and/or modified Lipid A (e.g. from large volumes of bacterial culture) prior to the detection of said Lipid A molecules (e.g. by mass spectrometry). Thus, the present invention is distinguished from prior art methods which require purification (e.g. pre-purification) of Lipid A and/or modified Lipid A.
The term "fragment" as used in the context of a bacterial membrane refers to a fragment comprising a non-lipid A component optionally in combination with a native Lipid A or modified Lipid A. In one embodiment a "fragment" is a non-Lipid A component optionally in combination with a modified Lipid A.
In one embodiment, a bacterial membrane is part of an intact bacterium.
The "test sample" may be a biological sample such as a clinical sample. In one embodiment a test sample is whole blood, serum, plasma, oral samples such as saliva, pus, vaginal sample, stool samples, vomitus, cerebrospinal fluid, tear fluid, synovial fluid, sputum, prepared or processed clinical samples (e.g. for the removal of salt), environmental samples (e.g. water, soil, air samples), bulk liquids, culled animal material, pharmaceuticals and biological matrices. A test sample may be prepared, for example where appropriate diluted or concentrated, or dialysed and stored by standard means. A test sample typically comprises or is suspected of comprising a bacterium (between $10^1$ to $10^{10}$ bacterial cells). A test sample also encompasses tissue homogenates, tissue sections and biopsy specimens from a live subject, or taken post-mortem. Alternatively, a test sample may be a bacterial colony or suspension recovered from any bacterial growth medium or clinical sample, prepared or processed clinical sample or environmental sample as outlined above.
The test sample may comprise, or may be suspected of comprising a bacterium resistant to a cyclic cationic polypeptide antibiotic. Suitably, the test sample may comprise a bacterium resistant a cyclic cationic polypeptide antibiotic.
A biological sample may be a clinical sample. The clinical sample may be a clinical sample that has been subjected to one or more processing steps. For example, dialysis (e.g. to to reduce the concentration of a salt in said sample). In one embodiment, the test sample is processed to remove salt. The reduced concentration of salt may allow the prevention of undesirable non-Lipid A species detection on a mass spectrum, thus improving the interpretability of the mass spectrum. Standard techniques for reducing the concentration of a salt (e.g. dialysis) are known in the art. In one embodiment, the test sample is subjected to several rounds of washing (e.g. centrifuging and resuspension in a low salt buffer or in a no salt buffer). A test sample may comprise a salt concentration of less than 200mM or 100mM. Suitably, a test sample may comprise a salt concentration of less than 50mM, 30mM or 10mM.

The term "detecting" as used herein means confirming the presence or absence of a cyclic cationic polypeptide antibiotic (e.g. polymyxin) resistant bacteria in a sample. Detecting may be performed on the test sample, or indirectly on an extract therefrom, or on a dilution or concentrate thereof. The term "identifying" as used herein means confirming the presence or absence of a peak assigned to a modified Lipid A in the mass spectrum output. Said identified peak also allows the quantification of the modified Lipid A. In methods of the invention, quantification may be performed by measuring the intensity (e.g. largest y-axis value) of a peak in a mass spectrum output.

The term "mass spectrometry analysis" encompasses any mass spectrometry technique suitable for the determination of the mass-to-charge ratio of a biological molecule, and embraces both negative and positive ion modes of mass spectrometry. Such mass spectrometry techniques include Matrix-assisted laser desorption/ionization time-of-flight mass spectrometry (MALDI-TOF MS), Surface Enhanced Laser Desorption Ionization time-of-flight mass spectrometry (SELDI-TOF MS), Accelerator Mass Spectrometry, Gas chromatography MS, liquid chromatography MS, Inductively coupled plasma MS, Isotope ratio mass spectrometry (IRMS), Rapid Evaporative Ionization Mass Spectrometry (REIMS), and Ion Mobility Spectrometry-MS. Preferably, mass spectrometry analysis comprises (or consists of) MALDI-TOF mass spectrometry.

MALDI is an ionization technique used in mass spectrometry, allowing the analysis of biomolecules (biopolymers such as DNA, proteins, peptides and sugars) and large organic molecules (such as polymers, dendrimers and other macromolecules), which tend to be fragile and fragment when ionized by other ionization methods. In one embodiment, the matrix solution acts as a proton source to encourage ionization of the analyte (e.g. Lipid A).

In one embodiment, mass spectrometry analysis comprises the ionisation of an analyte. In one embodiment, mass spectrometry analysis comprises the ionisation of Lipid A or a modified Lipid A.

The term "mass spectrum output" encompasses any data providing the mass-to-charge ratio (m/z) of a molecule (e.g. analyte) in a test sample together with an estimation of the quantity of said molecule in a test sample, for example across the range of 400 m/z to 30,000 m/z, 1,600 m/z to 2,200 m/z or 1,000 m/z to 3,000 m/z. The experimental procedure of the invention may be optimised to selectively provide the m/z of Lipid A molecules with minimal background (e.g. where "background" is m/z data on non-Lipid A species).

The term "native Lipid A" as used herein refers to a Lipid A that has not been modified, i.e. does not comprise a phosphoethanolamine and/or a 4-amino-L-arabinose modification. The term "native Lipid A" embraces (*inter alia*): hexa-acyl diphosphoryl Lipid A containing four C14:0 3-OH, one C14:0 and one C12:0; Lipid A with hydroxylation (-OH) of the C'-2 acyl-oxo-acyl chain; Lipid A with palmitoylation (-C-16) of the C-1 acyl-oxo-acyl chain; and Lipid A with hydroxylation (-OH) of the C'-2 acyl-oxo-acyl chain and palmitoylation (-C-16) of the C-1 acyl-oxo-acyl chain.

In one embodiment, native Lipid A comprises one or more of the following structures:

A
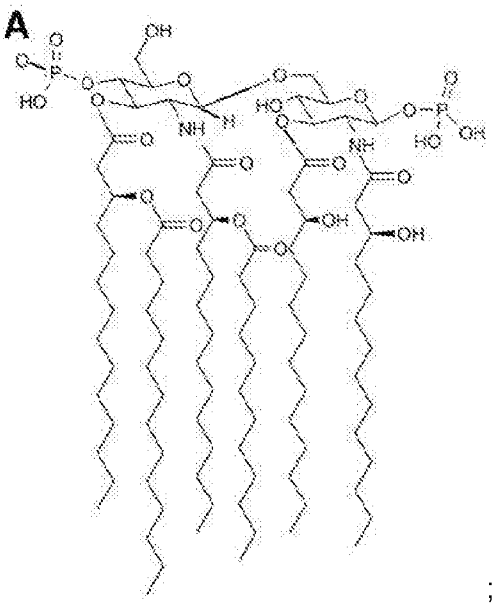
;

B
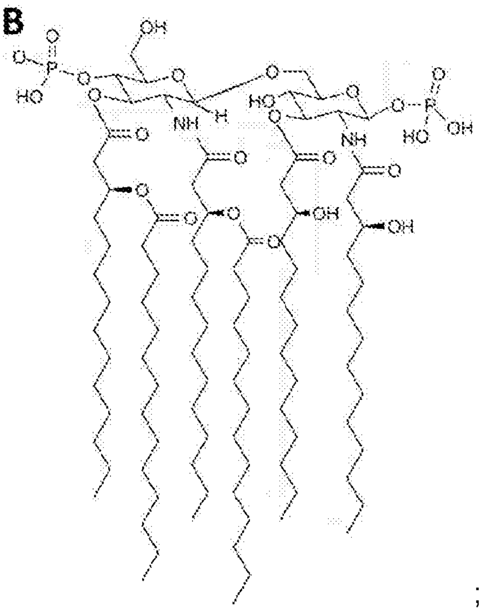
;

C

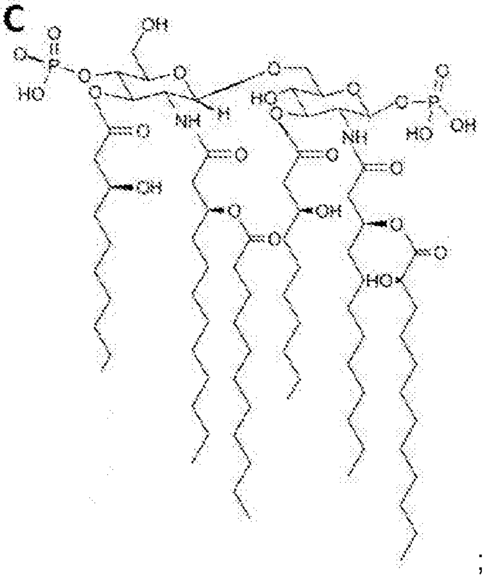

; or

D

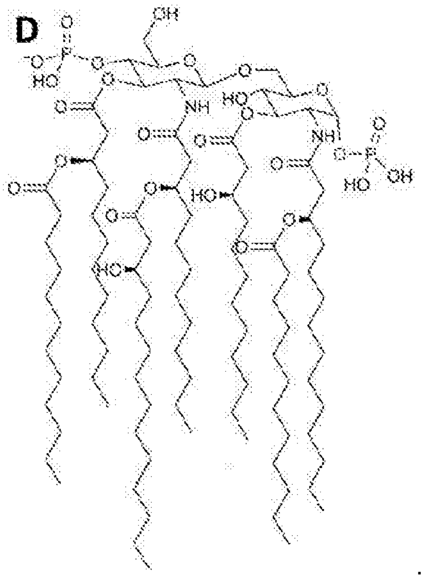

.

In embodiments where the bacterium is *Escherichia coli, Klebsiella spp., Shigella spp.,* or *Salmonella spp.,* native Lipid A comprises the structure as defined in (a) above.

In embodiments where the bacterium is *Klebsiella spp,* native Lipid A comprises the structure as defined in (b) above. In embodiments where the bacterium is *Pseudomonas aeruginosa,* native Lipid A comprises the structure as defined in (c) above.

In embodiments where the bacterium is *Acinetobacter baumannii,* native Lipid A comprises the structure as defined in (d) above.

In one embodiment, native Lipid A comprises one or more of the following structures:

A

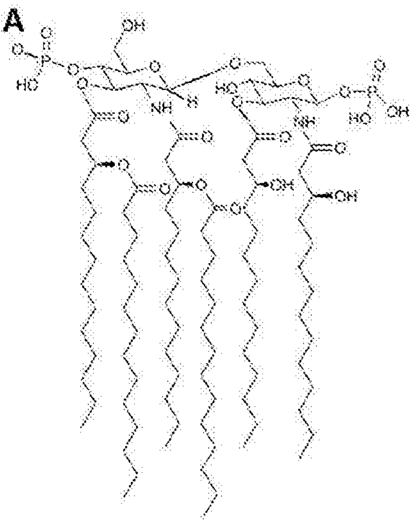

;

B

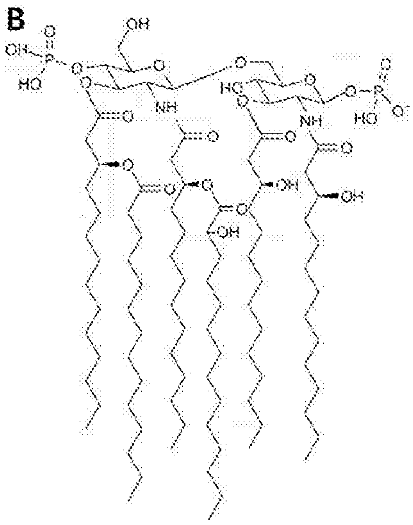

;

C

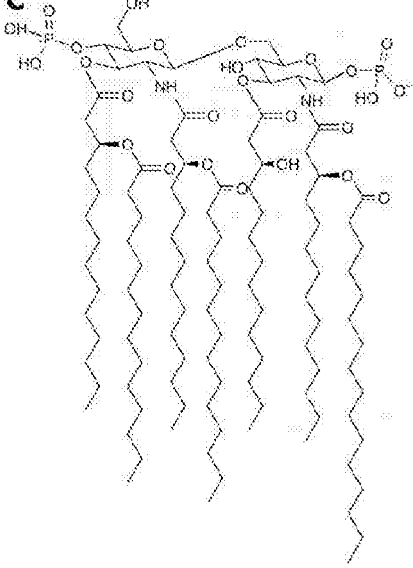

; or

D

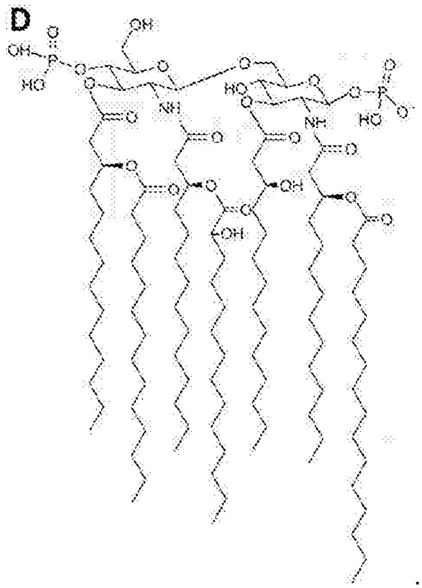

.

In embodiments where the bacterium is *Escherichia coli, Klebsiella spp., Shigella spp.,* or *Salmonella spp* (preferably *Klebsiella spp*,), native Lipid A comprises the structure as defined in any one of (a)-(d) above.

In one embodiment, Lipid A with hydroxylation (-OH) of the C'-2 acyl-oxo-acyl chain comprises the structure as defined in (b) above. Suitably, said Lipid A comprises a mass-to-charge ratio (m/z) of about 1837 to about 1843 m/z, preferably 1840 m/z. In one embodiment, Lipid A with palmitoylation (-C-16) of the C-1 acyl-oxo-acyl chain comprises the structure as defined in (c) above. Suitably, said Lipid A comprises a mass-to-charge ratio (m/z) of about 2060 to about 2066 m/z, preferably 2063 m/z. In one embodiment, Lipid A with hydroxylation (-OH) of the C'-2 acyl-oxo-acyl chain and palmitoylation (-C-16) of the C-1 acyl-oxo-acyl chain comprises the structure as defined in (d) above. Suitably, said Lipid A comprises a mass-to-charge ratio (m/z) of about 2076 to about 2082 m/z, preferably 2079 m/z.

The term "modified Lipid A" as used herein refers to a Lipid A that has been modified with a phosphoethanolamine and/or a 4-amino-L-arabinose. 4-amino-L-arabinose may alternatively be referred to as 4-amino-4-deoxy-L-arabinose. Lipid A modified with 4-amino-L-arabinoase and/or phosphoethanolamine may be a biomarker of cyclic cationic polypeptide antibiotic (e.g. polymyxin) resistance.

The structure of Lipid A is known in the art and can vary between bacteria (e.g. between genera, species or strains). The present invention involves different structures of Lipid A comprising a phosphoethanolamine modification and/or a 4-amino-L-arabinose at the 4' phosphate and/or the 1' phosphate position.

"Modified Lipid A" (e.g. modified with 4-amino-L-arabinoase and/or phosphoethanolamine) is distinct (both structurally and functionally) from a native Lipid A. Without wishing to be bound by theory, it is believed that modification of Lipid A with phosphoethanolamine and/or a 4-amino-L-arabinose leads to a lower negative charge of the outer membrane of a bacterium, leading to reduced interaction of this membrane with a cyclic cationic peptide antibiotic (e.g. polymyxin). Said modification of Lipid A with phosphoethanolamine and/or a 4-amino-L-arabinose is believed to cause remodelling of the bacterial outer membrane and decreases membrane permeability. Advantageously, the present invention provides a method that surprisingly allows detection of both a native Lipid A and a modified Lipid A in a test sample comprising a bacterial membrane or a fragment thereof. The ability to detect both a native Lipid A and a modified Lipid A in the same test sample is particularly advantageous, as the relative amounts (e.g. concentrations) of a native Lipid A and a modified Lipid A in the same test sample may be directly compared and contrasted.

In one embodiment Lipid A modified with phosphoethanolamine comprises a modification with phosphoethanolamine at the 1' phosphate and/or 4' phosphate of Lipid A. Suitably, Lipid A modified with phosphoethanolamine comprises a modification with phosphoethanolamine at the 1' phosphate.

In one embodiment Lipid A modified with phosphoethanolamine comprises a modification with phosphoethanolamine at the 1' phosphate of Lipid A with concomitant loss of the 4' phosphate group. In another embodiment Lipid A modified with phosphoethanolamine comprises a modification with phosphoethanolamine at the 4' phosphate group of Lipid A with concomitant loss of the 1' phosphate group. Suitably, Lipid A modified with phosphoethanolamine comprises a modification with phosphoethanolamine at the 1' phosphate group of Lipid A with concomitant loss of the 4' phosphate group.

In one embodiment, Lipid A modified with 4-amino-L-arabinose comprises a modification with 4-amino-L-arabinose at the 1' phosphate of Lipid A. In one embodiment, Lipid A modified with 4-amino-L-arabinose comprises a modification with 4-amino-L-arabinose at the 4' phosphate of Lipid A. Suitably, Lipid A modified with 4-amino-L-arabinose

comprises a modification with 4-amino-L-arabinose at the 4' phosphate of Lipid A.

In one embodiment, Lipid A modified with phosphoethanolamine and 4-amino-L-arabinose comprises a modification with phosphoethanolamine at the 1' phosphate and/or 4' phosphate of Lipid A. Suitably, Lipid A modified with phosphoethanolamine and 4-amino-L-arabinose comprises a modification with phosphoethanolamine at the 1' phosphate and a modification with 4-amino-L-arabinose at the 4' phosphate.

By way of example, an *Escherichia coli, Klebsiella spp., Shigella spp.,* or *Salmonella spp* Lipid A modified by phosphoethanolamine and 4-amino-L-arabinose may comprise the following structure:

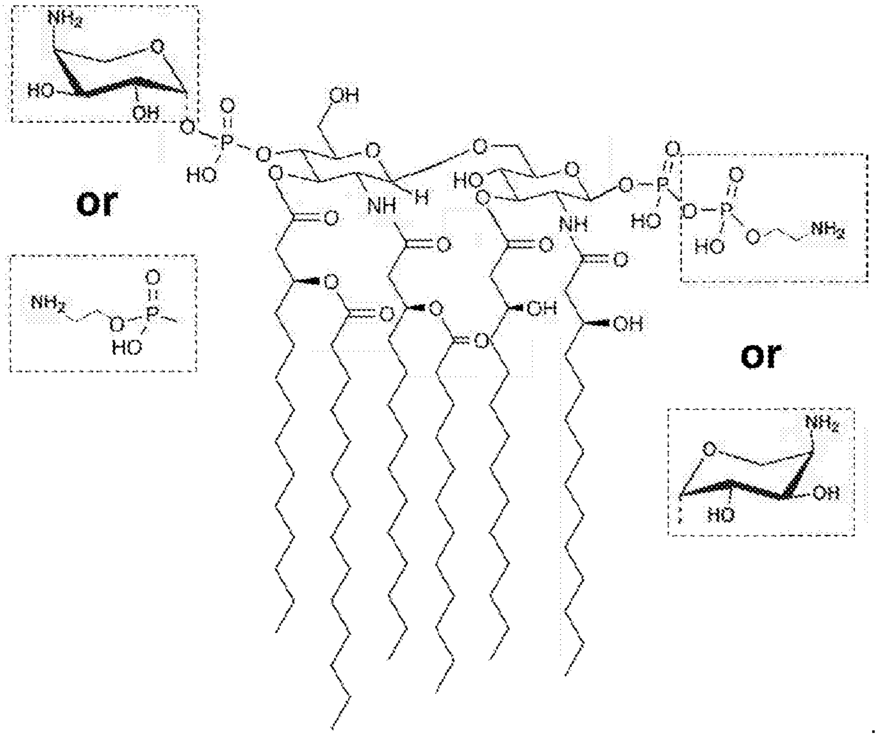
or
or

.

In one embodiment, Lipid A modified with 4-amino-L-arabinose comprises a modification with 4-amino-L-arabinose at the 1' phosphate and/or 4' phosphate of Lipid A, preferably at the 4' phosphate.

Preferably, Lipid A modified by phosphoethanolamine may comprise one or more of the following structures:

A
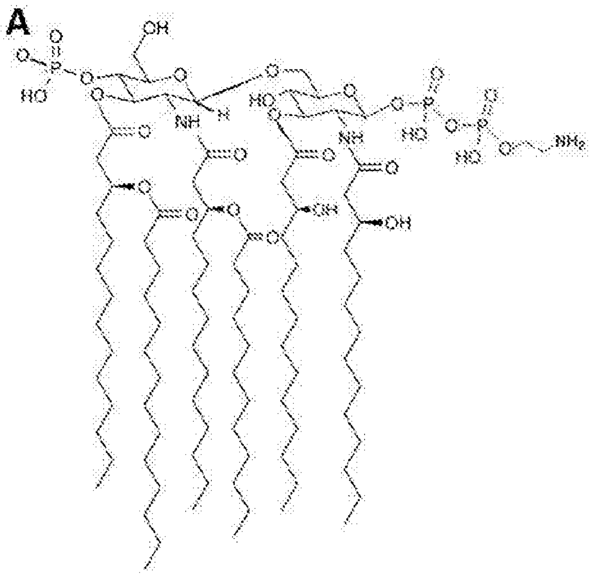
;

or

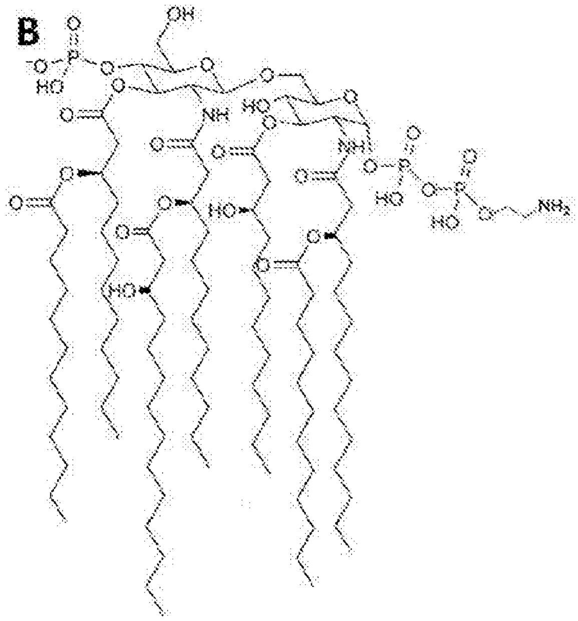

In embodiments where the bacterium is *Escherichia coli, Klebsiella spp., Shigella spp.,* or *Salmonella spp,* Lipid A modified by phosphoethanolamine may comprise the structure as defined in (a) above. In embodiments where the bacterium is *Acinetobacter baumannii,* Lipid A modified by phosphoethanolamine may comprise the structure as defined in (b) above. In one embodiment, Lipid A modified by phosphoethanolamine and 4-amino-L-arabinose comprises the structure as defined above for *E. coli, Klebsiella spp., Shigella spp., and Salmonella spp.*

Preferably, Lipid A modified with 4-amino-L-arabinose may comprise one or more of the following structures:

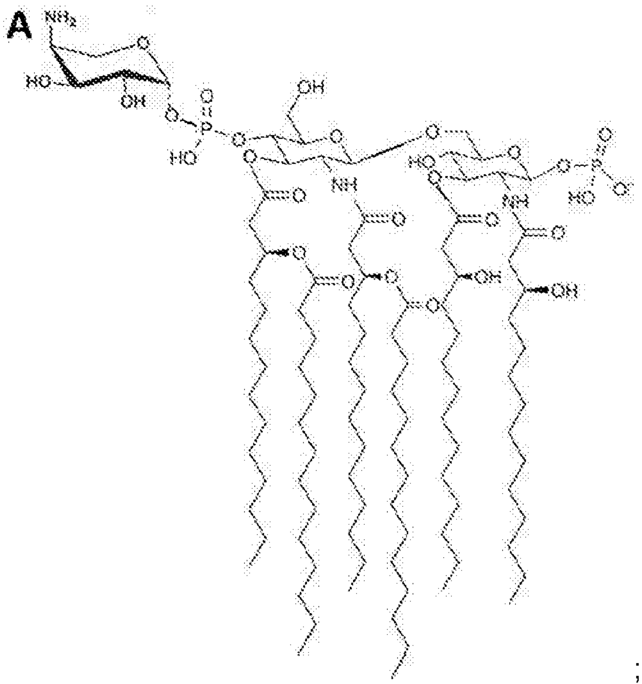
;

or

B

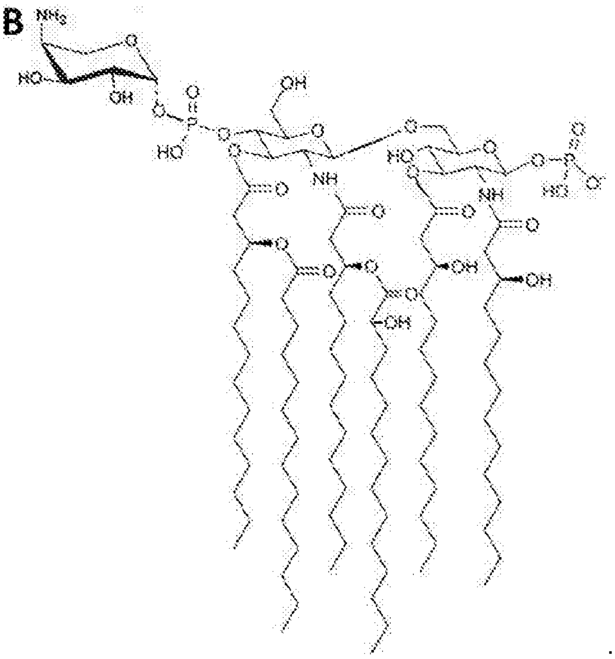

.

The method of the invention may be used for diagnosing infection of a subject with a bacterium resistant to a cyclic cationic polypeptide antibiotic through plasmid-encoded resistance, or infection of a subject with a bacterium resistant to a cyclic cationic polypeptide antibiotic through chromosome-encoded resistance.

The term "diagnosis" as used herein encompasses identification, confirmation and/or characterisation of cyclic cationic polypeptide resistant bacterial infections. Methods of diagnosis involving a method according to the invention are useful to confirm the existence of an infection. Methods of diagnosis are also useful in methods for assessment of clinical screening, prognosis, choice of therapy, evaluation of therapeutic benefit, i.e. for drug screening and drug development. Efficient diagnosis allows rapid identification of the most appropriate treatment (thus lessening unnecessary exposure to harmful drug side effects), and reducing relapse rates.

Also provided is a method for monitoring the efficacy of a therapy for cyclic cationic polypeptide antibiotic resistant bacterial infections, comprising identifying and/or quantifying the modified Lipid A in a test sample taken from a subject through a method of the present invention following therapy. In monitoring methods, test samples may be taken on one more occasion. The method may further comprise comparing the level of the modified Lipid A in a test sample with one or more control(s) and/or with one or more previous test sample(s) taken earlier from the same test subject, e.g. prior to commencement of therapy, and/or from the same test subject at an earlier stage of therapy. The method may comprise detecting a change in the level of a modified Lipid A in test samples taken on different occasions.

In one embodiment, the first defined peak indicative of the presence of Lipid A modified by phosphoethanolamine comprises a mass-to-charge ratio (m/z) of about 120 to about 125 m/z units (preferably about 123 m/z units) greater than a second defined peak indicative of the presence of native Lipid A.

In one embodiment, the second defined peak is selected from the group consisting of:

a. a peak comprising a mass-to-charge ratio (m/z) of about 1793 to about 1799 m/z, preferably 1796.2 m/z, for *Escherichia coli, Shigella, Klebsiella pneumoniae, Salmonella enterica, Enterobacter spp.* and *Klebsiella oxytoca;*
b. a peak comprising a mass-to-charge ratio (m/z) of about 1820 to about 1826 m/z, preferably 1823.9 m/z, for *Klebsiella pneumoniae;*
c. a peak comprising a mass-to-charge ratio (m/z) of about 1614 to about 1620 m/z, preferably 1617.2 m/z, for *Pseudomonas aeruginosa;* or
d. a peak comprising a mass-to-charge ratio (m/z) of about 1907 to about 1913 m/z, preferably 1910.3 m/z, for *Acinetobacter baumannii.*

In one embodiment, the second defined peak is selected from the group consisting of:

a. a peak comprising a mass-to-charge ratio (m/z) of about 1793 to about 1799 m/z, preferably 1796.2 m/z, for *Escherichia coli, Shigella, Klebsiella pneumoniae, Salmonella enterica, Enterobacter spp.* and *Klebsiella oxytoca;*
b. a peak comprising a mass-to-charge ratio (m/z) of about 1820 to about 1826 m/z, preferably 1823.9 m/z, for *Klebsiella pneumoniae;*

c. a peak comprising a mass-to-charge ratio (m/z) of about 1837 to about 1843 m/z, preferably 1840 m/z, for *Klebsiella pneumoniae;*

d. a peak comprising a mass-to-charge ratio (m/z) of about 1847 to about 1853 m/z, preferably 1850 m/z, for *Klebsiella pneumoniae;*

e. a peak comprising a mass-to-charge ratio (m/z) of about 2059 to about 2065 m/z, preferably 2062 m/z, for *Klebsiella pneumoniae;*

f. a peak comprising a mass-to-charge ratio (m/z) of about 2075 to about 2081 m/z, preferably 2078 m/z, for *Klebsiella pneumoniae;*

g. a peak comprising a mass-to-charge ratio (m/z) of about 1614 to about 1620 m/z, preferably 1617.2 m/z, for *Pseudomonas aeruginosa;*

h. a peak comprising a mass-to-charge ratio (m/z) of about 1907 to about 1913 m/z, preferably 1910.3 m/z, for *Acinetobacter baumannii.*

i. a peak comprising a mass-to-charge ratio (m/z) of about 1793 to about 1799 m/z, preferably 1796.2 m/z, for *Salmonella spp;*

j. a peak comprising a mass-to-charge ratio (m/z) of about 1820 to about 1826 m/z, preferably 1824 m/z, for *Salmonella spp;* or

k. a peak comprising a mass-to-charge ratio (m/z) of about 2031 to about 2037 m/z, preferably 2034 m/z, for *Salmonella spp.*

In one embodiment, native Lipid A comprises a mass-to-charge ratio (m/z) of about 1793 to about 1799 m/z, preferably about 1796.2 m/z for *Escherichia coli, Shigella, Klebsiella pneumoniae, Salmonella enterica, Enterobacter spp.* and *Klebsiella oxytoca*). In another embodiment, native Lipid A comprises a mass-to-charge ratio (m/z) of about 1820 to about 1826 m/z, preferably 1823.9 m/z for *Klebsiella pneumoniae.* In another embodiment, native Lipid A comprises a mass-to-charge ratio (m/z) of about 1837 to about 1843 m/z, preferably 1840 m/z for *Klebsiella pneumoniae.* In another embodiment, native Lipid A comprises a mass-to-charge ratio (m/z) of about 1847 to about 1853 m/z, preferably 1850 m/z for *Klebsiella pneumoniae.* In another embodiment, native Lipid A comprises a mass-to-charge ratio (m/z) of about 2059 to about 2065 m/z, preferably 2062 m/z for *Klebsiella pneumoniae.* In another embodiment, native Lipid A comprises a mass-to-charge ratio (m/z) of about 2075 to about 2081 m/z, preferably 2078 m/z for *Klebsiella pneumoniae.* In yet another embodiment, native Lipid A comprises a mass-to-charge ratio (m/z) of about 1614 to about 1620 m/z, preferably 1617.2 m/z, for *Pseudomonas aeruginosa.* In another embodiment, native Lipid A comprises a mass-to-charge ratio (m/z) of about 1907 to about 1913 m/z, preferably 1910.3 m/z, for *Acinetobacter baumannii.* In another embodiment, native Lipid A comprises a mass-to-charge ratio (m/z) of about 1793 to about 1799 m/z, preferably 1796.2 m/z, for *Salmonella spp.* In another embodiment, native Lipid A comprises a mass-to-charge ratio (m/z) of about 1820 to about 1826 m/z, preferably 1824 m/z, for *Salmonella spp.* In another embodiment, native Lipid A comprises a mass-to-charge ratio (m/z) of about 2031 to about 2037 m/z, preferably 2034 m/z, for *Salmonella spp.*

In some embodiments, the first defined peak indicative of the presence of Lipid A modified by phosphoethanolamine comprises a mass-to-charge ratio (m/z) of about 1960 to about 1966 m/z, preferably 1963 m/z for *Klebsiella pneumoniae;* between about 1970 to about 1976 m/z, preferably 1973 m/z for *Klebsiella pneumoniae;* between about 2182 to about 2188 m/z, preferably 2185 m/z for *Klebsiella pneumoniae;* between about 2198 to about 2204 m/z, preferably 2201 m/z for *Klebsiella pneumoniae;* between about 1918 and about 1920 m/z, preferably about 1919.2 m/z for *Salmonella spp.;* between about 1944 to about 1950 m/z, preferably 1947 m/z, for *Salmonella spp.* between about 2154 to about 2160 m/z, preferably 2157 m/z, for *Salmonella spp.;* between about 1913 and about 1924 m/z, or between about 1918 and about 1920 m/z, preferably about 1919.2 m/z for *Escherichia coli, Shigella, Klebsiella pneumoniae, Salmonella enterica, Enterobacter spp., Klebsiella oxytoca;* between about 1940 and about 1951 m/z, or between about 1946 and about 1947 m/z, preferably about 1946.9 m/z for *Klebsiella pneumoniae;* between about 1734 and about 1745 m/z, or between about 1939 and about 1741 m/z, preferably about 1740.2 m/z for *Pseudomonas aeruginosa;* and between about 2027 and about 2038 m/z, or between about 2032 and about 2034 m/z, preferably about 2033.3 m/z for *Acinetobacter baumannii.*

In some embodiments, the first defined peak indicative of the presence of Lipid A modified by phosphoethanolamine comprises a mass-to-charge ratio (m/z) of between about 1913 and about 1924 m/z, or between about 1918 and about 1920 m/z, preferably about 1919.2 m/z for *Escherichia coli, Shigella, Klebsiella pneumoniae, Salmonella enterica, Enterobacter spp., Klebsiella oxytoca;* between about 1940 and about 1951 m/z, or between about 1946 and about 1947 m/z, preferably about 1946.9 m/z for *Klebsiella pneumoniae;* between about 1734 and about 1745 m/z, or between about 1939 and about 1741 m/z, preferably about 1740.2 m/z for *Pseudomonas aeruginosa;* and between about 2027 and about 2038 m/z, or between about 2032 and about 2034 m/z, preferably about 2033.3 m/z for *Acinetobacter baumannii.*

In one embodiment, native Lipid A comprises a mass-to-charge ratio (m/z) of between about 1793 to about 1799 m/z, preferably 1796.2 m/z and a first defined peak indicative of the presence of Lipid A modified by phosphoethanolamine comprises a mass-to-charge ratio (m/z) of between about 1913 and about 1924 m/z, or between about 1918 and about 1920 m/z, preferably about 1919.2 m/z for *Escherichia coli, Shigella, Klebsiella pneumoniae, Salmonella enterica, Enterobacter spp., Klebsiella oxytoca.* In another embodiment, native Lipid A comprises a mass-to-charge ratio (m/z) of between about 1820 to about 1826 m/z, preferably 1823.9 m/z and a first defined peak indicative of the presence of Lipid A modified by phosphoethanolamine comprises a mass-to-charge ratio (m/z) of between about 1940 and about 1951 m/z, or between about 1946 m/z and about 1947 m/z, preferably about 1946.9 m/z for *Klebsiella pneumoniae.* In yet another embodiment, native Lipid A comprises a mass-to-charge ratio (m/z) of between about 1614 to about 1620 m/z, preferably 1617.2 m/z, and a first defined peak indicative of the presence of Lipid A modified by phosphoethanolamine comprises a mass-to-charge ratio (m/z) of between about 1734 and about 1745 m/z, or between about 1939 and about 1741 m/z, preferably about 1740.2 m/z for *Pseudomonas aeruginosa.* In another embodiment, native Lipid A comprises a mass-to-charge ratio (m/z) of between about 1907 to about 1913 m/z, preferably 1910.3 m/z, and a first defined peak indicative of the presence of Lipid A modified by phosphoethanolamine comprises a mass-to-charge ratio (m/z) between about 2027 and about 2038 m/z, or between about 2032 and about 2034 m/z, preferably about 2033.3 m/z for *Acinetobacter baumannii.* In another embodiment, native Lipid A comprises a mass-to-charge ratio (m/z) of about 1837 to about 1843 m/z, preferably 1840 m/z for *Klebsiella pneumoniae* and a first defined peak indicative of the presence of Lipid A modified by phosphoethanolamine comprises a mass-to-charge ratio (m/z) of about 1960 to about 1966 m/z, preferably 1963 m/z for *Klebsiella pneumoniae.* In another embodiment, native Lipid A comprises a mass-to-charge ratio (m/z) of about 1847 to about 1853 m/z, preferably 1850 m/z for *Klebsiella pneumoniae* and a first defined peak indicative of the presence of Lipid A modified by phosphoethanolamine comprises a mass-to-charge ratio (m/z) of between about 1970 to about 1976 m/z, preferably 1973 m/z for *Klebsiella pneumoniae.* In another embodiment, native Lipid A comprises a mass-to-charge ratio (m/z) of about 2059 to about 2065 m/z, preferably 2062 m/z for *Klebsiella pneumoniae* and a first defined peak indicative of the presence of Lipid A modified by phosphoethanolamine comprises a mass-to-charge ratio (m/z) of between about 2182 to about 2188 m/z, preferably 2185 m/z for *Klebsiella pneumoniae.* In another embodiment, native Lipid A comprises a mass-to-charge ratio (m/z) of about 2075 to about 2081 m/z, preferably 2078 m/z for *Klebsiella pneumoniae* and a first defined peak indicative of the presence of Lipid A modified by phosphoethanolamine comprises a mass-to-charge ratio (m/z) of between about 2198 to about 2204 m/z, preferably 2201 m/z for *Klebsiella pneumoniae.*

In another embodiment, native Lipid A comprises a mass-to-charge ratio (m/z) of about 1793 to about 1799 m/z, preferably 1796.2 m/z, for *Salmonella spp* and a first defined peak indicative of the presence of Lipid A modified by phosphoethanolamine comprises a mass-to-charge ratio (m/z) of between about 1918 and about 1920 m/z, preferably about 1919.2 m/z for *Salmonella spp.* In another embodiment, native Lipid A comprises a mass-to-charge ratio (m/z) of about 1820 to about 1826 m/z, preferably 1824 m/z, for *Salmonella spp* and a first defined peak indicative of the presence of Lipid A modified by phosphoethanolamine comprises a mass-to-charge ratio (m/z) of between about 1944 to about 1950 m/z, preferably 1947 m/z, for *Salmonella spp.* In another embodiment, native Lipid A comprises a mass-to-charge ratio (m/z) of about 2031 to about 2037 m/z, preferably 2034 m/z, for *Salmonella spp* and a first defined peak indicative of the presence of Lipid A modified by phosphoethanolamine comprises a mass-to-charge ratio (m/z) of between about 2154 to about 2160 m/z, preferably 2157 m/z, for *Salmonella spp.*

Intensity may be normalised to native Lipid A. In one embodiment, a ratio between a defined peak and native Lipid A may be calculated.

The term "intensity" refers to the highest value of a peak along the y-axis of a mass spectrum output, representing signal intensity of the ion (e.g. analyte).

The skilled person appreciates that a "peak" on a mass spectrum has a *de minimus* intensity value above the background. In one embodiment, a peak may be any *m/z* value(s) having an intensity of at least twice (e.g. 2x) the intensity of the average intensity of background peaks. Suitably, a peak may be any *m/z* value(s) having an intensity of at least six times (e.g. 6x) or eight times (e.g. 8x) the intensity of the average intensity of background peaks. Suitably, a peak may be any *m/z* value(s) having an intensity of at least ten times (e.g. 10x) or twelve times (e.g. 12x) the intensity of the average intensity of background peaks.

In one embodiment a ratio of intensity of the first defined peak (indicative of the presence of Lipid A modified by phosphoethanolamine) to the second defined peak (indicative of the presence of native Lipid A) is least 0.10.

Suitably, the ratio of intensity of the first defined peak (indicative of the presence of Lipid A modified by phosphoethanolamine) to the second defined peak may be at least about 0.10:1, 0.15:1, 0.30:1. 0.45:1, 1:1, 1.15:1, 1.30:1, 1.45:1, 2:1, 2,15:1, 2.30:1 or 2.45:1. Preferably, the ratio may be between 0.15:1 and 2.5:1, or the ratio may be between 0.15:1 and 1:1.

Advantageously, a ratio of at least about 0.10 as described above indicates the presence of bacterium resistant to a cyclic cationic polypeptide antibiotic (e.g. polymyxin). Calculation of this ratio may prevent false-positive detection of the presence of a bacterium resistant to a cyclic cationic polypeptide antibiotic (e.g. due to background peaks).

In one embodiment, a method of the invention further comprises identifying in a mass spectrum output a third defined peak comprising a mass-to-charge ratio (m/z) of about 22 to about 28 m/z units (preferably 25 m/z units) greater than a second defined peak. Without wishing to be bound by theory, it is believed that such a third defined peak is only

detecting in a test sample comprising a bacterium resistant to a cyclic cationic polypeptide antibiotic through plasmid-encoded resistance. Advantageously, the methods of the invention thus allow for the detection of both plasmid encoded and chromosome encoded cyclic cationic polypeptide antibiotic resistant bacteria in a single analysis. For example, wherein the bacteria have been isolated from an infected patient, this allows patients infected with such bacteria to be separated, for example for the quarantine of patients infected with plasmid encoded polymyxin resistant bacteria.

In one embodiment, said third defined peak as identified in a mass spectrum output in any method of the present invention is indicative of the presence of Lipid A modified by phosphoethanolamine. In a preferable embodiment, said third defined peak as identified in a mass spectrum output in any method of the present invention is indicative of the presence Lipid A modified by phosphoethanolamine at the 1’ phosphate group of Lipid A with concomitant loss of the 4’ phosphate group.

In one embodiment, the second defined peak is selected from the group consisting of:

a. a peak comprising a mass-to-charge ratio (m/z) of about 1793 to about 1799 m/z, preferably 1796.2 m/z, for *Escherichia coli, Shigella, Salmonella enterica, Enterobacter spp.* and *Klebsiella oxytoca;*
b. a peak comprising a mass-to-charge ratio (m/z) of about 1820 to about 1826 m/z, preferably 1823.9 m/z, for *Klebsiella pneumoniae.*
c. peak comprising a mass-to-charge ratio (m/z) of about 1837 to about 1843 m/z, preferably 1840 m/z, for *Klebsiella pneumoniae;*
d. peak comprising a mass-to-charge ratio (m/z) of about 1847 to about 1853 m/z, preferably 1850 m/z, for *Klebsiella pneumoniae;*
e. peak comprising a mass-to-charge ratio (m/z) of about 2059 to about 2065 m/z, preferably 2062 m/z, for *Klebsiella pneumoniae;*
f. a peak comprising a mass-to-charge ratio (m/z) of about 2075 to about 2081 m/z, preferably 2078 m/z, for *Klebsiella pneumoniae;*
g. a peak comprising a mass-to-charge ratio (m/z) of about 1793 to about 1799 m/z, preferably 1796.2 m/z, for *Salmonella spp;*
h. a peak comprising a mass-to-charge ratio (m/z) of about 1820 to about 1826 m/z, preferably 1824 m/z, for *Salmonella spp;* or
i. a peak comprising a mass-to-charge ratio (m/z) of about 2031 to about 2037 m/z, preferably 2034 m/z, for *Salmonella spp.*

In one embodiment, the second defined peak is selected from the group consisting of:

a. a peak comprising a mass-to-charge ratio (m/z) of about 1793 to about 1799 m/z, preferably 1796.2 m/z, for *Escherichia coli, Shigella, Salmonella enterica, Enterobacter spp.* and *Klebsiella oxytoca;*
b. a peak comprising a mass-to-charge ratio (m/z) of about 1820 to about 1826 m/z, preferably 1823.9 m/z, for *Klebsiella pneumoniae.*

In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 1793 to about 1799 m/z, or between about 1796 to about 1797 m/z, preferably 1796.2 m/z, for *Escherichia coli, Shigella, Klebsiella pneumoniae, Salmonella enterica, Enterobacter spp.* and *Klebsiella oxytoca.* In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 1820 to about 1826 m/z, preferably 1823.9 m/z, for *Klebsiella pneumoniae.* In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 1837 to about 1843 m/z, preferably 1840 m/z, for *Klebsiella pneumoniae.* In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 1847 to about 1853 m/z, preferably 1850 m/z, for *Klebsiella pneumoniae.* In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 2059 to about 2065 m/z, preferably 2062 m/z, for *Klebsiella pneumoniae.* In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 2075 to about 2081 m/z, preferably 2078 m/z, for *Klebsiella pneumoniae.* In one embodiment, a third defined peak comprises a mass-to-charge ratio (m/z) of between

about 22 and about 28 m/z units, preferably about 25 m/z units, greater than a second defined peak, wherein a second defined peak comprises a mass-to-charge ratio (m/z) of between about 1793 to about 1799 m/z, preferably 1796.2 m/z, for *Salmonella spp.* In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 1820 to about 1826 m/z, preferably 1824 m/z, for *Salmonella spp.* In one embodiment, a third defined peak comprises a mass-to-charge ratio (m/z) of between about 22 and about 28 m/z units, preferably about 25 m/z units, greater than a second defined peak, wherein a second defined peak comprises a mass-to-charge ratio (m/z) of between about 2031 to about 2037 m/z, preferably 2034 m/z, for *Salmonella spp.*

In one embodiment, a third defined peak is selected from the group consisting of:

a. a peak comprising a mass-to-charge ratio (m/z) of about 1818 to about 1824 m/z, preferably 1821 m/z, for *Escherichia coli, Shigella, Klebsiella pneumoniae, Salmonella enterica, Enterobacter spp.* and *Klebsiella oxytoca;*
b. a peak comprising a mass-to-charge ratio (m/z) of about 1845 to about 1852 m/z, preferably 1848.9 m/z, for *Klebsiella pneumoniae;*
c. peak comprising a mass-to-charge ratio (m/z) of about 1863 to about 1868 m/z, preferably 1865 m/z, for *Klebsiella pneumoniae;*
d. peak comprising a mass-to-charge ratio (m/z) of about 1872 to about 1878 m/z, preferably 1875 m/z, for *Klebsiella pneumoniae;*
e. peak comprising a mass-to-charge ratio (m/z) of about 2084 to about 2090 m/z, preferably 2087 m/z, for *Klebsiella pneumoniae;*
f. a peak comprising a mass-to-charge ratio (m/z) of about 2100 to about 2105 m/z, preferably 2103 m/z, for *Klebsiella pneumoniae;*
g. a peak comprising a mass-to-charge ratio (m/z) of about 1818 to about 1824 m/z, preferably 1821.2 m/z, for *Salmonella spp;*
h. a peak comprising a mass-to-charge ratio (m/z) of about 1846 to about 1852 m/z, preferably 1849 m/z, for *Salmonella spp;* or
i. a peak comprising a mass-to-charge ratio (m/z) of about 2056 to about 2062 m/z, preferably 2059 m/z, for *Salmonella spp.*

In one embodiment, native Lipid A (second defined peak) comprises a mass-to-charge ratio (m/z) of between about 1793 to about 1799 m/z, preferably 1796.2 m/z and a third defined peak comprises a mass-to-charge ratio (m/z) of between about 1818 to about 1824 m/z, preferably about 1821 m/z for *Escherichia coli, Shigella, Salmonella enterica, Enterobacter spp.* and *Klebsiella oxytoca.* In one embodiment, native Lipid A (second defined peak) comprises a mass-to-charge ratio (m/z) of between about 1820 to about 1826 m/z, preferably 1823.9 m/z and a third defined peak comprises a mass-to-charge ratio (m/z) of between 1845 to about 1852 m/z, preferably 1848.9 m/z, for *Klebsiella pneumoniae.* In one embodiment, native Lipid A (second defined peak) comprises a mass-to-charge ratio (m/z) of between about 1837 to about 1843 m/z, preferably 1840 m/z and a third defined peak comprises a mass-to-charge ratio (m/z) of between 1863 to about 1868 m/z, preferably 1865 m/z, for *Klebsiella pneumoniae.*

In one embodiment, native Lipid A (second defined peak) comprises a mass-to-charge ratio (m/z) of between about 1847 to about 1853 m/z, preferably 1850 m/z and a third defined peak comprises a mass-to-charge ratio (m/z) of between about 1872 to about 1878 m/z, preferably 1875 m/z, for *Klebsiella pneumoniae.* In one embodiment, native Lipid A (second defined peak) comprises a mass-to-charge ratio (m/z) of between about 2059 to about 2065 m/z, preferably 2062 m/z and a third defined peak comprises a mass-to-charge ratio (m/z) of between about 2084 to about 2090 m/z, preferably 2087 m/z, for *Klebsiella pneumoniae.* In one embodiment, native Lipid A (second defined peak) comprises a mass-to-charge ratio (m/z) of between about 2075 to about 2081 m/z, preferably 2078 m/z and a third defined peak comprises a mass-to-charge ratio (m/z) of between about 2100 to about 2105 m/z, preferably 2103 m/z, for *Klebsiella pneumoniae.* In one embodiment, native Lipid A (second defined peak) comprises a mass-to-charge ratio (m/z) of between about 1793 to about 1799 m/z, preferably 1796.2 m/z and a third defined peak comprises a mass-to-charge ratio (m/z) of between about 1818 to about 1824 m/z, preferably 1821.2 m/z, for *Salmonella spp.* In one embodiment, native Lipid A (second defined peak) comprises a mass-to-charge ratio (m/z) of between about 1820 to about 1826 m/z, preferably 1824 m/z and a third defined peak comprises a mass-to-charge ratio (m/z) of between about 1846 to about 1852 m/z, preferably 1849 m/z, for *Salmonella spp.* In one embodiment, native Lipid A (second defined peak) comprises a mass-to-charge ratio (m/z) of between about 1820 to about 1826 m/z, preferably 1824 m/z and a third defined peak comprises a mass-to-charge ratio (m/z) of between about 1846 to about 1852 m/z, preferably 1849 m/z, for *Salmonella spp.* In one embodiment, native Lipid A (second defined peak) comprises a mass-to-charge ratio (m/z) of between about 2031 to about 2037 m/z, preferably 2034 m/z and a third defined peak comprises a mass-to-charge ratio (m/z) of between 2056 to about 2062 m/z, preferably 2059 m/z, for *Salmonella spp.*

Advantageously, identification of the presence of the third defined as described above indicates the presence of a bacterium resistant to a cyclic cationic polypeptide antibiotic through plasmid-encoded resistance, preferably wherein said bacterium is of the family *Enterobacteriaceae.* Likewise, absence of the third defined peak may indicate the absence of a bacterium resistant to a cyclic cationic polypeptide through plasmid encoded resistance, preferably wherein said bacterium is of the family *Enterobacteriaceae.*

In one embodiment, the presence of said third defined peak indicates the presence of a bacterium of the family *Enterobacteriaceae* (e.g. *Escherichia coli, Klebsiella pneumoniae, Klebsiella oxytoca, Enterobacter aerogenes, Enterobacter cloacae, Enterobacter asburiae, Shigella sonnei, Shigella flexneri, Salmonella enterica, Citrobacter freundii, Citrobacter koseri, Citrobacter amalonaticus* or *Citrobacter youngae*) resistant to a cyclic cationic polypeptide antibiotic through plasmid-encoded resistance.

In another aspect of the invention, there is provided a method for detecting the presence or absence of a bacterium resistant to a cyclic cationic polypeptide antibiotic, comprising:

a. subjecting a test sample to mass spectrometry analysis and generating a mass spectrum output;
wherein said test sample comprises a bacterial membrane or a fragment thereof, wherein the fragment comprises a non-Lipid A component;
b. identifying in said mass spectrum output a first defined peak and/or a third defined peak indicative of the presence of Lipid A modified by phosphoethanolamine, wherein said first defined peak and/or third defined peak is a peak present in a mass spectrum output for Lipid A modified by phosphoethanolamine and wherein said first defined peak and or/said third defined peak is absent from a corresponding mass spectrum output for native Lipid A; and
c. wherein the presence of said first defined peak indicates the presence of a bacterium resistant to a cationic polypeptide antibiotic, and wherein the absence of said first defined peak indicates the absence of a bacterium resistant to a cyclic cationic polypeptide antibiotic; and/or
wherein the presence of said third defined peak indicates the presence of a bacterium resistant to a cationic polypeptide antibiotic through plasmid-encoded resistance, and wherein the absence of said third defined peak indicates the absence of a bacterium resistant to a cyclic cationic polypeptide through plasmid-encoded resistance.

In one embodiment, a ratio of intensity of:

the third defined peak; to
the second defined peak is at least about 0.15:1, or at least about 0.6:1.

Suitably, the ratio of intensity of the third defined peak to the second defined peak may be between at least about 0.15:1 to about 2.5:1. Suitably, the ratio may be between about 0.6:1 to about 1:1. Suitably, the ratio may be at least about 0.15:1, 0.3:1, 0.45:1, 0.6:1, 0.75:1, 0.9:1, 1.15:1, 1.3:1; 1.45:1, 1.6:1, 1.75:1, 1.9:1, 2.15:1, 2.3:1 or 2.45:1.

Thus, in one embodiment a ratio of intensity of the third defined peak to the second defined peak, wherein the ratio is at least about 0.15:1, indicates the presence of a bacterium (e.g. of the family Enterobacteriaceae) resistant to a cyclic cationic polypeptide antibiotic through plasmid-encoded resistance. Likewise, a ratio of less than 0.15:1 indicates the absence of a bacterium resistant to a cyclic cationic polypeptide through plasmid-encoded resistance.

In one embodiment, a ratio of:

the sum of the intensity of the first defined peak and the intensity of the third defined peak; to
the intensity of the second defined peak is at least about 0.15:1.

Suitably, the ratio of the sum of the intensity of the first defined peak and the intensity of the third defined peak to the intensity of the second defined peak is at least 0.15:1, 0.3:1, 0.45:1, 0.6:1, 0.75:1, 0.9:1, 1.15:1, 1.3:1; 1.45:1, 1.6:1, 1.75:1, 1.9:1, 2.15:1, 2.3:1 or 2.45:1.

In a preferable embodiment, a ratio of:

the sum of the intensity of the first defined peak and the intensity of the third defined peak; to
the intensity of the second defined peak is at least about 0.5:1.

Suitably, the ratio of the sum of the intensity of the first defined peak and the intensity of the third defined peak to the intensity of the second defined peak is at least about 0.5:1, 0.75:1, 0.9:1, 1.15:1, 1.3:1; 1.45:1, 1.6:1, 1.75:1, 1.9:1, 2.15:1, 2.3:1, 2.45:1, 2.6:1. 2.75:1, 2.9:1, 3.15:1, 3.3:1, 3.45:1, 3.6:1, 3.75:1, 3.9:1, 4.15:1, 4.3:1. 4.45:1, 4.6:1, 4.75:1, 4.9:1 or 5:1.

Advantageously, where a bacterium (e.g. of the family Enterobacteriaceae such as *Escherichia coli, Klebsiella pneumoniae, Shigella spp., Salmonella spp*) resistant to a cyclic cationic polypeptide antibiotic, comparing the ratio of

intensity of the third defined peak to the second defined peak provides further information as to the mechanism underlying the mechanism of resistance. The detection of a bacterium (e.g. of the family Enterobacteriaceae) resistant to a cyclic cationic polypeptide antibiotic through plasmid encoded resistance in a test sample is of particular importance, as said test samples or patients from which said test samples are derived should be safely contained/quarantined to prevent spread (or risk thereof) of the transmissible plasmid to another bacterium, thus spreading the plasmid encoded resistance.

In one embodiment, a ratio of the sum of the intensity of the first defined peak and the intensity of the third defined peak to the intensity of the second defined peak of at least about 0.5:1 is indicative of the presence of a bacterium resistant to a cyclic cationic polypeptide antibiotic through plasmid-encoded resistance.

In one embodiment, a ratio of:

the sum of the intensity of the first defined peak and the intensity of the third defined peak; to
the intensity of the second defined peak is between about 0.1:1 and 0.5:1.

Suitably, the ratio of the sum of the intensity of the first defined peak and the intensity of the third defined peak to the intensity of the second defined peak is between about 0.15:1 and 0.45:1, between about 0.2:1 and 0.4:1 or between about 0.25:1 and 0.35:1.

In one embodiment, a ratio of the sum of the intensity of the first defined peak and the intensity of the third defined peak to the intensity of the second defined peak of between about 0.1:1 and 0.5:1 is indicative of the presence of a bacterium resistant to a cyclic cationic polypeptide antibiotic through chromosome-encoded resistance.

In one embodiment, a ratio of:

the sum of the intensity of the first defined peak and the intensity of the third defined peak; to
the intensity of the second defined peak is less than about 0.1:1.

Suitably, the ratio of the sum of the intensity of the first defined peak and the intensity of the third defined peak to the intensity of the second defined peak is less than about 0.1:1, 0.08:1, 0.06:1, 0.04:1, 0.02:1 or 0.01:1.

In one embodiment, a ratio of the sum of the intensity of the first defined peak and the intensity of the third defined peak to the intensity of the second defined peak of less than about 0.1:1 is indicative of the absence of a bacterium resistant to a cyclic cationic polypeptide antibiotic.

In one embodiment, a method described herein further comprises identifying in said mass spectrum output a fourth defined peak indicative of the presence of Lipid A modified by 4-amino-L-arabinose, comprising a mass-to-charge ratio of about 129 to 133 m/z units (preferably about 131 m/z units) greater than the second defined peak indicative of the presence of native Lipid A.

In one embodiment, the second defined peak is selected from the group consisting of:

a. a peak comprising a mass-to-charge ratio (m/z) of about 1793 to about 1799 m/z, preferably 1796.2 m/z, for *Escherichia coli, Shigella, Klebsiella pneumoniae, Salmonella enterica, Enterobacter spp.* and *Klebsiella oxytoca;*
b. a peak comprising a mass-to-charge ratio (m/z) of about 1820 to about 1826 m/z, preferably 1823.9 m/z, for *Klebsiella pneumoniae;*
c. a peak comprising a mass-to-charge ratio (m/z) of about 1837 to about 1843 m/z, preferably 1840 m/z, for *Klebsiella pneumoniae;*
d. a peak comprising a mass-to-charge ratio (m/z) of about 1847 to about 1853 m/z, preferably 1850 m/z, for *Klebsiella pneumoniae;*
e. a peak comprising a mass-to-charge ratio (m/z) of about 2059 to about 2065 m/z, preferably 2062 m/z, for *Klebsiella pneumoniae;*
f. a peak comprising a mass-to-charge ratio (m/z) of about 2075 to about 2081 m/z, preferably 2078 m/z, for *Klebsiella pneumoniae;*
g. a peak comprising a mass-to-charge ratio (m/z) of about 1614 to about 1620 m/z, preferably 1617.2 m/z, for *Pseudomonas aeruginosa;*
h. a peak comprising a mass-to-charge ratio (m/z) of about 1907 to about 1913 m/z, preferably 1910.3 m/z, for *Acinetobacter baumannii;*
i. a peak comprising a mass-to-charge ratio (m/z) of about 1793 to about 1799 m/z, preferably 1796.2 m/z, for *Salmonella spp;*
j. a peak comprising a mass-to-charge ratio (m/z) of about 1820 to about 1826 m/z, preferably 1824 m/z, for *Salmonella spp;* or
k. a peak comprising a mass-to-charge ratio (m/z) of about 2031 to about 2037 m/z, preferably 2034 m/z, for *Salmonella spp.*

In one embodiment, the fourth defined peak indicative of the presence of Lipid A modified by 4-amino-L-arabinose is selected from the group consisting of:

a. a peak comprising a mass-to-charge ratio (m/z) of about 1924 to about 1930 m/z, preferably 1927.2 m/z, for *Escherichia coli, Shigella, Klebsiella pneumoniae, Salmonella enterica, Enterobacter spp.* and *Klebsiella oxytoca;*
b. a peak comprising a mass-to-charge ratio (m/z) of about 1951 to about 1957 m/z, preferably 1954.9 m/z, for *Klebsiella pneumoniae;*
c. a peak comprising a mass-to-charge ratio (m/z) of about 1968 to about 1974 m/z, preferably 1971 m/z, for *Klebsiella pneumoniae;*
d. a peak comprising a mass-to-charge ratio (m/z) of about 1978 to about 1984 m/z, preferably 1981 m/z, for *Klebsiella pneumoniae;*
e. a peak comprising a mass-to-charge ratio (m/z) of about 2190 to about 2196 m/z, preferably 2193 m/z, for *Klebsiella pneumoniae;*
f. a peak comprising a mass-to-charge ratio (m/z) of about 2206 to about 2212 m/z, preferably 2209 m/z, for *Klebsiella pneumoniae;*
g. a peak comprising a mass-to-charge ratio (m/z) of about 1745 to about 1751 m/z, preferably 1748.2 m/z, for *Pseudomonas aeruginosa;*
h. a peak comprising a mass-to-charge ratio (m/z) of about 2038 to about 2044 m/z, preferably 2041.3 m/z, for *Acinetobacter baumannii;*
i. a peak comprising a mass-to-charge ratio (m/z) of about 1924 to about 1930 m/z, preferably 1927.2 m/z, for *Salmonella spp;*
j. a peak comprising a mass-to-charge ratio (m/z) of about 1952 to about 1958 m/z, preferably 1955 m/z, for *Salmonella spp;* or
k. a peak comprising a mass-to-charge ratio (m/z) of about 2162 to about 2168 m/z, preferably 2165 m/z, for *Salmonella spp.*

In one embodiment, native Lipid A comprises a mass-to-charge ratio (m/z) of between about 1793 to about 1799 m/z, preferably 1796.2 m/z and a fourth defined peak indicative of the presence of Lipid A modified by 4-amino-L-arabinose comprises a mass-to-charge ratio (m/z) of between about 1924 to about 1930 m/z, preferably 1927.2 m/z, for *Escherichia coli, Shigella, Klebsiella pneumoniae, Salmonella enterica, Enterobacter spp.* and *Klebsiella oxytoca.* In one embodiment, native Lipid A comprises a mass-to-charge ratio (m/z) of between about 1820 to about 1826 m/z, preferably 1823.9 m/z and a fourth defined peak indicative of the presence of Lipid A modified by 4-amino-L-arabinose comprises a mass-to-charge ratio (m/z) of between about 1951 to about 1957 m/z, preferably 1954.9 m/z, for *Klebsiella pneumoniae.* In one embodiment, native Lipid A comprises a mass-to-charge ratio (m/z) of between about 1837 to about 1843 m/z, preferably 1840 m/z and a fourth defined peak indicative of the presence of Lipid A modified by 4-amino-L-arabinose comprises a mass-to-charge ratio (m/z) of between about 1968 to about 1974 m/z, preferably 1971 m/z, for *Klebsiella pneumoniae.* In one embodiment, native Lipid A comprises a mass-to-charge ratio (m/z) of between about 1847 to about 1853 m/z, preferably 1850 m/z and a fourth defined peak indicative of the presence of Lipid A modified by 4-amino-L-arabinose comprises a mass-to-charge ratio (m/z) of between about 1978 to about 1984 m/z, preferably 1981 m/z, for *Klebsiella pneumoniae.* In one embodiment, native Lipid A comprises a mass-to-charge ratio (m/z) of between about 2059 to about 2065 m/z, preferably 2062 m/z and a fourth defined peak indicative of the presence of Lipid A modified by 4-amino-L-arabinose comprises a mass-to-charge ratio (m/z) of between about 2190 to about 2196 m/z, preferably 2193 m/z, for *Klebsiella pneumoniae.* In one embodiment, native Lipid A comprises a mass-to-charge ratio (m/z) of between about 2075 to about 2081 m/z, preferably 2078 m/z and a fourth defined peak indicative of the presence of Lipid A modified by 4-amino-L-arabinose comprises a mass-to-charge ratio (m/z) of between about 2206 to about 2212 m/z, preferably 2209 m/z, for *Klebsiella pneumoniae.* In one embodiment, native Lipid A comprises a mass-to-charge ratio (m/z) of between about 1614 to about 1620 m/z, preferably 1617.2 m/z and a fourth defined peak indicative of the presence of Lipid A modified by 4-amino-L-arabinose comprises a mass-to-charge ratio (m/z) of between about 1745 to about 1751 m/z, preferably 1748.2 m/z, for *Pseudomonas aeruginosa.* In one embodiment, native Lipid A comprises a mass-to-charge ratio (m/z) of between about 1907 to about 1913 m/z, preferably 1910.3 m/z and a fourth defined peak indicative of the presence of Lipid A modified by 4-amino-L-arabinose comprises a mass-to-charge ratio (m/z) of between about 2038 to about 2044 m/z, preferably 2041.3 m/z, for *Acinetobacter baumannii.* In one embodiment, native Lipid A comprises a mass-to-charge ratio (m/z) of between about 1793 to about 1799 m/z, preferably 1796.2 m/z and a fourth defined peak indicative of the presence of Lipid A modified by 4-amino-L-arabinose comprises a mass-to-charge ratio (m/z) of between about 1924 to about 1930 m/z, preferably 1927.2 m/z, for *Salmonella spp.* In one embodiment, native Lipid A comprises a mass-to-charge ratio (m/z) of between about 1820 to about 1826 m/z, preferably 1824 m/z and a fourth defined peak indicative of the presence of Lipid A modified by 4-amino-L-arabinose comprises a mass-to-charge ratio (m/z) of between about 1952 to about 1958 m/z, preferably 1955 m/z, for *Salmonella spp.* In one embodiment, native Lipid A comprises a mass-to-charge ratio (m/z) of between about 2031 to about 2037 m/z, preferably 2034 m/z and a fourth defined peak indicative of the presence of Lipid A modified by 4-amino-L-arabinose comprises a mass-to-charge ratio (m/z) of between about 2162 to about 2168 m/z, preferably 2165 m/z, for *Salmonella spp.*

In one embodiment, the presence of said fourth defined peak indicates the presence of a bacterium resistant to a cyclic cationic polypeptide antibiotic through chromosome-encoded resistance. In one embodiment, the absence of said fourth defined peak indicates the absence of a bacterium resistant to a cyclic cationic polypeptide antibiotic through chromosome-encoded resistance.

In one embodiment, the presence of said first defined peak (e.g. indicative of the presence of Lipid A modified by phosphoethanolamine) and the absence of said fourth defined peak indicates the presence of a bacterium resistant to a cyclic cationic polypeptide antibiotic through plasmid-encoded resistance. In one embodiment, the absence of said first defined peak and the presence of said fourth defined peak indicates the presence of a bacterium resistant to a cyclic cationic polypeptide antibiotic through chromosome encoded resistance. Plasmid-encoded polymyxin resistance is typically associated with Lipid A modified by phosphoethanolamine (e.g. not additionally modified by 4-amino-L-arabinose).

In one embodiment, the presence of said third defined peak (e.g. indicative of the presence of Lipid A modified by phosphoethanolamine at the 1' phosphate group with concomitant loss of the 4' phosphate) and the absence of said fourth defined peak indicates the presence of a bacterium resistant to a cyclic cationic polypeptide antibiotic through plasmid-encoded resistance and the absence of a bacterium resistant to a cyclic cationic polypeptide antibiotic through chromosome-encoded resistance. In one embodiment, the absence of said third defined peak and the presence of said fourth defined peak indicates the presence of a bacterium resistant to a cyclic cationic polypeptide antibiotic through chromosome-encoded resistance and the absence of a bacterium resistant to a cyclic cationic polypeptide antibiotic through plasmid-encoded resistance.

In one aspect, there is proved a method for detecting the presence or absence of a bacterium resistant to a cyclic cationic polypeptide antibiotic, comprising:

a. subjecting a test sample to mass spectrometry analysis and generating a mass spectrum output; wherein said test sample comprises a bacterial membrane or a fragment thereof, wherein the fragment comprises a non-Lipid A component;

b. identifying in said mass spectrum output a fourth defined peak (e.g. any fourth defined peak as defined above) indicative of the presence of Lipid A modified by 4-amino-L-arabinose, wherein said fourth defined peak is a peak present in a mass spectrum output for Lipid A modified by 4-amino-L-arabinose and wherein said fourth defined peak is absent from a corresponding mass spectrum output for native Lipid A; and

c. wherein the presence of said fourth defined peak indicates the presence of a bacterium resistant to a cyclic cationic polypeptide antibiotic, and wherein the absence of said fourth defined peak indicates the absence of a bacterium resistant to a cyclic cationic polypeptide antibiotic.

In one embodiment, a method as described herein further comprises identifying in said mass spectrum output a fifth defined peak indicative of the presence of Lipid A modified by phosphoethanolamine and 4-amino-L-arabinose, comprising a mass-to-charge ratio of about 253 to 257 m/z units (preferably about 254 m/z units) greater than the second defined peak indicative of the presence of native Lipid A.

In one embodiment, the second defined peak is selected from the group consisting of:

a. a peak comprising a mass-to-charge ratio (m/z) of about 1793 to about 1799 m/z, preferably 1796.2 m/z, for *Escherichia coli, Shigella, Klebsiella pneumoniae, Salmonella enterica, Enterobacter spp.* and *Klebsiella oxytoca;*

b. a peak comprising a mass-to-charge ratio (m/z) of about 1820 to about 1826 m/z, preferably 1823.9 m/z, for *Klebsiella pneumoniae;*

c. a peak comprising a mass-to-charge ratio (m/z) of about 1837 to about 1843 m/z, preferably 1840 m/z, for *Klebsiella pneumoniae;*

d. a peak comprising a mass-to-charge ratio (m/z) of about 1847 to about 1853 m/z, preferably 1850 m/z, for *Klebsiella pneumoniae;*

e. a peak comprising a mass-to-charge ratio (m/z) of about 2059 to about 2065 m/z, preferably 2062 m/z, for *Klebsiella pneumoniae;*

f. a peak comprising a mass-to-charge ratio (m/z) of about 2075 to about 2081 m/z, preferably 2078 m/z, for *Klebsiella pneumoniae;*

g. a peak comprising a mass-to-charge ratio (m/z) of about 1614 to about 1620 m/z, preferably 1617.2 m/z, for *Pseudomonas aeruginosa;*

h. a peak comprising a mass-to-charge ratio (m/z) of about 1907 to about 1913 m/z, preferably 1910.3 m/z, for *Acinetobacter baumannii;*

i. a peak comprising a mass-to-charge ratio (m/z) of about 1793 to about 1799 m/z, preferably 1796.2 m/z, for

*Salmonella spp;*
j. a peak comprising a mass-to-charge ratio (m/z) of about 1820 to about 1826 m/z, preferably 1824 m/z, for *Salmonella spp;* or
k. a peak comprising a mass-to-charge ratio (m/z) of about 2031 to about 2037 m/z, preferably 2034 m/z, for *Salmonella spp.*

In one embodiment, the fifth defined peak indicative of the presence of Lipid A modified by phosphoethanolamine and 4-amino-L-arabinose is selected from the group consisting of:

a. a peak comprising a mass-to-charge ratio (m/z) of about 2047 to about 2053 m/z, preferably 2050 m/z, for *Escherichia coli, Shigella, Klebsiella pneumoniae, Salmonella enterica, Enterobacter spp.* and *Klebsiella oxytoca;*
b. a peak comprising a mass-to-charge ratio (m/z) of about 2074 to about 2080 m/z, preferably 2077 m/z, for *Klebsiella pneumoniae;*
c. a peak comprising a mass-to-charge ratio (m/z) of about 2091 to about 2097 m/z, preferably 2094 m/z, for *Klebsiella pneumoniae;*
d. a peak comprising a mass-to-charge ratio (m/z) of about 2101 to about 2107 m/z, preferably 2104 m/z, for *Klebsiella pneumoniae;*
e. a peak comprising a mass-to-charge ratio (m/z) of about 2313 to about 2319 m/z, preferably 2316 m/z, for *Klebsiella pneumoniae;*
f. a peak comprising a mass-to-charge ratio (m/z) of about 2329 to about 2335 m/z, preferably 2332 m/z, for *Klebsiella pneumoniae;*
g. a peak comprising a mass-to-charge ratio (m/z) of about 1868 to about 1874 m/z, preferably 1871.2 m/z, for *Pseudomonas aeruginosa;*
h. a peak comprising a mass-to-charge ratio (m/z) of about 2161 to about 2167 m/z, preferably 2164.3 m/z, for *Acinetobacter baumannii;*
i. a peak comprising a mass-to-charge ratio (m/z) of about 2047 to about 2053 m/z, preferably 2050.2 m/z, for *Salmonella spp;*
j. a peak comprising a mass-to-charge ratio (m/z) of about 2075 to about 2081 m/z, preferably 2078 m/z, for *Salmonella spp;* or
k. a peak comprising a mass-to-charge ratio (m/z) of about 2285 to about 2291 m/z, preferably 2288 m/z, for *Salmonella spp.*

In one embodiment, native Lipid A comprises a mass-to-charge ratio (m/z) of between about 1793 to about 1799 m/z, preferably 1796.2 m/z and a fifth defined peak indicative of the presence of Lipid A modified by phosphoethanolamine and 4-amino-L-arabinose comprises a mass-to-charge ratio (m/z) of between about 2047 to about 2053 m/z, preferably 2050 m/z, for *Escherichia coli, Shigella, Klebsiella pneumoniae, Salmonella enterica, Enterobacter spp.* and *Klebsiella oxytoca.* In one embodiment, native Lipid A comprises a mass-to-charge ratio (m/z) of between about 1820 to about 1826 m/z, preferably 1823.9 m/z and a fifth defined peak indicative of the presence of Lipid A modified by phosphoethanolamine and 4-amino-L-arabinose comprises a mass-to-charge ratio (m/z) of between about 2074 to about 2080 m/z, preferably 2077 m/z, for *Klebsiella pneumoniae.* In one embodiment, native Lipid A comprises a mass-to-charge ratio (m/z) of between about 1837 to about 1843 m/z, preferably 1840 m/z and a fifth defined peak indicative of the presence of Lipid A modified by phosphoethanolamine and 4-amino-L-arabinose comprises a mass-to-charge ratio (m/z) of between about 2091 to about 2097 m/z, preferably 2094 m/z, for *Klebsiella pneumoniae.* In one embodiment, native Lipid A comprises a mass-to-charge ratio (m/z) of between about 1847 to about 1853 m/z, preferably 1850 m/z and a fifth defined peak indicative of the presence of Lipid A modified by phosphoethanolamine and 4-amino-L-arabinose comprises a mass-to-charge ratio (m/z) of between about 2101 to about 2107 m/z, preferably 2104 m/z, for *Klebsiella pneumoniae.* In one embodiment, native Lipid A comprises a mass-to-charge ratio (m/z) of between about 2059 to about 2065 m/z, preferably 2062 m/z and a fifth defined peak indicative of the presence of Lipid A modified by phosphoethanolamine and 4-amino-L-arabinose comprises a mass-to-charge ratio (m/z) of between about 2313 to about 2319 m/z, preferably 2316 m/z, for *Klebsiella pneumoniae.* In one embodiment, native Lipid A comprises a mass-to-charge ratio (m/z) of between about 2075 to about 2081 m/z, preferably 2078 m/z and a fifth defined peak indicative of the presence of Lipid A modified by phosphoethanolamine and 4-amino-L-arabinose comprises a mass-to-charge ratio (m/z) of between about 2329 to about 2335 m/z, preferably 2332 m/z, for *Klebsiella pneumoniae.* In one embodiment, native Lipid A comprises a mass-to-charge ratio (m/z) of between about 1614 to about 1620 m/z, preferably 1617.2 m/z and a fifth defined peak indicative of the presence of Lipid A modified by phosphoethanolamine and 4-amino-L-arabinose comprises a mass-to-charge ratio (m/z) of between about 1868 to about 1874 m/z, preferably 1871.2 m/z, for *Pseudomonas aeruginosa.* In one embodiment, native Lipid A comprises a mass-to-charge ratio (m/z) of between about 1907 to about 1913 m/z, preferably 1910.3 m/z and a fifth defined peak indicative of the presence of Lipid A modified by phosphoethanolamine and 4-amino-L-arabinose comprises a mass-to-charge ratio (m/z) of between about 2161 to about 2167 m/z, preferably 2164.3 m/z, for *Acinetobacter baumannii.* In one embodiment, native Lipid A comprises a mass-to-charge ratio (m/z) of between about 1793 to about 1799 m/z, preferably 1796.2 m/z and a fifth defined peak indicative of the presence of Lipid A modified by phosphoethanolamine and 4-amino-L-arabinose comprises a mass-to-charge ratio (m/z) of between about 2047 to about 2053 m/z, preferably 2050.2 m/z, for *Salmonella spp.* In one embodiment, native Lipid A comprises a mass-to-charge ratio (m/z) of between about 2075 to about 2081 m/z, preferably 2078 m/z and a fifth defined peak indicative of the presence of Lipid A modified by phosphoethanolamine and 4-amino-L-arabinose comprises a mass-to-charge ratio (m/z) of between about 2075 to about 2081 m/z, preferably 2078 m/z, for *Salmonella spp.* In one embodiment, native Lipid A comprises a mass-to-charge ratio (m/z) of between about 2031 to about 2037 m/z, preferably 2034 m/z and a fifth defined peak indicative of the presence of Lipid A modified by phosphoethanolamine and 4-amino-L-arabinose comprises a mass-to-charge ratio (m/z) of between about 2285 to about 2291 m/z, preferably 2288 m/z, for *Salmonella spp.*

In one embodiment, the presence of said fifth defined peak indicates the presence of a bacterium resistant to a cyclic cationic polypeptide antibiotic through chromosome-encoded resistance. In one embodiment, the absence of said fifth defined peak indicates the absence of a bacterium resistant to a cyclic cationic polypeptide antibiotic through chromosome-encoded resistance. Chromosome-encoded polymyxin resistance may typically be associated with Lipid A modified by phosphoethanolamine, and/or Lipid A modified with both phosphoethanolamine and 4-amino-L-arabinose (e.g. the presence of both the first defined peak and the fifth defined peak).

In one embodiment, the presence of said first defined peak (e.g. indicative of the presence of Lipid A modified by phosphoethanolamine) and the absence of said fifth defined peak indicates the presence of a bacterium resistant to a cyclic cationic polypeptide antibiotic through plasmid encoded resistance. In one embodiment, the absence of said first defined peak and the presence of said fifth defined peak indicates the presence of a bacterium resistant to a cyclic cationic polypeptide antibiotic through chromosome encoded resistance.

In one embodiment, the presence of said third defined peak (e.g. indicative of the presence of Lipid A modified by phosphoethanolamine at the 1' phosphate group with concomitant loss of the 4' phosphate) and the absence of said fifth defined peak indicates the presence of a bacterium resistant to a cyclic cationic polypeptide antibiotic through plasmid encoded resistance and the absence of a bacterium resistant to a cyclic cationic polypeptide antibiotic through chromosome encoded resistance. In one embodiment, the absence of said third defined peak and the presence of said fifth defined peak indicates the presence of a bacterium resistant to a cyclic cationic polypeptide antibiotic through chromosome-encoded resistance and the absence of a bacterium resistant to a cyclic cationic polypeptide antibiotic through plasmid-encoded resistance.

Without wishing to be bound by theory it is believed that a bacterium resistant to a cyclic cationic polypeptide antibiotic through plasmid-encoded resistance comprises a plasmid having an *mcr-1, mcr-2,* an mcr-like gene or a combination thereof. A bacterium resistant to a cyclic cationic polypeptide antibiotic through plasmid-encoded resistance may comprise a plasmid having an *mcr-1* gene (e.g. *mcr-1, mcr-1.1, mcr-1.2, mcr-1.3, mcr-1.4, mcr-1.5, mcr-1.6, mcr-1.7, mcr-1.8, mcr-1.9* and/or *mcr-1.10*), an *mcr-2* gene (e.g. *mcr-2* and/or *mcr-2.2*), an gene *mcr-3* (e.g. *mcr-3* and/or *mcr-3.2*), an *mcr-4* gene (e.g. *mcr-4*), an *mcr-5 gene* (e.g. *mcr-5),* an mcr-like gene or a combination thereof. Suitably, Lipid A modified with phosphoethanolamine at the 1' phosphate group of Lipid A with concomitant loss of the 4' phosphate group (e.g. as per the third defined peak as described herein) is only detectable in a bacterium comprising a plasmid comprising one or more of said *mcr* genes, and is not detectable in a bacterium lacking such a plasmid.

In one embodiment, a bacterium resistant to a cyclic cationic polypeptide antibiotic through plasmid-encoded resistance or a bacterium of the family *Enterobacteriaceae* resistant to a cyclic cationic polypeptide antibiotic through plasmid-encoded resistance comprises a plasmid having a mobilised colistin resistance gene. In one embodiment, a mobilised colistin resistance gene comprises one of more of *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4, mcr-5 gene* or an mcr-like gene. In one embodiment, a mobilised colistin resistance gene comprises one of more of *mcr-1, mcr-2* or an mcr-like gene.

An "mcr-like gene" as used herein is a gene comprising a sequence having a nucleotide sequence encoding a functionally and/or structurally equivalent polypeptide to the polypeptide encoded by a *mcr-1* or *mcr-2* gene. An "mcr-like gene" as used herein is a gene comprising a sequence having a nucleotide sequence encoding a functionally and/or structurally equivalent polypeptide to the polypeptide encoded by *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4* or *mcr-5.*

In one embodiment an mcr-like gene is a gene comprising a sequence having at least 50% sequence identity to *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4* or *mcr-5.* In one embodiment, said mcr-like gene comprises at least 60% or 70% sequence identity to *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4* or *mcr-5.* Suitably, mcr-like gene comprises at least 80% or 90% (e.g. at least 95%) sequence identity to *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4* or *mcr-5.*

In one embodiment an mcr-like gene is a gene comprising a sequence having at least 50% sequence identity to *mcr-1* or *mcr-2.* In one embodiment, said mcr-like gene comprises at least 60% or 70% sequence identity to *mcr-1* or *mcr-2.* Suitably, mcr-like gene comprises at least 80% or 90% (e.g. at least 95%) sequence identity to *mcr-1* or *mcr-2.*

In one embodiment, an *mcr-1* gene has a sequence selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO: 10 or a sequence having at least 50% sequence identity thereto, suitably at least 60%, 70%, 80% or 90% sequence identity thereto. In one embodiment, an *mcr-1* gene has the sequence SEQ ID NO: 1, or a sequence having at least 50% sequence identity thereto, suitably at least 60%, 70%, 80% or 90% sequence identity thereto.

In one embodiment, an *mcr-2* gene has the sequence SEQ ID NO: 11, SEQ ID NO: 12, or a sequence having at least 50% sequence identity thereto, suitably at least 60%, 70%, 80% or 90% sequence identity thereto. In one embodiment, an *mcr-2* gene has the sequence SEQ ID NO: 11, or a sequence having at least 50% sequence identity thereto, suitably at least 60%, 70%, 80% or 90% sequence identity thereto.

In one embodiment, an *mcr-3* gene has the sequence SEQ ID NO: 13, SEQ ID NO: 14 or a sequence having at least 50% sequence identity thereto, suitably at least 60%, 70%, 80% or 90% sequence identity thereto.

In one embodiment, an *mcr-4* gene has the sequence SEQ ID NO: 15, or a sequence having at least 50% sequence identity thereto, suitably at least 60%, 70%, 80% or 90% sequence identity thereto.

In one embodiment, an *mcr-5* gene has the sequence SEQ ID NO: 16, or a sequence having at least 50% sequence identity thereto, suitably at least 60%, 70%, 80% or 90% sequence identity thereto.

A bacterium may be resistant to a cyclic cationic polypeptide antibiotic through chromosome-encoded resistance. Without wishing to be bound by theory, it is believed that a bacterium resistant to a cyclic cationic polypeptide antibiotic through chromosome-encoded resistance comprises a mutation in one or more gene selected from *pmrA, pmrB, pmrC, pmrD, pmrE, pmrR, phoP, phoQ, mgrB, arnA, arnB, arnC, arnD, arnE, arnF, arnF, arnT, cptA, eptB* and combinations thereof. Said mutations may result, either directly or indirectly, in the modification of Lipid A with 4-amino-L-arabinose and/or phosphoethanolamine. A "mutation" encompasses any alteration of the nucleotide sequence of a gene, a point mutation, a missense mutation, a nonsense mutation, an insertion, a deletion, a duplication, a frameshift mutation or a repeat expansion.

In one embodiment, a test sample is admixed with a matrix solution prior to subjecting said test sample to mass spectrometry analysis. A matrix solution facilitates mass spectrometry of a test sample, suitably wherein the mass spectrometry is MALDI-TOF mass spectrometry.

In one embodiment, a matrix solution as described herein allows for the selective extraction, co-crystallization and ionisation of native Lipid A and/or modified Lipid A as an integral part of a bacterial membrane. In one embodiment, a matrix solution as described herein allows for the identification of a peak assigned to native Lipid A and/or modified Lipid A (e.g. by allowing the selective extraction, co-crystallization and ionisation of native Lipid A and/or modified Lipid A) as an integral part of a bacterial membrane.

Thus in one aspect the present disclosure provides a prepared composition for use in mass spectrometry, said composition comprising a bacterial membrane or fragment thereof and a matrix solution.

In one embodiment, a matrix solution comprises 2,5-dihydroxybenzoic acid suspended in an organic solvent. In one embodiment, a matrix solution comprises 2,5-dihydroxybenzoic acid suspended in an organic solvent at a concentration of about 1 to 100 mg/ml. In one embodiment, a matrix solution comprises 2,5-dihydroxybenzoic acid suspended in an organic solvent at a concentration of about 7 to 13 mg/ml. Preferably 2,5-dihydroxybenzoic acid is suspended in an organic solvent at a concentration of about 10 mg/ml.

In one embodiment, a matrix solution comprises one or more selected from norharmane (NRM), 3-Hydroxymethyl-β-carboline, 3-Methyl-a-carboline, Ethyl 2,3,4,9-tetrahydro-1H-β-carboline-3-carboxylate, Ethyl β-carboline-3-carboxylate, 1-Methylindole-2-carboxylic acid, norharman methiodide, β-Carboline-3-carboxylic acid N-methylamide, harmaline hydrochloride dehydrate, 1,2,3,4-tetrahydro-beta-carboline-1-carboxylic acid, 1,2,3,4-Tetrahydro-9H-pyrido[3,4-b]indole, harmane, harmine, harmaline or a combination thereof suspended in an organic solvent. In one embodiment, a matrix solution comprises one or more selected from norharmane (NRM), 3-Hydroxymethyl-β-carboline, 3-Methyl-a-carboline, Ethyl 2,3,4,9-tetrahydro-1H-β-carboline-3-carboxylate, Ethyl β-carboline-3-carboxylate, 1-Methylindole-2-carboxylic acid, norharman methiodide, β-Carboline-3-carboxylic acid N-methylamide, harmaline hydrochloride dehydrate, 1,2,3,4-tetrahydro-beta-carboline-1-carboxylic acid, 1,2,3,4-Tetrahydro-9H-pyrido[3,4-b]indole, harmane, harmine, harmaline or a combination thereof suspended in an organic solvent at a concentration of 1 to 100 mg/ml. In one embodiment, a matrix solution comprises one or more selected from norharmane (NRM), 3-Hydroxymethyl-β-carboline, 3-Methyl-a-carboline, Ethyl 2,3,4,9-tetrahydro-1H-β-carboline-3-carboxylate, Ethyl β-carboline-3-carboxylate, 1-Methylindole-2-carboxylic acid, norharman methiodide, β-Carboline-3-carboxylic acid N-methylamide, harmaline hydrochloride dehydrate, 1,2,3,4-tetrahydro-beta-carboline-1-carboxylic acid, 1,2,3,4-Tetrahydro-9H-pyrido[3,4-b]indole, harmane, harmine, harmaline or a combination thereof suspended in an organic solvent at a concentration of 7 to 13 mg/ml. In a preferable embodiment, a matrix solution comprises one or more selected from norharmane (NRM), 3-Hydroxymethyl-β-carboline, 3-Methyl-a-carboline, Ethyl 2,3,4,9-tetrahydro-1H-β-carboline-3-carboxylate, Ethyl β-carboline-3-carboxylate, 1-Methylindole-2-carboxylic acid, norharman methiodide, β-Carboline-3-carboxylic acid N-methylamide, harmaline hydrochloride dehydrate, 1,2,3,4-tetrahydro-beta-carboline-1-carboxylic acid, 1,2,3,4-Tetrahydro-9H-pyrido[3,4-b]indole, harmane, harmine, harmaline or a combination thereof suspended in an organic solvent at a concentration of 10 mg/ml.

In one embodiment, a matrix solution comprises norharmane (NRM) suspended in an organic solvent. In one embodiment, a matrix solution comprises norharmane suspended in an organic solvent at a concentration of 1 to 100 mg/ml. In one embodiment, a matrix solution comprises norharmane suspended in an organic solvent at a concentration of 7 to 13 mg/ml. In a preferable embodiment, a matrix solution comprises norharmane suspended in an organic solvent at a concentration of 10 mg/ml.

In one embodiment, an organic solvent comprises chloroform and methanol at a ratio of about 6:1 to about 12:1, or about 8:1 to about 10:1. Suitably an organic solvent comprises chloroform and methanol at a ratio of about 9:1 v/v.

In one embodiment, the organic solvent comprises one or more of chloroform, methanol, dichloromethane, ether, diethyl-ether, petroleoum ether, isopropanol, butanol, hexane or a combination thereof.

In one embodiment, the organic solvent comprises one or more of acetic acid, acetone, acetonitrile, benzene, 1-butanol, 2-butanol, 2-butanone, t-butyl alcohol, carbon tetrachloride, chlorobenzene, chloroform, cyclohexane, 1,2-dichloroethane, diethylene glycol, diethyl ether, diglyme (diethylene glycol dimethyl ether), 1,2-dimethoxy-ethane (glyme, DME), dimethylformamide (DMF), dimethyl sulfoxide (DMSO), 1,4-dioxane, ethanol, ethyl acetate, ethylene glycol, glycerine, heptane, Hexamethylphosphoramide (HMPA), Hexamethylphosphorous triamide (HMPT), hexane, methanol, methyl t-butyl ether (MTBE), methylene chloride, N-methyl-2-pyrrolidinone (NMP), pentane, Petroleum ether (ligroine), 1-propanol, 2-propanol, pyridine, tetrahydrofuran (THF), toluene, triethyl amine, water, water (heavy), o-xylene, m-xylene, p-xylene.

In one embodiment, the ratio of the test sample to the matrix solution is between about 0.1:1 to about 2:1 v/v, or about 0.5:1 to about 0.7:1 v/v. Suitably, the ratio of the test sample to the matrix solution is about 0.66:1 v/v.

In one embodiment, a test sample comprises less than about $10^{10}$ bacterial cells. Suitably, a test sample may comprise less than about $10^9$, $10^8$, $10^7$, $10^6$, $10^5$, $10^4$, $10^3$, $10^2$ or $10^1$ bacterial cells.

In one embodiment a test sample comprises between about $10^1$ to about $10^{10}$ bacterial cells. Suitably, a test sample comprises between about $10^2$ to about $10^8$, about $10^3$ to about $10^6$, or about $10^4$ to about $10^5$ bacterial cells.

In one embodiment, the cyclic cationic polypeptide antibiotic is a polymyxin antibiotic. Suitably, a polymyxin antibiotic may be one or more of Colistin (Polymyxin E), Polymyxin B, Mattacin (Polymyxin M), or a salt thereof.

Preferably a polymyxin antibiotic may be Colistin (Polymyxin E) or a salt thereof. A salt of Colistin (Polymyxin E) may be Colistin sulfate or Colistimethate sodium.

In one embodiment a bacterium as used herein may be one or more selected from the following genera: *Escherichia, Klebsiella, Enterobacter, Pseudomonas, Acinetobacter, Shigella, Salmonella, Citrobacter, Raoultella* or combinations thereof.

Suitably a bacterium may be one or more selected from the following species: *Escherichia coli, Klebsiella pneumoniae, Klebsiella oxytoca, Enterobacter aerogenes, Enterobacter cloacae, Enterobacter asburiae, Pseudomonas aeruginosa, Acinetobacter baumannii, Shigella sonnei, Shigella flexneri, Salmonella enterica, Citrobacter freundii, Citrobacter koseri, Citrobacter amalonaticus, Citrobacter youngae* or combinations thereof.

Any strain of such genera or species may be suitable for use in a method of the invention.

In one embodiment, a bacterium is heat inactivated. A bacterium may be heat inactivated using any technique known in the art, for example by using a dry bath or a water bath. Where a dry bath or water bath is used a bacterium may be heat inactivated by heating for between about 10 to about 50 minutes at 70 to 90°C (suitably for 30 min at 80°C). In some embodiments a bacterium may be heat inactivated by heating for at least about 45 minutes (preferably at least 1 hour) to at least about 80 °C (preferably at least 90 °C).

In some embodiments, the bacterium is not heat inactivated.

In one embodiment, a method of the invention comprises the step of recording the data obtained in step (a) on a suitable data carrier. Thus the present invention also provides a data carrier comprising data obtained in step (a) of a method herein.

In one aspect of the invention, there is provided a screening method for identifying an inhibitor of cyclic cationic polypeptide antibiotic resistance in a bacterium, comprising:

a. incubating a sample comprising a bacterium resistant to a cyclic cationic polypeptide antibiotic with a candidate inhibitor;
b. subjecting said sample to mass spectrometry analysis according to a method of the present invention and generating a mass spectrum output; and
c. identifying the presence or absence of said first defined peak in the mass spectrum output;

wherein the presence of said first defined peak indicates said candidate inhibitor is not a substance capable of inhibiting cyclic cationic polypeptide antibiotic resistance in a bacterium, and wherein the absence of said first defined peak

indicates said candidate inhibitor is a substance capable of inhibiting cyclic cationic polypeptide antibiotic resistance in a bacterium.

In one aspect of the invention, there is provided a screening method for identifying an inhibitor of cyclic cationic polypeptide antibiotic resistance in a bacterium, comprising:

a. incubating a sample comprising a bacterium resistant to a cyclic cationic polypeptide antibiotic with a candidate inhibitor;
b. subjecting said sample to mass spectrometry analysis according to a method of the present invention and generating a mass spectrum output; and
c. identifying the presence or absence of said first defined peak and/or said third defined peak in the mass spectrum output;

wherein the presence of said first defined peak and/or said third defined peak indicates said candidate inhibitor is not a substance capable of inhibiting cyclic cationic polypeptide antibiotic resistance in a bacterium, and wherein the absence of said first defined peak and/or said third defined peak indicates said candidate inhibitor is a substance capable of inhibiting cyclic cationic polypeptide antibiotic resistance in a bacterium.

In one aspect of the disclosure there is provided a screening method for identifying an inhibitor of cyclic cationic polypeptide antibiotic resistance in a bacterium, comprising:

a. incubating a sample comprising a bacterium resistant to a cyclic cationic polypeptide antibiotic with a candidate inhibitor;
b. subjecting said sample to mass spectrometry analysis according to a method of the present invention and generating a mass spectrum output; and
c. identifying the presence or absence of said first defined peak, said third defined peak, said fourth defined peak and/or said fifth defined peak in the mass spectrum output;

wherein the presence of said first defined peak, said third defined peak, said fourth defined peak and/or said fifth defined peak indicates said candidate inhibitor is not a substance capable of inhibiting cyclic cationic polypeptide antibiotic resistance in a bacterium, and wherein the absence of said first defined peak and/or said third defined peak indicates said candidate inhibitor is a substance capable of inhibiting cyclic cationic polypeptide antibiotic resistance in a bacterium.

Said screening method(s) embraces the corresponding use of mass spectrometry for identifying an inhibitor of cyclic cationic polypeptide antibiotic resistance in a bacterium.

In one embodiment, wherein said screening method fails to identify an inhibitor of cyclic cationic polypeptide antibiotic (e.g. polymyxin) resistance in a bacterium, steps a. - c. are repeated with a different candidate inhibitor. This sequence may be repeated iteratively until the absence of the first defined peak is identified, indicating the candidate inhibitor is a substance capable of inhibiting cyclic cationic polypeptide antibiotic resistance in a bacterium. Once the absence of the first defined peak is identified, said candidate inhibitor may be used as an inhibitor of cyclic cationic polypeptide antibiotic resistance in a bacterium.

In one embodiment, a screening method for identifying an inhibitor of cyclic cationic polypeptide antibiotic resistance in a bacterium comprises incubating a sample comprising a bacterium resistant to a cyclic cationic polypeptide antibiotic with a candidate inhibitor and subsequently testing said bacterium for susceptibility to a cyclic cationic polypeptide antibiotic, wherein mass spectrometry analysis according to a method of the present invention is used to confirm whether a substance is capable or is not capable of inhibiting cyclic cationic polypeptide antibiotic resistance in a bacterium by identifying the presence or absence of said first defined peak and/or said third defined peak in a mass spectrum output.

In one embodiment a bacterium resistant to a cyclic cationic polypeptide antibiotic may be isolated from a clinical sample. In an alternative embodiment, the bacterium resistant to a cyclic cationic polypeptide antibiotic may be cultured under laboratory conditions.

A candidate inhibitor may be capable of directly or indirectly inhibiting modification of Lipid A with 4-amino-L-arabinose and/or phosphoethanolamine.

In one embodiment a candidate inhibitor is selected from a small molecule inhibitor, a peptide, a monoclonal or a polyclonal antibody or an antibody fragment.

In one embodiment a candidate inhibitor is a known chemical or pharmaceutical substance selected from a library of such candidate inhibitors.

In one embodiment a sample comprising a bacterium resistant to a cyclic cationic polypeptide antibiotic with a candidate inhibitor is a sample from a human or a non-human animal. A non-human animal may be, for example, a non-human primate, horse, cow, goat, cat, dog, sheep, rodent (e.g. mouse, rate or Guinea pig), fish or amphibian (e.g. *Xenopus*).

In one aspect, there is provided the use of a test sample in mass spectrometry analysis for detection of the

presence or absence of a bacterium resistant to a cyclic cationic polypeptide antibiotic (e.g. in said test sample).

Methods of determining nucleic acid percentage sequence identity are known in the art. By way of example, when assessing nucleic acid sequence identity, a sequence having a defined number of contiguous nucleotides may be aligned with a nucleic acid sequence (having the same number of contiguous nucleotides) from the corresponding portion of a nucleic acid sequence of the present disclosure. Tools known in the art for determining nucleic acid percentage sequence identity include Nucleotide BLAST.

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position- Specific Gap Penalties and Weight Matrix Choice, 22(22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 -509 (1992); Gibbs sampling, see, e.g., C. E. Lawrence et al., Detecting Subtle Sequence Signals: A Gibbs Sampling Strategy for Multiple Alignment, 262(5131) Science 208-214 (1993); Align-M, see, e.g., Ivo Van Walle et al., Align-M - A New Algorithm for Multiple Alignment of Highly Divergent Sequences, 20(9) Bioinformatics:1428-1435 (2004).

Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992. Briefly, two amino acid sequences are aligned to optimize the alignment scores using a gap opening penalty of 10, a gap extension penalty of 1, and the "blosum 62" scoring matrix of Henikoff and Henikoff (ibid.) as shown below (amino acids are indicated by the standard one-letter codes).

<u>**ALIGNMENT SCORES FOR DETERMINING SEQUENCE IDENTITY**</u>

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 | | | | | | | | | | | | | | | | | | | |
| R | -1 | 5 | | | | | | | | | | | | | | | | | | |
| N | -2 | 0 | 6 | | | | | | | | | | | | | | | | | |
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | -1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | 1 | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 11 | | |
| Y | -2 | -2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

The percent identity is then calculated as:

$$\frac{\text{Total number of identical matches}}{\text{[length of the longer sequence plus the number of gaps introduced into the longer sequence in order to align the two sequences]}} \times 100$$

Substantially homologous polypeptides are characterized as having one or more amino acid substitutions, deletions or additions. These changes are preferably of a minor nature, that is conservative amino acid substitutions (see below) and other substitutions that do not significantly affect the folding or activity of the polypeptide; small deletions, typically of one to about 30 amino acids; and small amino- or carboxyl-terminal extensions, such as an amino-terminal methionine residue, a small linker peptide of up to about 20-25 residues, or an affinity tag.

CONSERVATIVE AMINO ACID SUBSTITUTIONS

Basic: arginine

(continued)

| | |
|---|---|
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

In addition to the 20 standard amino acids, non-standard amino acids (such as 4-hydroxyproline, 6-N-methyl lysine, 2-aminoisobutyric acid, isovaline and $\alpha$-methyl serine) may be substituted for amino acid residues of the polypeptides described herein. A limited number of non-conservative amino acids, amino acids that are not encoded by the genetic code, and unnatural amino acids may be substituted for polypeptide amino acid residues. The polypeptides described herein can also comprise non-naturally occurring amino acid residues.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell free system comprising an E. coli S30 extract and commercially available enzymes and other reagents. Proteins are purified by chromatography. See, for example, Robertson et al., J. Am. Chem. Soc. 113:2722, 1991; Ellman et al., Methods Enzymol. 202:301, 1991; Chung et al., Science 259:806-9, 1993; and Chung et al., Proc. Natl. Acad. Sci. USA 90:10145-9, 1993). In a second method, translation is carried out in Xenopus oocytes by microinjection of mutated mRNA and chemically aminoacylated suppressor tRNAs (Turcatti et al., J. Biol. Chem. 271:19991-8, 1996). Within a third method, E. coli cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the polypeptide in place of its natural counterpart. See, Koide et al., Biochem. 33:7470-6, 1994. Naturally occurring amino acid residues can be converted to non-naturally occurring species by in vitro chemical modification. Chemical modification can be combined with site-directed mutagenesis to further expand the range of substitutions (Wynn and Richards, Protein Sci. 2:395-403, 1993).

A limited number of non-conservative amino acids, amino acids that are not encoded by the genetic code, non-naturally occurring amino acids, and unnatural amino acids may be substituted for amino acid residues of polypeptides described herein.

Essential amino acids in the polypeptides described herein can be identified according to procedures known in the art, such as site-directed mutagenesis or alanine-scanning mutagenesis (Cunningham and Wells, Science 244: 1081-5, 1989). Sites of biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-12, 1992; Smith et al., J. Mol. Biol. 224:899-904, 1992; Wlodaver et al., FEBS Lett. 309:59-64, 1992. The identities of essential amino acids can also be inferred from analysis of homologies with related components (e.g. the translocation or protease components) of the polypeptides described herein.

Multiple amino acid substitutions can be made and tested using known methods of mutagenesis and screening,

such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide the skilled person with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

The headings provided herein are not limitations of the various aspects or embodiments of this disclosure.

Other definitions of terms may appear throughout the specification. Before the exemplary embodiments are described in more detail, it is to be understood that this disclosure is not limited to particular embodiments described, and as such may vary. It is also to be understood that the terminology used herein is for the purpose of describing particular embodiments only, and is not intended to be limiting, since the scope of the present disclosure will be defined only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "a bacterium" includes a plurality of such a bacterium and reference to "the bacterium" includes reference to one or more bacterial cells and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The invention will now be described, by way of example only, with reference to the following Examples.

## FIGURES

Embodiments of the invention will now be described, by way of example only, with reference to accompanying figures, in which:

**Figure 1** shows a scheme representing the analysis of lipids on intact bacteria through mass spectrometry. The test sample is placed (11) onto the MALDI target (10) and overlaid with a suitable matrix solution (12). Mass spectrometry in negative or positive ion mode is undertaken using any suitable mass spectrometry machine (12) (e.g. 4800 MALDI TOF/TOF Analyser from Applied Biosystems). MS may involve use of atypical solvents matrix (organic solvents, $CHCL_3$, MeOH, PE, DE). MS = mass spectrometry; $CHCl_3$ = Chlorine; MeOH = Methanol; PE = Petroleum Ether; DE = diethyl-ether.

**Figure 2 (A)** shows typical structures of: native Lipid A (21) having a size of 1976 m/z; Lipid A modified with phosphoethanolamine (pETN) at the 1' phosphate with concomitant loss of the 4' phosphate (22) having a size of 1821 m/z; **(B)** shows Lipid A modified with pETN having a size of 1919 m/z (23); and Lipid A modified with 4-amino-L-arabinose (L-Ara4N) and pETN. Numbers indicate the number of carbon atoms in each fatty acid chain.

**Figure 3** shows a schematic of Lipid A modifications related to cyclic polypeptide antibiotic (e.g. polymyxin) resistance in a bacterium of the family *Enterobacteriaceae.* The modification of Lipid A (31) with phosphoethanolamine (pETN) occurs in bacteria comprising a plasmid having a Mobilised colistin resistance (MCR) or mcr-like gene (32). The modification of Lipid A with 4-amino-L-arabinose (L-Ara4N) and/or phosphoethanolamine (33) occurs in bacteria comprising certain alterations in genes (e.g. *pmrA, pmrB, pmrC, pmrD, pmrE, pmrR, phoP, phoQ, mgrB, arnA, arnB, arnC, arnD, arnE, arnF, arnF, arnT, cptA, eptB).*

**Figure 4** shows examples of MALDI mass spectra generated by a method of the present invention, demonstrating the discrimination between polymyxin susceptible *Escherichia coli* **(A),** chromosome-encoded polymyxin resistant *E. coli* **(B),** and plasmid-encoded polymyxin resistant (e.g. *mcr-1* positive) *E. coli* **(C).** For each mass spectrum, the peak at *m*/*z* 1796.2 was assigned to native Lipid A, the peak at *m*/*z* 1919.2 was assigned to Lipid A modified by phosphoethanolamine, and the peak at 1821.2 was assigned to Lipid A modified by phosphoethanolamine at the 1' position with concomitant loss of the 4' phosphate.

**Figure 5** shows examples of MALDI mass spectra generated by a method of the present invention, demonstrating the discrimination between polymyxin susceptible *Escherichia coli* (e.g. *E. coli* J53) (top panel) and plasmid-encoded polymyxin resistant (e.g. *mcr-1* positive) *E. coli* (e.g. *E. coli* R4) (bottom panel). For each mass spectrum, the peak at *m*/*z* 1796.2 (50) was assigned to native Lipid A (e.g. a second defined peak), the peak at *m*/*z* 1919.2 (51) was assigned to Lipid A modified by phosphoethanolamine (e.g. a first defined peak).

**Figure 6** shows distribution of the Polymyxin Resistance Ratios (PPR) for the 79 *E.coli* strains tested (see Figure 18). Three independent experiments were performed for each strain. Cut-off values for discrimination between polymyxin-resistance and polymyxin-susceptibility (0.1) and for discrimination between chromosome- and MCR-encoded resistance to polymyxin (0.5) are indicated by grey and black dotted lines, respectively. $PRR_{E.\ coli} = (I_{1919}+I_{1821}) / I_{1796}$. I = Intensity (e.g. peak intensity on a mass spectrum).

**Figure 7** shows examples of MALDI mass spectra generated by a method of the present invention, demonstrating the discrimination between polymyxin susceptible *Klebsiella pneumoniae* (top panel) and plasmid encoded polymyxin-resistant (e.g. *mcr-1* positive) *K.*

*pneumoniae* (bottom panel). For each mass spectrum, the peak at *m*/*z* 1796.2 and *m*/*z* 1840 (71) is assigned to native Lipid A, the peak at *m*/*z* 1919.2 (72) is assigned to Lipid A modified by phosphoethanolamine.

**Figure 8** shows examples of MALDI mass spectra generated by a method of the present invention, demonstrating the discrimination between polymyxin susceptible *Klebsiella* spp. (first panel), chromosome encoded polymyxin-resistant *Klebsiella* spp. (second panel), plasmid encoded polymyxin-resistant (e.g. *mcr-1* positive) *Klebsiella* spp. (third panel) and *Klebsiella* spp. which are both chromosome encoded plasmid encoded polymyxin-resistant (bottom panel). For each mass spectrum, the peaks at *m*/*z* 1824, *m*/*z* 1840, m/z 2062, and m/z 2078 were assigned to native Lipid A, the peaks at m/z 1971 and m/z 2209 were assigned to Lipid A modified by L-Ara4N and the peaks at *m*/*z* 1963 and m/z 2201 were assigned to Lipid A modified by pETN.

**Figure 9** shows examples of MALDI mass spectra generated with the *A. baumannii* strains S1 and R1. For each mass spectrum, the peak at *m*/*z* 1910.3 (90) is assigned to native Lipid A, the peak at *m*/*z* 2033.3 (91) is assigned to Lipid A modified by phosphoethanolamine. Top panel represents mass spectra obtained from polymyxin susceptible *A. baumannii* S1. Bottom panel represents mass spectra obtained from polymyxin resistant *A. baumannii* R1.

**Figure 10** shows examples of MALDI mass spectra generated with the *E. coli* strains S1 J53, R4 J53, R4 and R1. For each mass spectrum, the peak at *m*/*z* 1796.2 is assigned to native Lipid A (e.g. a second defined peak), the peak at *m*/*z* 1919.2 is assigned to Lipid A modified by phosphoethanolamine (e.g. a first defined peak). The peak at *m*/*z* 1821 (+ 25 m/z from the native lipid A) (e.g. a third defined peak) is typical of a mass spectrum generated through a method of the invention with plasmid encoded polymyxin-resistant *Enterobacteriaceae* (e.g. *Escherichia coli, Klebsiella pneumoniae, Klebsiella oxytoca, Enterobacter aerogenes, Enterobacter cloacae, Enterobacter asburiae, Shigella sonnei, Shigella flexneri, Salmonella enterica, Citrobacter freundii, Citrobacter koseri, Citrobacter amalonaticus* or *Citrobacter youngae*) bacteria. Top panel represents mass spectra obtained from polymyxin susceptible *E. coli* J53. Middle panel represents mass spectra obtained from plasmid-encoded polymyxin resistant *E. coli* R4. Bottom panel represents mass spectra obtained from polymyxin resistant *E. coli* R1.

**Figure 11** shows examples of MALDI mass spectra generated with the *K. pneumoniae* strains S32, R46, R42. For each mass spectrum, the peak at *m*/*z* 1796.2 is assigned to native Lipid A (e.g. a second defined peak), the peak at *m*/*z* 1919.2 is assigned to Lipid A modified by phosphoethanolamine (e.g. a first defined peak). The peak at *m*/*z* 1821 (+ 25 m/z from the native lipid A) (e.g. a third defined peak) is typical of a mass spectrum generated through a method if the invention with plasmid encoded polymyxin-resistant *Enterobacteriaceae* (e.g. *Escherichia coli, Klebsiella pneumoniae, Klebsiella oxytoca, Enterobacter aerogenes, Enterobacter cloacae, Enterobacter asburiae, Shigella sonnei, Shigella flexneri, Salmonella enterica, Citrobacter freundii, Citrobacter koseri, Citrobacter amalonaticus* or *Citrobacter youngae*) bacteria. Top panel represents mass spectra obtained from polymyxin susceptible *K. pneumoniae* S32. Middle panel represents mass spectra obtained from plasmid-encoded polymyxin resistant *K. pneu-*

*moniae* R46. Bottom panel represents mass spectra obtained from chromosome-encoded polymyxin resistant *K. pneumoniae* KpR42.

**Figure 12** shows examples of MALDI mass spectra generated by a method of the present invention, demonstrating the discrimination between polymyxin susceptible *Salmoella spp.* (top panel), chromosome-encoded polymyxin resistant *Salmonella spp.* (middle panel), and plasmid-encoded polymyxin resistant *Salmonella spp* (bottom panel). For each mass spectrum, the peak at *m/z* 1796 was assigned to native Lipid A, the peaks at *m/z* 1919 and m/z 2034 were assigned to Lipid A modified by phosphoethanolamine, the peak at m/z was assign to Lipid A modified with L-Ara4N, and the peaks at 1919 and 2157 were assigned to Lipid A modified by phosphoethanolamine.

**Figure 13** shows a plot of example ratios of phosphoethanolamine modified Lipid A to native Lipid A, obtained for polymyxin susceptible (n = 44) and polymyxin-resistant (n=8) *E. coli,* calculated as follows: Intensity of Lipid A modified by phosphoethanolamine peak / Intensity of native Lipid A peak, e.g. Intensity of *m/z* 1919 peak / Intensity of *m/z* 1796 peak. The box plot represents the data by its quartiles.

**Figure 14** shows examples of MALDI mass spectra generated by a method of the present invention from *E.coli* grown on various clinically relevant media. **(A)** shows mass spectra for bacteria grown on Mueller-Hinton media; representative mass spectra are shown for polymyxin susceptible bacteria (top panel) having $PRR_{E.coli}$ = 0, plasmid-encoded polymyxin resistant bacteria (middle panel) having $PRR_{E.coli}$ = 3.83, and chromosome-encoded polymyxin resistant bacteria (bottom panel) having $PRR_{E.coli}$ = 0.24. **(B)** shows mass spectra for bacteria grown on Lysogeny Broth media; representative mass spectra are shown for polymyxin susceptible bacteria (top panel) having $PRR_{E.coli}$ = 0, plasmid-encoded polymyxin resistant bacteria (middle panel) having $PRR_{E.coli}$ = 2.89, and chromosome-encoded polymyxin resistant bacteria (bottom panel) having $PRR_{E.coli}$ = 0.35. **(C)** shows mass spectra for bacteria grown on Mueller-Hinton + Polymyxin B (1 mg/ml) media; representative mass spectra are shown for plasmid-encoded polymyxin resistant bacteria (top panel) having $PRR\text{-}_{E.coli}$ = 4.58, and chromosome-encoded polymyxin resistant bacteria (bottom panel) having $PRR_{E.coli}$ = 0.37. **(D)** shows mass spectra for bacteria grown on Mueller-Hinton + Polymyxin B (2 mg/ml) media; representative mass spectra are shown for plasmid-encoded polymyxin resistant bacteria (top panel) having $PRR_{E.coli}$ = 5.69, and chromosome-encoded polymyxin resistant bacteria (bottom panel) having $PRR_{E.coli}$ = 0.21. E. coli strains used were: J53 (susceptible), J53 + *mcr-1* (plasmid-encoded resistant) and CNR20160235 (chromosome-encoded resistant).

**Figure 15** Resume of the peaks of interest (e.g. particular interest) profiles observed in *Escherichia coli, Klebsiella pneumoniae, Salmonella* spp (e.g. *Salmonella enterica*) and *Acinetobacter baumannii.*

**Figure 16** shows alignment of the nucleic acid sequences of MCR variants.

**Figure 17** shows divergence of MCR variant from different families.

**Figure 18** shows characteristics and MALDIxin test results for *E. coli* strains.[a] Laboratory strains are underlined, all other strains are clinical isolates. [b] For unknown mechanisms absence of mutation in *mgrB, pmrA, pmrB, phoP* and *phoQ* have been checked by PCR and sequencing, and the strain was negative for mcr-like genes. [c] Carbapenemases are shown in bold and extended spectrum β-lactamases are underlined. [d] PRR stands for Polymyxin Resistance Ratio. [e] The MCR-2 producing *E. coli* R12 F5 has been previously described by Xavier BB et al. (Euro surveillance 2016; 21(27)). * natural plasmids, the natural *mcr-5* carrying plasmid has been previously described by Borowiak M et al. (Journal of Antimicrobial Chemotherapy (2017), 72(12),3317-3324). ** functional *mcr-like* gene cloned into pDM1, an IPTG-inducible (final concentration 0.5 mM), $Tet^R$ derivative of pACYC184. The genes *mcr-1, mcr-2* and *mcr-5* were cloned into the SacI/XmaI sites of the vector, while for *mcr-3,* the NdeI/XmaI sites were used.

**Figure 19** shows results of a method of the invention (e.g. the MALDIxin test) on bacterial colonies after 24 hours, 48 hours, one week and two weeks. [a] Laboratory strains are underlined, all other strains are clinical isolates. [b] For unknown mechanisms absence of mutation in *mgrB, pmrA, pmrB, phoP* and *phoQ* have be checked by PCR and sequencing, and the strain was negative for mcr-like genes. PRR stands for Polymyxin Resistance Ratio. * natural plasmid.

## EXAMPLES

### Materials & methods

#### Bacterial Strains

The following bacterial strains, representative of clinical isolates, were used in a method of the invention: *E. coli* S1 J53, *E. coli* R4 J53, *E. coli* R1, *K. pneumoniae* R46, *K. pneumoniae* R42, A. *baumannii* S1 and A. *baumannii* R1.

Additionally, a collection of 79 *E. coli* strains were used, including 33 polymyxin-resistant isolates, of which 29 were MCR producers (18 MCR-1, two MCR-1.5, three MCR-2, two MCR-3 and four MCR-5). The 46 polymyxin-susceptible E. coli strains were of various phenotypes, from wild-type to carbapenemase producers (Figure 18). The MALDIxin test was prospectively evaluated using a collection of 78 isolates of carbapenemase-producing E. coli received during

October and November 2016, from the French National Reference Centre (NRC) for Antimicrobial Resistance (Table 2).

Plasmid encoded polymyxin-resistant bacterial strains comprise a plasmid having an mcr-like gene, such as *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4* or mcr-5gene, or a sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, or SEQ ID NO: 16. Addition of phosphoethanolamine to Lipid A in such strains is plasmid mediated through the mcr-like gene, which confers plasmid-encoded resistance to polymyxin antibiotics (e.g. colistin) in isolates from humans and animals.

Chromosome encoded polymyxin-resistant bacterial strains comprise a mutation and/or truncation in any one of the genes *pmrA, pmrB, pmrC, pmrD, pmrE, pmrR, phoP, phoQ, mgrB, arnA, arnB, arnC, arnD, arnE, arnF, arnF, arnT, cptA, eptB.* For example, missense mutations in *pmrA* or *pmrB* can cause the constitutive activation of the PmrA/PmrB system, leading to upregulation of *pmrC* and the *arnBCADTEF* operon, and thus synthesis and addition of phosphoethanolamine and 4-amino-L-arabinose to Lipid A. Disruption (e.g. mutation or truncation) of *mgrB* prevents negative feedback on the PhoP/PhoQ regulatory system, leading to the addition of phosphoethanolamine and 4-amino-L-arabinose to Lipid A. Disruption of *mgrB* can be due to insertional inactivation (e.g. with IS*Kpn25*).

Susceptibility Testing

Minimal inhibitory concentrations (MICs) were determined by BMD according to the guidelines of the CLSI and EUCAST joint subcommittee. Results were interpreted using EUCAST breakpoint as updated in 2017.

Methods

A one microliter loop (equivalent to one or less than one colony equivalent to $10^4$ to $10^7$ bacterial cells) of bacteria, recovered from colonies grown on any bacteria culture medium or from any enrichment liquid medium (including blood cultures or other clinical samples with enough amount of bacteria) was collected. This colony was transferred into an Eppendorf tube (0.5 to 2mL) containing 100 microliter of water (distilled or double-distilled). The culture medium was Luria Broth agar; or other non-selective media such as blood agar or chocolate blood agar, Gram-negative selective media such as Drigalski or MacConkey media, and several chromogenic media may be used. Strains may be cultivated under aerobic conditions in Luria Broth agar medium at 37 °C overnight.

Bacteria were then heat inactivated (facultative step) using a dry bath or a water bath for 30 min at 80°C, or alternatively for 1 h at 90 °C.

Bacteria were pelleted and washed at least once by 200 μl double-distilled water to remove salts excess resulting from the culture medium. Failure to wash the bacterial pellet to remove the remaining salts can lead to an undesirable background on the final MALDI-TOF spectrum, obscuring or removing the interpretability of the results. Bacteria may alternatively be washed through a commercially available solution (e.g. salt absorbing columns). Bacteria may be washed three times with 0.5 ml of double distilled water and centrifuged at 9000 xg for 5 min.

The washed bacteria were resuspended in 50 μl of double-distilled water to provide a bacterial suspension at a final concentration of about $10^4$ to $10^7$ bacteria per μl and 0.4 μl was loaded on the MALDI-TOF target. Such classical MALDI-TOF targets as used in the average clinical microbiology lab are suitable for use with the present invention.

0.6 μl (or 0.8 μl) of a designed atypical matrix was admixed with the 0.4 μl of bacterial suspension. Suitably, the atypical matrix comprises 2,5-dihydroxybenzoic acid at a final concentration of 10 mg/ml suspended in organic solvents, usually Chloroform/Methanol 9:1 v/v. Additional organic solvents suitable for use with the invention include chloroform, dichloromethane, methanol, ether, diethyl-ether, petroleum ether, isopropanol, butanol, hexane. Table 1 provides examples of solvents suitable for use with the present invention. The sample and matrix solution were deposited on the target (e.g. MALDI target), mixed with a micropipette and dried gently under a stream of air. After optimization, this solvent system and solvent system to sample ratio allows selective ionization of Lipid A (e.g. on intact bacteria).

Alternatively, two colonies of bacterial cells grown on any bacteria culture medium or from any enrichment liquid medium (including blood cultures or other clinical samples with enough amount of bacteria) are collected and transferred into an Eppendorf tube (0.5 to 2ml). This may be followed by suspended the colonies in water (distilled or double distilled) followed by pelleting and washing the bacteria at least once (e.g. 2-5 times) with distilled or double distilled water, e.g. to remove excess salts. The bacteria may be resuspended in 2% acetic acid in water (e.g. 200μl 2% acetic acid in water). The bacterial suspension may then be heated by any suitable means, for example using a heat-block, preferably for 2 hours at 100°C. This may be followed by pelleting the bacteria (e.g. by centrifuging for 5 seconds at 15,000 g) and resuspending the bacteria in distilled or double distilled water (e.g. 50μl $ddH_20$). Norharmane (e.g. N6252 Sigma) matrix or any of its derivatives (e.g. 3-Hydroxymethyl-β-carboline; 3-Methyl-a-carboline; Ethyl 2,3,4,9-tetrahydro-1H-β-carboline-3-carboxylate; Ethyl β-carboline-3-carboxylate; 1-Methylindole-2-carboxylic acid; norharman methiodide; β-Carboline-3-carboxylic acid N-methylamide; Harmaline hydrochloride dehydrate; 1,2,3,4-tetrahydro-beta-carboline-1-carboxylic acid; 1,2,3,4-Tetrahydro-9H-pyrido[3,4-b]indole; Harmane; Harmine; Harmaline) may be admixed with the bacterial suspension. Suitably, 0.6μl of said matrix may be admixed with 0.4μl of bacterial suspension. Suitably, the Norharmane matrix or any of its derivatives (as outlined above) is present at a final concentration of 10 mg/ml suspended in organic solvents, usually Chloroform/Methanol 9:1 v/v. This protocol is particularly suitable for use in a method of the invention where the Lipid A composition of Klebsiella and/or Salmonella bacteria are being investigated.

A mass spectrum was generated. Typical MALDI-TOF mass spectrometry machines suitable for use in a method of the present invention include the Bioyper from Bruker Daltonics and VITEK-MS from bioMerieux. Alternatively, MALDI-TOF MS analysis is performed on a 4800 Proteomics Analyzer (with TOF-TOF Optics, Applied Biosystems) (e.g. using the reflectron mode). Samples are typically analyzed operating at 20 kV in the negative ion mode using an extraction delay time set at 20 ns. Typically, spectra from 500 to 2000 laser shots are summed to obtain the final spectrum. All experiments are typically carried out on three independent samples and in three technical replicates. The negative control consisted of 0.5 μl of double distilled water and 0.5 μl of the matrix solution. Mass spectrometry data are typically analyzed using Data Explorer version 4.9 from Applied Biosystems. The mass spectrum may scanned between m/z 1000 and 2200, preferably between m/z 1,500 and 2,500.

The resulting mass spectrum was analysed, and peaks corresponding to intact Lipid A (e.g. 1796 m/z for *Escherichia coli*) and modified Lipid A (addition of phosphoethanolamine (pETN) [+ 123 m/z]) were identified and used to calculate intensity ratios. The modified Lipid A to native Lipid A ratio is used to discriminate between polymyxin-susceptible and polymyxin-resistant bacteria. For polymyxin-resistant Enterobacteriaceae discrimination between plasmid encoded resistance and chromosome encoded resistance has been assessed using the third peak [+ 25 m/z] (e.g. 1821 m/z for *Escherichia coli* and *Klebsiella pneumoniae*).

Patients infected with plasmid encoded polymyxin-resistant bacteria, as determined by a method of the present invention, were quarantined to prevent transmission to other patients and/or subjects.

This method (e.g. according to the claims) allowed the detection of polymyxin resistance directly on bacteria (e.g. using samples comprising a bacterial membrane) in less than 15 minutes.

Statistical analysis

Data were compared two-by-two using unpaired Welch's t-test. P values $< 0.05$ were considered statistically different.

Table 1

Table 1: List of suitable organic solvents suitable for use in a matrix solution of the invention.

| Solvent | Formula | MW | Boiling point (°C) | Melting point (°C) | Density (g/mL) | Solubility in water (g/100g) | Dielectric Constant 3,4 | Flash point (°C) |
|---|---|---|---|---|---|---|---|---|
| acetic acid | $C_2H_4O_2$ | 60.052 | 118 | 16.6 | 1.0446 | Miscible | 6.2 | 39 |
| acetone | $C_3H_6O$ | 58.079 | 56.05 | -94.7 | 0.7845 | Miscible | 21.01 | -20 |
| acetonitrile | $C_2H_3N$ | 41.052 | 81.65 | -43.8 | 0.7857 | Miscible | 36.64 | 6 |
| benzene | $C_6H_6$ | 78.11 | 80.1 | 5.5 | 0.8765 | 0.18 | 2.28 | -11 |
| 1-butanol | $C_4H_{10}O$ | 74.12 | 117.7 | -88.6 | 0.8095 | 6.3 | 17.8 | 37 |
| 2-butanol | $C_4H_{10}O$ | 74.12 | 99.5 | -88.5 | 0.8063 | 15 | 17.26 | 24 |
| 2-butanone | $C_4H_8O$ | 72.11 | 79.6 | -86.6 | 0.7999 | 25.6 | 18.6 | -9 |
| t-butyl alcohol | $C_4H_{10}O$ | 74.12 | 82.4 | 25.7 | 0.7887 | Miscible | 12.5 | 11 |
| carbon tetrachloride | $CCl_4$ | 153.82 | 76.8 | -22.6 | 1.594 | 0.08 | 2.24 | -- |
| chlorobenzen | $C_6H_5Cl$ | 112.56 | 131.7 | -45.3 | 1.1058 | 0.05 | 5.69 | 28 |
| e | | | | | | | | |
| chloroform | $CHCl_3$ | 119.38 | 61.2 | -63.4 | 1.4788 | 0.795 | 4.81 | -- |
| cyclohexane | $C_6H_{12}$ | 84.16 | 80.7 | 6.6 | 0.7739 | <0.1 | 2.02 | -20 |
| 1,2-dichloroethan e | $C_2H_4Cl_2$ | 98.96 | 83.5 | -35.7 | 1.245 | 0.861 | 10.42 | 13 |
| diethylene glycol | $C_4H_{10}O_3$ | 106.12 | 246 | -10 | 1.1197 | 10 | 31.8 | 124 |
| diethyl ether | $C_4H_{10}O$ | 74.12 | 34.5 | -116.2 | 0.713 | 7.5 | 4.267 | -45 |
| diglyme (diethylene glycol dimethyl ether) | $C_6H_{14}O_3$ | 134.17 | 162 | -68 | 0.943 | Miscible | 7.23 | 67 |
| 1,2-dimethoxyethane (glyme, DME) | $C_4H_{10}O_2$ | 90.12 | 84.5 | -69.2 | 0.8637 | Miscible | 7.3 | -2 |
| dimethylformamide (DMF) | $C_3H_7NO$ | 73.09 | 153 | -60.48 | 0.9445 | Miscible | 38.25 | 58 |
| dimethyl sulfoxide (DMSO) | $C_2H_6OS$ | 78.13 | 189 | 18.4 | 1.092 | 25.3 | 47 | 95 |
| 1,4-dioxane | $C_4H_8O_2$ | 88.11 | 101.1 | 11.8 | 1.033 | Miscible | 2.21(25) | 12 |
| ethanol | $C_2H_6O$ | 46.07 | 78.5 | -114.1 | 0.789 | Miscible | 24.6 | 13 |

(continued)

| Solvent | Formula | MW | Boiling point (°C) | Melting point (°C) | Density (g/mL) | Solubility in water (g/100g) | Dielectric Constant 3,4 | Flash point (°C) |
|---|---|---|---|---|---|---|---|---|
| ethyl acetate | $C_4H_8O_2$ | 88.11 | 77 | -83.6 | 0.895 | 8.7 | 6(25) | -4 |
| ethylene glycol | $C_2H_6O_2$ | 62.07 | 195 | -13 | 1.115 | Miscible | 37.7 | 111 |
| glycerin | $C_3H_8O_3$ | 92.09 | 290 | 17.8 | 1.261 | Miscible | 42.5 | 160 |
| heptane | $C_7H_{16}$ | 100.2 | 98 | -90.6 | 0.684 | 0.01 | 1.92 | -4 |
| Hexamethylph osphoramide (HMPA) | $C_6H_{18}N_3OP$ | 179.2 | 232.5 | 7.2 | 1.03 | Miscible | 31.3 | 105 |
| Hexamethylph osphorous triamide (HMPT) | $C_6H_{18}N_3P$ | 163.2 | 150 | -44 | 0.898 | Miscible | -- | 26 |
| hexane | $C_6H_{14}$ | 86.18 | 69 | -95 | 0.659 | 0.014 | 1.89 | -22 |
| methanol | $CH_4O$ | 32.04 | 64.6 | -98 | 0.791 | Miscible | 32.6(25) | 12 |
| methyl t-butyl ether (MTBE) | $C_5H_{12}O$ | 88.15 | 55.2 | -109 | 0.741 | 5.1 | -- | -28 |
| methylene chloride | $CH_2Cl_2$ | 84.93 | 39.8 | -96.7 | 1.326 | 1.32 | 9.08 | 1.6 |
| N-methyl-2-pyrrolidinone (NMP) | $CH_5H_9NO$ | 99.13 | 202 | -24 | 1.033 | 10 | 32 | 91 |
| pentane | $C_5H_{12}$ | 72.15 | 36.1 | -129.7 | 0.626 | 0.04 | 1.84 | -49 |
| Petroleum ether (ligroine) | -- | -- | 30-60 | -40 | 0.656 | -- | -- | -30 |
| 1-propanol | $C_3H_8O$ | 88.15 | 97 | -126 | 0.803 | Miscible | 20.1(25) | 15 |
| 2-propanol | $C_3H_8O$ | 88.15 | 82.4 | -88.5 | 0.785 | Miscible | 18.3(25) | 12 |
| pyridine | $C_5H_5N$ | 79.1 | 115.2 | -41.6 | 0.982 | Miscible | 12.3(25) | 17 |
| tetrahydrofura n (THF) | $C_4H_8O$ | 72.106 | 65 | -108.4 | 0.8833 | 30 | 7.52 | -14 |
| toluene | $C_7H_8$ | 92.14 | 110.6 | -93 | 0.867 | 0.05 | 2.38(25) | 4 |
| triethyl amine | $C_6H_{15}N$ | 101.19 | 88.9 | -114.7 | 0.728 | 0.02 | 2.4 | -11 |
| water | $H_2O$ | 18.02 | 100 | 0 | 0.998 | -- | 78.54 | -- |
| water, heavy | $D_2O$ | 20.03 | 101.3 | 4 | 1.107 | Miscible | -- | -- |
| o-xylene | $C_8H_{10}$ | 106.17 | 144 | -25.2 | 0.897 | Insoluble | 2.57 | 32 |

(continued)

| Solvent | Formula | MW | Boiling point (°C) | Melting point (°C) | Density (g/mL) | Solubility in water (g/100g) | Dielectric Constant 3,4 | Flash point (°C) |
|---|---|---|---|---|---|---|---|---|
| m-xylene | $C_8H_{10}$ | 106.17 | 139.1 | -47.8 | 0.868 | Insoluble | 2.37 | 27 |
| p-xylene | $C_8H_{10}$ | 106.17 | 138.4 | 13.3 | 0.861 | Insoluble | 2.27 | 27 |

## EXAMPLE 1

**Detection of the presence or absence of a polymyxin-resistant *Escherichia coli* in a test sample**

Test samples comprising intact bacterial cells of *E. coli* (e.g. bacterial suspensions, washed to remove salt), as follows, were subjected mass spectrometry according to a method of the present invention: (i) Polymyxin-susceptible *E. coli*; (ii) chromosome encoded polymyxin-resistant *E. coli*; and (iii) plasmid encoded polymyxin-resistant *E. coli* (comprising a plasmid having the *mcr-1* gene).

For polymyxin susceptible *E. coli,* the mass spectrum was dominated by a peak at *m*/*z* 1796.2 (Figure 5, top panel; and Figure 10, top panel) assigned to native Lipid A. The absence of a peak indicating the presence of Lipid A modified with phosphoethanolamine at *m*/*z* 1919.2 (i.e. 123 mass units greater than native Lipid A) successfully indicated the absence of a bacterium resistant to a polymyxin antibiotic.

In plasmid encoded polymyxin-resistant *E. coli,* the mass spectrum further comprised peaks at *m*/*z* 1919.2 (i.e. 123 m/z units greater than native Lipid A), assigned to Lipid A modified by phosphoethanolamine and an unassigned peak at *m*/*z* 1821.2 (i.e. 25 mass units greater than native Lipid A, *m*/*z* 1796.2) (Figure 5, bottom panel; and Figure 10, middle panel). In one embodiment, said unassigned peak at *m*/*z* 1821.2 is assigned to Lipid A modified with pETN at 1' phosphate group with concomitant loss of the 4' phosphate group.

For chromosome encoded polymyxin-resistant *E. coli,* the mass spectrum further comprised peaks at *m*/*z* 1919.2 (i.e. 123 mass units greater than native Lipid A), assigned to Lipid A modified with phosphoethanolamine, and did not comprise the unassigned peak (which the present inventors have assigned to Lipid A modified with pETN at 1' phosphate group with concomitant loss of the 4' phosphate group) at *m*/*z* 1821.2.

The method of the present invention was further validated with strains of *E. coli* S1 J53, *E. coli* R4 J53 and *E. coli* R1 (Figure 10).

## EXAMPLE 2

**Detection of the presence or absence of a polymyxin-resistant *Klebsiella pneumoniae* in a test sample**

Test samples comprising intact bacterial cells of *K. pneumoniae* (e.g. bacterial suspensions, washed to remove salt), as follows, were subjected to mass spectrometry according to a method of the present invention: (i) chromosome encoded polymyxin-resistant *K. pneumoniae* R46; and (ii) plasmid encoded polymyxin-resistant *K. pneumoniae* R42 (comprising a plasmid comprising the *mcr-1* gene).

For polymyxin susceptible *K. pneumoniae,* the mass spectrum was dominated by a peak at *m*/*z* 1840.2 and *m*/*z* 1796.2 (Figure 7, top panel; and Figure 11, top panel) assigned to native Lipid A. The absence of a peak indicating the presence of Lipid A modified with phosphoethanolamine at *m*/*z* 1919.2 (i.e. 123 mass units greater than native Lipid A) successfully indicated the absence of a bacterium resistant to a polymyxin antibiotic.

In polymyxin-resistant *K. pneumoniae,* the mass spectrum comprised a peak at *m*/*z* 1919.2 (i.e. 123 mass units greater than native Lipid A, *m*/*z* 1796.2), assigned to Lipid A modified by phosphoethanolamine (Figure 7, bottom panel; and Figure 11, middle panel). The presence of a peak indicating the presence of Lipid A modified by phosphoethanolamine successfully indicated the presence of a bacterium resistant to a polymyxin antibiotic.

Similarly to *E. coli,* an unassigned peak (which the present inventors have assigned to Lipid A modified with pETN at 1' phosphate group with concomitant loss of the 4' phosphate group) at *m*/*z* 1821.2 (i.e. 25 mass units greater than native Lipid A, *m*/*z* 1796) was also indicative of the presence of a *K. pneumoniae* bacterium (e.g. *Enterobacteriaceae*) resistant to a polymyxin antibiotic through plasmid encoded polymyxin-resistance (Figure 11, middle panel). Said *m*/*z* 1821.2 was not present for a *K. pneumoniae* bacterium (e.g. *Enterobacteriaceae*) resistant to a polymyxin antibiotic through chromosome encoded polymyxin-resistance (Figure 11, bottom panel; and Figure 15), highlighting the applicability of this peak in discriminating between the two resistance mechanisms (plasmid - and chromosome encoded resistance).

## EXAMPLE 3

**Detection of the presence or absence of a polymyxin-resistant *Acinetobacter baumanni* in a test sample**

Test samples comprising intact bacterial cells of A. *baumanni* (e.g. bacterial suspensions, washed to remove salt), as follows, were subjected to mass spectrometry according to a method of the present invention: (i) Polymyxin susceptible *A. baumanni;* (ii) polymyxin-resistant *A. baumanni.*

In polymyxin susceptible *A. baumanni,* the mass spectrum was dominated by a peak at *m*/*z* 1910.3 (Figure 9, top panel) assigned to native Lipid A. The absence of a peak indicating the presence of phosphoethanolamine successfully

indicated the absence of a bacterium resistant to a polymyxin antibiotic.

In polymyxin-resistant *A. baumanni,* the mass spectrum further comprised a peak at *m/z* 2033.3 (i.e. 123 mass units greater than native Lipid A), assigned to Lipid A modified by phosphoethanolamine (Figure 9, bottom panel).

## EXAMPLE 4

### Peak intensity ratios allow the detection of the presence or absence of a polymyxin-resistant bacterium in a test sample

Calculation of the ratio of the intensity of Lipid A modified by phosphoethanolamine to the intensity of the peak corresponding to native Lipid A, allows the detection of the presence or absence of a polymyxin-resistant bacterium in a test sample.

This ratio was shown to typically be between 0.10 to 1.7 in a polymyxin resistant bacterium, and close to 0 (typically less than 0.05) in a polymyxin-susceptible bacterium (Figure 13).

## EXAMPLE 5

### Further detection of the presence or absence of a polymyxin-resistant *Escherichia coli* in a test sample

In polymyxin-susceptible *E. coli* strains the negative mass spectrum scanned between m/z 1600 and 2200 was dominated by a set of peaks assigned to bis-phosphorylated hexa-acyl lipid A (Figure 4). The major peak at m/z 1796.2 is known to correspond to the hexa-acyl diphosphoryl lipid A containing four C14:0 3-OH, one C14:0 and one C12:0, and referred to as native lipid A (Figure 2A (21)).

In all polymyxin-resistant E. coli strains, an additional peak at m/z 1919.2 was observed (Figure 4B and 4C) independent of the resistance mechanism involved (chromosome- or plasmid-encoded). This peak corresponds to the addition of one pETN moiety onto the phosphate group at position 4 of native lipid A (Figure 2B (23)), leading to an increase of 123 mass units compared to the mass assigned to native lipid A (Figure 4B and C).

In the case of plasmid-encoded resistance (mcr-like genes in Enterobacteriaceae), a third peak at m/z 1821·2 was systematically observed in addition to the peaks corresponding to the native lipid A (m/z 1796·2) and the pETN-modified lipid A (m/z 1919·2) (Figure 4C). This m/z 1821·2 peak was assigned to the addition of a pETN moiety onto the phosphate group at position 1' of native lipid A with concomitant loss of the phosphate group on position 4 (Figure 2A (22)), and is a specific marker of MCR-like enzymes.

To further support this observation, 73 E. coli isolates were analysed including 46 polymyxin susceptible strains with various antimicrobial resistance phenotypes. Of these strains four strains possess chromosome-encoded resistance to polymyxin and 23 are MCR-producers (Figure 18). The intensity of the peaks corresponding to the native lipid A (m/z 1796·2), the pETN-modified lipid A (m/z 1919·2) and the specific marker of MCR resistance (m/z 1821.2) were recorded from three independent experiments. The ratio (termed Polymyxin Resistance Ratio (PRR) from here after) of the sum of the intensities of the peaks associated with modified lipid A (for E. coli peaks at m/z 1919·2 and m/z 1821·2) over the intensity of the peak of native lipid A (for E. coli m/z 1796·2) allows accurate distinction between polymyxin-susceptible and polymyxin-resistant isolates, but also discrimination between chromosome-encoded and MCR-related (i.e. plasmid-encoded) resistance to polymyxin (Figure 6). The PRR value for all susceptible *E. coli* strains was found to be 0 (Figure 18 and Figure 6). The ratio ranged from 0.109 to 0·481 (average of 0·245) for *E. coli* strains with chromosome-encoded resistance to polymyxin, and from 0·533 to 4·844 (average of 2.340) for all MCR-producers (Figure 18 and Figure 6). The distribution of the $PRR_{E.coli}$ values of polymyxin-susceptible isolates compared to *E. coli* strains with chromosome-encoded resistance to polymyxin and MCR-producers were all significantly different (p<0·001, Welch's t-test). Analysis of ROC curves allowed definition of two cut-off values for $PRR_{E.coli}$ (0·1 and 0·5) which discriminate polymyxin-susceptible ($PRR_{E.coli}$ < 0·1) and polymyxin resistant isolates ($PRR_{E.coli}$ > 0·1) and also allows further discrimination between chromosome-encoded (0·1 < $PRR_{E.coli}$ 0·5) and plasmid-encoded resistance ($PRR_{E.coli}$> 0·5).

## EXAMPLE 6

### Detection of the presence or absence of a polymyxin-resistant *Escherichia coli* grown on clinically-relevant media

The performance of methods of the invention (e.g. the "MALDIxin test") was tested using colonies grown on media routinely used to test for antimicrobial susceptibility, *i.e.* Mueller-Hinton (MH) agar. In addition, because of the rapid rise of MCR-1-producing *Enterobacteriaceae* since 2016, it has also been suggested to screen infected patients using polymyxin-supplemented media. Accordingly, methods of the invention were conducted on three *E. coli* strains (a

wild-type strain, a strain with chromosome-encoded resistance and an MCR-1 producing strain), grown on LB agar, MH agar and polymyxin B-supplemented MH agar at final concentrations of either 1 mg/L or 2 mg/L. As shown in Figure 14, similar spectra were obtained independently of the growth medium, with the $PRR_{E.coli}$ being between 2·89 and 5·69 for MCR-1-producing strains and between 0·21 and 0·37 for strains harbouring chromosome-encoded resistance. To identify any impact of the age of the colonies grown on LB and MH agar, a method of the invention (e.g. the MALDIxin test) was performed on fresh colonies (overnight incubation), colonies aged by 48 hours, one week and two weeks (agar plates were kept at 4°C). Similar spectra were obtained for all tested colonies leading to no significant differences in their $PRR_{E.coli}$ (Figure 19).

## EXAMPLE 7

Validation of the method on a prospective collection of carbapenemase producing ***E. coli* from the French NRC**

In order to validate the methods of the invention (e.g. the MALDIxin test), all carbapenemase-producing *E. coli* received from the French NRC during October and November 2016 were blindly analysed in triplicate (three colonies selected from the same MH agar plate). The collection of 78 carbapenemase producing *E. coli* strains that were obtained included two KPC-producers, eleven NDM producers, two VIM-producers, 63 OXA-48-like producers, and one isolate producing two carbapenemases (Table 2). Among these isolates, two NDM-1 and one OXA-48 producer were also found to be resistant to colistin with MICs of 4 mg/L. PCR analysis revealed that these three isolates were positive for the *mcr-1* gene (Table 2). When analysed using the methods of the invention (e.g. the MALDIxin test), all polymyxin-susceptible isolates (n=75) showed a $PRR_{E.coli}$ of 0, corresponding to polymyxin susceptibility, while for the three MCR-1-producing isolates a $PRR_{E.coli}$ above 0·5 was obtained (more specifically 1·73 ± 0·23 and 2·34 ± 0·12 for the two NDM-1-producers and 1·29 ± 0·63 for the OXA-48-producer), accurately classifying them as resistant to polymyxin by a plasmid-encoded mechanism (Table 2). Therefore, in this study, the methods of the invention (e.g. the MALDIxin test) reliably and rapidly identified all MCR-producing strains where conventional methods would have needed 24 to 48 hours (broth dilution method (around 16 to 24 hours) and subsequent DNA extraction and PCR for mcr-1 and mcr-2 on resistant isolates (around 4 hours)).

| Carbapenemase | N | Polymyxin resistance | | MALDI-TOF results | |
|---|---|---|---|---|---|
| | | Colistin MIC (mg/L) | *mcr-1/-2* PCR | $PRR_{E. coli}$ | Interpretation |
| KPC-2 | 1 | 0·25 | - | 0 | Susceptible |
| KPC-3[*] | 1 | 0·25 | - | 0 | Susceptible |
| NDM-1 | 3 | 0·25 | - | 0 | Susceptible |
| NDM-1 | 1 | 0·5 | - | 0 | Susceptible |
| **NDM-1** | **1** | **4** | + (*mcr-1*) | **1·73 ± 0·23** | **Plasmid-encoded resistance** |
| **NDM-1** | **1** | **4** | + (*mcr-1*) | **2·34 ± 0·12** | **Plasmid-encoded resistance** |
| NDM-5 | 2 | 0·25 | - | 0 | Susceptible |
| NDM-5 | 3 | 0·5 | - | 0 | Susceptible |
| VIM-1 | 1 | 0·5 | - | 0 | Susceptible |
| VIM-4 | 1 | 0·25 | - | 0 | Susceptible |
| OXA-48 | 27 | 0·25 | - | 0 | Susceptible |
| OXA-48 | 26 | 0·5 | - | 0 | Susceptible |
| OXA-48 | 1 | 1 | - | 0 | Susceptible |
| **OXA-48**[§] | **1** | **4** | + (*mcr-1*) | **1·29 ± 0·63** | **Plasmid-encoded resistance** |
| OXA-181 | 4 | 0·25 | - | 0 | Susceptible |
| OXA-204 | 1 | 0·25 | - | 0 | Susceptible |
| OXA-244 | 1 | 0·25 | - | 0 | Susceptible |
| OXA-244 | 1 | 0·5 | - | 0 | Susceptible |
| OXA-181 + NDM-5 | 1 | 0·25 | - | 0 | Susceptible |

Table 2. Detection and characterization of polymyxin resistance using conventional techniques (MICs and PCR) and a method of the present invention (e.g. the MALDIxin test) on 78 carbapenemase-producing *E. coli* strains received by the French NRC during October and November 2016. N, number of isolates; MIC, minimal inhibition concentration; PPR, Polymyxin Resistance Ratio. +, positive PCR; - negative PCR for the gene indicated in parenthesis. *These two strains were isolated from the same patient.

## EXAMPLE 8

### Analysis of ***mcr* family genes**

Chromosome-encoded genes associated with polymyxin resistance are numerous, and the gene modifications (disruptions, deletions mutations) involved are not systematically described nor characterized. As for plasmid-encoded resistance, five families of *mcr* genes are known in *Enterobacteriaceae*.

Sequence homology of MCR-2, MCR-3, MCR-4 and MCR-5 with MCR-1 was analysed. MCR-2, MCR-3, MCR-4 and MCR-5 share only 81%, 34%, 33% and 31% amino acid identity with MCR-1, respectively (Figure 17). This diversity would inevitably lead to failure in systematic detection of polymyxin resistance, if it relies on using available molecular biology tools dedicated to *mcr-1* and/or *mcr-2* detection (Figure 16).

These issues are overcome by methods of the present invention (e.g. the MALDIxin test), which provides a cost-effective tool (method) based on MALDI-TOF technology that aims to detect polymyxin resistance using a single Gram-negative bacterial colony, in less than 15 minutes.

## SEQUENCES

SEQ ID NO: 1 (*mcr-1*)

ATGATGCAGCATACTTCTGTGTGGTACCGACGCTCGGTCAGTCCGTTTGTTCTTGTGGC GAGTGTTGCCGTTTTCTTGACCGCGACCGCCAATCTTACCTTTTTTGATAAAATCAGCCA AACCTATCCCATCGCGGACAATCTCGGCTTTGTGCTGACGATCGCTGTCGTGCTCTTTG GCGCGATGCTACTGATCACCACGCTGTTATCATCGTATCGCTATGTGCTAAAGCCTGTG TTGATTTTGCTATTAATCATGGGCGCGGTGACCAGTTATTTTACTGACACTTATGGCACG GTCTATGATACGACCATGCTCCAAAATGCCCTACAGACCGACCAAGCCGAGACCAAGG ATCTATTAAACGCAGCGTTTATCATGCGTATCATTGGTTTGGGTGTGCTACCAAGTTTGC TTGTGGCTTTTGTTAAGGTGGATTATCCGACTTGGGGCAAGGGTTTGATGCGCCGATTG GGCTTGATCGTGGCAAGTCTTGCGCTGATTTTACTGCCTGTGGTGGCGTTCAGCAGTCA TTATGCCAGTTTCTTTCGCGTGCATAAGCCGCTGCGTAGCTATGTCAATCCGATCATGC CAATCTACTCGGTGGGTAAGCTTGCCAGTATTGAGTATAAAAAAGCCAGTGCGCCAAAA GATACCATTTATCACGCCAAAGACGCGGTACAAGCAACCAAGCCTGATATGCGTAAGCC ACGCCTAGTGGTGTTCGTCGTCGGTGAGACGGCACGCGCCGATCATGTCAGCTTCAAT GGCTATGAGCGCGATACTTTCCCACAGCTTGCCAAGATCGATGGCGTGACCAATTTTAG CAATGTCACATCGTGCGGCACATCGACGGCGTATTCTGTGCCGTGTATGTTCAGCTATC TGGGCGCGGATGAGTATGATGTCGATACCGCCAAATACCAAGAAAATGTGCTGGATAC GCTGGATCGCTTGGGCGTAAGTATCTTGTGGCGTGATAATAATTCGGACTCAAAAGGCG TGATGGATAAGCTGCCAAAAGCGCAATTTGCCGATTATAAATCCGCGACCAACAACGCC ATCTGCAACACCAATCCTTATAACGAATGCCGCGATGTCGGTATGCTCGTTGGCTTAGA TGACTTTGTCGCTGCCAATAACGGCAAAGATATGCTGATCATGCTGCACCAAATGGGCA ATCACGGGCCTGCGTATTTTAAGCGATATGATGAAAAGTTTGCCAAATTCACGCCAGTG TGTGAAGGTAATGAGCTTGCCAAGTGCGAACATCAGTCCTTGATCAATGCTTATGACAAT GCCTTGCTTGCCACCGATGATTTCATCGCTCAAAGTATCCAGTGGCTGCAGACGCACAG CAATGCCTATGATGTCTCAATGCTGTATGTCAGCGATCATGGCGAAAGTCTGGGTGAGA ACGGTGTCTATCTACATGGTATGCCAAATGCCTTTGCACCAAAAGAACAGCGCAGTGTG CCTGCATTTTTCTGGACGGATAAGCAAACTGGCATCACGCCAATGGCAACCGATACCGT CCTGACCCATGACGCGATCACGCCGACATTATTAAAGCTGTTTGATGTCACCGCGGACA AAGTCAAAGACCGCACCGCATTCATCCGCTGA

SEQ ID NO: 2 (*mcr-1.2*)

ATGATGCTGCATACTTCTGTGTGGTACCGACGCTCGGTCAGTCCGTTTGTTCTTGTGGC GAGTGTTGCCGTTTTCTTGACCGCGACCGCCAATCTTACCTTTTTTGATAAAATCAGCCA

AACCTATCCCATCGCGGACAATCTCGGCTTTGTGCTGACGATCGCTGTCGTGCTCTTTG
GCGCGATGCTACTGATCACCACGCTGTTATCATCGTATCGCTATGTGCTAAAGCCTGTG
TTGATTTTGCTATTAATCATGGGCGCGGTGACCAGTTATTTTACTGACACTTATGGCACG
GTCTATGATACGACCATGCTCCAAAATGCCCTACAGACCGACCAAGCCGAGACCAAGG
ATCTATTAAACGCAGCGTTTATCATGCGTATCATTGGTTTGGGTGTGCTACCAAGTTTGC
TTGTGGCTTTTGTTAAGGTGGATTATCCGACTTGGGGCAAGGGTTTGATGCGCCGATTG
GGCTTGATCGTGGCAAGTCTTGCGCTGATTTTACTGCCTGTGGTGGCGTTCAGCAGTCA
TTATGCCAGTTTCTTTCGCGTGCATAAGCCGCTGCGTAGCTATGTCAATCCGATCATGC
CAATCTACTCGGTGGGTAAGCTTGCCAGTATTGAGTATAAAAAAGCCAGTGCGCCAAAA
GATACCATTTATCACGCCAAAGACGCGGTACAAGCAACCAAGCCTGATATGCGTAAGCC
ACGCCTAGTGGTGTTCGTCGTCGGTGAGACGGCACGCGCCGATCATGTCAGCTTCAAT
GGCTATGAGCGCGATACTTTCCCACAGCTTGCCAAGATCGATGGCGTGACCAATTTTAG
CAATGTCACATCGTGCGGCACATCGACGGCGTATTCTGTGCCGTGTATGTTCAGCTATC
TGGGCGCGGATGAGTATGATGTCGATACCGCCAAATACCAAGAAAATGTGCTGGATAC
GCTGGATCGCTTGGGCGTAAGTATCTTGTGGCGTGATAATAATTCGGACTCAAAAGGCG
TGATGGATAAGCTGCCAAAAGCGCAATTTGCCGATTATAAATCCGCGACCAACAACGCC
ATCTGCAACACCAATCCTTATAACGAATGCCGCGATGTCGGTATGCTCGTTGGCTTAGA
TGACTTTGTCGCTGCCAATAACGGCAAAGATATGCTGATCATGCTGCACCAAATGGGCA
ATCACGGGCCTGCGTATTTTAAGCGATATGATGAAAAGTTTGCCAAATTCACGCCAGTG
TGTGAAGGTAATGAGCTTGCCAAGTGCGAACATCAGTCCTTGATCAATGCTTATGACAAT
GCCTTGCTTGCCACCGATGATTTCATCGCTCAAAGTATCCAGTGGCTGCAGACGCACAG
CAATGCCTATGATGTCTCAATGCTGTATGTCAGCGATCATGGCGAAAGTCTGGGTGAGA
ACGGTGTCTATCTACATGGTATGCCAAATGCCTTTGCACCAAAAGAACAGCGCAGTGTG
CCTGCATTTTTCTGGACGGATAAGCAAACTGGCATCACGCCAATGGCAACCGATACCGT
CCTGACCCATGACGCGATCACGCCGACATTATTAAAGCTGTTTGATGTCACCGCGGACA
AAGTCAAAGACCGCACCGCATTCATCCGCTGA

SEQ ID NO: 3 *(mcr-1.3)*

ATGATGCAGCATACTTCTGTGTGGTACCGACGCTCGGTCAGTCCGTTTGTTCTTGTGGC
GAGTGTTGCCGTTTTCTTGACCGCGACCGCCAATCTTACCTTTTTTGATAAGGTCAGCCA
AACCTATCCCATCGCGGACAATCTCGGCTTTGTGCTGACGATCGCTGTCGTGCTCTTTG
GCGCGATGCTACTGATCACCACGCTGTTATCATCGTATCGCTATGTGCTAAAGCCTGTG
TTGATTTTGCTATTAATCATGGGCGCGGTGACCAGTTATTTTACTGACACTTATGGCACG
GTCTATGATACGACCATGCTCCAAAATGCCCTACAGACCGACCAAGCCGAGACCAAGG
ATCTATTAAACGCAGCGTTTATCATGCGTATCATTGGTTTGGGTGTGCTACCAAGTTTGC
TTGTGGCTTTTGTTAAGGTGGATTATCCGACTTGGGGCAAGGGTTTGATGCGCCGATTG

GGCTTGATCGTGGCAAGTCTTGCGCTGATTTTACTGCCTGTGGTGGCGTTCAGCAGTCA TTATGCCAGTTTCTTTCGCGTGCATAAGCCGCTGCGTAGCTATGTCAATCCGATCATGC CAATCTACTCGGTGGGTAAGCTTGCCAGTATTGAGTATAAAAAAGCCAGTGCGCCAAAA GATACCATTTATCACGCCAAAGACGCGGTACAAGCAACCAAGCCTGATATGCGTAAGCC ACGCCTAGTGGTGTTCGTCGTCGGTGAGACGGCACGCGCCGATCATGTCAGCTTCAAT GGCTATGAGCGCGATACTTTCCCACAGCTTGCCAAGATCGATGGCGTGACCAATTTTAG CAATGTCACATCGTGCGGCACATCGACGGCGTATTCTGTGCCGTGTATGTTCAGCTATC TGGGCGCGGATGAGTATGATGTCGATACCGCCAAATACCAAGAAAATGTGCTGGATAC GCTGGATCGCTTGGGCGTAAGTATCTTGTGGCGTGATAATAATTCGGACTCAAAAGGCG TGATGGATAAGCTGCCAAAAGCGCAATTTGCCGATTATAAATCCGCGACCAACAACGCC ATCTGCAACACCAATCCTTATAACGAATGCCGCGATGTCGGTATGCTCGTTGGCTTAGA TGACTTTGTCGCTGCCAATAACGGCAAAGATATGCTGATCATGCTGCACCAAATGGGCA ATCACGGGCCTGCGTATTTTAAGCGATATGATGAAAAGTTTGCCAAATTCACGCCAGTG TGTGAAGGTAATGAGCTTGCCAAGTGCGAACATCAGTCCTTGATCAATGCTTATGACAAT GCCTTGCTTGCCACCGATGATTTCATCGCTCAAAGTATCCAGTGGCTGCAGACGCACAG CAATGCCTATGATGTCTCAATGCTGTATGTCAGCGATCATGGCGAAAGTCTGGGTGAGA ACGGTGTCTATCTACATGGTATGCCAAATGCCTTTGCACCAAAAGAACAGCGCAGTGTG CCTGCATTTTTCTGGACGGATAAGCAAACTGGCATCACGCCAATGGCAACCGATACCGT CCTGACCCATGACGCGATCACGCCGACATTATTAAAGCTGTTTGATGTCACCGCGGACA AAGTCAAAGACCGCACCGCATTCATCCGCTGA

SEQ ID NO: 4 (*mcr-1.4*)

ATGATGCAGCATACTTCTGTGTGGTACCGACGCTCGGTCAGTCCGTTTGTTCTTGTGGC GAGTGTTGCCGTTTTCTTGACCGCGACCGCCAATCTTACCTTTTTTGATAAAATCAGCCA AACCTATCCCATCGCGGACAATCTCGGCTTTGTGCTGACGATCGCTGTCGTGCTCTTTG GCGCGATGCTACTGATCACCACGCTGTTATCATCGTATCGCTATGTGCTAAAGCCTGTG TTGATTTTGCTATTAATCATGGGCGCGGTGACCAGTTATTTTACTGACACTTATGGCACG GTCTATGATACGACCATGCTCCAAAATGCCCTACAGACCGACCAAGCCGAGACCAAGG ATCTATTAAACGCAGCGTTTATCATGCGTATCATTGGTTTGGGTGTGCTACCAAGTTTGC TTGTGGCTTTTGTTAAGGTGGATTATCCGACTTGGGGCAAGGGTTTGATGCGCCGATTG GGCTTGATCGTGGCAAGTCTTGCGCTGATTTTACTGCCTGTGGTGGCGTTCAGCAGTCA TTATGCCAGTTTCTTTCGCGTGCATAAGCCGCTGCGTAGCTATGTCAATCCGATCATGC CAATCTACTCGGTGGGTAAGCTTGCCAGTATTGAGTATAAAAAAGCCAGTGCGCCAAAA GATACCATTTATCACGCCAAAGACGCGGTACAAGCAACCAAGCCTGATATGCGTAAGCC ACGCCTAGTGGTGTTCGTCGTCGGTGAGACGGCACGCGCCGATCATGTCAGCTTCAAT GGCTATGAGCGCGATACTTTCCCACAGCTTGCCAAGATCGATGGCGTGACCAATTTTAG

CAATGTCACATCGTGCGGCACATCGACGGCGTATTCTGTGCCGTGTATGTTCAGCTATCTGGGCGCGGATGAGTATGATGTCGATACCGCCAAATACCAAGAAAATGTGCTGGATACGCTGGATCGCTTGGGCGTAAGTATCTTGTGGCGTGATAATAATTCGGACTCAAAAGGCGTGATGGATAAGCTGCCAAAAGCGCAATTTGCCGATTATAAATCCGCGACCAACAACGCCATCTGCAACACCAATCCTTATAACGAATGCCGCGATGTCGGTATGCTCGTTGGCTTAGATGACTTTGTCGCTGCCAATAACGGCAAAGATATGCTGATCATGCTGCACCAAATGGGCAATCACGGGCCTGCGTATTTTAAGCGATATGATGAAAAGTTTGCCAAATTCACGCCAGTGTGTGAAGGTAATGAGCTTGCCAAGTGCGAACATCAGTCCTTGATCAATGCTTATGACAATGCCTTGCTTGCCACCGATAATTTCATCGCTCAAAGTATCCAGTGGCTGCAGACGCACAGCAATGCCTATGATGTCTCAATGCTGTATGTCAGCGATCATGGCGAAAGTCTGGGTGAGAACGGTGTCTATCTACATGGTATGCCAAATGCCTTTGCACCAAAAGAACAGCGCAGTGTGCCTGCATTTTTCTGGACGGATAAGCAAACTGGCATCACGCCAATGGCAACCGATACCGTCCTGACCCATGACGCGATCACGCCGACATTATTAAAGCTGTTTGATGTCACCGCGGACAAAGTCAAAGACCGCACCGCATTCATCCGCTGA

SEQ ID NO: 5 (*mcr-1.5*)

ATGATGCAGCATACTTCTGTGTGGTACCGACGCTCGGTCAGTCCGTTTGTTCTTGTGGCGAGTGTTGCCGTTTTCTTGACCGCGACCGCCAATCTTACCTTTTTTGATAAAATCAGCCAAACCTATCCCATCGCGGACAATCTCGGCTTTGTGCTGACGATCGCTGTCGTGCTCTTTGGCGCGATGCTACTGATCACCACGCTGTTATCATCGTATCGCTATGTGCTAAAGCCTGTGTTGATTTTGCTATTAATCATGGGCGCGGTGACCAGTTATTTTACTGACACTTATGGCACGGTCTATGATACGACCATGCTCCAAAATGCCCTACAGACCGACCAAGCCGAGACCAAGGATCTATTAAACGCAGCGTTTATCATGCGTATCATTGGTTTGGGTGTGCTACCAAGTTTGCTTGTGGCTTTTGTTAAGGTGGATTATCCGACTTGGGGCAAGGGTTTGATGCGCCGATTGGGCTTGATCGTGGCAAGTCTTGCGCTGATTTTACTGCCTGTGGTGGCGTTCAGCAGTCATTATGCCAGTTTCTTTCGCGTGCATAAGCCGCTGCGTAGCTATGTCAATCCGATCATGCCAATCTACTCGGTGGGTAAGCTTGCCAGTATTGAGTATAAAAAAGCCAGTGCGCCAAAAGATACCATTTATCACGCCAAAGACGCGGTACAAGCAACCAAGCCTGATATGCGTAAGCCACGCCTAGTGGTGTTCGTCGTCGGTGAGACGGCACGCGCCGATCATGTCAGCTTCAATGGCTATGAGCGCGATACTTTCCCACAGCTTGCCAAGATCGATGGCGTGACCAATTTTAGCAATGTCACATCGTGCGGCACATCGACGGCGTATTCTGTGCCGTGTATGTTCAGCTATCTGGGCGCGGATGAGTATGATGTCGATACCGCCAAATACCAAGAAAATGTGCTGGATACGCTGGATCGCTTGGGCGTAAGTATCTTGTGGCGTGATAATAATTCGGACTCAAAAGGCGTGATGGATAAGCTGCCAAAAGCGCAATTTGCCGATTATAAATCCGCGACCAACAACGCCATCTGCAACACCAATCCTTATAACGAATGCCGCGATGTCGGTATGCTCGTTGGCTTAGATGACTTTGTCGCTGCCAATAACGGCAAAGATATGCTGATCATGCTGCACCAAATGGGCA

ATCACGGGCCTGCGTATTTTAAGCGATATGATGAAAAGTTTGCCAAATTCACGCCAGTG
TGTGAAGGTAATGAGCTTGCCAAGTGCGAACATCAGTCCTTGATCAATGCTTATGACAAT
GCCTTGCTTGCCACCGATGATTTCATCGCTCAAAGTATCCAGTGGCTGCAGACGTACAG
CAATGCCTATGATGTCTCAATGCTGTATGTCAGCGATCATGGCGAAAGTCTGGGTGAGA
ACGGTGTCTATCTACATGGTATGCCAAATGCCTTTGCACCAAAAGAACAGCGCAGTGTG
CCTGCATTTTTCTGGACGGATAAGCAAACTGGCATCACGCCAATGGCAACCGATACCGT
CCTGACCCATGACGCGATCACGCCGACATTATTAAAGCTGTTTGATGTCACCGCGGACA
AAGTCAAAGACCGCACCGCATTCATCCGCTGA

SEQ ID NO: 6 (*mcr-1.6*)

ATGATGCAGCATACTTCTGTGTGGTACCGACGCTCGGTCAGTCCGTTTGTTCTTGTGGC
GAGTGTTGCCGTTTTCTTGACCGCGACCGCCAATCTTACCTTTTTTGATAAAATCAGCCA
AACCTATCCCATCGCGGACAATCTCGGCTTTGTGCTGACGATCGCTGTCGTGCTCTTTG
GCGCGATGCTACTGATCACCACGCTGTTATCATCGTATCGCTATGTGCTAAAGCCTGTG
TTGATTTTGCTATTAATCATGGGCGCGGTGACCAGTTATTTTACTGACACTTATGGCACG
GTCTATGATACGACCATGCTCCAAAATGCCCTACAGACCGACCAAGCCGAGACCAAGG
ATCTATTAAACGCAGCGTTTATCATGCGTATCATTGGTTTGGGTGTGCTACCAAGTTTGC
TTGTGGCTTTTGTTAAGGTGGATTATCCGACTTGGGGCAAGGGTTTGATGCGCCGATTG
GGCTTGATCGTGGCAAGTCTTGCGCTGATTTTACTGCCTGTGGTGGCGTTCAGCAGTCA
TTATGCCAGTTTCTTTCGCGTGCATAAGCCGCTGCGTAGCTATGTCAATCCGATCATGC
CAATCTACTCGGTGGGTAAGCTTGCCAGTATTGAGTATAAAAAAGCCAGTGCGCCAAAA
GATACCATTTATCACGCCAAAGACGCGGTACAAGCAACCAAGCCTGATATGCGTAAGCC
ACGCCTAGTGGTGTTCGTCGTCGGTGAGACGGCACGCGCCGATCATGTCAGCTTCAAT
GGCTATGAGCGCGATACTTTCCCACAGCTTGCCAAGATCGATGGCGTGACCAATTTTAG
CAATGTCACATCGTGCGGCACATCGACGGCGTATTCTGTGCCGTGTATGTTCAGCTATC
TGGGCGCGGATGAGTATGATGTCGATACCGCCAAATACCAAGAAAATGTGCTGGATAC
GCTGGATCGCTTGGGCGTAAGTATCTTGTGGCGTGATAATAATTCGGACTCAAAAGGCG
TGATGGATAAGCTGCCAAAAGCGCAATTTGCCGATTATAAATCCGCGACCAACAACGCC
ATCTGCAACACCAATCCTTATAACGAATGCCGCGATGTCGGTATGCTCGTTGGCTTAGA
TGACTTTGTCGCTGCCAATAACGGCAAAGATATGCTGATCATGCTGCACCAAATGGGCA
ATCACGGGCCTGCGTATTTTAAGCGATATGATGAAAAGTTTGCCAAATTCACGCCAGTG
TGTGAAGGTAATGAGCTTGCCAAATGCGAACATCAGTCCTTGATCAATGCTTATGACAAT
GCCTTGCTTGCCACCGATGATTTCATCGCTCAAAGTATCCAGTGGCTGCAGACGCACAG
CAATGCCTATGATGTCTCAATGCTGTATGTCAGCGATCATGGCGAAAGTCTGGGTGAGA
ACGGTGTCTATCTACATGGTATGCCAAATGCCTTTGCACCAAAAGAACAGCGCAGTGTG
CCTGCATTTTTCTGGACGGATAAGCAAACTGGCATCACGCCAATGGCAACCGATACCGT

CCTGACCCATGACGCGATCACGCCGACATTATTAAAGCTGTTTGATGTCACCGCGGACA
AAGTCAAAGACCACACCGCATTCATCCGCTGA

SEQ ID NO: 7 (*mcr-1.7*)

ATGATGCAGCATACTTCTGTGTGGTACCGACGCTCGGTCAGTCCGTTTGTTCTTGTGGC
GAGTGTTGCCGTTTTCTTGACCGCGACCGCCAATCTTACCTTTTTTGATAAAATCAGCCA
AACCTATCCCATCGCGGACAATCTCGGCTTTGTGCTGACGATCGCTGTCGTGCTCTTTG
GCGCGATGCTACTGATCACCACGCTGTTATCATCGTATCGCTATGTGCTAAAGCCTGTG
TTGATTTTGCTATTAATCATGGGCGCGGTGACCAGTTATTTTACTGACACTTATGGCACG
GTCTATGATACGACCATGCTCCAAAATGCCCTACAGACCGACCAAGCCGAGACCAAGG
ATCTATTAAACGCAGCGTTTATCATGCGTATCATTGGTTTGGGTGTGCTACCAAGTTTGC
TTGTGGCTTTTGTTAAGGTGGATTATCCGACTTGGGGCAAGGGTTTGATGCGCCGATTG
GGCTTGATCGTGGCAAGTCTTGCGCTGATTTTACTGCCTGTGGTGGCGTTCAGCAGTCA
TTATGCCAGTTTCTTTCGCGTGCATAAGCCGCTGCGTAGCTATGTCAATCCGATCATGC
CAATCTACTCGGTGGGTAAGCTTGCCAGTATTGAGTATAAAAAAGCCAGTACGCCAAAA
GATACCATTTATCACGCCAAAGACGCGGTACAAGCAACCAAGCCTGATATGCGTAAGCC
ACGCCTAGTGGTGTTCGTCGTCGGTGAGACGGCACGCGCCGATCATGTCAGCTTCAAT
GGCTATGAGCGCGATACTTTCCCACAGCTTGCCAAGATCGATGGCGTGACCAATTTTAG
CAATGTCACATCGTGCGGCACATCGACGGCGTATTCTGTGCCGTGTATGTTCAGCTATC
TGGGCGCGGATGAGTATGATGTCGATACCGCCAAATACCAAGAAAATGTGCTGGATAC
GCTGGATCGCTTGGGCGTAAGTATCTTGTGGCGTGATAATAATTCGGACTCAAAAGGCG
TGATGGATAAGCTGCCAAAAGCGCAATTTGCCGATTATAAATCCGCGACCAACAACGCC
ATCTGCAACACCAATCCTTATAACGAATGCCGCGATGTCGGTATGCTCGTTGGCTTAGA
TGACTTTGTCGCTGCCAATAACGGCAAAGATATGCTGATCATGCTGCACCAAATGGGCA
ATCACGGGCCTGCGTATTTTAAGCGATATGATGAAAAGTTTGCCAAATTCACGCCAGTG
TGTGAAGGTAATGAGCTTGCCAAGTGCGAACATCAGTCCTTGATCAATGCTTATGACAAT
GCCTTGCTTGCCACCGATGATTTCATCGCTCAAAGTATCCAGTGGCTGCAGACGCACAG
CAATGCCTATGATGTCTCAATGCTGTATGTCAGCGATCATGGCGAAAGTCTGGGTGAGA
ACGGTGTCTATCTACATGGTATGCCAAATGCCTTTGCACCAAAAGAACAGCGCAGTGTG
CCTGCATTTTTCTGGACGGATAAGCAAACTGGCATCACGCCAATGGCAACCGATACCGT
CCTGACCCATGACGCGATCACGCCGACATTATTAAAGCTGTTTGATGTCACCGCGGACA
AAGTCAAAGACCGCACCGCATTCATCCGCTGA

SEQ ID NO: 8 (*mcr-1.8*)

ATGATGCGGCATACTTCTGTGTGGTACCGACGCTCGGTCAGTCCGTTTGTTCTTGTGGC
GAGTGTTGCCGTTTTCTTGACCGCGACCGCCAATCTTACCTTTTTTGATAAAATCAGCCA
AACCTATCCCATCGCGGACAATCTCGGCTTTGTGCTGACGATCGCTGTCGTGCTCTTTG
GCGCGATGCTACTGATCACCACGCTGTTATCATCGTATCGCTATGTGCTAAAGCCTGTG
TTGATTTTGCTATTAATCATGGGCGCGGTGACCAGTTATTTTACTGACACTTATGGCACG
GTCTATGATACGACCATGCTCCAAAATGCCCTACAGACCGACCAAGCCGAGACCAAGG
ATCTATTAAACGCAGCGTTTATCATGCGTATCATTGGTTTGGGTGTGCTACCAAGTTTGC
TTGTGGCTTTTGTTAAGGTGGATTATCCGACTTGGGGCAAGGGTTTGATGCGCCGATTG
GGCTTGATCGTGGCAAGTCTTGCGCTGATTTTACTGCCTGTGGTGGCGTTCAGCAGTCA
TTATGCCAGTTTCTTTCGCGTGCATAAGCCGCTGCGTAGCTATGTCAATCCGATCATGC
CAATCTACTCGGTGGGTAAGCTTGCCAGTATTGAGTATAAAAAAGCCAGTGCGCCAAAA
GATACCATTTATCACGCCAAAGACGCGGTACAAGCAACCAAGCCTGATATGCGTAAGCC
ACGCCTAGTGGTGTTCGTCGTCGGTGAGACGGCACGCGCCGATCATGTCAGCTTCAAT
GGCTATGAGCGCGATACTTTCCCACAGCTTGCCAAGATCGATGGCGTGACCAATTTTAG
CAATGTCACATCGTGCGGCACATCGACGGCGTATTCTGTGCCGTGTATGTTCAGCTATC
TGGGCGCGGATGAGTATGATGTCGATACCGCCAAATACCAAGAAAATGTGCTGGATAC
GCTGGATCGCTTGGGCGTAAGTATCTTGTGGCGTGATAATAATTCGGACTCAAAAGGCG
TGATGGATAAGCTGCCAAAAGCGCAATTTGCCGATTATAAATCCGCGACCAACAACGCC
ATCTGCAACACCAATCCTTATAACGAATGCCGCGATGTCGGTATGCTCGTTGGCTTAGA
TGACTTTGTCGCTGCCAATAACGGCAAAGATATGCTGATCATGCTGCACCAAATGGGCA
ATCACGGGCCTGCGTATTTTAAGCGATATGATGAAAAGTTTGCCAAATTCACGCCAGTG
TGTGAAGGTAATGAGCTTGCCAAGTGCGAACATCAGTCCTTGATCAATGCTTATGACAAT
GCCTTGCTTGCCACCGATGATTTCATCGCTCAAAGTATCCAGTGGCTGCAGACGCACAG
CAATGCCTATGATGTCTCAATGCTGTATGTCAGCGATCATGGCGAAAGTCTGGGTGAGA
ACGGTGTCTATCTACATGGTATGCCAAATGCCTTTGCACCAAAAGAACAGCGCAGTGTG
CCTGCATTTTTCTGGACGGATAAGCAAACTGGCATCACGCCAATGGCAACCGATACCGT
CCTGACCCATGACGCGATCACGCCGACATTATTAAAGCTGTTTGATGTCACCGCGGACA
AAGTCAAAGACCGCACCGCATTCATCCGCTGA

SEQ ID NO: 9 (*mcr-1.9*)

GTGTGGTACCGACGCTCGGTCAGTCCGTTTGTTCTTGTGGCGAGTGTTGCCGTTTTCTT
GACCGCGACCGCCAATCTTACCTTTTTTGATAAAATCAGCCAAACCTATCCCATCGCGG
ACAATCTCGGCTTTGTGCTGACGATCGCTGTCGTGCTCTTTGGCGCGATGCTACTGATC
ACCACGCTGTTATCATCGTATCGCTATGTGCTAAAGCCTGTGTTGATTTTGCTATTAATC
ATGGGCGCGGTGACCAGTTATTTTACTGACACTTATGGCACGGTCTATGATACGACCAT

GCTCCAAAATGCCCTACAGACCGACCAAGCCGAGACCAAGGATCTATTAAACGCAGCG
TTTATCATGCGTATCATTGGTTTGGGTGTGCTACCAAGTTTGCTTGTGGCTTTTGTTAAG
GTGGATTATCCGACTTGGGGCAAGGGTTTGATGCGCCGATTGGGCTTGATCGTGGCAA
GTCTTGCGCTGATTTTACTGCCTGTGGTGGCGTTCAGCAGTCATTATGCCAGTTTCTTTC
GCGTGCATAAGCCGCTGCGTAGCTATGTCAATCCGATCATGCCAATCTACTCGGTGGGT
AAGCTTGCCAGTATTGAGTATAAAAAAGCCAGTGCGCCAAAAGATACCATTTATCACGC
CAAAGACGCGGTACAAGCAACCAAGCCTGATATGCGTAAGCCACGCCTAGTGGTGTTC
GTCGTCGGTGAGACGGCACGCGCCGATCATGTCAGCTTCAATGGCTATGAGCGCGATA
CTTTCCCACAGCTTGCCAAGATCGATGGCGTGACCAATTTTAGCAATGTCACATCGTGC
GGCACATCGACGGCGTATTCTGTGCCGTGTATGTTCAGCTATCTGGGCGCGGATGAGT
ATGATGTCGATACCGCCAAATACCAAGAAAATGTGCTGGATACGCTGGATCGCTTGGGC
GTAAGTATCTTGTGGCGTGATAATAATTCGGACTCAAAAGGCGTGATGGATAAGCTGCC
AAAAGCGCAATTTGCCGATTATAAATCCGCGACCAACAACGCCATCTGCAACACCAATC
CTTATAACGAATGCCGCGATGTCGGTATGCTCGTTGGCTTAGATGACTTTGTCGCTGCC
AATAACGGCAAAGATATGCTGATCATGCTGCACCAAATGGGCAATCACGGGCCTGCGTA
TTTTAAGCGATATGATGAAAAGTTTGCCAAATTCACGCCAGCGTGTGAAGGTAATGAGCT
TGCCAAGTGCGAACATCAGTCCTTGATCAATGCTTATGACAATGCCTTGCTTGCCACCG
ATGATTTCATCGCTCAAAGTATCCAGTGGCTGCAGACGCACAGCAATGCCTATGATGTC
TCAATGCTGTATGTCAGCGATCATGGCGAAAGTCTGGGTGAGAACGGTGTCTATCTACA
TGGTATGCCAAATGCCTTTGCACCAAAAGAACAGCGCAGTGTGCCTGCATTTTTCTGGA
CGGATAAGCAAACTGGCATCACGCCAATGGCAACCGATACCGTCCTGACCCATGACGC
GATCACGCCGACATTATTAAAGCTGTTTGATGTCACCGCGGACAAAGTCAAAGACCGCA
CCGCATTCATCCGCTGA

SEQ ID NO: 10 (*mcr-1.10*)

GTGTGGTACCGACGCTCGGTCAGTCCGTTTGTTCTTGTGGCGAGTGTTGCCGTTTTCTT
GACCGCGACCGCCAATCTTACCTTTTTTGATAAAATCAGCCAAACCTATCCCATCGCGG
ACAATCTCGGCTTTGTGCTGACGATCGCTGTCGTGCTCTTTGGCGCGATGCTACTGATC
ACCACGCTGTTATCATCGTATCGCTATGTGCTAAAGCCTGTGTTGATTTTGCTATTAATC
ATGGGCGCGGTGACCAGTTATTTTACTGACACTTATGGCACGGTCTATGATACGACCAT
GCTCCAAAATGCCCTACAGACCGACCAAGCCGAGACCAAGGATCTATTAAACGCAGCG
TTTATCATGCGTATCATTGGTTTGGGTGTGCTACCAAGTTTGCTTGTGGCTTTTGTTAAG
GTGGATTATCCGACTTGGGGCAAGGGTTTGATGCGCCGATTGGGCTTGATCGTGGCAA
GTCTTGCGCTGATTTTACTGCCTGTGGTGGCGTTCAGCAGTCATTATGCCAGTTTCTTTC
GCGTGCATAAGCCGCTGCGTAGCTATGTCAATCCGATCATGCCAATCTACTCGGTGGGT
AAGCTTGCCAGTATTGAGTATAAAAAAGCCAGTGCGCCAAAAGATACCATTTATCACGC

CAAAGACGCGGTACAAGCAACCAAGCCTGATATGCGTAAGCCACGCCTAGTGGTGTTC
GTCGTCGGTGAGACGGCACGCGCCGATCATGTCAGCTTCAATGGCTATGAGCGCGATA
CTTTCCCACAGCTTGCCAAGATCGATGGCGTGACCAATTTTAGCAATGTCACATCGTGC
GGCACATCGACGGCGTATTCTGTGCCGTGTATGTTCAGCTATCTGGGCGCGGATGAGT
ATGATGTCGATACCGCCAAATACCAAGAAAATGTGCTGGATACGCTGGATCGCTTGGGC
GTAAGTATCTTGTGGCGTGATAATAATTCGGACTCAAAAGGCGTGATGGATAAGCTGCC
AAAAGCGCAATTTGCCGATTATAAATCCGCGACCAACAACGCCATCTGCAACACCAATC
CTTATAACGAATGCCGCGATGTCGGTATGCTCGTTGGCTTAGATGACTTTGTCGCTGCC
AATAACGGCAAAGATATGCTGATCATGCTGCACCAAATGGGCAATCACGGGCCTGCGTA
TTTTAAGCGATATGATGAAAAGTTTGCCAAATTCACGCCAGCGTGTGAAGGTAATGAGCT
TGCCAAGTGCGAACATCAGTCCTTGATCAATGCTTATGACAATGCCTTGCTTGCCACCG
ATGATTTCATCGCTCAAAGTATCCAGTGGCTGCAGACGCACAGCAATGCCTATGATGTC
TCAATGCTGTATGTCAGCGATCATGGCGAAAGTCTGGGTGAGAACGGTGTCTATCTACA
TGGTATGCCAAATGCCTTTGCACCAAAAGAACAGCGCAGTGTGCCTGCATTTTTCTGGA
CGGATAAGCAAACTGGCATCACGCCAATGGCAACCGATACCGTCCTGACCCATGACGC
GATCACGCCGACATTATTAAAGCTGTTTGATGTCACCGCGGACAAAGTCAAAGACCGCA
CCGCATTCATCCGCTGA

SEQ ID NO: 11 (*mcr-2*)

ATGACATCACATCACTCTTGGTATCGCTATTCTATCAATCCTTTTGTGCTGATGGGTTTG
GTGGCGTTATTTTTGGCAGCGACAGCGAACCTGACATTTTTTGAAAAAGCGATGGCGGT
CTATCCTGTATCGGATAACTTAGGCTTTATCATCTCAATGGCGGTGGCGGTGATGGGTG
CTATGCTACTGATTGTCGTGCTGTTATCCTATCGCTATGTGCTAAAGCCTGTCCTGATTT
TGCTACTGATTATGGGTGCGGTGACGAGCTATTTTACCGATACTTATGGCACGGTCTAT
GACACCACCATGCTCCAAAATGCCATGCAAACCGACCAAGCCGAGTCTAAGGACTTGAT
GAATTTGGCGTTTTTTGTGCGAATTATCGGGCTTGGCGTGTTGCCAAGTGTGTTGGTCG
CAGTTGCCAAAGTCAATTATCCAACATGGGGCAAAGGTCTGATTCAGCGTGCGATGACA
TGGGGTGTCAGCCTTGTGCTGTTGCTTGTGCCGATTGGACTATTTAGCAGTCAGTATGC
GAGTTTCTTTCGGGTGCATAAGCCAGTGCGTTTTTATATCAACCCGATTACGCCGATTTA
TTCGGTGGGTAAGCTTGCCAGTATCGAGTACAAAAAAGCCACTGCGCCAACAGACACCA
TCTATCATGCCAAAGACGCCGTGCAGACCACCAAGCCGAGCGAGCGTAAGCCACGCCT
AGTGGTGTTCGTCGTCGGTGAGACGGCGCGTGCTGACCATGTGCAGTTCAATGGCTAT
GGCCGTGAGACTTTCCCGCAGCTTGCCAAAGTTGATGGCTTGGCGAATTTTAGCCAAGT
GACATCGTGTGGCACATCGACGGCGTATTCTGTGCCGTGTATGTTCAGCTATTTGGGTC
AAGATGACTATGATGTCGATACCGCCAAATACCAAGAAAATGTGCTAGATACGCTTGAC

CGCTTGGGTGTGGGTATCTTGTGGCGTGATAATAATTCAGACTCAAAAGGCGTGATGGA
TAAGCTACCTGCCACGCAGTATTTTGATTATAAATCAGCAACCAACAATACCATCTGTAA
CACCAATCCCTATAACGAATGCCGTGATGTCGGTATGCTTGTCGGGCTAGATGACTATG
TCAGCGCCAATAATGGCAAAGATATGCTCATCATGCTACACCAAATGGGCAATCATGGG
CCGGCGTACTTTAAGCGTTATGATGAGCAATTTGCCAAATTCACCCCCGTGTGCGAAGG
CAACGAGCTTGCCAAATGCGAACACCAATCACTCATCAATGCCTATGACAATGCGCTAC
TTGCGACTGATGATTTTATCGCCAAAAGCATCGATTGGCTAAAAACGCATGAAGCGAAC
TACGATGTCGCCATGCTCTATGTCAGTGACCACGGCGAGAGCTTGGGCGAAAATGGTG
TCTATCTGCATGGTATGCCAAATGCCTTTGCACCAAAAGAACAGCGAGCTGTGCCTGCG
TTTTTTTGGTCAAATAATACGACATTCAAGCCAACTGCCAGCGATACTGTGCTGACGCAT
GATGCGATTACGCCAACACTGCTTAAGCTGTTTGATGTCACAGCGGGCAAGGTCAAAGA
CCGCGCGGCATTTATCCAGTAA

SEQ ID NO: 12 (*mcr-2.2*)

GTGTGGTACCGACGCTCGGTCAGTCCGTTTGTTCTTGTGGCGAGTGTTGCCGTTTTCTT
GACCGCGACCGCCAATCTTACCTTTTTTGATAAAATCAGCCAAACCTATCCCATCGCGG
ACAATCTCGGCTTTGTGCTGACGATCGCTGTCGTGCTCTTTGGCGCGATGCTACTGATC
ACCACGCTGTTATCATCGTATCGCTATGTGCTAAAGCCTGTGTTGATTTTGCTATTAATC
ATGGGCGCGGTGACCAGTTATTTTACTGACACTTATGGCACGGTCTATGATACGACCAT
GCTCCAAAATGCCCTACAGACCGACCAAGCCGAGACCAAGGATCTATTAAACGCAGCG
TTTATCATGCGTATCATTGGTTTGGGTGTGCTACCAAGTTTGCTTGTGGCTTTTGTTAAG
GTGGATTATCCGACTTGGGGCAAGGGTTTGATGCGCCGATTGGGCTTGATCGTGGCAA
GTCTTGCGCTGATTTTACTGCCTGTGGTGGCGTTCAGCAGTCATTATGCCAGTTTCTTTC
GCGTGCATAAGCCGCTGCGTAGCTATGTCAATCCGATCATGCCAATCTACTCGGTGGGT
AAGCTTGCCAGTATTGAGTATAAAAAAGCCAGTGCGCCAAAAGATACCATTTATCACGC
CAAAGACGCGGTACAAGCAACCAAGCCTGATATGCGTAAGCCACGCCTAGTGGTGTTC
GTCGTCGGTGAGACGGCACGCGCCGATCATGTCAGCTTCAATGGCTATGAGCGCGATA
CTTTCCCACAGCTTGCCAAGATCGATGGCGTGACCAATTTTAGCAATGTCACATCGTGC
GGCACATCGACGGCGTATTCTGTGCCGTGTATGTTCAGCTATCTGGGCGCGGATGAGT
ATGATGTCGATACCGCCAAATACCAAGAAAATGTGCTGGATACGCTGGATCGCTTGGGC
GTAAGTATCTTGTGGCGTGATAATAATTCGGACTCAAAAGGCGTGATGGATAAGCTGCC
AAAAGCGCAATTTGCCGATTATAAATCCGCGACCAACAACGCCATCTGCAACACCAATC
CTTATAACGAATGCCGCGATGTCGGTATGCTCGTTGGCTTAGATGACTTTGTCGCTGCC
AATAACGGCAAAGATATGCTGATCATGCTGCACCAAATGGGCAATCACGGGCCTGCGTA
TTTTAAGCGATATGATGAAAAGTTTGCCAAATTCACGCCAGCGTGTGAAGGTAATGAGCT
TGCCAAGTGCGAACATCAGTCCTTGATCAATGCTTATGACAATGCCTTGCTTGCCACCG

ATGATTTCATCGCTCAAAGTATCCAGTGGCTGCAGACGCACAGCAATGCCTATGATGTC
TCAATGCTGTATGTCAGCGATCATGGCGAAAGTCTGGGTGAGAACGGTGTCTATCTACA
TGGTATGCCAAATGCCTTTGCACCAAAAGAACAGCGCAGTGTGCCTGCATTTTTCTGGA
CGGATAAGCAAACTGGCATCACGCCAATGGCAACCGATACCGTCCTGACCCATGACGC
GATCACGCCGACATTATTAAAGCTGTTTGATGTCACCGCGGACAAAGTCAAAGACCGCA
CCGCATTCATCCGCTGA

SEQ ID NO: 13 (*mcr-3*)

ATGCCTTCCCTTATAAAAATAAAAATTGTTCCGCTTATGTTCTTTTTGGCACTGTATTTTG
CATTTATGCTGAACTGGCGTGGAGTTCTCCATTTTTACGAAATCCTTTACAAATTAGAAG
ATTTTAAGTTTGGTTTCGCCATTTCATTACCAATATTGCTTGTTGCAGCGCTTAACTTTGT
ATTTGTTCCATTTTCGATACGGTATTTAATAAAGCCTTTTTTTGCACTTCTTATCGCACTTA
GTGCAATCGTTAGTTACACAATGATGAAGTATAGAGTCTTGTTTGATCAAAACATGATTC
AGAATATTTTTGAAACCAATCAAAATGAGGCGTTAGCATATTTAAGCTTACCAATTATAGT
ATGGGTTACTATTGCTGGTTTTATCCCTGCCATTTTACTTTTCTTTGTTGAAATTGAATAT
GAGGAAAAATGGTTCAAAGGGATTCTAACTCGTGCCCTATCGATGTTTGCATCACTTATA
GTGATTGCGGTTATTGCAGCACTATACTATCAAGATTATGTGTCAGTGGGGCGCAACAA
TTCAAACCTCCAGCGTGAGATTGTTCCAGCCAATTTCGTTAATAGTACCGTTAAATACGT
TTACAATCGTTATCTTGCTGAACCAATCCCATTTACAACTTTAGGTGATGATGCAAAACG
GGATACTAATCAAAGTAAGCCCACGTTGATGTTTCTGGTCGTTGGTGAAACCGCTCGTG
GTAAAAATTTCTCGATGAATGGCTATGAGAAAGACACCAATCCATTTACCAGTAAATCTG
GTGGCGTGATCTCCTTTAATGATGTTCGTTCGTGTGGGACTGCAACCGCTGTATCCGTC
CCCTGCATGTTCTCCAATATGGGGAGAAAGGAGTTTGATGATAATCGCGCTCGCAATAG
CGAGGGCCTGCTAGATGTGTTGCAAAAAACGGGGATCTCCATTTTTTGGAAGGAGAACG
ATGGAGGCTGCAAAGGCGTCTGCGACCGAGTACCTAACATCGAAATCGAACCAAAGGA
TCACCCTAAGTTCTGCGATAAAAACACATGCTATGACGAGGTTGTCCTTCAAGACCTCG
ATAGTGAAATTGCTCAAATGAAAGGGGATAAGCTGGTTGGCTTCCACCTGATAGGTAGC
CATGGCCCAACCTACTACAAGCGCTACCCTGATGCTCATCGTCAGTTCACCCCTGACTG
TCCACGCAGTGATATTGAAAACTGCACAGATGAAGAGCTCACCAACACCTATGACAACA
CCATCCGCTACACCGATTTCGTGATTGGAGAGATGATTGCCAAGTTGAAAACCTACGAA
GATAAGTACAACACCGCGTTGCTCTACGTCTCCGATCATGGTGAATCACTGGGAGCATT
AGGGCTTTACCTACACGGTACACCGTACCAGTTTGCACCGGATGATCAGACCCGTGTTC
CTATGCAGGTGTGGATGTCACCTGGATTTACCAAAGAGAAAGGCGTTGATATGGCGTGT
TTGCAGCAGAAAGCCGCTGATACTCGTTACTCACACGATAATATTTTCTCATCTGTATTG
GGTATCTGGGACGTCAAAACATCAGTTTACGAAAAGGGTCTAGATATTTTCAGTCAATGT
CGTAATGTTCAATAA

SEQ ID NO: 14 (*mcr-3.2*)

ATGCCTTCCCTTATAAAAATAAAAATTGTTCCGCTTATGTTCTTTTTGGCACTGTATTTTG
CATTTATGCTGAACTGGCGTGGAGTTCTCCATTTTTACGAAATCCTTTACAAATTAGAAG
ATTTTAAGTTTGGTTTCGCCATTTCATTACCAATATTGCTTGTTGCAGCGCTTAACTTTGT
ATTTGTTCCATTTTCGATACGGTATTTAATAAAGCCTTTTTTTGCACTTCTTATCGCACTTA
GTGCAATCGTTAGTTACACAATGATGAAGTATAGAGTCTTGTTTGATCAAAACATGATTC
AGAATATTTTTGAAACCAATCAAAATGAGGCGTTAGCATATTTAAGCTTACCAATTATAGT
ATGGGTTACTATTGCTGGTTTTATCCCTGCCATTTTACTTTTCTTTGTTGAAATTGAATAT
GAGGAAAAATGGTTCAAAGGGATTCTAACTCGTGCCCTATCGATGTTTGCATCACTTATA
GTGATTGCGGTTATTGCAGCACTATACTATCAAGATTATGTGTCAGTGGGGCGCAACAA
TTCAAACCTCCAGCGTGAGATTGTTCCAGCCAATTTCGTTAATAGTACCGTTAAATACGT
TTACAATCGTTATCTTGCTGAACCAATCCCATTTACAACTTTAGGTGATGATGCAAAACG
GGATACTAATCAAAGTAAGCCCACGTTGATGTTTCTGGTCGTTGGTGAAACCGCTCGTG
GTAAAAATTTCTCGATGAATGGCTATGAGAAAGACACCAATCCATTTACCAGTAAATCTG
GTGGCGTGATCTCCTTTAATGATGTTCGTTCGTGTGGGACTGCAACCGCTGTATCCGTC
CCCTGCATGTTCTCCAATATGGGGAGAAAGGAGTTTGATGAAAATCGCGCTCGCAATAG
CGAGGGCCTGCTAGATGTGTTGCAAAAAACGGGGATCTCCATTTTTTGGAAGGAGAACG
ATGGAGGCTGCAAAGGCGTCTGCGACCGAGTACCTAACATCGAAATCGAACCAAAGGA
TCACCCTAAGTTCTGCGATAAAAACACATGCTATGACGAGGTTGTCCTTCAAGACCTCG
ATAGTGAAATTGCTCAAATGAAAGGGGATAAGCTGGTTGGCTTCCACCTGATAGGTAGC
CATGGCCCAACCTACTACAAGCGCTACCCTGATGCTCATCGTCAGTTCACCCCTGACTG
TCCACGCAGTGATATTGAAAACTGCACAGATGAAGAGCTCACCAACACCTATGACAACA
CCATCCGCTACACCGATTTCGTGATTGGAGAGATGATTGCCAAGTTGAAAACCTACGAA
GATAAGTACAACACCGCGTTGCTCTACGTCTCCGATCATGGTGAATCACTGGGAGCATT
AGGGCTTTACCTACACGGTACACCGTACCAGTTTGCACCGGATGATCAGACCCGTGTTC
CTATGCAGGTGTGGATGTCACCTGGATTTACCAAAGAGAAAGGCGTTGATATGGCGTGT
TTGCAGCAGAAAGCCGCTGATACTCGTTACTCACACGATAATATTTTCTCATCTGTATTG
GGTATCTGGGACGTCAAAACATCAGTTTACGAAAAGGGTCTAGATATTTTCAGTCAATGT
CGTAATGTTCAATAA

SEQ ID NO: 15 (*mcr-4*)

GTGATTTCTAGATTTAAGACGTTATCGGTTAACCAATTCACTTTCATCACTGCGTTGTTTT
ATGTTGCCATTTTCAATCTACCGCTCTTTGGTATAGTGCGAAAAGGAATTGAAAAACAAC
CAGAAGTTGATCCCCTTTTCATCGCATCTATGCCGCTATTTTTAACATTTGCGCTGAGTT
TTTTGTTTTCAATTTTTACCGTCAAATACCTGCTGAAGCCCTTTTTTATCGTATTGACGTT

ACTTTCCTCAAGTGTATTTTTTGCAGCCTATCAATACAATGTCGTGTTTGACTACGGCAT
GATAGAAAACACGTTTCAAACACATCCTGCTGAAGCATTGATGTATGTAAATCTTGCATC
AATTACCAATCTACTGCTGACTGGGCTATTACCGTCATATCTTATTTATAAGGCCGATATT
CATTATCAGCCCTTTTTTAAGGAGTTATTGCATAAATTAGCCTTTATGCTGCTAATGTTCG
TTGGCATTGGGATAGTCGCCTTTTTTTACTATCAAGATTATGCTGCATTTGTTCGAAACAA
CAGTGAGTTAAGGCGTTACATTGTCCCTACCTATTTTGTCAGTAGTGCATCTAAATATCT
CAATGAGCACTATTTGCAGACGCCCATGGAATACCAACAACTTGGCCTAGATGCGAAGA
ATGCCAGTCGTAACCCGAACACTAAACCTAACTTATTAGTGGTTGTTGTGGGTGAAACT
GCGCGCTCAATGAGCTATCAATATTATGGATATAACAAGCCAACCAATGCTCATACCCAA
AATCAGGGGCTGATTGCGTTTAACGATACTAGCTCATGCGGCACGGCCACGGCGGTGT
CTCTACCCTGTATGTTTTCACGAATGGGGCGGGCAGACTATGATCCTCGCCGTGCTAAT
GCTCAAGACACAGTGATTGATGTGTTAAGTCATAGTGGTATAAAAGTACAGTGGTTTGAT
AATGATTCTGGCTGTAAAGGTGTGTGTGATCAGGTTGAAAATCTCACGATAGATTTGAAG
AGTGATCCGAAGCTGTGTTCTGGCCAATATTGTTTTGACCAAGTATTGCTCAACAAATTA
GATAAAATTCTGGCAGTAGCACCAAGTCAAGATACAGTAATTTTTTTGCATATCATTGGT
AGTCATGGACCAACTTATTATCTTAGATACCCGCCAGAGCATCGTAAATTTATACCGGAT
TGTCCGCGCAGTGATATTCAAAATTGCAGTCAAGAAGAACTGATTAACACCTACGACAA
CACTATTCTATATACGGATTTTATTCTCAGTGAAGTGGTGAATAAATTAAAAGGTAAGCA
GGATATGTTCGATACTGCAATGCTGTATCTCTCTGACCATGGTGAGTCTTTGGGTGAAAA
GGGCATGTATTTACATGGTGCGCCCTATAGTATTGCACCGAAAGAACAAACTAGCGTAC
CAATGCTGGCTTGGGTATCTAATGACTTTAGCCAAGATAATCAGTTGAACATGACTTGTG
TTGCACAGCGAGCAGAACAGGGCGGCTTTTCCCACGACAATTTGTTCGACAGTTTGCTA
GGACTTATGAATGTAAAAACCACCGTCTATCAGAGCCAACTCGATATTTTTGCACCTTGC
AGGTATTAG

SEQ ID NO: 16 (*mcr-5*)

ATGCGGTTGTCTGCATTTATCACTTTCTTGAAAATGCGCCCGCAAGTGCGCACTGAATTT
TTGACTCTGTTCATCAGCCTTGTGTTCACCCTGCTGTGCAATGGCGTGTTTTGGAATGC
CCTTCTTGCTGGACGCGACTCCCTAACTTCTGGAACATGGCTAATGCTCCTTTGCACTG
GGTTGCTGATCACCGGGCTGCAATGGTTGTTGCTCCTTCTGGTGGCCACGCGCTGGAG
TGTCAAGCCACTACTGATTCTGCTTGCTGTCATGACGCCCGCCGCCGTTTATTTCATGC
GCAACTACGGGGTTTATCTCGACAAGGCCATGCTGCGGAATCTGATGGAGACGGACGT
CAGGGAAGCCAGTGAGCTGTTGCAATGGAGAATGCTGCCCTACTTGTTGGTTGCAGCC
GTATCCGTGTGGTGGATTGCGAGAGTCAGGGTTTTACGAACGGGCTGGAAACAAGCGG
TAATGATGCGCAGCGCTTGTCTGGCTGGCGCTCTCGCCATGATTTCCATGGGTCTGTG
GCCAGTCATGGATGTGCTGATACCCACGCTTCGTGAAAACAAGCCGCTTCGCTATTTGA

TCACTCCTGCAAACTACGTCATCTCGGGCATTCGGGTTTTGACTGAACAGGCGTCATCG
TCAGCAGACGAAGCAAGGGAAGTCGTTGCAGCCGATGCGCATCGAGGGCCTCAAGAAC
AAGGCCGCCGTCCTCGTGCTCTCGTACTGGTTGTCGGGGAAACCGTCAGGGCGGCTAA
TTGGGGGTTGAGCGGCTATGAACGACAAACCACCCCTGAGTTGGCCGCACGCGACGTG
ATCAATTTTTCCGATGTCACCAGTTGCGGGACGGATACGGCTACATCCCTTCCCTGCAT
GTTTTCCCTCAATGGTCGGCGCGACTACGACGAACGCCAGATTCGTCGGCGCGAGTCC
GTGCTGCACGTTTTAAACCGTAGTGACGTCAACATTCTCTGGCGCGATAACCAGTCGGG
CTGTAAAGGCGTCTGTGATGGACTGCCCTTTGAAAACCTGTCTTCGGCAGGCCATCCCA
CACTGTGCCATGGCGAGCGCTGCCTGGATGAAATTCTGCTCGAAGGGTTGGCCGAGAA
GATAACAACAAGCCGCAGCGATATGCTGATCGTTCTGCATATGCTGGGCAATCACGGCC
CAGCGTATTTCCAGCGCTATCCCGCAAGCTACCGACGCTGGTCGCCAACCTGCGACAC
CACCGATCTGGCCAGCTGTTCGCATGAAGCCTTGGTGAACACCTACGACAACGCCGTG
CTTTACACCGATCATGTGCTTGCCCGTACCATTGACCTGCTGTCCGGCATCCGCTCACA
CGACACGGCGCTGCTGTACGTTTCCGATCATGGGGAATCGCTCGGCGAGAAAGGCCTG
TATCTCCATGGCATACCTTACGTCATCGCGCCGGATGAGCAGATCAAGGTGCCGATGAT
CTGGTGGCAGTCGAGTCAGGTTTATGCCGACCAAGCCTGTATGCAAACTCATGCCTCTC
GGGCACCGGTAAGTCACGATCACCTGTTTCACACCTTGCTCGGGATGTTCGACGTGAAA
ACCGCTGCCTACACGCCAGAGTTGGACCTTCTGGCAACATGCAGAAAAGGACAACCAC
AATGA

## Claims

1. A method for detecting the presence or absence of a bacterium resistant to a cyclic cationic polypeptide antibiotic, comprising:

   a. subjecting a test sample to mass spectrometry analysis and generating a mass spectrum output; wherein said test sample comprises a bacterial membrane or a fragment thereof, wherein the fragment comprises a non-Lipid A component;
   b. identifying in said mass spectrum output a first defined peak indicative of the presence of Lipid A modified by phosphoethanolamine, wherein said first defined peak is a peak present in a mass spectrum output for Lipid A modified by phosphoethanolamine and wherein said first defined peak is absent from a corresponding mass spectrum output for native Lipid A; and
   c. wherein the presence of said first defined peak indicates the presence of a bacterium resistant to a cyclic cationic polypeptide antibiotic, and wherein the absence of said first defined peak indicates the absence of a bacterium resistant to a cyclic cationic polypeptide antibiotic.

2. A method according to claim 1, wherein said Lipid A modified by phosphoethanolamine is an integral part of a bacterial membrane or a fragment thereof; and/or
   wherein said mass spectrometry analysis comprises MALDI-TOF mass spectrometry analysis; and/or
   wherein said first defined peak comprises a mass-to-charge ratio (m/z) of 120 to 125 m/z units greater than a second defined peak indicative of the presence of native Lipid A.

3. A method according to claim 2, wherein said second defined peak is selected from the group consisting of:

   a. a peak comprising a mass-to-charge ratio (m/z) of 1793 to 1799 m/z, preferably 1796.2 m/z, for *Escherichia coli, Shigella, Klebsiella pneumoniae, Salmonella enterica, Enterobacter spp.* and *Klebsiella oxytoca;*
   b. a peak comprising a mass-to-charge ratio (m/z) of 1820 to 1826 m/z, preferably 1823.9 m/z, for *Klebsiella pneumoniae;*
   c. a peak comprising a mass-to-charge ratio (m/z) of 1837 to 1843 m/z, preferably 1840 m/z, for *Klebsiella pneumoniae;*
   d. a peak comprising a mass-to-charge ratio (m/z) of 1847 to 1853 m/z, preferably 1850 m/z, for *Klebsiella pneumoniae;*
   e. a peak comprising a mass-to-charge ratio (m/z) of 2059 to 2065 m/z, preferably 2062 m/z, for *Klebsiella pneumoniae;*
   f. a peak comprising a mass-to-charge ratio (m/z) of 2075 to 2081 m/z, preferably 2078 m/z, for *Klebsiella pneumoniae;*
   g. a peak comprising a mass-to-charge ratio (m/z) of 1614 to 1620 m/z, preferably 1617.2 m/z, for *Pseudomonas aeruginosa;*
   h. a peak comprising a mass-to-charge ratio (m/z) of 1907 to 1913 m/z, preferably 1910.3 m/z, for *Acinetobacter baumannii;*
   i. a peak comprising a mass-to-charge ratio (m/z) of 1793 to 1799 m/z, preferably 1796.2 m/z, for *Salmonella spp;*
   j. a peak comprising a mass-to-charge ratio (m/z) of 1820 to 1826 m/z, preferably 1824 m/z, for *Salmonella spp;* or
   k. a peak comprising a mass-to-charge ratio (m/z) of 2031 to 2037 m/z, preferably 2034 m/z, for *Salmonella spp;* and/or
   wherein a ratio of intensity of:

      the first defined peak; to
      the second defined peak is least 0.10:1.

4. A method according to any one of the preceding claims, further comprising identifying in said mass spectrum output a third defined peak comprising a mass-to-charge ratio (m/z) of 22 to 28 m/z units greater than a second defined peak indicative of the presence of native Lipid A.

5. A method according to claim 4, wherein said second defined peak is selected from the group consisting of:

a. a peak comprising a mass-to-charge ratio (m/z) of 1793 to 1799 m/z, preferably 1796.2 m/z, for *Escherichia coli, Shigella, Klebsiella pneumoniae, Salmonella enterica, Enterobacter spp*. and *Klebsiella oxytoca;*
b. a peak comprising a mass-to-charge ratio (m/z) of 1820 to 1826 m/z, preferably 1823.9 m/z, for *Klebsiella pneumoniae;*
c. a peak comprising a mass-to-charge ratio (m/z) of 1837 to 1843 m/z, preferably 1840 m/z, for *Klebsiella pneumoniae;*
d. a peak comprising a mass-to-charge ratio (m/z) of 1847 to 1853 m/z, preferably 1850 m/z, for *Klebsiella pneumoniae;*
e. a peak comprising a mass-to-charge ratio (m/z) of 2059 to 2065 m/z, a preferably 2062 m/z, for *Klebsiella pneumoniae;*
f. a peak comprising a mass-to-charge ratio (m/z) of 2075 to 2081 m/z, preferably 2078 m/z, for *Klebsiella pneumoniae;*
g. a peak comprising a mass-to-charge ratio (m/z) of 1793 to 1799 m/z, preferably 1796.2 m/z, for *Salmonella spp;*
h. a peak comprising a mass-to-charge ratio (m/z) of 1820 to 1826 m/z, preferably 1824 m/z, for *Salmonella spp;* or
i. a peak comprising a mass-to-charge ratio (m/z) of 2031 to 2037 m/z, preferably 2034 m/z, for *Salmonella spp.*

**6.** A method according to any one of the preceding claims, wherein a ratio of intensity of:

the third defined peak; to
the second defined peak is at least 0.15:1, or at least 0.6:1; and/or
wherein a ratio of:

the sum of the intensity of said first defined peak and the intensity of said third defined peak; to
the intensity of said second defined peak is least 0.15:1; preferably at least 0.5:1.

**7.** A method according to any one of the preceding claims, wherein a bacterium resistant to a cyclic cationic polypeptide antibiotic through plasmid-encoded resistance comprises a plasmid having a mobilised colistin resistance gene; optionally
wherein said mobilised colistin gene comprises one or more of an *mcr*-like gene, *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4, mcr-5,* SEQ ID:1, SEQ ID:2, SEQ ID:3, SEQ ID:4, SEQ ID:5, SEQ ID:6, SEQ ID:7, SEQ ID:8, SEQ ID:9, SEQ ID:10, SEQ ID:11, SEQ ID:12, SEQ ID:13, SEQ ID:14, SEQ ID:15, SEQ ID:16, or a sequence having at least 50% homology thereto.

**8.** A method according to any one of the preceding claims, wherein said test sample is admixed with a matrix solution prior to subjecting said test sample to mass spectrometry analysis, optionally wherein said matrix solution allows for the selective extraction, co-crystallization and ionisation of native Lipid A and/or modified Lipid A as an integral part of a bacterial membrane; and/or
wherein the matrix solution comprises 2,5-dihydroxybenzoic acid suspended in an organic solvent, optionally wherein said organic solvent comprises chloroform and methanol at a ratio of 6:1 to 12:1, or at a ratio of 9:1 v/v.
wherein said organic solvent comprises chloroform, methanol, dichloromethane, ether, diethyl-ether, petroleum ether, isopropanol, butanol, hexane or a combination thereof; optionally

**9.** A method according to claim 8, wherein the ratio of the test sample to the matrix solution is between 0.1:1 to 2:1 v/v, or 0.66:1 v/v.

**10.** A method according to any one of the preceding claims, wherein said test sample comprises between $10^1$ to $10^{10}$ bacterial cells; and/or
wherein said cyclic cationic polypeptide antibiotic is a polymyxin antibiotic; preferably
wherein said polymyxin antibiotic is one or more of Colistin (Polymyxin E), Polymyxin B, Mattacin (Polymyxin M), or a salt thereof.

**11.** A method according to any one of the preceding claims, wherein said bacterium is selected from the following genera: *Escherichia, Klebsiella, Enterobacter, Pseudomonas, Acinetobacter, Shigella, Salmonella, Citrobacter, Raoultella* and combinations thereof; and/or
wherein said bacterium is selected from the following species: *Escherichia coli, Klebsiella pneumoniae, Klebsiella oxytoca, Enterobacter aerogenes, Enterobacter cloacae, Enterobacter asburiae, Pseudomonas aeruginosa, Acinetobacter baumannii, Shigella sonnei, Shigella flexneri, Salmonella enterica, Citrobacter freundii, Citrobacter koseri, Citrobacter amalonaticus, Citrobacter youngae* and combinations thereof.

12. A method according to any one of the preceding claims, wherein said bacterium is heat inactivated or wherein said bacterium is not heat inactivated; and/or wherein said test sample is processed to remove salt; and/or further comprising the step of recording the data obtained in step (a) on a suitable data carrier.

13. A screening method for identifying an inhibitor of cyclic cationic polypeptide antibiotic resistance in a bacterium, comprising:

a. incubating a sample comprising a bacterium resistant to a cyclic cationic polypeptide antibiotic with a candidate inhibitor;
b. subjecting said sample to mass spectrometry analysis according to any one of claims 1-12 and generating a mass spectrum output; and
c. identifying the presence or absence of said first defined peak in the mass spectrum output;

wherein the presence of said first defined peak indicates said candidate inhibitor is not a substance capable of inhibiting cyclic cationic polypeptide antibiotic resistance in a bacterium, and wherein the absence of said first defined peak indicates said candidate inhibitor is a substance capable of inhibiting cyclic cationic polypeptide antibiotic resistance in a bacterium.

14. A method according to claim 13, wherein said candidate inhibitor is selected from any one of a natural compound, a synthetic chemical compound, peptide, a monoclonal or polyclonal antibody or an antibody fragment, preferably wherein said candidate inhibitor is a known chemical or pharmaceutical substance selected from a library of such candidate inhibitors; and/or wherein said sample is from a human or a non-human animal.

## Patentansprüche

1. Verfahren zum Nachweis der An- oder Abwesenheit eines gegen ein zyklisches kationisches Polypeptid-Antibiotikum resistenten Bakteriums, umfassend:

a. Unterziehen einer Testprobe einer Massenspektrometrieanalyse und Generieren eines massenspektrometrischen Outputs;
wobei die genannte Testprobe eine bakterielle Membran oder ein Fragment davon umfasst, wobei das Fragment eine Nicht-Lipid-A-Komponente umfasst;
b. Identifizieren im genannten massenspektrometrischen Output einen ersten definierten Peak, der auf die Anwesenheit von durch Phosphoethanolamin modifiziertes Lipid A schließen lässt, wobei der genannte erste definierte Peak ein Peak ist, der in einem massenspektrometrischen Output für durch Phosphoethanolamin modifiziertes Lipid A anwesend ist und wobei der genannte erste definierte Peak von einem entsprechenden massenspektrometrischen Output für natives Lipid A abwesend ist; und
c. wobei die Anwesenheit des genannten ersten definierten Peaks auf die Anwesenheit eines gegen ein zyklisches kationisches Polypeptid-Antibiotikum resistenten Bakteriums hindeutet, und wobei die Abwesenheit des genannten ersten definierten Peaks auf die Abwesenheit eines gegen ein zyklisches kationisches Polypeptid-Antibiotikum resistenten Bakteriums hindeutet.

2. Verfahren nach Anspruch 1, wobei das genannte durch Phosphoethanolamin modifizierte Lipid A ein integraler Teil einer bakteriellen Membran oder ein Fragment davon ist; und/oder
wobei die genannte Massenspektrometrieanalyse eine MALDI-TOF-Massenspektrometrieanalyse umfasst; und/oder
wobei der genannte erste definierte Peak ein Masse-Ladungs-Verhältnis (m/z) von 120 bis 125 m/z-Einheiten größer als ein zweiter definierter Peak umfasst, was auf die Anwesenheit von nativem Lipid A schließen lässt.

3. Verfahren nach Anspruch 2, wobei der genannte zweite definierte Peak aus der Gruppe ausgewählt ist, bestehend aus:

a. einem Peak, umfassend ein Masse-Ladungs-Verhältnis (m/z) von 1793 bis 1799 m/z, bevorzugt 1796,2 m/z, für *Escherichia coli, Shigella, Klebsiella pneumoniae, Salmonella enterica, Enterobacter spp.* und *Klebsiella oxytoca;*
b. einem Peak, umfassend ein Masse-Ladungs-Verhältnis (m/z) von 1820 bis 1826 m/z, bevorzugt 1823,9 m/z, für *Klebsiella pneumoniae;*

c. einem Peak, umfassend ein Masse-Ladungs-Verhältnis (m/z) von 1837 bis 1843 m/z, bevorzugt 1840 m/z, für *Klebsiella pneumoniae;*
d. einem Peak, umfassend ein Masse-Ladungs-Verhältnis (m/z) von 1847 bis 1853 m/z, bevorzugt 1850 m/z, für *Klebsiella pneumoniae;*
e. einem Peak, umfassend ein Masse-Ladungs-Verhältnis (m/z) von 2059 bis 2065 m/z, bevorzugt 2062 m/z, für *Klebsiella pneumoniae;*
f. einem Peak, umfassend ein Masse-Ladungs-Verhältnis (m/z) von 2075 bis 2081 m/z, bevorzugt 2078 m/z, für *Klebsiella pneumoniae;*
g. einem Peak, umfassend ein Masse-Ladungs-Verhältnis (m/z) von 1614 bis 1620 m/z, bevorzugt 1617,2 m/z, für *Pseudomonas aeruginosa;*
h. einem Peak, umfassend ein Masse-Ladungs-Verhältnis (m/z) von 1907 bis 1913 m/z, bevorzugt 1910,3 m/z, für *Acinetobacter baumannii;*
i. einem Peak, umfassend ein Masse-Ladungs-Verhältnis (m/z) von 1793 bis 1799 m/z, bevorzugt 1796,2 m/z, für *Salmonella spp.;*
j. einem Peak, umfassend ein Masse-Ladungs-Verhältnis (m/z) von 1820 bis 1826 m/z, bevorzugt 1824 m/z, für *Salmonella spp.;* oder
k. einem Peak, umfassend ein Masse-Ladungs-Verhältnis (m/z) von 2031 bis 2037 m/z, bevorzugt 2034 m/z, für *Salmonella spp.;* und/oder
wobei ein Intensitätsverhältnis von:

dem ersten definierten Peak; zu
dem zweiten definierten Peak mindestens 0,10:1 beträgt.

**4.** Verfahren nach einem der vorangehenden Ansprüche, weiter umfassend die Identifizierung im genannten massenspektrometrischen Output eines dritten definierten Peaks, umfassend ein Masse-Ladungs-Verhältnis (m/z) von 22 bis 28 m/z-Einheiten größer als ein zweiter definierter Peak, was auf die Anwesenheit von nativem Lipid A schließen lässt.

**5.** Verfahren nach Anspruch 4, wobei der genannte zweite definierte Peak aus der Gruppe ausgewählt ist, bestehend aus:

a. einem Peak, umfassend ein Masse-Ladungs-Verhältnis (m/z) von 1793 bis 1799 m/z, bevorzugt 1796,2 m/z, für *Escherichia coli, Shigella, Klebsiella pneumoniae, Salmonella enterica, Enterobacter spp.* und *Klebsiella oxytoca;*
b. einem Peak, umfassend ein Masse-Ladungs-Verhältnis (m/z) von 1820 bis 1826 m/z, bevorzugt 1823,9 m/z, für *Klebsiella pneumoniae;*
c. einem Peak, umfassend ein Masse-Ladungs-Verhältnis (m/z) von 1837 bis 1843 m/z, bevorzugt 1840 m/z, für *Klebsiella pneumoniae;*
d. einem Peak, umfassend ein Masse-Ladungs-Verhältnis (m/z) von 1847 bis 1853 m/z, bevorzugt 1850 m/z, für *Klebsiella pneumoniae;*
e. einem Peak, umfassend ein Masse-Ladungs-Verhältnis (m/z) von 2059 bis 2065 m/z, bevorzugt 2062 m/z, für *Klebsiella pneumoniae;*
f. einem Peak, umfassend ein Masse-Ladungs-Verhältnis (m/z) von 2075 bis 2081 m/z, bevorzugt 2078 m/z, für *Klebsiella pneumoniae;*
g. einem Peak, umfassend ein Masse-Ladungs-Verhältnis (m/z) von 1793 bis 1799 m/z, bevorzugt 1796,2 m/z, für *Salmonella spp.;*
h. einem Peak, umfassend ein Masse-Ladungs-Verhältnis (m/z) von 1820 bis 1826 m/z, bevorzugt 1824 m/z, für *Salmonella spp.;* oder
i. einem Peak, umfassend ein Masse-Ladungs-Verhältnis (m/z) von 2031 bis 2037 m/z, bevorzugt 2034 m/z, für *Salmonella spp.*

**6.** Verfahren nach einem der vorangehenden Ansprüche, wobei ein Intensitätsverhältnis von:

dem dritten definierten Peak; zu
dem zweiten definierten Peak mindestens 0,15:1, oder mindestens 0,6:1 beträgt; und/oder
wobei ein Verhältnis von:

der Summe der Intensität des genannten ersten definierten Peaks und der Intensität des genannten dritten

definierten Peaks; zu
der Intensität des genannten zweiten definierten Peaks mindestens 0,15:1; bevorzugt mindestens 0,5:1 beträgt.

**7.** Verfahren nach einem der vorangehenden Ansprüche, wobei ein gegen ein zyklisches kationisches Polypeptid-Antibiotikum durch Plasmid-kodierte Resistenz resistentes Bakterium ein Plasmid mit einem mobilisierten Colistin-Resistenzgen umfasst; gegebenenfalls
wobei das genannte mobilisierte Colistin-Resistenzgen Folgendes umfasst: eines oder mehr von einem mcr-ähnlichen Gen, *mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4, mcr-5,* SEQ ID:1, SEQ ID:2, SEQ ID:3, SEQ ID:4, SEQ ID:5, SEQ ID:6, SEQ ID:7, SEQ ID:8, SEQ ID:9, SEQ ID:10, SEQ ID:11, SEQ ID:12, SEQ ID:13, SEQ ID:14, SEQ 10:15, SEQ ID:16, oder eine Sequenz mit einer mindestens 50%igen Homologie dazu.

**8.** Verfahren nach einem der vorangehenden Ansprüche, wobei die genannte Testprobe vor dem Unterziehen der genannten Testprobe der Massenspektrometrieanalyse mit einer Matrixlösung versetzt wird, gegebenenfalls wobei die genannte Matrixlösung die selektive Extraktion, Cokristallisation und Ionisation von nativem Lipid A und/oder modifiziertem Lipid A als einen integralen Teil einer bakteriellen Membran zulässt; und/oder
wobei die Matrixlösung 2,5-Dihydroxybenzoesäure suspendiert in einem organischen Lösungsmittel umfasst, gegebenenfalls wobei das genannte organische Lösungsmittel Chloroform und Methanol in einem Verhältnis von 6:1 bis 12:1 oder in einem Verhältnis von 9:1 v/v umfasst,
gegebenenfalls wobei das genannte organische Lösungsmittel Chloroform, Methanol, Dichlormethan, Ether, Diethylether, Petrolether, Isopropanol, Butanol, Hexan oder eine Kombination davon umfasst.

**9.** Verfahren nach Anspruch 8, wobei das Verhältnis der Testprobe zur Matrixlösung zwischen 0,1:1 bis 2:1 v/v, oder 0,66:1 v/v liegt.

**10.** Verfahren nach einem der vorangehenden Ansprüche, wobei die genannte Testprobe zwischen $10^1$ bis $10^{10}$ Bakterienzellen umfasst; und/oder
wobei das genannte zyklische kationische Polypeptid-Antibiotikum ein Polymyxin-Antibiotikum ist; bevorzugt wobei das genannte Polymyxin-Antibiotikum eines oder mehr von Colistin (Polymyxin E), Polymyxin B, Mattacin (Polymyxin M) oder ein Salz davon ist.

**11.** Verfahren nach einem der vorangehenden Ansprüche, wobei das genannte Bakterium aus den folgenden Gattungen ausgewählt ist: *Escherichia, Klebsiella, Enterobacter, Pseudomonas, Acinetobacter, Shigella, Salmonella, Citrobacter, Raoultella* und Kombinationen davon; und/oder
wobei das genannte Bakterium aus den folgenden Arten ausgewählt ist: *Escherichia coli, Klebsiella pneumoniae, Klebsiella oxytoca, Enterobacter aerogenes, Enterobacter cloacae, Enterobacter asburiae, Pseudomonas aeruginosa, Acinetobacter baumannii, Shigella sonnei, Shigella flexneri, Salmonella enterica, Citrobacter freundii, Citrobacter koseri, Citrobacter amalonaticus, Citrobacter youngae* und Kombinationen davon.

**12.** Verfahren nach einem der vorangehenden Ansprüche, wobei das genannte Bakterium hitzeinaktiviert ist oder wobei das genannte Bakterium nicht hitzeinaktiviert ist; und/oder wobei die genannte Testprobe zum Entfernen von Salz verarbeitet wird; und/oder
weiter den Schritt des Aufzeichnens der in Schritt (a) erfassten Daten auf einem geeigneten Datenträger umfasst.

**13.** Screening-Verfahren zum Identifizieren eines Inhibitors einer zyklischen kationischen Polypeptid-Antibiotikaresistenz in einem Bakterium, umfassend:

a. Inkubieren einer Probe, umfassend ein gegen ein zyklisches kationisches Polypeptid-Antibiotikum resistentes Bakterium, mit einem Kandidateninhibitor;
b. Unterziehen der genannten Probe einer Massenspektrometrieanalyse nach einem der Ansprüche 1 bis 12 und Generieren eines massenspektrometrischen Outputs; und
c. Identifizieren der An- oder Abwesenheit des genannten ersten definierten Peaks in dem massenspektrometrischen Output;

wobei die Anwesenheit des genannten ersten definierten Peaks darauf hindeutet, dass der genannte Kandidateninhibitor keine Substanz ist, die zur Inhibition der zyklischen kationischen Polypeptid-Antibiotikaresistenz in einem Bakterium fähig ist, und wobei die Abwesenheit des genannten ersten definierten Peaks darauf hinweist, dass der

genannte Kandidateninhibitor eine Substanz ist, die zur Inhibition der zyklischen kationischen Polypeptid-Antibiotikaresistenz in einem Bakterium fähig ist.

**14.** Verfahren nach Anspruch 13, wobei der genannte Kandidateninhibitor ausgewählt ist aus einer/einem von Folgendem: einer natürlichen Verbindung, einer synthetischen chemischen Verbindung, einem Peptid, einem monoklonalen oder polyklonalen Antikörper oder einem Antikörperfragment, bevorzugt wobei der genannte Kandidateninhibitor eine bekannte chemische oder pharmazeutische Substanz ist, die aus einer Bibliothek von solchen Kandidateninhibitoren ausgewählt ist; und/oder wobei die genannte Probe von einem Menschen oder einem nicht humanen Tier stammt.

## Revendications

**1.** Procédé destiné à détecter la présence ou l'absence d'une bactérie résistante à un antibiotique polypeptidique cationique cyclique, comprenant :

a. la soumission d'un échantillon d'essai à une analyse de spectrométrie de masse et la génération d'une sortie de spectre de masse ;
dans lequel ledit échantillon d'essai comprend une membrane bactérienne ou un fragment de celle-ci, dans lequel le fragment comprend un composant A non lipidique ;
b. l'identification dans ladite sortie de spectre de masse d'un premier pic défini indiquant la présence d'un Lipide A modifié par de la phosphoéthanolamine, dans lequel ledit premier pic défini est un pic présent dans une sortie de spectre de masse pour le Lipide A modifié par de la phosphoéthanolamine et dans lequel ledit premier pic défini est absent d'une sortie de spectre de masse correspondante pour le Lipide A natif ; et
c. dans lequel la présence dudit premier pic défini indique la présence d'une bactérie résistante à un antibiotique polypeptidique cationique cyclique, et dans lequel l'absence dudit premier pic défini indique l'absence d'une bactérie résistante à un antibiotique polypeptidique cationique cyclique.

**2.** Procédé selon la revendication 1, dans lequel ledit Lipide A modifié par de la phosphoéthanolamine fait partie intégrante d'une membrane bactérienne ou d'un fragment de celle-ci ; et/ou
dans lequel ladite analyse de spectrométrie de masse comprend une analyse de spectrométrie de masse MALDI-TOF ; et/ou
dans lequel ledit premier pic défini comprend un rapport masse sur charge (m/z) de 120 à 125 m/z unités de plus qu'un deuxième pic défini indiquant la présence d'un Lipide A natif.

**3.** Procédé selon la revendication 2, dans lequel ledit deuxième pic défini est choisi parmi le groupe constitué par :

a. un pic comprenant un rapport masse sur charge (m/z) de 1793 à 1799 m/z, de préférence 1796,2 m/z, pour *Escherichia coli, Shigella, Klebsiella pneumoniae, Salmonella enterica, Enterobacter spp.* et *Klebsiella oxytoca ;*
b. un pic comprenant un rapport masse sur charge (m/z) de 1820 à 1826 m/z, de préférence 1823,9 m/z, pour *Klebsiella pneumoniae ;*
c. un pic comprenant un rapport masse sur charge (m/z) de 1837 à 1843 m/z, de préférence 1840 m/z, pour *Klebsiella pneumoniae ;*
d. un pic comprenant un rapport masse sur charge (m/z) de 1847 à 1853 m/z, de préférence 1850 m/z, pour *Klebsiella pneumoniae ;*
e. un pic comprenant un rapport masse sur charge (m/z) de 2059 à 2065 m/z, de préférence 2062 m/z, pour *Klebsiella pneumoniae ;*
f. un pic comprenant un rapport masse sur charge (m/z) de 2075 à 2081 m/z, de préférence 2078 m/z, pour *Klebsiella pneumoniae ;*
g. un pic comprenant un rapport masse sur charge (m/z) de 1614 à 1620 m/z, de préférence 1617,2 m/z, pour *Pseudomonas aeruginosa ;*
h. un pic comprenant un rapport masse sur charge (m/z) de 1907 à 1913 m/z, de préférence 1910,3 m/z, pour *Acinetobacter baumannii ;*
i. un pic comprenant un rapport masse sur charge (m/z) de 1793 à 1799 m/z, de préférence 1796,2 m/z, pour *Salmonella spp ;*
j. un pic comprenant un rapport masse sur charge (m/z) de 1820 à 1826 m/z, de préférence 1824 m/z, pour *Salmonella spp* ; ou
k. un pic comprenant un rapport masse sur charge (m/z) de 2031 à 2037 m/z, de préférence 2034 m/z, pour

*Salmonella spp* ; et/ou
dans lequel un rapport d'intensité entre :

le premier pic défini ; et
le deuxième pic défini est au moins 0,10/1.

**4.** Procédé selon l'une quelconque des revendications précédentes, comprenant en outre l'identification dans ladite sortie de spectre de masse d'un troisième pic défini comprenant un rapport masse sur charge (m/z) de 22 à 28 m/z unités de plus qu'un deuxième pic défini indiquant la présence d'un Lipide A natif.

**5.** Procédé selon la revendication 4, dans lequel ledit deuxième pic défini est choisi parmi le groupe constitué par :

a. un pic comprenant un rapport masse sur charge (m/z) de 1793 à 1799 m/z, de préférence 1796,2 m/z, pour *Escherichia coli, Shigella, Klebsiella pneumoniae, Salmonella enterica, Enterobacter spp.* et *Klebsiella oxytoca ;*
b. un pic comprenant un rapport masse sur charge (m/z) de 1820 à 1826 m/z, de préférence 1823,9 m/z, pour *Klebsiella pneumoniae ;*
c. un pic comprenant un rapport masse sur charge (m/z) de 1837 à 1843 m/z, de préférence 1840 m/z, pour *Klebsiella pneumoniae ;*
d. un pic comprenant un rapport masse sur charge (m/z) de 1847 à 1853 m/z, de préférence 1850 m/z, pour *Klebsiella pneumoniae ;*
e. un pic comprenant un rapport masse sur charge (m/z) de 2059 à 2065 m/z, de préférence 2062 m/z, pour *Klebsiella pneumoniae ;*
f. un pic comprenant un rapport masse sur charge (m/z) de 2075 à 2081 m/z, de préférence 2078 m/z, pour *Klebsiella pneumoniae ;*
g. un pic comprenant un rapport masse sur charge (m/z) de 1793 à 1799 m/z, de préférence 1796,2 m/z, pour *Salmonella spp ;*
h. un pic comprenant un rapport masse sur charge (m/z) de 1820 à 1826 m/z, de préférence 1824 m/z, pour *Salmonella spp* ; ou
i. un pic comprenant un rapport masse sur charge (m/z) de 2031 à 2037 m/z, de préférence 2034 m/z, pour *Salmonella spp.*

**6.** Procédé selon l'une quelconque des revendications précédentes, dans lequel un rapport d'intensité entre :

le troisième pic défini ; et
le deuxième pic défini est au moins 0,15/1, ou au moins 0,6/1 ; et/ou
dans lequel un rapport entre :

la somme de l'intensité dudit premier pic défini et de l'intensité dudit troisième pic défini ; et
l'intensité dudit deuxième pic défini est au moins 0,15/1 ; de préférence au moins 0,5/1.

**7.** Procédé selon l'une quelconque des revendications précédentes, dans lequel une bactérie résistante à un antibiotique polypeptidique cationique cyclique par le biais d'une résistance codée par un plasmide comprend un plasmide ayant un gène de résistance à la colistine mobilisé ; facultativement
dans lequel ledit gène de résistance à la colistine mobilisé comprend un ou plusieurs d'un gène semblable à *mcr, mcr-1, mcr-1.1, mcr-1.2, mcr-1.3, mcr-1.4, mcr-1.5, mcr-1.6, mcr-1.7, mcr-1.8, mcr-1.9, mcr-1.10, mcr-2, mcr-2.2, mcr-3, mcr-3.2, mcr-4, mcr-5,* SEQ ID : 1, SEQ ID : 2, SEQ ID : 3, SEQ ID : 4, SEQ ID : 5, SEQ ID : 6, SEQ ID : 7, SEQ ID : 8, SEQ ID : 9, SEQ ID : 10, SEQ ID : 11, SEQ ID : 12, SEQ ID : 13, SEQ ID : 14, SEQ ID : 15, SEQ ID : 16, ou une séquence ayant au moins 50 % d'homologie avec celles-ci.

**8.** Procédé selon l'une quelconque des revendications précédentes, dans lequel ledit échantillon d'essai est mélangé avec une solution de matrice avant la soumission dudit échantillon d'essai à l'analyse de spectrométrie de masse, facultativement dans lequel ladite solution de matrice permet l'extraction sélective, la cocristallisation et l'ionisation du Lipide A natif et/ou du Lipide A modifié comme une partie intégrante d'une membrane bactérienne ; et/ou
dans lequel la solution de matrice comprend de l'acide 2,5-dihydroxybenzoïque en suspension dans un solvant organique, facultativement dans lequel ledit solvant organique comprend du chloroforme et du méthanol dans un rapport de 6/1 à 12/1, ou dans un rapport de 9/1 v/v.
facultativement dans lequel ledit solvant organique comprend le chloroforme, le méthanol, le dichlorométhane, l'éther, l'éther diéthylique, l'éther de pétrole, l'isopropanol, le butanol, l'hexane ou une combinaison de ceux-ci.

**9.** Procédé selon la revendication 8, dans lequel le rapport entre l'échantillon d'essai et la matrice est entre 0,1/1 à 2/1 v/v, ou 0,66/1 v/v.

**10.** Procédé selon l'une quelconque des revendications précédentes, dans lequel ledit échantillon d'essai comprend entre $10^1$ à $10^{10}$ cellules bactériennes ; et/ou
dans lequel ledit antibiotique polypeptidique cationique cyclique est un antibiotique de polymyxine ; de préférence dans lequel ledit antibiotique de polymyxine est un ou plusieurs parmi la Colistine (Polymyxine E), la Polymyxine B, la Mattacine (Polymyxine M), ou un sel de celles-ci.

**11.** Procédé selon l'une quelconque des revendications précédentes, dans lequel ladite bactérie est choisie parmi les genres suivants : *Escherichia, Klebsiella, Enterobacter, Pseudomonas, Acinetobacter, Shigella, Salmonella, Citrobacter, Raoultella* et des combinaisons de ceux-ci ; et/ou
dans lequel ladite bactérie est choisie parmi les espèces suivantes : *Escherichia coli, Klebsiella pneumoniae, Klebsiella oxytoca, Enterobacter aerogenes, Enterobacter cloacae, Enterobacter asburiae, Pseudomonas aeruginosa, Acinetobacter baumannii, Shigella sonnei, Shigella flexneri, Salmonella enterica, Citrobacter freundii, Citrobacter koseri, Citrobacter amalonaticus, Citrobacter youngae* et des combinaisons de celles-ci.

**12.** Procédé selon l'une quelconque des revendications précédentes, dans lequel ladite bactérie est inactivée à la chaleur ou dans lequel ladite bactérie n'est pas inactivée à la chaleur ; et/ou dans lequel ledit échantillon d'essai est traité pour éliminer le sel ; et/ou comprenant en outre l'étape d'enregistrement des données obtenues dans l'étape (a) sur un support de données adaptés.

**13.** Procédé de criblage destiné à identifier un inhibiteur de la résistance à un antibiotique polypeptidique cationique cyclique dans une bactérie, comprenant :

a. l'incubation d'un échantillon comprenant une bactérie résistante à un antibiotique polypeptidique cationique cyclique avec un inhibiteur candidat ;
b. la soumission dudit échantillon à une analyse de spectrométrie de masse selon l'une quelconque des revendications 1 à 12 et la génération d'une sortie de spectre de masse ; et
c. l'identification de la présence ou de l'absence dudit premier pic défini dans la sortie de spectre de masse ;

dans lequel la présence dudit premier pic défini indique que ledit inhibiteur candidat n'est pas une substance capable d'inhiber la résistance à un antibiotique polypeptidique cationique cyclique dans une bactérie, et dans lequel l'absence dudit premier pic défini indique que ledit inhibiteur candidat est une substance capable d'inhiber la résistance à un antibiotique polypeptidique cationique cyclique dans une bactérie.

**14.** Procédé selon la revendication 13, dans lequel ledit inhibiteur candidat est choisi parmi l'un quelconque d'un composé naturel, d'un composé chimique synthétique, d'un peptide, d'un anticorps monoclonal ou polyclonal ou d'un fragment d'anticorps, de préférence dans lequel ledit inhibiteur candidat est une substance chimique ou pharmaceutique connue choisie parmi une banque de tels inhibiteurs candidats ; et/ou dans lequel ledit échantillon provient d'un humain ou d'un animal non humain.

FIGURE 1

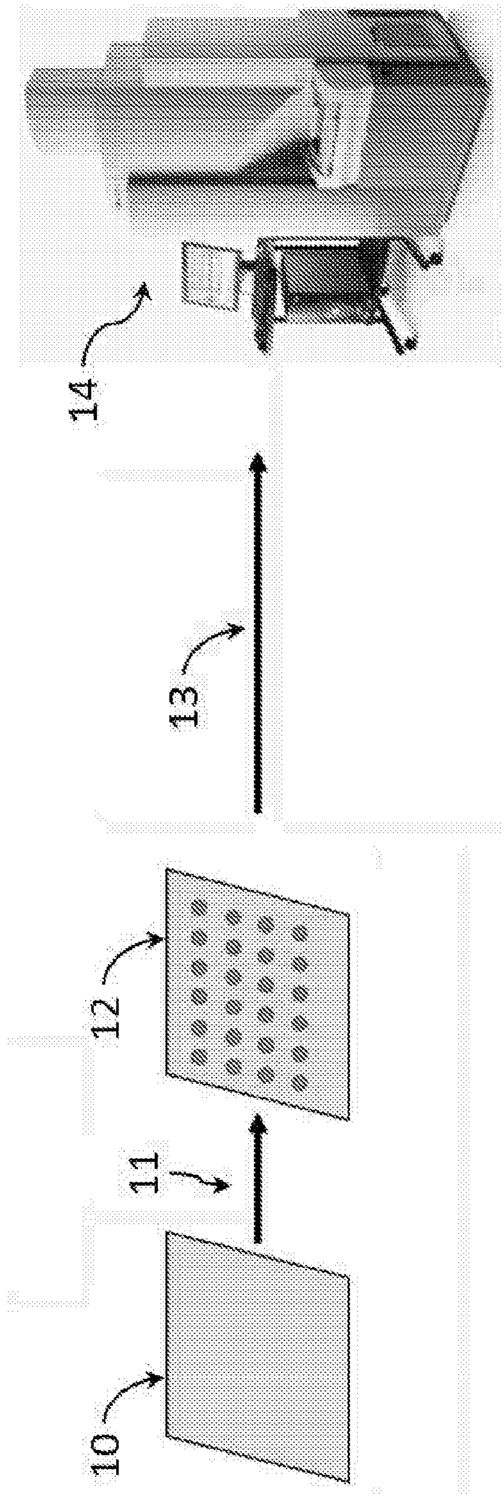

FIGURE 2A

21

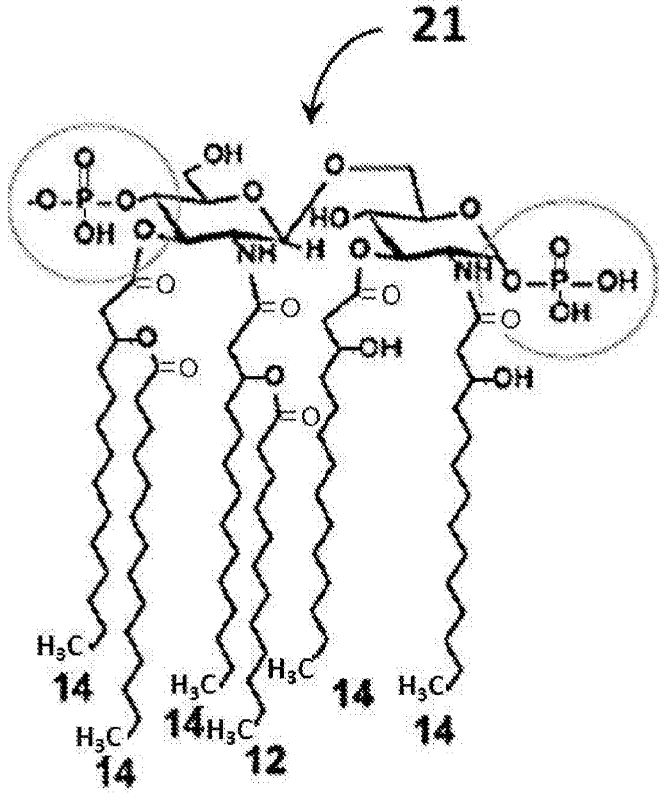

22

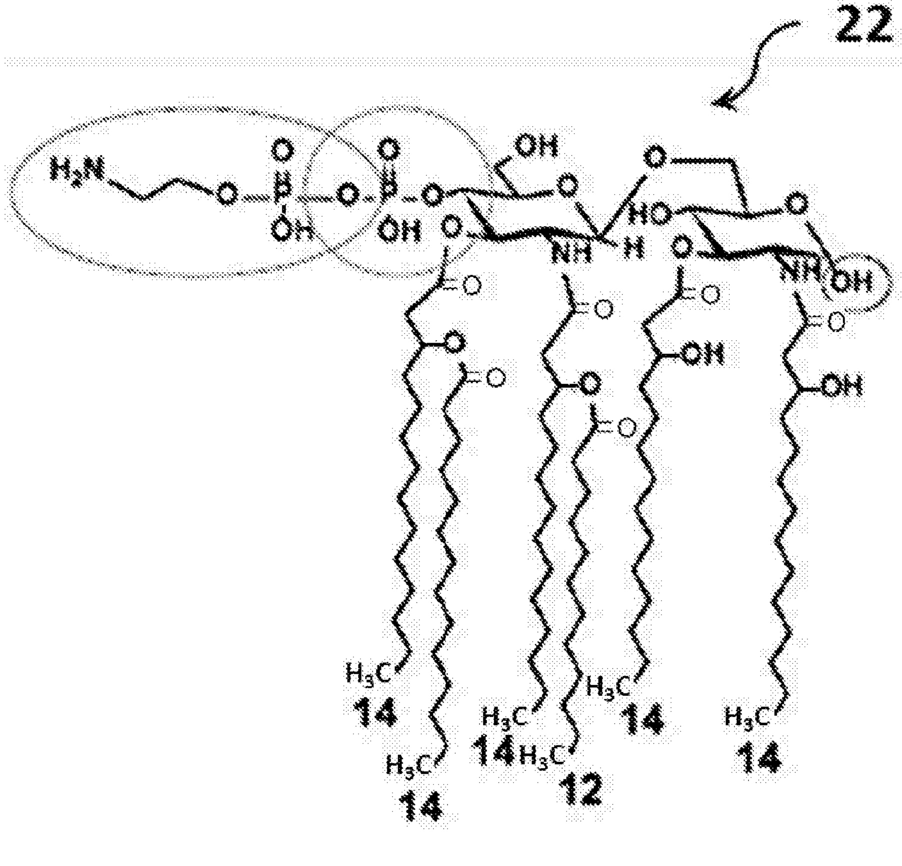

FIGURE 2B

23

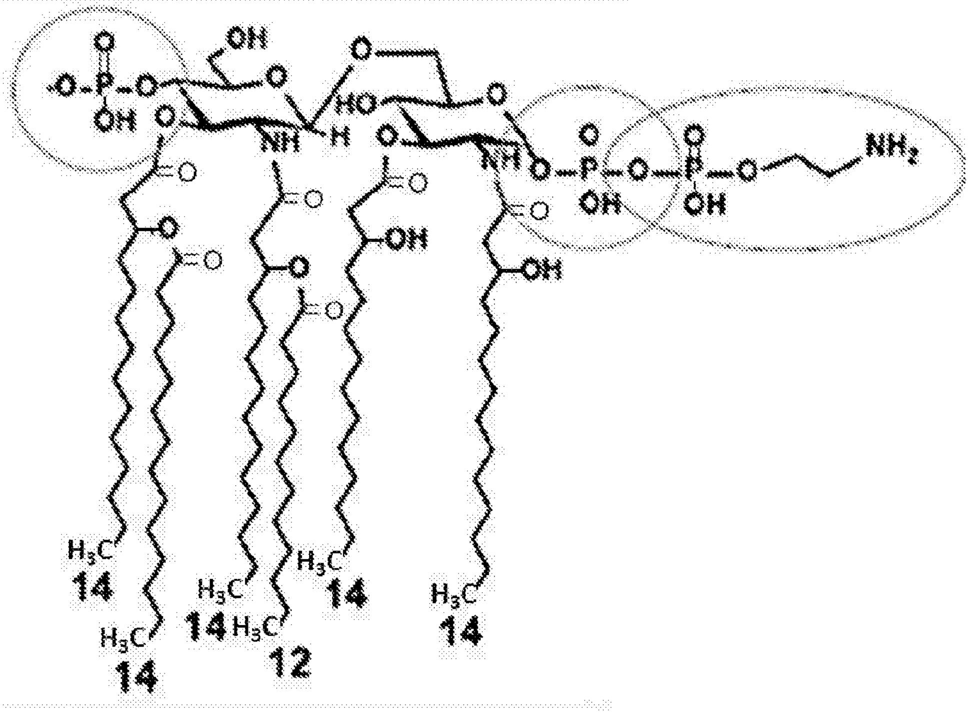

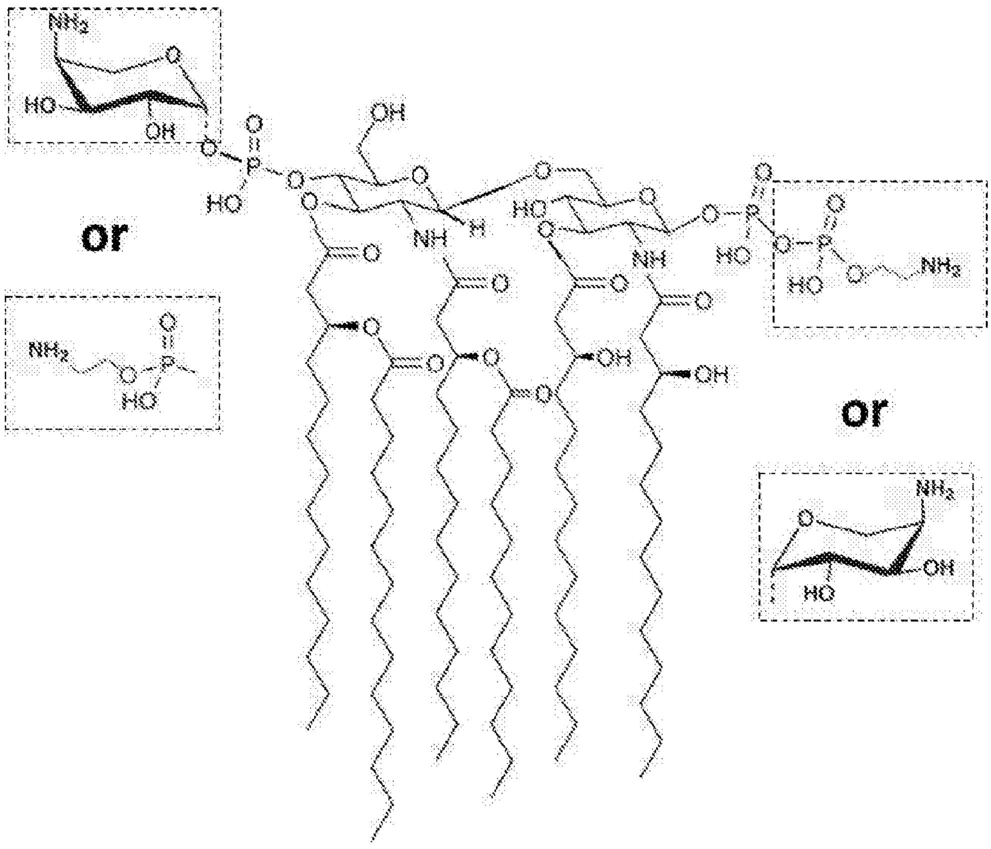

or

or

FIGURE 3

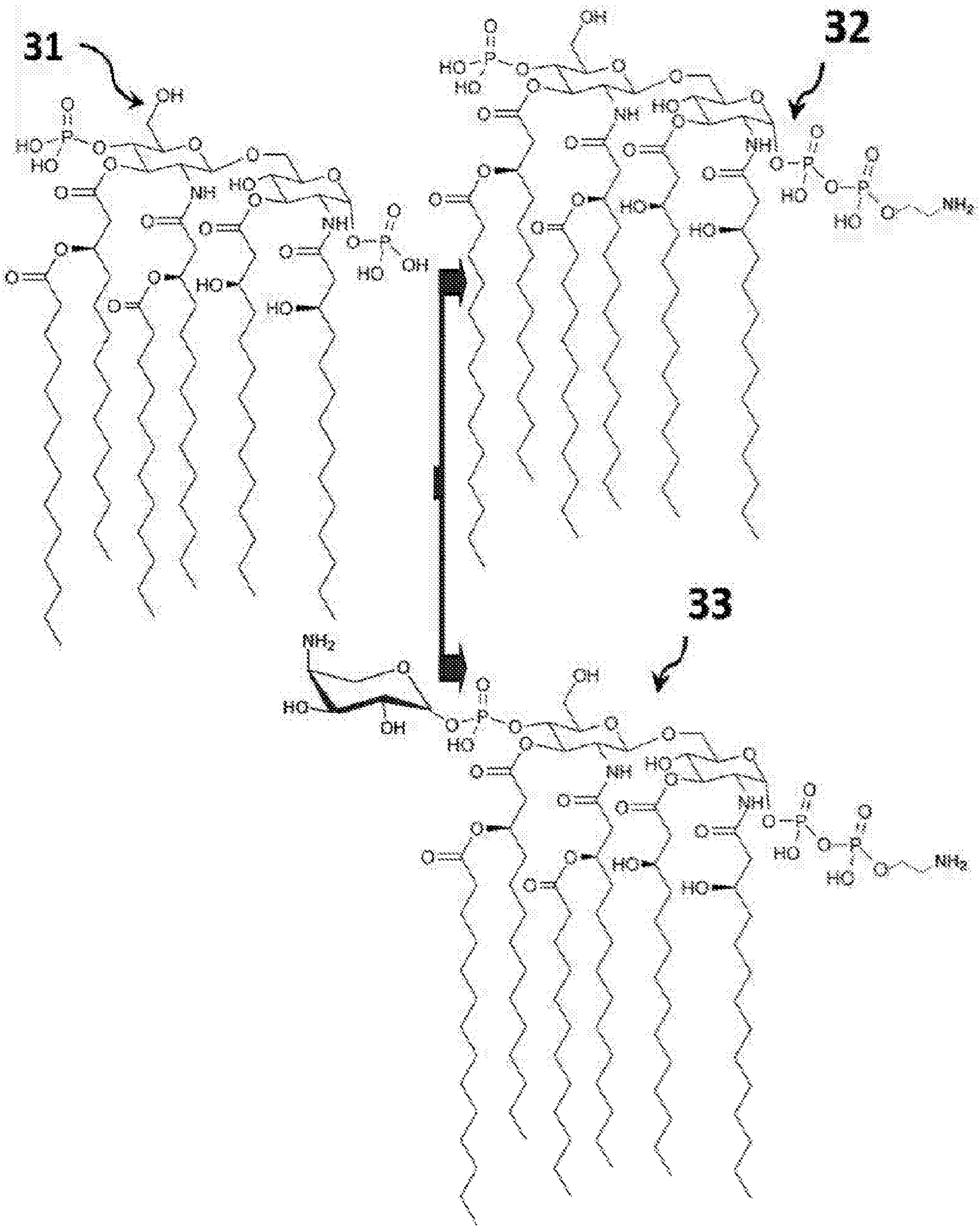

31

32

33

FIGURE 4

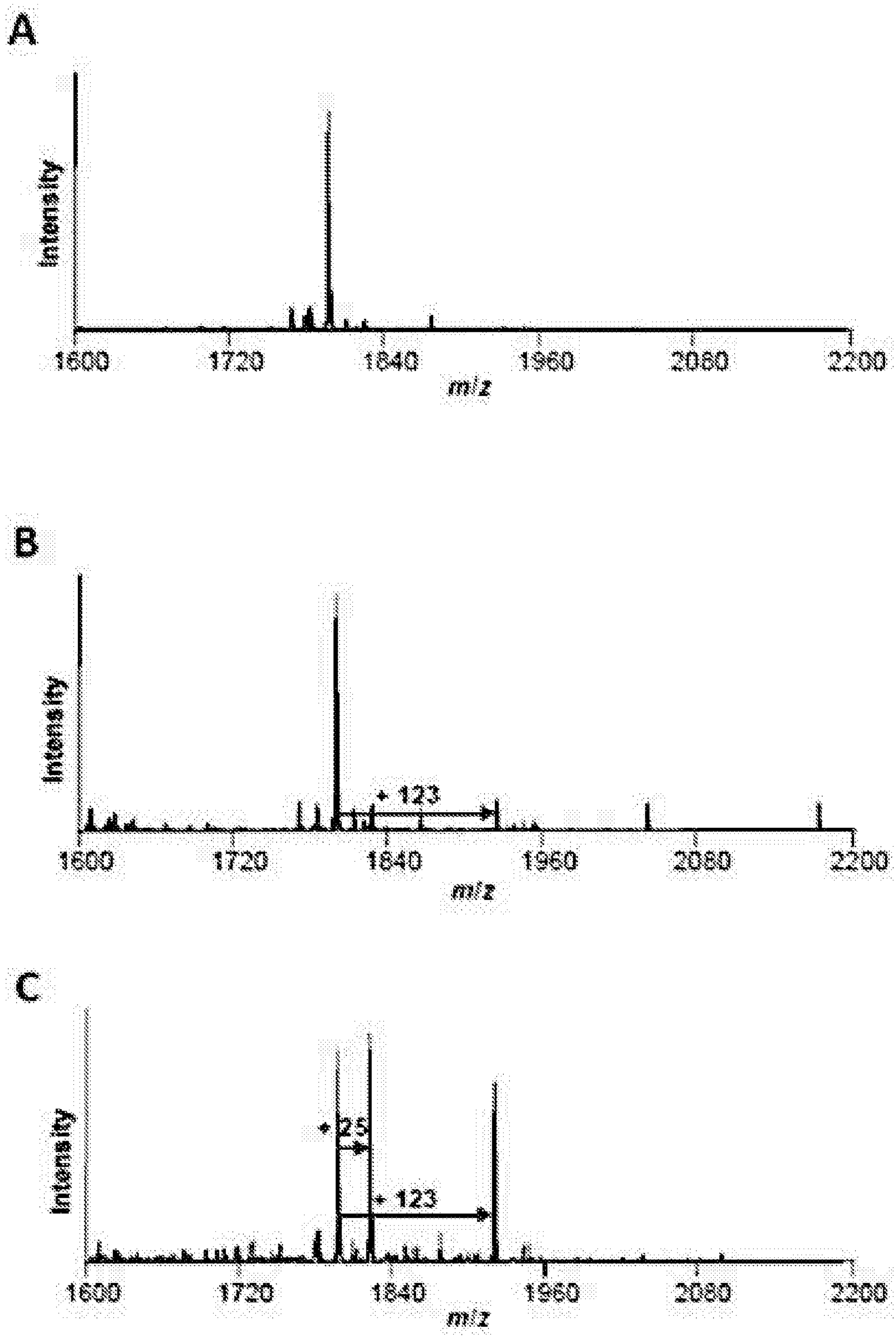
A
Intensity
1600
1720
1840
1960
2080
2200
m/z
B
Intensity
+ 123
1600
1720
1840
1960
2080
2200
m/z
C
Intensity
+ 25
+ 123
1600
1720
1840
1960
2080
2200
m/z

FIGURE 5

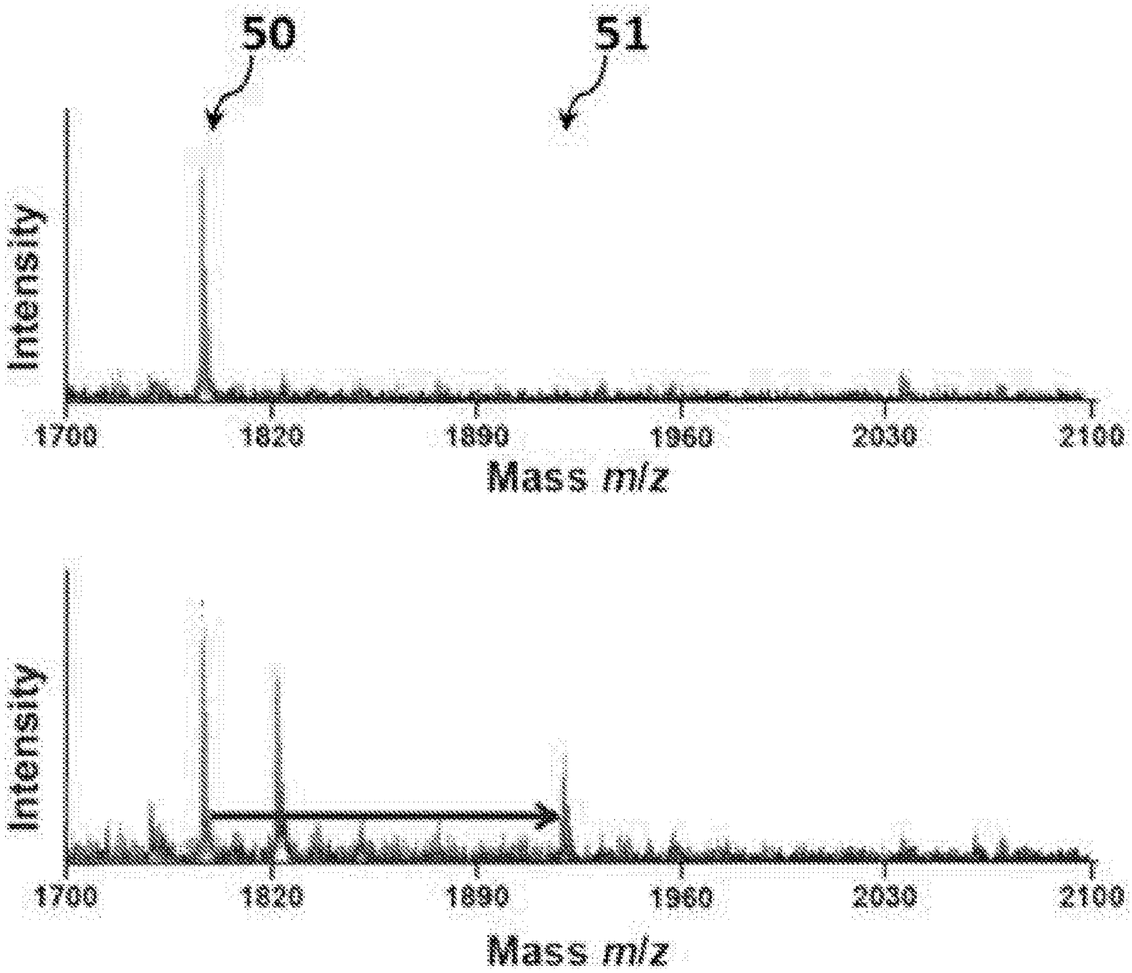
50
51
Intensity
1700
1820
1890
1960
2030
2100
Mass m/z
Intensity
1700
1820
1890
1960
2030
2100
Mass m/z

FIGURE 6

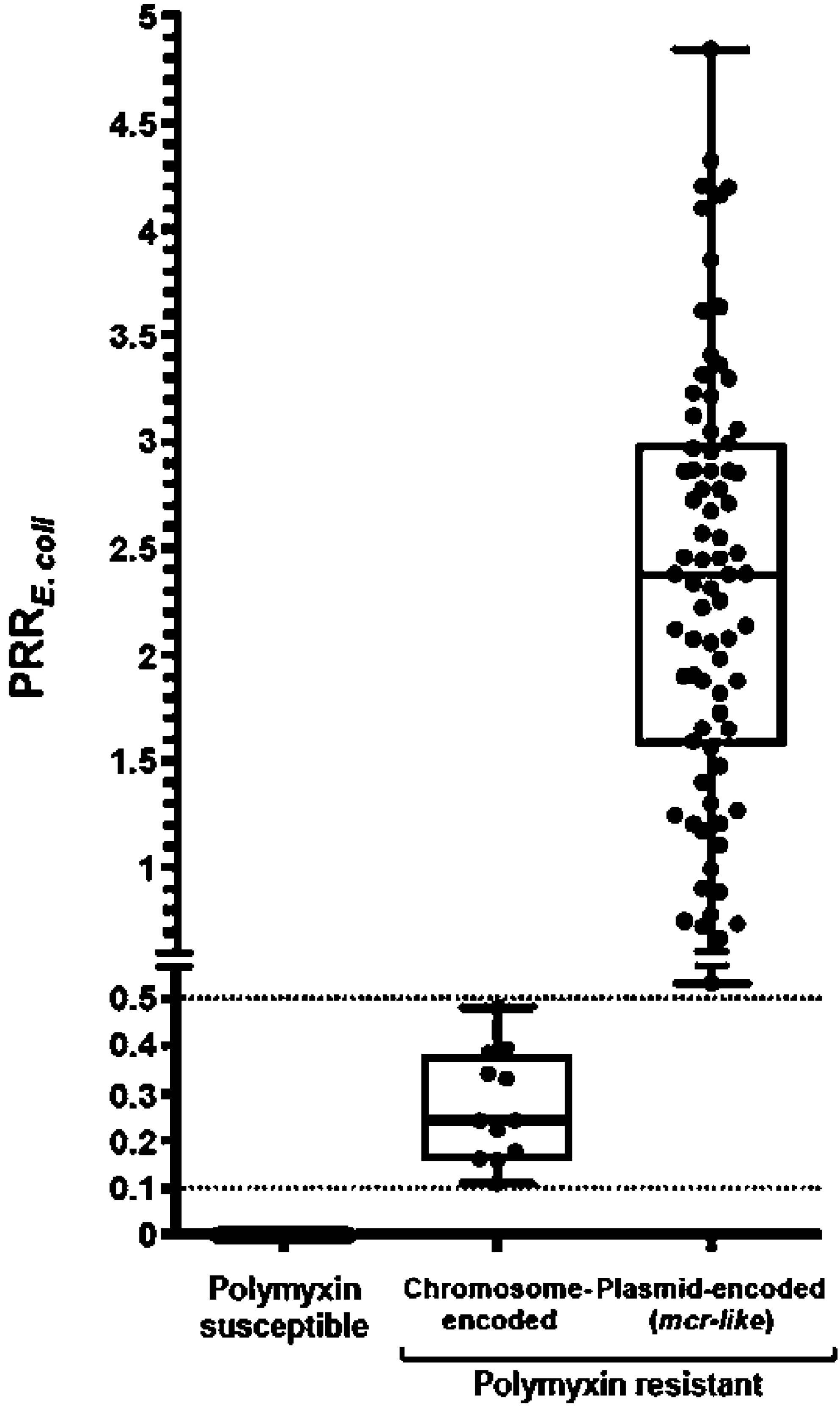
PRR E. coli
5
4.5
4
3.5
3
2.5
2
1.5
1
0.5
0.4
0.3
0.2
0.1
0
Polymyxin susceptible
Chromosome-encoded
Plasmid-encoded (mcr-like)
Polymyxin resistant

FIGURE 7

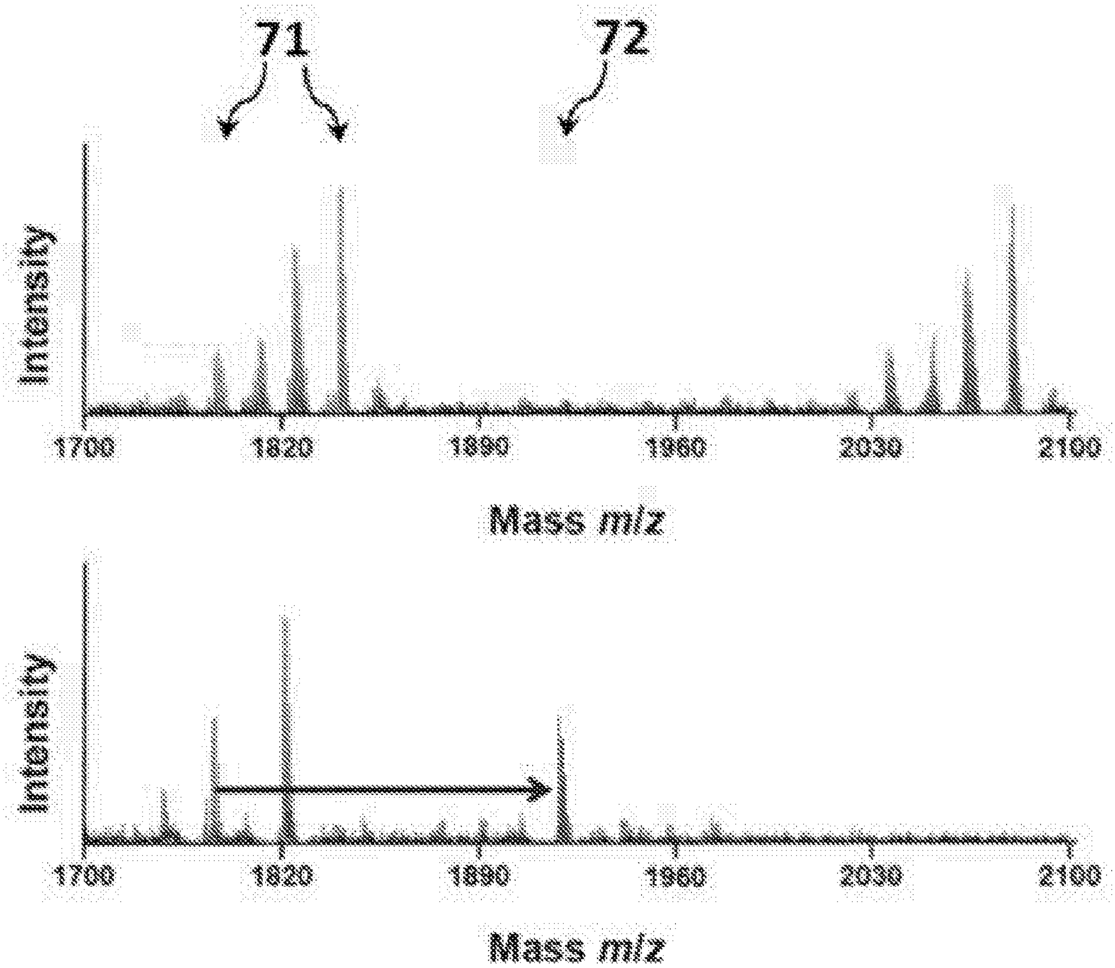
71
72
Intensity
1700
1820
1890
1960
2030
2100
Mass m/z
Intensity
1700
1820
1890
1960
2030
2100
Mass m/z

FIGURE 8

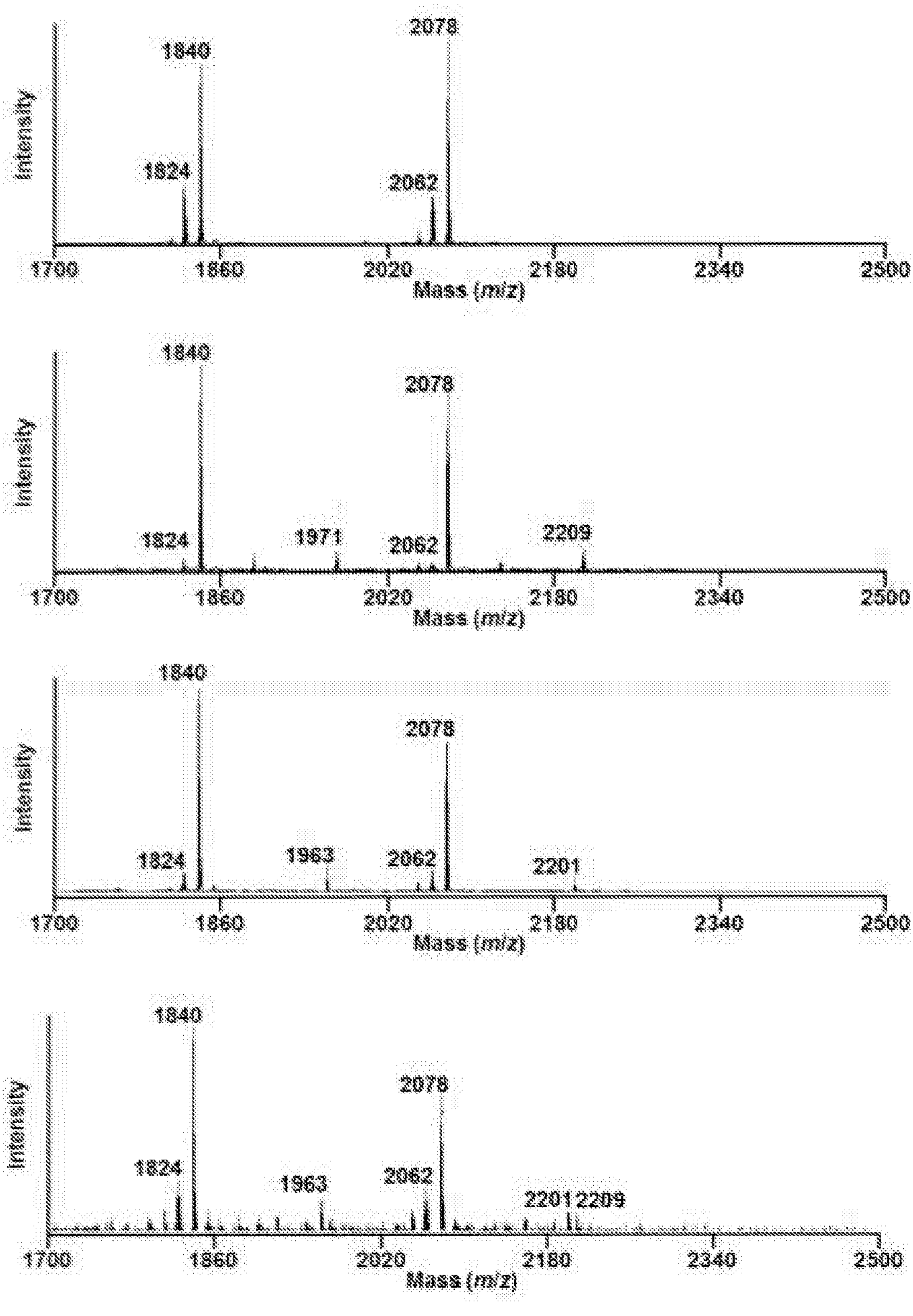
1840
2078
1824
2062
Intensity
1971
2209
1963
2201
1700
1860
2020
2180
2340
2500
Mass (m/z)

FIGURE 9

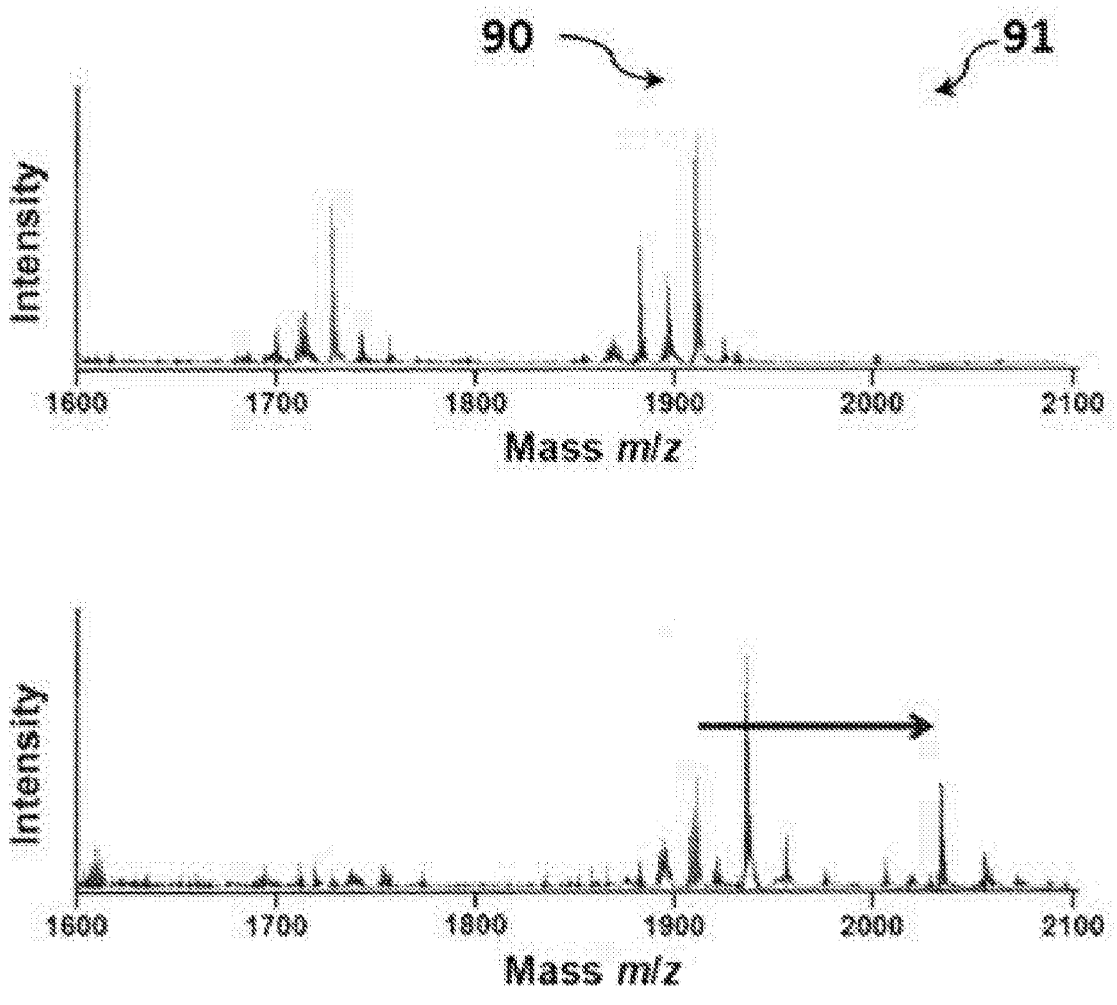
90
91
Intensity
1600
1700
1800
1900
2000
2100
Mass m/z
Intensity
1600
1700
1800
1900
2000
2100
Mass m/z

FIGURE 10

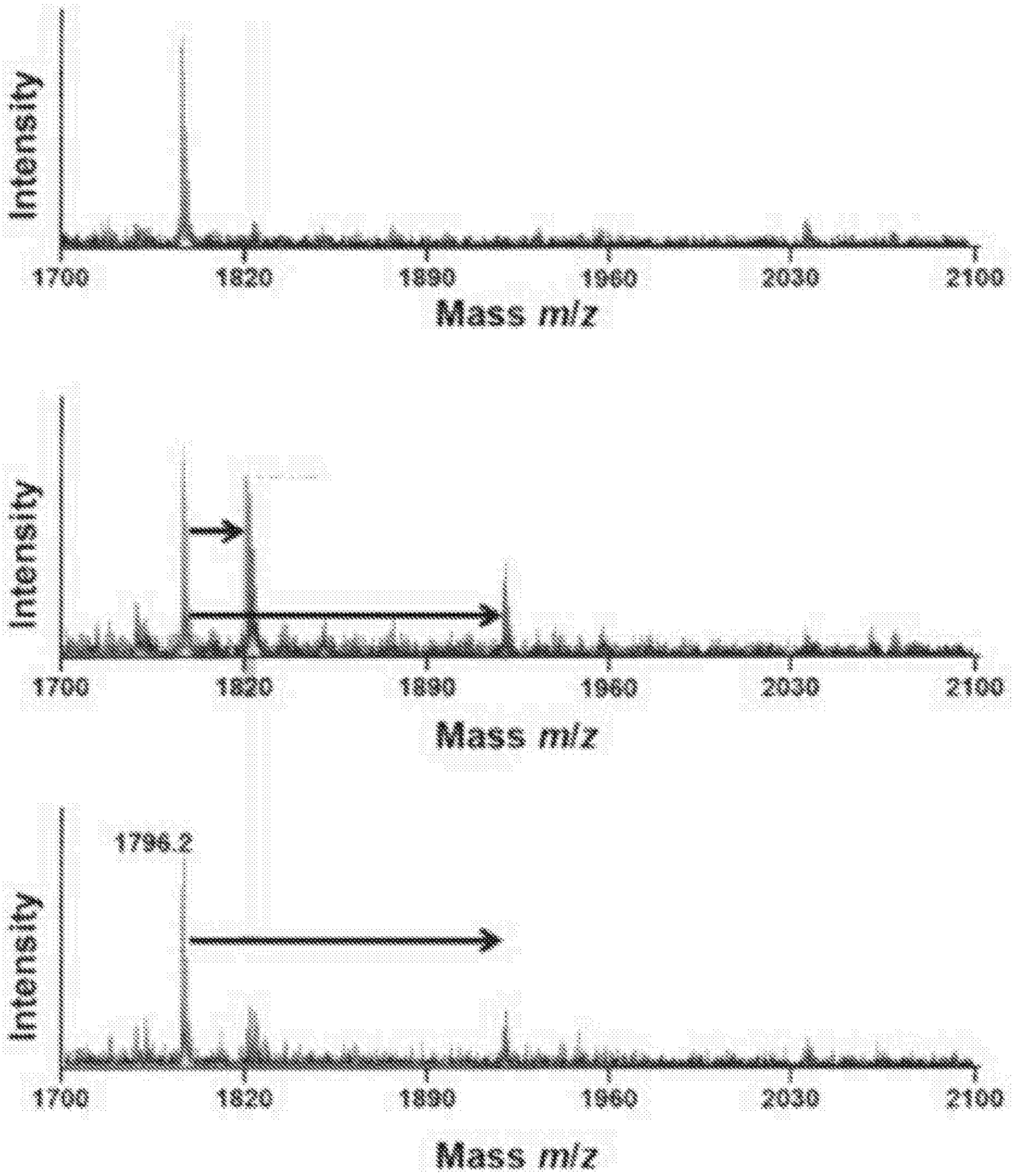
Intensity
1700
1820
1890
1960
2030
2100
Mass m/z
Intensity
1700
1820
1890
1960
2030
2100
Mass m/z
1796.2
Intensity
1700
1820
1890
1960
2030
2100
Mass m/z

FIGURE 11

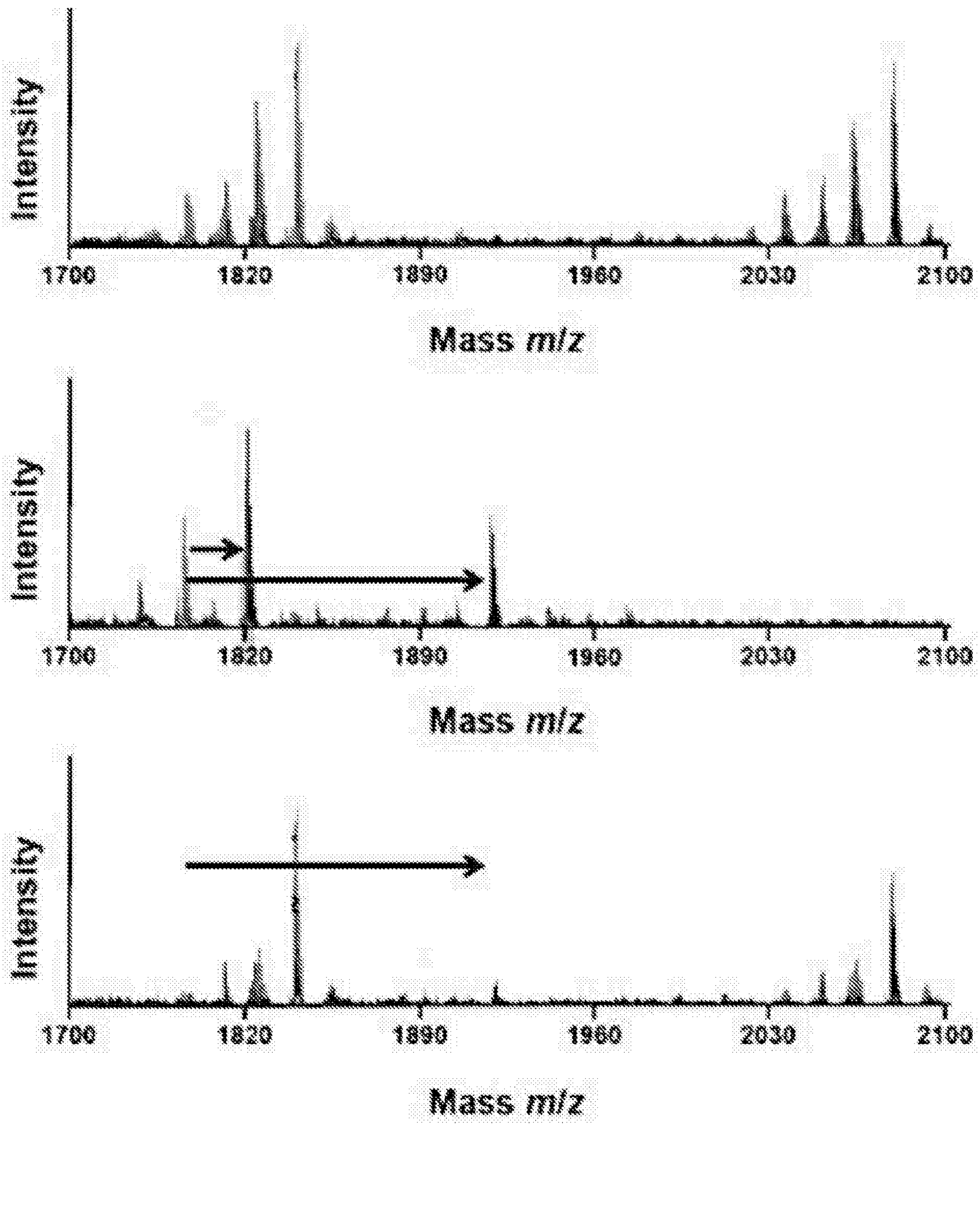
Intensity
1700
1820
1890
1960
2030
2100
Mass m/z
Intensity
1700
1820
1890
1960
2030
2100
Mass m/z
Intensity
1700
1820
1890
1960
2030
2100
Mass m/z

FIGURE 12

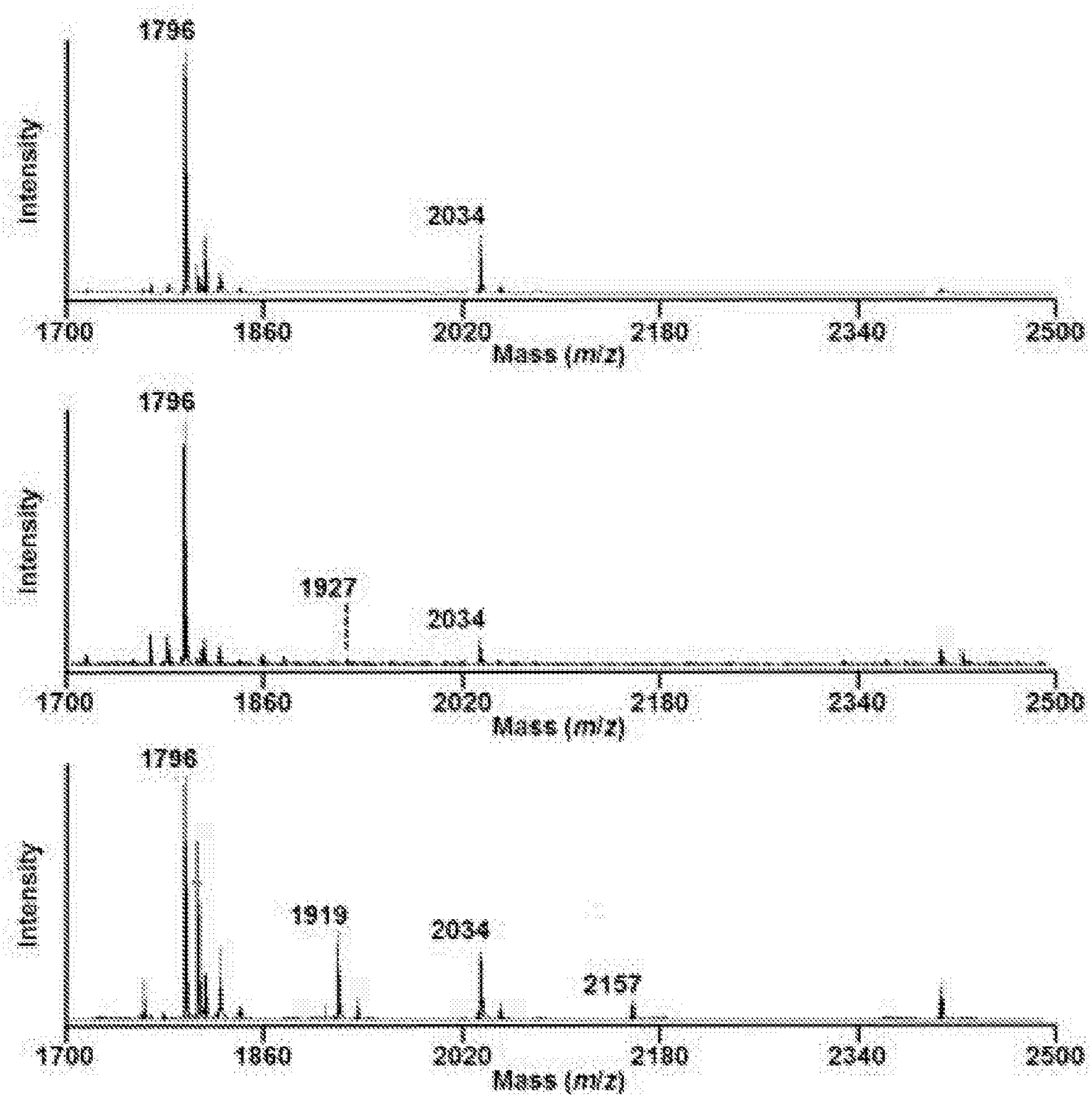
1796
2034
Intensity
1700
1860
2020
2180
2340
2500
Mass (m/z)
1796
1927
2034
Intensity
1700
1860
2020
2180
2340
2500
Mass (m/z)
1796
1919
2034
2157
Intensity
1700
1860
2020
2180
2340
2500
Mass (m/z)

FIGURE 13

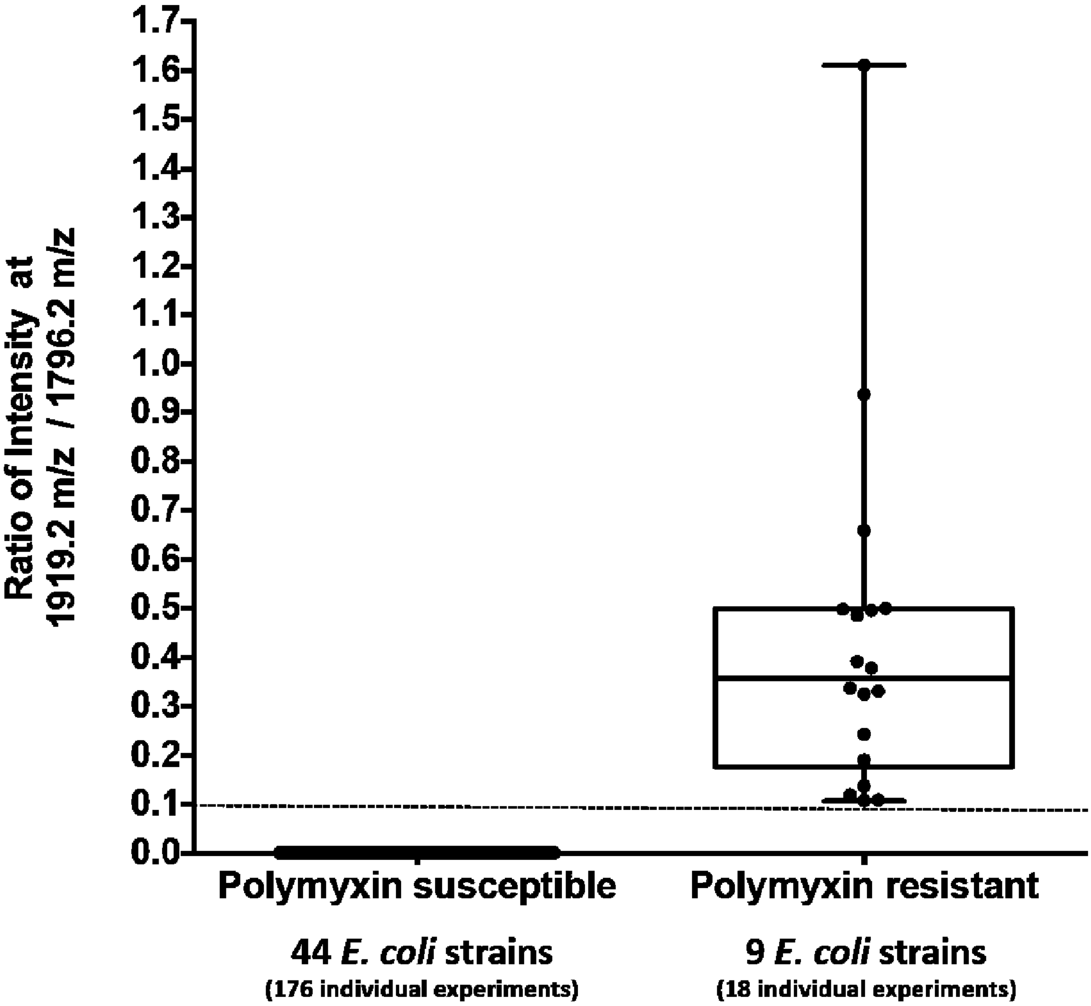

Ratio of Intensity at
1919.2 m/z / 1796.2 m/z
1.7
1.6
1.5
1.4
1.3
1.2
1.1
1.0
0.9
0.8
0.7
0.6
0.5
0.4
0.3
0.2
0.1
0.0
Polymyxin susceptible
44 E. coli strains
(176 individual experiments)
Polymyxin resistant
9 E. coli strains
(18 individual experiments)

FIGURE 14A

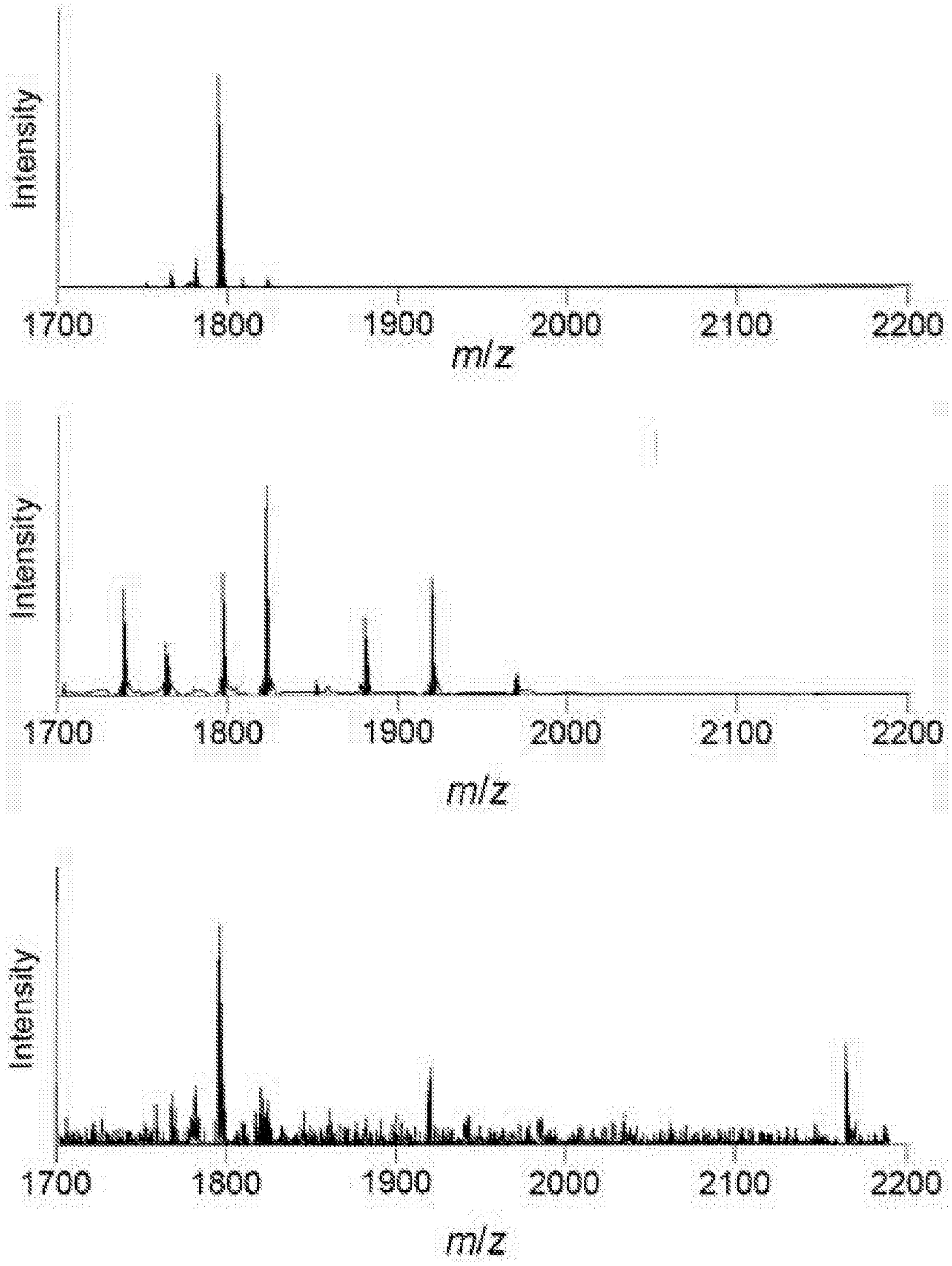
Intensity
1700
1800
1900
2000
2100
2200
m/z
Intensity
1700
1800
1900
2000
2100
2200
m/z
Intensity
1700
1800
1900
2000
2100
2200
m/z

FIGURE 14B

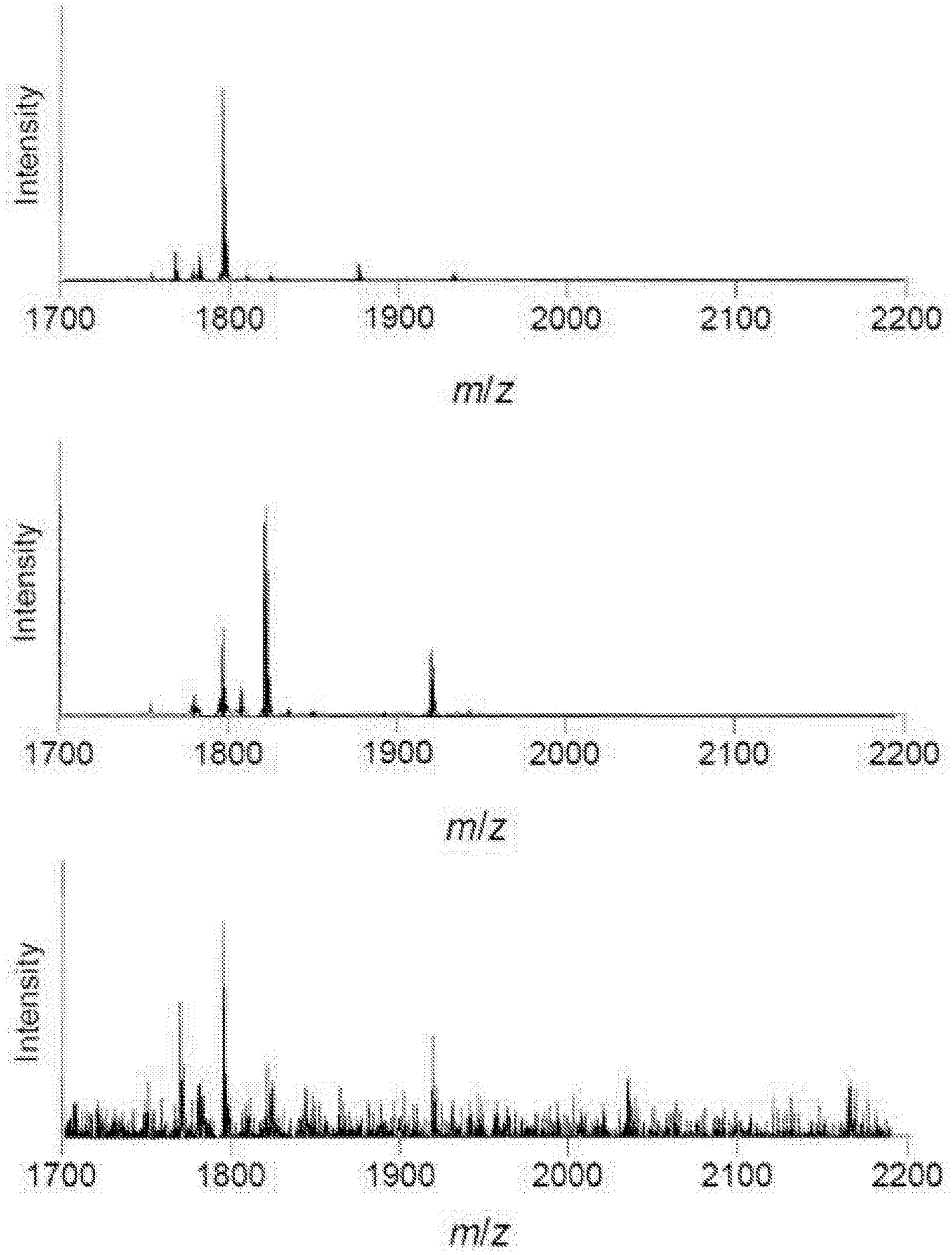
Intensity
1700
1800
1900
2000
2100
2200
m/z
Intensity
1700
1800
1900
2000
2100
2200
m/z
Intensity
1700
1800
1900
2000
2100
2200
m/z

FIGURE 14C

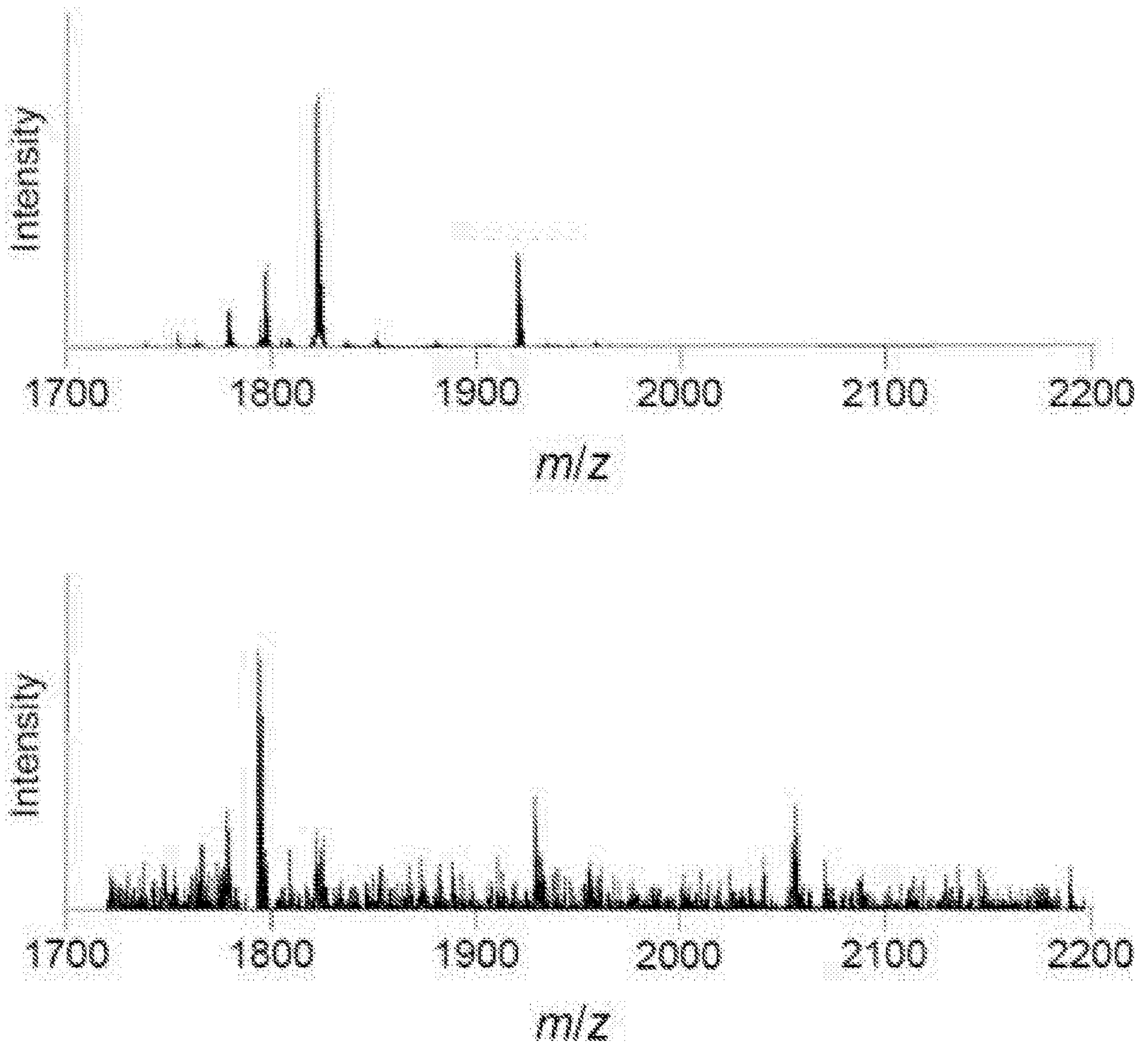
Intensity
1700
1800
1900
2000
2100
2200
m/z
Intensity
1700
1800
1900
2000
2100
2200
m/z

FIGURE 14D

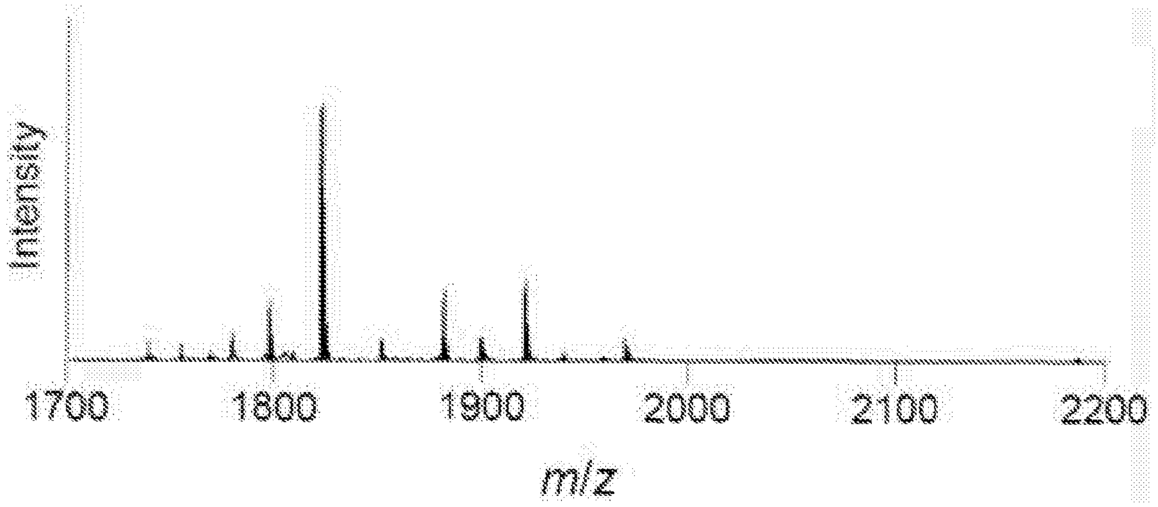
Intensity
1700
1800
1900
2000
2100
2200
m/z

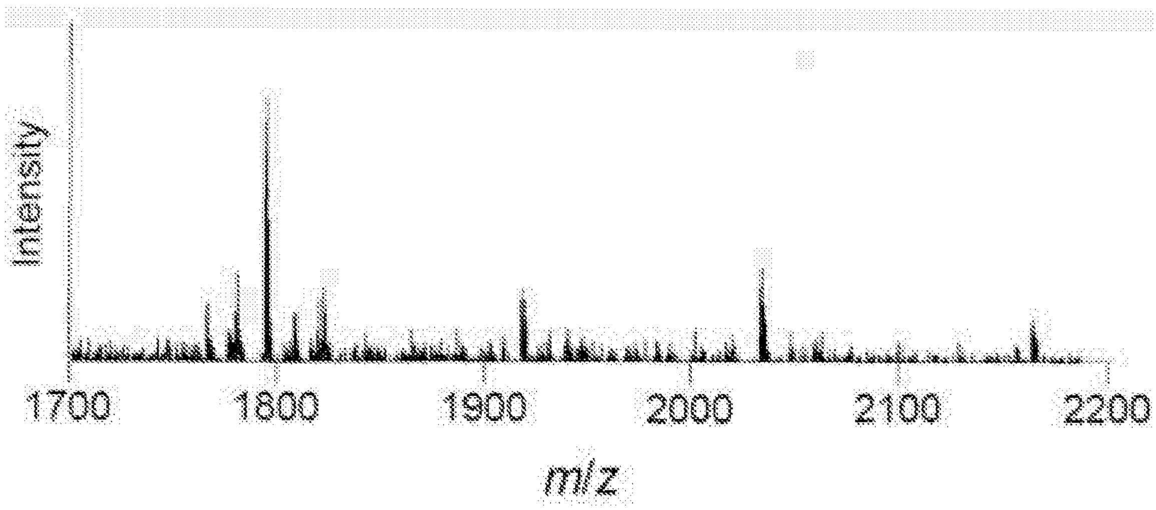
Intensity
1700
1800
1900
2000
2100
2200
m/z

FIGURE 15

| Species | susceptibility to polymixins | Resistance mechanism | Peaks | | | | | | | | |
|---|---|---|---|---|---|---|---|---|---|---|---|
| | | Chromosome - vs plasmid-encoded | 1796 | 1821 | 1824 | 1840 | 1850 | 1919 | 1910 | 1927 | 1955 |
| *E. coli* | Susceptible | | ++ | - | | - | | - | | | |
| | Resistant | Plasmid | + | ++ | | - | | ++ | | | |
| | Resistant | Chromosome | + | - | | - | | + | | | |
| *K. pneumoniae* | Susceptible | | +/- | - | + | ++ | + | - | | | - |
| | Resistant | Plasmid | + | ++ | + | - | + | ++ | | | - |
| | Resistant | Chromosome | + | - | + | ++ | + | + | | | + |
| *A. baumannii* | Susceptible | | | | | | | | ++ | | |
| | Resistant | Plasmid | | | | | | | | | |
| | Resistant | Chromosome | | | | | | | + | | |
| *Salmonella Spp.* | Susceptible | | ++ | | + | | | - | | - | |
| | Resistant | Plasmid | ++ | | + | | | + | | - | |
| | Resistant | Chromosome | ++ | | + | | | | | + | |

| Species | susceptibility to polymixins | Chromosome - vs plasmid-encoded | 1963 | 1971 | 2033 | 2034 | 2062 | 2078 | 2157 | 2193 | 2201 | 2209 |
|---|---|---|---|---|---|---|---|---|---|---|---|---|
| *E. coli* | Susceptible | | | | | | | | | | | |
| | Resistant | Plasmid | | | | | | | | | | |
| | Resistant | Chromosome | | | | | | | | | | |
| *K. pneumoniae* | Susceptible | | - | - | | | + | ++ | | - | - | - |
| | Resistant | Plasmid | + | - | | | + | ++ | | - | + | - |
| | Resistant | Chromosome | +/- | + | | | + | ++ | | + | +/- | + |
| *A. baumannii* | Susceptible | | | | - | | | | | | | |
| | Resistant | Plasmid | | | | | | | | | | |
| | Resistant | Chromosome | | | ++ | | | | | | | |
| *Salmonella Spp.* | Susceptible | | | | | + | | | - | | | |
| | Resistant | Plasmid | | | | + | | | + | | | |
| | Resistant | Chromosome | | | | + | | | | | | |

FIGURE 16

```
MCR-1.2   ATGATGCTGCATACTTCTGTGTGGTACCGACGCTCGGTCAGTCCGTTTGTTCTTGTGGC-
MCR-1.8   ATGATGCGGCATACTTCTGTGTGGTACCGACGCTCGGTCAGTCCGTTTGTTCTTGTGGC-
MCR-1     ATGATGCAGCATACTTCTGTGTGGTACCGACGCTCGGTCAGTCCGTTTGTTCTTGTGGC-
MCR-1.7   ATGATGCAGCATACTTCTGTGTGGTACCGACGCTCGGTCAGTCCGTTTGTTCTTGTGGC-
MCR-1.5   ATGATGCAGCATACTTCTGTGTGGTACCGACGCTCGGTCAGTCCGTTTGTTCTTGTGGC-
MCR-1.4   ATGATGCAGCATACTTCTGTGTGGTACCGACGCTCGGTCAGTCCGTTTGTTCTTGTGGC-
MCR-1.6   ATGATGCAGCATACTTCTGTGTGGTACCGACGCTCGGTCAGTCCGTTTGTTCTTGTGGC-
MCR-1.3   ATGATGCAGCATACTTCTGTGTGGTACCGACGCTCGGTCAGTCCGTTTGTTCTTGTGGC-
MCR-1.10  ------------------GTGTGGTACCGACGCTCGGTCAGTCCGTTTGTTCTTGTGGC-
MCR-1.9   ------------------GTGTGGTACCGACGCTCGGTCAGTCCGTTTGTTCTTGTGGC-
MCR-2.2   ------------------GTGTGGTACCGACGCTCGGTCAGTCCGTTTGTTCTTGTGGC-
MCR-2     ATGACATCACATCACTCT---TGGTATCGCTATTCTATCAATCCTTTTGTGCTGATGG--
MCR-5     ------ATGCGGTTGTCTGCATT-TATCACTTTCTTGAAAATGCGCCCGCAAGTGCGCAC
MCR-3     ----------------ATGCCTTCCCTTATAAAAATAAAAATTGTTCCGCTTATGTTCT-
MCR-3.2   ----------------ATGCCTTCCCTTATAAAAATAAAAATTGTTCCGCTTATGTTCT-
MCR-4     -------------GTGATTTCTAGATTTAAGACGTTATCGGTTAACCAATTCACTTTCA-
                               *                    *

          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAGGTCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GAGTGTTGCCGTT-TTCTTGACCGCGACCGCCAATCTTA-CCTTTTTTGATAAAATCA
          --GTTTGGTGGCGTTATTTTTGGCAGCGACAGCGAACCTGA-CATTTTTTGAAAAAGCGA
          TGAATTTTTGACTCTGTTCATCAGCCTTGTGTTCACCCTGC-TGTGCAATGGCGTGTTTT
          -----TTTTGGCACTGTATTTTGCATTTATGCTGAACTGGCGTGGAGTTCTCCATTTT-T
          -----TTTTGGCACTGTATTTTGCATTTATGCTGAACTGGCGTGGAGTTCTCCATTTT-T
          -----TCACTGCGTTGTTTTATGTTGCCATTTTCAATCTAC-CGCTCTTTGGTATAGTGC
               *       *   *  *                    *

MCR-1.2   GCCAAACCTATCCCATCGCGGACAATCTCGGCTTTGTGCTGACGATCGCTG-TCGTGCTC
MCR-1.8   GCCAAACCTATCCCATCGCGGACAATCTCGGCTTTGTGCTGACGATCGCTG-TCGTGCTC
MCR-1     GCCAAACCTATCCCATCGCGGACAATCTCGGCTTTGTGCTGACGATCGCTG-TCGTGCTC
MCR-1.7   GCCAAACCTATCCCATCGCGGACAATCTCGGCTTTGTGCTGACGATCGCTG-TCGTGCTC
MCR-1.5   GCCAAACCTATCCCATCGCGGACAATCTCGGCTTTGTGCTGACGATCGCTG-TCGTGCTC
MCR-1.4   GCCAAACCTATCCCATCGCGGACAATCTCGGCTTTGTGCTGACGATCGCTG-TCGTGCTC
MCR-1.6   GCCAAACCTATCCCATCGCGGACAATCTCGGCTTTGTGCTGACGATCGCTG-TCGTGCTC
MCR-1.3   GCCAAACCTATCCCATCGCGGACAATCTCGGCTTTGTGCTGACGATCGCTG-TCGTGCTC
MCR-1.10  GCCAAACCTATCCCATCGCGGACAATCTCGGCTTTGTGCTGACGATCGCTG-TCGTGCTC
MCR-1.9   GCCAAACCTATCCCATCGCGGACAATCTCGGCTTTGTGCTGACGATCGCTG-TCGTGCTC
MCR-2.2   GCCAAACCTATCCCATCGCGGACAATCTCGGCTTTGTGCTGACGATCGCTG-TCGTGCTC
MCR-2     TGGCGGTCTATCCTGTATCGGATAACTTAGGCTTTATCATCTCAATGCCGG-TGGCGGTG
MCR-5     GGAATGCCCTTCTTGCTGGACGCGACT-----CCCTAACTTCTGGAACATGGCTAATGCTC
MCR-3     ACGAAATCCTTTACAAATTAGAAGATTTAAGTTTGGTTTCGCCATTTCAT-TACCAATA
MCR-3.2   ACGAAATCCTTTACAAATTAGAAGATTTAAGTTTGGTTTCGCCATTTCAT-TACCAATA
MCR-4     GAAAAGGAATTGAAAAACAACCAGAAGTTGATCCCCTTTTCATCGCATCTA-TGCCGCTA
                      *              *              *          *    *
```

FIGURE 16 (continued)

```
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         TTTGGCGCGATGCTACTGATCACCACGCTGTTATCATCGT-----------ATCGCTATG
         ATGGGTGCTATGCTACTGATTGTCGTGCTGTTATC---CT-----------ATCGCTATG
         CTTTGCACTGGGTTGCTGATCACCGGGCTGCAATGGTTGTTGCTCCTTCTGGTGGCCACG
         TTGCTTGTTGCAGCGCTTAACTTTGTATTTGTTCCATTTT--------CGATACGGTATT
         TTGCTTGTTGCAGCGCTTAACTTTGTATTTGTTCCATTTT--------CGATACGGTATT
         TTTTTAACATTTGCGCTGAGTTTTTTGTTTTCAATTTTTA--------CCGTCAAATACC
          *             ** *         *                            *
MCR-1.2  TGCTA-------AAGCCTGTGTTGATTTTGCTATTAATCATGGGCGCGGTGACCAGTTATT
MCR-1.8  TGCTA-------AAGCCTGTGTTGATTTTGCTATTAATCATGGGCGCGGTGACCAGTTATT
MCR-1    TGCTA-------AAGCCTGTGTTGATTTTGCTATTAATCATGGGCGCGGTGACCAGTTATT
MCR-1.7  TGCTA-------AAGCCTGTGTTGATTTTGCTATTAATCATGGGCGCGGTGACCAGTTATT
MCR-1.5  TGCTA-------AAGCCTGTGTTGATTTTGCTATTAATCATGGGCGCGGTGACCAGTTATT
MCR-1.4  TGCTA-------AAGCCTGTGTTGATTTTGCTATTAATCATGGGCGCGGTGACCAGTTATT
MCR-1.6  TGCTA-------AAGCCTGTGTTGATTTTGCTATTAATCATGGGCGCGGTGACCAGTTATT
MCR-1.3  TGCTA-------AAGCCTGTGTTGATTTTGCTATTAATCATGGGCGCGGTGACCAGTTATT
MCR-1.10 TGCTA-------AAGCCTGTGTTGATTTTGCTATTAATCATGGGCGCGGTGACCAGTTATT
MCR-1.9  TGCTA-------AAGCCTGTGTTGATTTTGCTATTAATCATGGGCGCGGTGACCAGTTATT
MCR-2.2  TGCTA-------AAGCCTGTGTTGATTTTGCTATTAATCATGGGCGCGGTGACCAGTTATT
MCR-2    TGCTA-------AAGCCTGTCCTGATTTTGCTACTGATTATGGGTGCGGTGACGAGCTATT
MCR-5    CGCTGGAGTGTCAAGCCACTACTGATTCTGCTTGCTGTCATGACGCCCGCCGCCGTTTATT
MCR-3    TAATA-------AAGCCTTTTTTTGCACTTCTTATCGCACTTAGTGCAATCGTTAGTTACA
MCR-3.2  TAATA-------AAGCCTTTTTTTGCACTTCTTATCGCACTTAGTGCAATCGTTAGTTACA
MCR-4    TGCTG-------AAGCCCTTTTTTATCCTATTGACGTTACTTTCCTCAAGTGTATTTTTTG
            *        *****  *  *     *  *        *     *          *
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACTGACACTTATGGCACGGTCTATGATACGACCATGCTCCAAAATGCCCTACAGACC
         TTACCGATACTTATGGCACGGTCTATGACACCACCATGCTCCAAAATGCCATGCAAACC
         TCATGCGCAACTACGGGGTTTATCTCGACAAGGCCATGCTGCGGAATCTGATGGAGACG
         CAATGATGAAGTATAGAGTCTTGTTTGATCAAAACATGATTCAGAATATTTTTGAAACC
         CAATGATGAAGTATAGAGTCTTGTTTGATCAAAACATGATTCAGAATATTTTTGAAACC
         CAGCCTATCAATACAATGTCGTGTTTGACTACGGCATGATAGAAAACACGTTTCAAACA
                    **             **      **** *    **     *  * **
```

FIGURE 16 (continued)

```
MCR-1.2   GACCAAGCCGAGACCAAGGATCTATTAAACGCAGCGTTTATCATGCGTATCATTGGTTTGG
MCR-1.8   GACCAAGCCGAGACCAAGGATCTATTAAACGCAGCGTTTATCATGCGTATCATTGGTTTGG
MCR-1     GACCAAGCCGAGACCAAGGATCTATTAAACGCAGCGTTTATCATGCGTATCATTGGTTTGG
MCR-1.7   GACCAAGCCGAGACCAAGGATCTATTAAACGCAGCGTTTATCATGCGTATCATTGGTTTGG
MCR-1.5   GACCAAGCCGAGACCAAGGATCTATTAAACGCAGCGTTTATCATGCGTATCATTGGTTTGG
MCR-1.4   GACCAAGCCGAGACCAAGGATCTATTAAACGCAGCGTTTATCATGCGTATCATTGGTTTGG
MCR-1.6   GACCAAGCCGAGACCAAGGATCTATTAAACGCAGCGTTTATCATGCGTATCATTGGTTTGG
MCR-1.3   GACCAAGCCGAGACCAAGGATCTATTAAACGCAGCGTTTATCATGCGTATCATTGGTTTGG
MCR-1.10  GACCAAGCCGAGACCAAGGATCTATTAAACGCAGCGTTTATCATGCGTATCATTGGTTTGG
MCR-1.9   GACCAAGCCGAGACCAAGGATCTATTAAACGCAGCGTTTATCATGCGTATCATTGGTTTGG
MCR-2.2   GACCAAGCCGAGACCAAGGATCTATTAAACGCAGCGTTTATCATGCGTATCATTGGTTTGG
MCR-2     GACCAAGCCGAGTCTAAGGACTTGATGAATTTGGCGTTTTTTGTGCGAATTATCGGCTTG
MCR-5     GACGTCAGGGAAGCCAGTGAGCTGTTGCA-ATGGAGAATGCTGCCCTACTTGTTGGTTGCA
MCR-3     AATCAAAATGAGGCGTTAGCATATTTAAGCTTACCAATTATAGTATGGGTTACTATTGCTG
MCR-3.2   AATCAAAATGAGGCGTTAGCATATTTAAGCTTACCAATTATAGTATGGGTTACTATTGCTG
MCR-4     CATCCTGCTGAAGCATTGATGTATGTAAATCTTGCATCAATTAC-CAATCTACTGCTGACT
           *       **  *           *
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GTG-TGCTACCAAGTTTGCTTGTGGCTTTTGTTAAGGTGG---ATTATCCGACTTGGGG
          GCG-TGTTGCCAAGTGTGTTGGTCGCAGTTGCCAAAGTCA---ATTATCCAACATGGGG
          GC--CGTATCCGTGTG-GTGGATTGCGAGAGTCAGGTTTT---ACGAACGGGCTGGAAA
          GTTTTATCCCTGCCATTTACTTTTCTTTG-TTGAAATTGAATATGAGGAAAAATGGTT
          GTTTTATCCCTGCCATTTACTTTTCTTTG-TTGAAATTGAATATGAGGAAAAATGGTT
          GGCTATTACCGTCATATCTTATTTATAAGGCCGATATTCATTATCAGCC---CTTTTT
          *     *               *              *     *  *
MCR-1.2   CAAGGGTT--TGATGCGCCGATTGGGCTTGATCGTGGCAAGTCTTGCGCTGATTTACTGC
MCR-1.8   CAAGGGTT--TGATGCGCCGATTGGGCTTGATCGTGGCAAGTCTTGCGCTGATTTACTGC
MCR-1     CAAGGGTT--TGATGCGCCGATTGGGCTTGATCGTGGCAAGTCTTGCGCTGATTTACTGC
MCR-1.7   CAAGGGTT--TGATGCGCCGATTGGGCTTGATCGTGGCAAGTCTTGCGCTGATTTACTGC
MCR-1.5   CAAGGGTT--TGATGCGCCGATTGGGCTTGATCGTGGCAAGTCTTGCGCTGATTTACTGC
MCR-1.4   CAAGGGTT--TGATGCGCCGATTGGGCTTGATCGTGGCAAGTCTTGCGCTGATTTACTGC
MCR-1.6   CAAGGGTT--TGATGCGCCGATTGGGCTTGATCGTGGCAAGTCTTGCGCTGATTTACTGC
MCR-1.3   CAAGGGTT--TGATGCGCCGATTGGGCTTGATCGTGGCAAGTCTTGCGCTGATTTACTGC
MCR-1.10  CAAGGGTT--TGATGCGCCGATTGGGCTTGATCGTGGCAAGTCTTGCGCTGATTTACTGC
MCR-1.9   CAAGGGTT--TGATGCGCCGATTGGGCTTGATCGTGGCAAGTCTTGCGCTGATTTACTGC
MCR-2.2   CAAGGGTT--TGATGCGCCGATTGGGCTTGATCGTGGCAAGTCTTGCGCTGATTTACTGC
MCR-2     CAAAGGTC--TGATTCAGCGTGCGATGACATGGGGTGTCAGCCTTGTGCTGTTGCTTGTGC
MCR-5     CAAGCGGTAATGATGCGCAGCGCTTGTCTGGCTGGCGC---TCTCGCCATGATTTCCATGG
MCR-3     CAAAGGGA--TTCTAACTCGTGCCCTATCGATGTTTGCATCACTTATAGTGATTGCGGTTA
MCR-3.2   CAAAGGGA--TTCTAACTCGTGCCCTATCGATGTTTGCATCACTTATAGTGATTGCGGTTA
MCR-4     TAAGGAGT--TATTGCATAAATTAGCCTTTATGCTGCTAATGTTCGTTGGCATTGGATAG
           **       *  *                             *        *     *
```

FIGURE 16 (continued)

```
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CTGTGGTGGCGTTCAGCAGTCATTATGCCAGTTTCTTTCGCGTGCATAAGCCGCTGCGT
           CGATTGGACTATTTAGCAGTCAGTATGCGAGTTTCTTTCGGGTGCATAAGCCAGTGCGT
           GTCTGTGGCCAGTCATGGATGTGCTGATACCCACGCTTCGTGAAAACAAGCCGCTTCGC
           TTGCAGCACTATACTATCAAGATTATGTGTCAGTGGGGCGCAACAATTCAAACCTCCAG
           TTGCAGCACTATACTATCAAGATTATGTGTCAGTGGGGCGCAACAATTCAAACCTCCAG
           TCGCCTTTTTTTACTATCAAGATTATGCTGCATTTGTTCGAAACAACAGTGAGTTAAGG
                                                   **     *       *

MCR-1.2    AGCTATGTCAATCCGATCATGCCAATCTACTCGGTGGGTAAGCTTGCCAGTATTGAGTATA
MCR-1.8    AGCTATGTCAATCCGATCATGCCAATCTACTCGGTGGGTAAGCTTGCCAGTATTGAGTATA
MCR-1      AGCTATGTCAATCCGATCATGCCAATCTACTCGGTGGGTAAGCTTGCCAGTATTGAGTATA
MCR-1.7    AGCTATGTCAATCCGATCATGCCAATCTACTCGGTGGGTAAGCTTGCCAGTATTGAGTATA
MCR-1.5    AGCTATGTCAATCCGATCATGCCAATCTACTCGGTGGGTAAGCTTGCCAGTATTGAGTATA
MCR-1.4    AGCTATGTCAATCCGATCATGCCAATCTACTCGGTGGGTAAGCTTGCCAGTATTGAGTATA
MCR-1.6    AGCTATGTCAATCCGATCATGCCAATCTACTCGGTGGGTAAGCTTGCCAGTATTGAGTATA
MCR-1.3    AGCTATGTCAATCCGATCATGCCAATCTACTCGGTGGGTAAGCTTGCCAGTATTGAGTATA
MCR-1.10   AGCTATGTCAATCCGATCATGCCAATCTACTCGGTGGGTAAGCTTGCCAGTATTGAGTATA
MCR-1.9    AGCTATGTCAATCCGATCATGCCAATCTACTCGGTGGGTAAGCTTGCCAGTATTGAGTATA
MCR-2.2    AGCTATGTCAATCCGATCATGCCAATCTACTCGGTGGGTAAGCTTGCCAGTATTGAGTATA
MCR-2      TTTTATATCAACCCGATTACGCCGATTTATTCGGTGGGTAAGCTTGCCAGTATCGAGTACA
MCR-5      TATTTGATCACTCCTGCAAACTACGTCATCTCGGGCATTCGGGTTTTGACTGAACAG-GCG
MCR-3      CGTGAGATTGTTCCAGCCAATTTCGTTAATAGTACCGTTAAATACGTTTACAATCGTTATC
MCR-3.2    CGTGAGATTGTTCCAGCCAATTTCGTTAATAGTACCGTTAAATACGTTTACAATCGTTATC
MCR-4      CGTTACATTGTCCCTACCTATTTTGTCAGTAGTGCATCTAAATATCTCAATGAGCACTAT-
                   *    **           *                 *

           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTACGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCAGTGCGCCAAAAGATACCATTTATCACGCCAAAGACGCGGTACAAGCAACC
           AAAAAGCCACTGCGCCAACAGACACCATCTATCATGCCAAAGACGCCGTGCAGACCACC
           TCATCGTCAGCAGACGAAGCAAGGGAAGTCGTTGCAGCCGATGCGCATCGAGGGCCTCA
           TTGCTGA-ACCAATCCCATTT--ACAACTTTAGGTG---ATGATGCAAAACGGGATAC-
           TTGCTGA-ACCAATCCCATTT--ACAACTTTAGGTG---ATGATGCAAAACGGGATAC-
           TTGCAGACGCCCATGGAATACCAACAACTTGGCCTA---GATGCGAAGAATGCCAGTCG
               *         *       *              *           *
```

FIGURE 16 (continued)

```
MCR-1.2   AAGCCTGATATGCGTAAGCCACGCCTAGTGGTGTTCGTCGTCGGTGAGACGGCACGCGCCG
MCR-1.8   AAGCCTGATATGCGTAAGCCACGCCTAGTGGTGTTCGTCGTCGGTGAGACGGCACGCGCCG
MCR-1     AAGCCTGATATGCGTAAGCCACGCCTAGTGGTGTTCGTCGTCGGTGAGACGGCACGCGCCG
MCR-1.7   AAGCCTGATATGCGTAAGCCACGCCTAGTGGTGTTCGTCGTCGGTGAGACGGCACGCGCCG
MCR-1.5   AAGCCTGATATGCGTAAGCCACGCCTAGTGGTGTTCGTCGTCGGTGAGACGGCACGCGCCG
MCR-1.4   AAGCCTGATATGCGTAAGCCACGCCTAGTGGTGTTCGTCGTCGGTGAGACGGCACGCGCCG
MCR-1.6   AAGCCTGATATGCGTAAGCCACGCCTAGTGGTGTTCGTCGTCGGTGAGACGGCACGCGCCG
MCR-1.3   AAGCCTGATATGCGTAAGCCACGCCTAGTGGTGTTCGTCGTCGGTGAGACGGCACGCGCCG
MCR-1.10  AAGCCTGATATGCGTAAGCCACGCCTAGTGGTGTTCGTCGTCGGTGAGACGGCACGCGCCG
MCR-1.9   AAGCCTGATATGCGTAAGCCACGCCTAGTGGTGTTCGTCGTCGGTGAGACGGCACGCGCCG
MCR-2.2   AAGCCTGATATGCGTAAGCCACGCCTAGTGGTGTTCGTCGTCGGTGAGACGGCACGCGCCG
MCR-2     AAGCCGAGCGAGCGTAAGCCACGCCTAGTGGTGTTCGTCGTCGGTGAGACGGCGCGTGCTG
MCR-5     AGAACAAG--GCCGCCGTCCTCGTGCTCTCGTACTGGTTGTCGGGGAAACCGTCAGGGCGG
MCR-3     TAATCAAA--G----TAAGCCCACGTTGATGTTTCTGGTCGTTGGTGAAACCGCTCGTGGTA
MCR-3.2   TAATCAAA--G----TAAGCCCACGTTGATGTTTCTGGTCGTTGGTGAAACCGCTCGTGGTA
MCR-4     TAACCCGA--ACACTAAACCTAACTTATTAGTGGTTGTTGTGGGTGAAACTGCGCGCT-CA
              *             **        *  *  * ** ** ** ** ** *   *
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ATCATGTCAGCTTCA-ATGGCTATGAGCGCGATACTTTCCCACAGCTTGCCAAGATCGA
          ACCATGTGCAGTTCA-ATGGCTATGGCCGTGAGACTTTCCCGCAGCTTGCCAAAGTTGA
          CTAATTGGGGTTGA-GCGGCTATGAACGACAAACCACCCCTGAGTTGGCCGCACGCGA
          AAAATTTCTCGATGA-ATGGCTATGAGAAAGACACCAATCCATTTACCAGTAAATCTGG
          AAAATTTCTCGATGA-ATGGCTATGAGAAAGACACCAATCCATTTACCAGTAAATCTGG
          ATGAGCTATCAATATTATGGATATAACAAGCCAACCAATGCTCATACC--CAAAATCAG
             *        *    **  ***        **      *
MCR-1.2   TGGCGTGACCAATTTTAGCAATGTCACATCGTGCGGCACATCGACGGCGTATTCTGTGCCG
MCR-1.8   TGGCGTGACCAATTTTAGCAATGTCACATCGTGCGGCACATCGACGGCGTATTCTGTGCCG
MCR-1     TGGCGTGACCAATTTTAGCAATGTCACATCGTGCGGCACATCGACGGCGTATTCTGTGCCG
MCR-1.7   TGGCGTGACCAATTTTAGCAATGTCACATCGTGCGGCACATCGACGGCGTATTCTGTGCCG
MCR-1.5   TGGCGTGACCAATTTTAGCAATGTCACATCGTGCGGCACATCGACGGCGTATTCTGTGCCG
MCR-1.4   TGGCGTGACCAATTTTAGCAATGTCACATCGTGCGGCACATCGACGGCGTATTCTGTGCCG
MCR-1.6   TGGCGTGACCAATTTTAGCAATGTCACATCGTGCGGCACATCGACGGCGTATTCTGTGCCG
MCR-1.3   TGGCGTGACCAATTTTAGCAATGTCACATCGTGCGGCACATCGACGGCGTATTCTGTGCCG
MCR-1.10  TGGCGTGACCAATTTTAGCAATGTCACATCGTGCGGCACATCGACGGCGTATTCTGTGCCG
MCR-1.9   TGGCGTGACCAATTTTAGCAATGTCACATCGTGCGGCACATCGACGGCGTATTCTGTGCCG
MCR-2.2   TGGCGTGACCAATTTTAGCAATGTCACATCGTGCGGCACATCGACGGCGTATTCTGTGCCG
MCR-2     TGGCTTGGCGAATTTTAGCCAAGTGACATCGTGTGGCACATCGACGGCGTATTCTGTGCCG
MCR-5     ---CGTGATCAATTTTTCCGATGTCACCAGTTGCGGGACGGATACGGCTACATCCCTTCCC
MCR-3     TGGCGTGATCTCCTTTAATGATGTTCGTTCGTGTGGGACTGCAACCGCTGTATCCGTCCCC
MCR-3.2   TGGCGTGATCTCCTTTAATGATGTTCGTTCGTGTGGGACTGCAACCGCTGTATCCGTCCCC
MCR-4     GGGC-TGATTGCGTTTAACGATACTAGCTCATGCGGCACGGCCACGGCGGTGTCTCTACCC
             * **      ***    *          ** ** **    ** **    ** * **
```

FIGURE 16 (continued)

```
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATCTGGGCGCGGATGAGTATGATGTCGATACCGCCAAATACCAAGA
          TGTATGTTCAGCTATTTGGGTCAAGATGACTATGATGTCGATACCGCCAAATACCAAGA
          TGCATGTTTTCCCTCAATGGTCGGCGCCGACTACGACGAACGCCAGATTCGTCGGCGCGA
          TGCATGTTCTCCAATATGGGGAGAAAGGAGTTTGATGATAATCGCGCTCGCAATAGCGA
          TGCATGTTCTCCAATATGGGGAGAAAGGAGTTTGATGAAAATCGCGCTCGCAATAGCGA
          TGTATGTTTTCACGAATGGGCGGGCAGACTATGATCCTCGCCGTGCTAATGCTCAAGA
          ** *****             **        ** *   **                 **
MCR-1.2   AAATGTGCTGGATACGCTGGATCGCTTGGCCGTAAGTATCTTGTGGCCTGATAATAATTCG
MCR-1.8   AAATGTGCTGGATACGCTGGATCGCTTGGCCGTAAGTATCTTGTGGCCTGATAATAATTCG
MCR-1     AAATGTGCTGGATACGCTGGATCGCTTGGGCGTAAGTATCTTGTGGCGTGATAATAATTCG
MCR-1.7   AAATGTGCTGGATACGCTGGATCGCTTGGGCGTAAGTATCTTGTGGCGTGATAATAATTCG
MCR-1.5   AAATGTGCTGGATACGCTGGATCGCTTGGGCGTAAGTATCTTGTGGCGTGATAATAATTCG
MCR-1.4   AAATGTGCTGGATACGCTGGATCGCTTGGGCGTAAGTATCTTGTGGCGTGATAATAATTCG
MCR-1.6   AAATGTGCTGGATACGCTGGATCGCTTGGGCGTAAGTATCTTGTGGCGTGATAATAATTCG
MCR-1.3   AAATGTGCTGGATACGCTGGATCGCTTGGGCGTAAGTATCTTGTGGCGTGATAATAATTCG
MCR-1.10  AAATGTGCTGGATACGCTGGATCGCTTGGGCGTAAGTATCTTGTGGCGTGATAATAATTCG
MCR-1.9   AAATGTGCTGGATACGCTGGATCGCTTGGGCGTAAGTATCTTGTGGCGTGATAATAATTCG
MCR-2.2   AAATGTGCTGGATACGCTGGATCGCTTGGGCGTAAGTATCTTGTGGCGTGATAATAATTCG
MCR-2     AAATGTGCTAGATACGCTTGACCGCTTGGGTGTGGGTATCTTGTGGCGTGATAATAATTCA
MCR-5     GTCCGTGCTGCACGTTTTAAACCGTAGTGACGTCAACATTCTCTGGCGCGATAACCAGTCG
MCR-3     GGGCCTGCTAGATGTGTTGCAAAAAACGGGGATCTCCATTTTTTGGAAGGAGAACGATGGA
MCR-3.2   GGGCCTGCTAGATGTGTTGCAAAAAACGGGGATCTCCATTTTTTGGAAGGAGAACGATGGA
MCR-4     CACAGTGATTGATGTGTTAAGTCATAGTGGTATAAAAGTACAGTGGTTTGATAATGATTCT
              ** *  *     *           *   *     *    ***   ** **  *
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTGCCAAAAGCGCAATTTGCCGATTATAAATCCGC
          GACTCAAAAGGCGTGATGGATAAGCTACCTGCCACGCAGTATTTTGATTATAAATCAGC
          GGCTGTAAAGGCGTCTGTGATGGACTGCC-------------CTTTGA----AAACCTGT
          GGCTGCAAAGGCGTCTGCGACCGAGTACCTAA----------CATCGA----AATC----
          GGCTGCAAAGGCGTCTGCGACCGAGTACCTAA----------CATCGA----AATC----
          GGCTGTAAAGGTGTGTGTGATCAGGTTGAAAA----------TCTCAC----GATA----
          * **   ***** **      **       *                        *
```

FIGURE 16 (continued)

```
MCR-1.2   GACCAACAACGCCATCTGCAACACCAATCCTTATAACGAATGCCGCGATGTCGGTATGCTC
MCR-1.8   GACCAACAACGCCATCTGCAACACCAATCCTTATAACGAATGCCGCGATGTCGGTATGCTC
MCR-1     GACCAACAACGCCATCTGCAACACCAATCCTTATAACGAATGCCGCGATGTCGGTATGCTC
MCR-1.7   GACCAACAACGCCATCTGCAACACCAATCCTTATAACGAATGCCGCGATGTCGGTATGCTC
MCR-1.5   GACCAACAACGCCATCTGCAACACCAATCCTTATAACGAATGCCGCGATGTCGGTATGCTC
MCR-1.4   GACCAACAACGCCATCTGCAACACCAATCCTTATAACGAATGCCGCGATGTCGGTATGCTC
MCR-1.6   GACCAACAACGCCATCTGCAACACCAATCCTTATAACGAATGCCGCGATGTCGGTATGCTC
MCR-1.3   GACCAACAACGCCATCTGCAACACCAATCCTTATAACGAATGCCGCGATGTCGGTATGCTC
MCR-1.10  GACCAACAACGCCATCTGCAACACCAATCCTTATAACGAATGCCGCGATGTCGGTATGCTC
MCR-1.9   GACCAACAACGCCATCTGCAACACCAATCCTTATAACGAATGCCGCGATGTCGGTATGCTC
MCR-2.2   GACCAACAACGCCATCTGCAACACCAATCCTTATAACGAATGCCGCGATGTCGGTATGCTC
MCR-2     AACCAACAATACCATCTGTAACACCAATCCCTATAACGAATGCCGTGATGTCGGTATGCTT
MCR-5     CTTCGGCAG-GCCATC--CCACACTGTGCC--ATGGCGAGCGCTGCCTGGATGAAATTCT-
MCR-3     GAACCAAAGGATCACCCTAAGTTCTGCGATA-AAAACACATGCTATGACGAGGTTGTCCTT
MCR-3.2   GAACCAAAGGATCACCCTAAGTTCTGCGATA-AAAACACATGCTATGACGAGGTTGTCCTT
MCR-4     GATTTGAAGAGTGATCCGAAGCTGTGTTCTG-GCCAATATTGTTTTGACCAAGTATTGCTC
                 *     * *                         *          *  * **
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTTGGCTTAGATGACTTTGTCGCTGCCAATAA-CGGCAAAGATATGCTGATCATGCTGC
          GTCGGCTAGATGACTATGTCAGCGCCAATAA-TGGCAAAGATATGCTCATCATGCTAC
          GCTCGAAGGGTTGGCCGAGAAGATAACAACAAGCCGCAGCGATATGCTGATCGTTCTGC
          CAAGACCTCGATAG-TGAAATTGCTCAAATGAAAGG----GGATAAGCTGGTTGGCTTCC
          CAAGACCTCGATAG-TGAAATTGCTCAAATGAAAGG----GGATAAGCTGGTTGGCTTCC
          AACAAATTAGATAA-AATTCTGGCAGTAGCACCAAGTCAAGATACAGTAATTTTTTTGC
                   * *             *       *    ****   *  *     * *
MCR-1.2   ACCAAATGGGCAATCACGGGCCTGCGTATTTTAAGCGATATGATGAAAAGTTTGCCAAATTC
MCR-1.8   ACCAAATGGGCAATCACGGGCCTGCGTATTTTAAGCGATATGATGAAAAGTTTGCCAAATTC
MCR-1     ACCAAATGGGCAATCACGGGCCTGCGTATTTTAAGCGATATGATGAAAAGTTTGCCAAATTC
MCR-1.7   ACCAAATGGGCAATCACGGGCCTGCGTATTTTAAGCGATATGATGAAAAGTTTGCCAAATTC
MCR-1.5   ACCAAATGGGCAATCACGGGCCTGCGTATTTTAAGCGATATGATGAAAAGTTTGCCAAATTC
MCR-1.4   ACCAAATGGGCAATCACGGGCCTGCGTATTTTAAGCGATATGATGAAAAGTTTGCCAAATTC
MCR-1.6   ACCAAATGGGCAATCACGGGCCTGCGTATTTTAAGCGATATGATGAAAAGTTTGCCAAATTC
MCR-1.3   ACCAAATGGGCAATCACGGGCCTGCGTATTTTAAGCGATATGATGAAAAGTTTGCCAAATTC
MCR-1.10  ACCAAATGGGCAATCACGGGCCTGCGTATTTTAAGCGATATGATGAAAAGTTTGCCAAATTC
MCR-1.9   ACCAAATGGGCAATCACGGGCCTGCGTATTTTAAGCGATATGATGAAAAGTTTGCCAAATTC
MCR-2.2   ACCAAATGGGCAATCACGGGCCTGCGTATTTTAAGCGATATGATGAAAAGTTTGCCAAATTC
MCR-2     ACCAAATGGGCAATCATGGGCCGGCGTACTTTAAGCGTTATGATGAGCAATTTGCCAAATTC
MCR-5     ATATGCTGGGCAATCACGGCCCAGCGTATTTCCAGCGCTATCCCGCAAGCTACCGACGCTGG
MCR-3     ACCTGATAGGTAGCCATGGCCCAACCTACTACAAGCGCTACCCTGATGCTCATCGTCAGTTC
MCR-3.2   ACCTGATAGGTAGCCATGGCCCAACCTACTACAAGCGCTACCCTGATGCTCATCGTCAGTTC
MCR-4     ATATCATTGGTAGTCATGGACCAACTTATTATCTTAGATACCCGCCAGAGCATCGTAAATTT
          *     * ** *  ** ** **  * ** *      * **                    *
```

FIGURE 16 (continued)

```
         ACGCCAGTGTGTGAAGGTAATGAGCTTGCCAAGTGCGAACATCAGTCCTTGATCAATG
         ACGCCAGTGTGTGAAGGTAATGAGCTTGCCAAGTGCGAACATCAGTCCTTGATCAATG
         ACGCCAGTGTGTGAAGGTAATGAGCTTGCCAAGTGCGAACATCAGTCCTTGATCAATG
         ACGCCAGTGTGTGAAGGTAATGAGCTTGCCAAGTGCGAACATCAGTCCTTGATCAATG
         ACGCCAGTGTGTGAAGGTAATGAGCTTGCCAAGTGCGAACATCAGTCCTTGATCAATG
         ACGCCAGTGTGTGAAGGTAATGAGCTTGCCAAGTGCGAACATCAGTCCTTGATCAATG
         ACGCCAGTGTGTGAAGGTAATGAGCTTGCCAAATGCGAACATCAGTCCTTGATCAATG
         ACGCCAGTGTGTGAAGGTAATGAGCTTGCCAAGTGCGAACATCAGTCCTTGATCAATG
         ACGCCAGCGTGTGAAGGTAATGAGCTTGCCAAGTGCGAACATCAGTCCTTGATCAATG
         ACGCCAGCGTGTGAAGGTAATGAGCTTGCCAAGTGCGAACATCAGTCCTTGATCAATG
         ACGCCAGCGTGTGAAGGTAATGAGCTTGCCAAGTGCGAACATCAGTCCTTGATCAATG
         ACCCCCGTGTGCGAAGGCAACGAGCTTGCCAAATGCGAACACCAATCACTCATCAATG
         TCGCCAACCTGCGACACCACCGATCTGGCCAGCTGTTCGCATGAAGCCTTGGTGAACA
         ACCCCTGACTGTCCACGCAGTGATATTGAAAACTGCACAGATGAAGAGCTCACCAACA
         ACCCCTGACTGTCCACGCAGTGATATTGAAAACTGCACAGATGAAGAGCTCACCAACA
         ATACCGGATTGTCCGCGCAGTGATATTCAAAATTGCAGTCAAGAAGAACTGATTAACA
            **    **      *  **  *    *  **    *  *     *    **
MCR-1.2  CTTATGACAATGCCTTGCTTGCCACCGATGATTTCATCGCTCAAAGTATCCAGTGGCTGCAG
MCR-1.8  CTTATGACAATGCCTTGCTTGCCACCGATGATTTCATCGCTCAAAGTATCCAGTGGCTGCAG
MCR-1    CTTATGACAATGCCTTGCTTGCCACCGATGATTTCATCGCTCAAAGTATCCAGTGGCTGCAG
MCR-1.7  CTTATGACAATGCCTTGCTTGCCACCGATGATTTCATCGCTCAAAGTATCCAGTGGCTGCAG
MCR-1.5  CTTATGACAATGCCTTGCTTGCCACCGATGATTTCATCGCTCAAAGTATCCAGTGGCTGCAG
MCR-1.4  CTTATGACAATGCCTTGCTTGCCACCGATAATTTCATCGCTCAAAGTATCCAGTGGCTGCAG
MCR-1.6  CTTATGACAATGCCTTGCTTGCCACCGATGATTTCATCGCTCAAAGTATCCAGTGGCTGCAG
MCR-1.3  CTTATGACAATGCCTTGCTTGCCACCGATGATTTCATCGCTCAAAGTATCCAGTGGCTGCAG
MCR-1.10 CTTATGACAATGCCTTGCTTGCCACCGATGATTTCATCGCTCAAAGTATCCAGTGGCTGCAG
MCR-1.9  CTTATGACAATGCCTTGCTTGCCACCGATGATTTCATCGCTCAAAGTATCCAGTGGCTGCAG
MCR-2.2  CTTATGACAATGCCTTGCTTGCCACCGATGATTTCATCGCTCAAAGTATCCAGTGGCTGCAG
MCR-2    CCTATGACAATGCGCTACTTGCGACTGATGATTTTATCGCCAAAAGCATCGATTGGCTAAAA
MCR-5    CCTACGACAACGCCGTGCTTTACACCGATCATGTGCTTGCCCGTACCATTGACCTGCTGT--
MCR-3    CCTATGACAACACCATCCGCTACACCGATTTCGTGATTGGAGAGATGATTGCCAAGTTGAAA
MCR-3.2  CCTATGACAACACCATCCGCTACACCGATTTCGTGATTGGAGAGATGATTGCCAAGTTGAAA
MCR-4    CCTACGACAACACTATTCTATATACGGATTTTATTCTCAGTGAAGTGGTGAATAAATTAAAA
         * ** *****  *  * *     ** ***    *  *           *        *
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGTACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCACAGCAATGCCTATGATGTCTCAATGCTGTATGTCAGCGATCATGGCGAAAGTC
         ACGCATGAAGCGAACTACGATGTCGCCATGCTCTATGTCAGTGACCACGGCGAGAGCT
         -CCGGCATCCGCTCACACGACACGGCGCTGCTGTACGTTTCCGATCATGGGGAATCGC
         ACCTACGAAGATAAGTACAACACCGCGTTGCTCTACGTCTCCGATCATGGTGAATCAC
         ACCTACGAAGATAAGTACAACACCGCGTTGCTCTACGTCTCCGATCATGGTGAATCAC
         GGTAAGCAGGATATGTTCGATACTGCAATGCTGTATCTCTCTGACCATGGTGAGTCTT
                             *    *  **** **  *    ** ** ** **
```

FIGURE 16 (continued)

```
MCR-1.2   TGGGTGAGAACGGTGTCTATCTACATGGTATGCCAAATGCCTTTGCACCAAAAGAACAGCGC
MCR-1.8   TGGGTGAGAACGGTGTCTATCTACATGGTATGCCAAATGCCTTTGCACCAAAAGAACAGCGC
MCR-1     TGGGTGAGAACGGTGTCTATCTACATGGTATGCCAAATGCCTTTGCACCAAAAGAACAGCGC
MCR-1.7   TGGGTGAGAACGGTGTCTATCTACATGGTATGCCAAATGCCTTTGCACCAAAAGAACAGCGC
MCR-1.5   TGGGTGAGAACGGTGTCTATCTACATGGTATGCCAAATGCCTTTGCACCAAAAGAACAGCGC
MCR-1.4   TGGGTGAGAACGGTGTCTATCTACATGGTATGCCAAATGCCTTTGCACCAAAAGAACAGCGC
MCR-1.6   TGGGTGAGAACGGTGTCTATCTACATGGTATGCCAAATGCCTTTGCACCAAAAGAACAGCGC
MCR-1.3   TGGGTGAGAACGGTGTCTATCTACATGGTATGCCAAATGCCTTTGCACCAAAAGAACAGCGC
MCR-1.10  TGGGTGAGAACGGTGTCTATCTACATGGTATGCCAAATGCCTTTGCACCAAAAGAACAGCGC
MCR-1.9   TGGGTGAGAACGGTGTCTATCTACATGGTATGCCAAATGCCTTTGCACCAAAAGAACAGCGC
MCR-2.2   TGGGTGAGAACGGTGTCTATCTACATGGTATGCCAAATGCCTTTGCACCAAAAGAACAGCGC
MCR-2     TGGGCGAAAATGGTGTCTATCTGCATGGTATGCCAAATGCCTTTGCACCAAAAGAACAGCGA
MCR-5     TCGGCGAGAAAGGCCTGTATCTCCATGGCATACCTTACGTCATCGCGCCGGATGAGCAGATC
MCR-3     TGGGAGCATTAGGGCTTTACCTACACGGTACACCGTACCAGTTTGCACCGGATGATCAGACC
MCR-3.2   TGGGAGCATTAGGGCTTTACCTACACGGTACACCGTACCAGTTTGCACCGGATGATCAGACC
MCR-4     TGGGTGAAAAGGGCATGTATTTACATGGTGCGCCCTATAGTATTGCACCGAAAGAACAAACT
          * ** *     **  * **  * ** **    **  *     * ** **  * ** **
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          AGTGTGCCTGCATTTTTCTGG---ACGGAT-------------AAGCAAACTGGCATC
          GCTGTGCCTGCGTTTTTTTGG---TCAAAT-------------A---ATACGACATTC
          AAGGTGCCGATGATCTGGTGGCAGTCGAGTC------------AGGTTTATGCCGACC
          CGTGTTCCTATGCAGGTGTGGATGTCACCTGGATTTACCAAAGAGAAAGGCGTTGATA
          CGTGTTCCTATGCAGGTGTGGATGTCACCTGGATTTACCAAAGAGAAAGGCGTTGATA
          AGCGTACCAATGCTGGCTTGGGTATCTAATGACTTTAGCCAAGATAATCAGTTGAACA
              ** **         ***     *   *             *
MCR-1.2   ACGCCA--ATGGCAACCGATACCGTCCTGACCC----ATGACGCGATCACG-----CCGACA
MCR-1.8   ACGCCA--ATGGCAACCGATACCGTCCTGACCC----ATGACGCGATCACG-----CCGACA
MCR-1     ACGCCA--ATGGCAACCGATACCGTCCTGACCC----ATGACGCGATCACG-----CCGACA
MCR-1.7   ACGCCA--ATGGCAACCGATACCGTCCTGACCC----ATGACGCGATCACG-----CCGACA
MCR-1.5   ACGCCA--ATGGCAACCGATACCGTCCTGACCC----ATGACGCGATCACG-----CCGACA
MCR-1.4   ACGCCA--ATGGCAACCGATACCGTCCTGACCC----ATGACGCGATCACG-----CCGACA
MCR-1.6   ACGCCA--ATGGCAACCGATACCGTCCTGACCC----ATGACGCGATCACG-----CCGACA
MCR-1.3   ACGCCA--ATGGCAACCGATACCGTCCTGACCC----ATGACGCGATCACG-----CCGACA
MCR-1.10  ACGCCA--ATGGCAACCGATACCGTCCTGACCC----ATGACGCGATCACG-----CCGACA
MCR-1.9   ACGCCA--ATGGCAACCGATACCGTCCTGACCC----ATGACGCGATCACG-----CCGACA
MCR-2.2   ACGCCA--ATGGCAACCGATACCGTCCTGACCC----ATGACGCGATCACG-----CCGACA
MCR-2     AAGCCA--ACTGCCAGCGATACTGTGCTGACGC----ATGATGCGATTACG-----CCAACA
MCR-5     AAGCCTGTATGCAAACTCATGCCTCTCGGGCACCGGTAAGTCACGATCACCTGTTTCACACC
MCR-3     TGGCGTGTTTGCAGCAGAAAGCCGCTGATACTCGTTACTCACACGATAATATTTTCTCATCT
MCR-3.2   TGGCGTGTTTGCAGCAGAAAGCCGCTGATACTCGTTACTCACACGATAATATTTTCTCATCT
MCR-4     TGACTTGTGTTGCACAGCGAGCAGAACAGGGCGGCTTTCCCACGACAATTTGTTCGACAGT
             *                *                    ***   *
```

FIGURE 16 (continued)

```
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCACACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            TTATTAAAGCTGTTTGATGTCA---CCGCGGACAAA--GTCAAAG----ACCGCACCG
            CTGCTTAAGCTGTTTGATGTCA---CAGCGGGCAAG--GTCAAAG----ACCGCGCGG
            TTGCTCGGATGTTCGACGTGAAAACCGCTGCCTACACGCCAGAGTTGGACCTTCTGG
            GTATTGGGTATCTGGACGTCAAAACATCAGTTTACGAAAAGGGTCTAGATATTTTTCA
            GTATTGGGTATCTGGACGTCAAAACATCAGTTTACGAAAAGGGTCTAGATATTTTTCA
            TTGCTAGGACTTATGAATGTAAAAACCACCGTCTATCAGAGCCAACTCGATATTTTTC
             *  *      *      * ** *    *  * *   *              *
MCR-1.2     CATTCATCCGCTGA----------------
MCR-1.8     CATTCATCCGCTGA----------------
MCR-1       CATTCATCCGCTGA----------------
MCR-1.7     CATTCATCCGCTGA----------------
MCR-1.5     CATTCATCCGCTGA----------------
MCR-1.4     CATTCATCCGCTGA----------------
MCR-1.6     CATTCATCCGCTGA----------------
MCR-1.3     CATTCATCCGCTGA----------------
MCR-1.10    CATTCATCCGCTGA----------------
MCR-1.9     CATTCATCCGCTGA----------------
MCR-2.2     CATTCATCCGCTGA----------------
MCR-2       CATTTATCCAGTAA----------------
MCR-5       CAA-CATGCAGAAAAGGACAACCACAATGA
MCR-3       GTC-AATGTCGTAATGTTCAATAA------
MCR-3.2     GTC-AATGTCGTAATGTTCAATAA------
MCR-4       CAC-CTTGCAG--GTATTAG----------
                  *
```

FIGURE 17

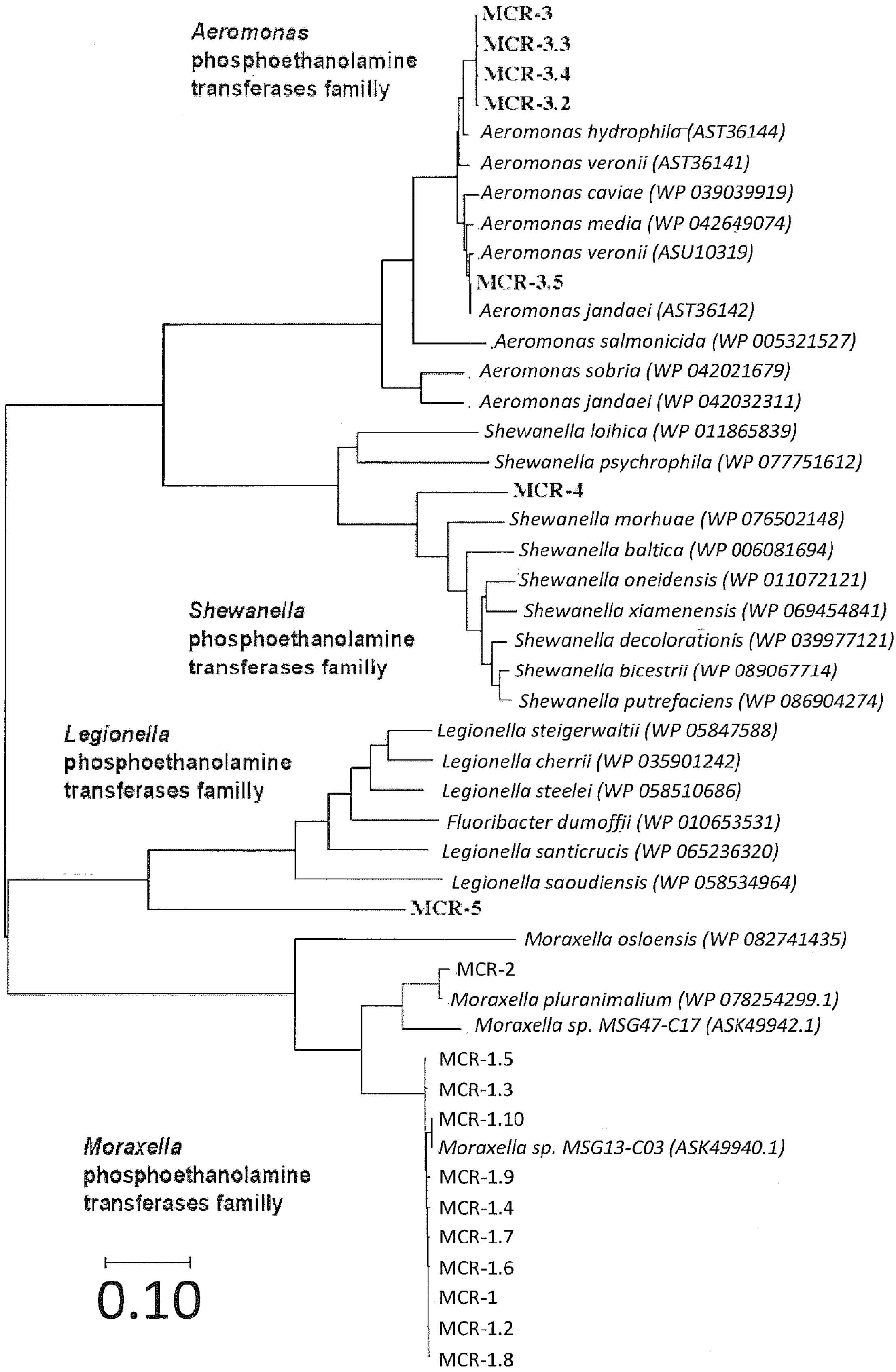
*Aeromonas* phosphoethanolamine transferases familly
MCR-3
MCR-3.3
MCR-3.4
MCR-3.2
Aeromonas hydrophila (AST36144)
Aeromonas veronii (AST36141)
Aeromonas caviae (WP 039039919)
Aeromonas media (WP 042649074)
Aeromonas veronii (ASU10319)
MCR-3.5
Aeromonas jandaei (AST36142)
Aeromonas salmonicida (WP 005321527)
Aeromonas sobria (WP 042021679)
Aeromonas jandaei (WP 042032311)
Shewanella loihica (WP 011865839)
Shewanella psychrophila (WP 077751612)
MCR-4
Shewanella morhuae (WP 076502148)
Shewanella baltica (WP 006081694)
Shewanella oneidensis (WP 011072121)
Shewanella xiamenensis (WP 069454841)
Shewanella decolorationis (WP 039977121)
Shewanella bicestrii (WP 089067714)
Shewanella putrefaciens (WP 086904274)
*Shewanella* phosphoethanolamine transferases familly
*Legionella* phosphoethanolamine transferases familly
Legionella steigerwaltii (WP 05847588)
Legionella cherrii (WP 035901242)
Legionella steelei (WP 058510686)
Fluoribacter dumoffii (WP 010653531)
Legionella santicrucis (WP 065236320)
Legionella saoudiensis (WP 058534964)
MCR-5
Moraxella osloensis (WP 082741435)
MCR-2
Moraxella pluranimalium (WP 078254299.1)
Moraxella sp. MSG47-C17 (ASK49942.1)
MCR-1.5
MCR-1.3
MCR-1.10
Moraxella sp. MSG13-C03 (ASK49940.1)
MCR-1.9
MCR-1.4
MCR-1.7
MCR-1.6
MCR-1
MCR-1.2
MCR-1.8
*Moraxella* phosphoethanolamine transferases familly
0.10

FIGURE 18

| Strain[a] | Polymyxin resistance | | | Acquired resistance to β-lactams[c] | $^{d}PRR_{E. coli}$ |
|---|---|---|---|---|---|
| | Colistin MIC (mg/L) | Chromosome / Plasmid | Mechanism[b] | | |
| CNR 111J7 | 6 | Chromosome | PmrB mutations (D14N, S71C and V83A) | CTX-M-15 + TEM-1 | 0·25 ± 0·08 |
| CNR20160039 | 4 | Chromosome | Unknown | None | 0·38 ± 0·02 |
| CNR20160235 | 8 | Chromosome | MgrB mutation (V8A) | TEM-1 | 0·19 ± 0·05 |
| CNR 1728 | 8 | Chromosome | PmrB mutation (G160E) | None | 0·30 ± 0·08 |
| 41489 | 4 | Plasmid | MCR-1 | None | 0·92 ± 0·29 |
| J53 + *mcr-1*[*] | 8 | Plasmid | MCR-1 | None | 2·43 ± 1·10 |
| CNR20140385 | 4 | Plasmid | MCR-1 | **OXA-48** | 1·88 ± 0·28 |
| S08-056 | 4 | Plasmid | MCR-1 | **OXA-48** | 3·70 ± 0·40 |
| CNR 117G7 | 4 | Plasmid | MCR-1 | **NDM-1** | 2·28 ± 0·59 |
| CNR 121G9 | 4 | Plasmid | MCR-1 | **OXA-48** | 0·71 ± 0·04 |
| CNR 1745 | 4 | Plasmid | MCR-1 | SHV-12 | 2·08 ± 0·24 |
| CNR 1604 | 4 | Plasmid | MCR-1 | CTX-M-15 | 3·48 ± 0·25 |
| CNR 1667 | 4 | Plasmid | MCR-1 | **OXA-48** + KPC-28 | 1·67 ± 0·80 |
| CNR 1790 | 4 | Plasmid | MCR-1 | TEM-15 | 2·69 ± 0·63 |
| CNR 1859 | 4 | Plasmid | MCR-1 | CTX-M-15 + SHV-12 + TEM-1 | 2·86 ± 0·37 |
| CNR 1886 | 4 | Plasmid | MCR-1 | CTX-M-1 + TEM-1 | 2·61 ± 0·30 |
| 4222 | 4 | Plasmid | MCR-1 | CTX-M-2 | 4·32 ± 0·51 |
| 4070 | 4 | Plasmid | MCR-1 | TEM-1B | 1·65 ± 0·28 |
| 979 | 4 | Plasmid | MCR-1 | CTX-M-2 | 1·21 ± 0·14 |
| 1724 | 4 | Plasmid | MCR-1 | None | 2·07 ± 0·37 |
| DH5α + pDM1-*mcr-1*[**] | 4 | Plasmid | MCR-1 | None | 2·82 ± 0·92 |

FIGURE 18 (continued)

| | | | | | |
|---|---|---|---|---|---|
| MC1000 + pDM1- *mcr-1*** | 4 | Plasmid | MCR-1 | None | 3·13 ± 0·33 |
| 6383 | 4 | Plasmid | MCR-1.5 | TEM-1B | 2·85 ± 0·76 |
| 1670 | 4 | Plasmid | MCR-1.5 | CTX-M-2 | 0·92 ± 0·18 |
| [e]R12 F5 | 4 | Plasmid | MCR-2 | None | 3·13 ± 1·05 |
| DH5α + pDM1- *mcr-2*** | 4 | Plasmid | MCR-2 | None | 2·24 ± 0·51 |
| MC1000 + pDM1- *mcr-2*** | 4 | Plasmid | MCR-2 | None | 1·80 ± 0·35 |
| DH5α + pDM1- *mcr-3*** | 4 | Plasmid | MCR-3 | None | 2·62 ± 0·47 |
| MC1000 + pDM1- *mcr-3*** | 4 | Plasmid | MCR-3 | None | 2·80 ± 0·15 |
| DH5α + pDM1- *mcr-5*** | 4 | Plasmid | MCR-5 | None | 2·14 ± 0·04 |
| MC1000 + pDM1- *mcr-5** | 4 | Plasmid | MCR-5 | None | 3·12 ± 0·57 |
| TOP 10 + *mcr-5** | 8 | Plasmid | MCR-5 | None | 1·59 ± 0·29 |
| J53 + *mcr-5** | 8 | Plasmid | MCR-5 | None | 1·54 ± 0·36 |
| J53 | 0·5 | - | - | None | 0·00 ± 0·00 |
| TOP 10 | 0·25 | - | - | None | 0·00 ± 0·00 |
| DH5α | 0·5 | - | - | None | 0·00 ± 0·00 |
| DH5α + pDM1-Empty | 0·5 | - | - | None | 0·00 ± 0·00 |
| MC1000 + pDM1-Empty | 0·5 | - | - | None | 0·00 ± 0·00 |
| 1608071881 | 0·25 | - | - | None | 0·00 ± 0·00 |
| 1608072264 | 0·25 | - | - | None | 0·00 ± 0·00 |
| 1608073740 | 0·25 | - | - | None | 0·00 ± 0·00 |

FIGURE 18 (continued)

| | | | | | |
|---|---|---|---|---|---|
| 1608070579 | 0·5 | - | - | None | 0·00 ± 0·00 |
| 1608073561 | 0·25 | - | - | None | 0·00 ± 0·00 |
| 1608073733 | 0·5 | - | - | None | 0·00 ± 0·00 |
| 1608073937 | 0·25 | - | - | None | 0·00 ± 0·00 |
| 1608079010 | 0·25 | - | - | None | 0·00 ± 0·00 |
| 1608079029 | 0·25 | - | - | None | 0·00 ± 0·00 |
| 1608079018 | 0·25 | - | - | None | 0·00 ± 0·00 |
| 1608075385 | 0·12 | - | - | TEM-1 | 0·00 ± 0·00 |
| 1608078198 | 0·25 | - | - | TEM-1 | 0·00 ± 0·00 |
| 1608073301 | 0·25 | - | - | TEM-1 | 0·00 ± 0·00 |
| 1608078105 | 0·25 | - | - | TEM-1 | 0·00 ± 0·00 |
| 1608073228 | 0·25 | - | - | TEM-1 | 0·00 ± 0·00 |
| 1608078635 | 0·25 | - | - | TEM-1 | 0·00 ± 0·00 |
| 1608078858 | 0·25 | - | - | TEM-1 | 0·00 ± 0·00 |
| 1608062671 | 0·25 | - | - | TEM-1 | 0·00 ± 0·00 |
| 1608064819 | 0·25 | - | - | TEM-1 | 0·00 ± 0·00 |
| 1608062559 | 0·12 | - | - | TEM-1 | 0·00 ± 0·00 |
| 2E6 | 0·25 | - | - | CTX-M-1 | 0·00 ± 0·00 |
| 2E7 | 0·25 | - | - | CTX-M-3 | 0·00 ± 0·00 |
| 2E9 | 0·25 | - | - | CTX-M-14 | 0·00 ± 0·00 |
| 2E10 | 0·25 | - | - | CTX-M-14 | 0·00 ± 0·00 |
| 2H6 | 0·25 | - | - | CTX-M-15 | 0·00 ± 0·00 |
| 2F2 | 0·25 | - | - | CTX-M-15 | 0·00 ± 0·00 |
| 2F3 | 0·25 | - | - | CTX-M-15 | 0·00 ± 0·00 |
| LAN 10.48 | 0·25 | - | - | SHV-2a | 0·00 ± 0·00 |
| LEC 11.8 | 0·5 | - | - | SHV-12 | 0·00 ± 0·00 |
| VER 9.39 | 0·25 | - | - | TEM-3 | 0·00 ± 0·00 |
| NEG 11.87 | 0·25 | - | - | TEM-24 | 0·00 ± 0·00 |
| 1F1 | 0·25 | - | - | **KPC-2** | 0·00 ± 0·00 |
| 1A2 | 0·5 | - | - | **NDM-1** + OXA-1 + TEM-1 | 0·00 ± 0·00 |

FIGURE 18 (continued)

| | | | | | |
|---|---|---|---|---|---|
| 1A6 | 0·25 | - | - | **NDM-4** + CTX-M-15 + OXA-1 | 0·00 ± 0·00 |
| 1A8 | 0·25 | - | - | **NDM-5** + CTX-M-15 + TEM-1 | 0·00 ± 0·00 |
| 1C2 | 0·5 | - | - | **VIM-1** | 0·00 ± 0·00 |
| 1D10 | 0·25 | - | - | **IMP-8** + SHV-12 | 0·00 ± 0·00 |
| 2A1 | 0·25 | - | - | **OXA-48** + CTX-M-15 | 0·00 ± 0·00 |
| 2C4 | 0·5 | - | - | **OXA-181** | 0·00 ± 0·00 |
| 2D5 | 0·25 | - | - | **OXA-204** + CMY-4 + CTX-M-15 | 0·00 ± 0·00 |
| 2D9 | 0·25 | - | - | **OXA-244** | 0·00 ± 0·00 |

FIGURE 19

| Strain[a] | Polymyxin resistance | | | $PRR_{E. coli}$ for bacterial colonies aged of : | | | |
|---|---|---|---|---|---|---|---|
| | Colistin MIC (mg/L) | Chromosome / Plasmid | Mechanism[b] | 24h | 48h | 1 week | 2 weeks |
| CNR 111J7 | 6 | Chromosome | PmrB mutations (D14N, S71C and V83A) | 0·25 ± 0·08 | 0·20 ± 0·06 | 0·22 ± 0·05 | 0·25 ± 0·10 |
| CNR20160039 | 4 | Chromosome | Unknown | 0·38 ± 0·02 | 0·36 ± 0·05 | 0·32 ± 0·04 | 0·30 ± 0·08 |
| CNR20160235 | 8 | Chromosome | MgrB mutation (V8A) | 0·19 ± 0·05 | 0·22 ± 0·06 | 0·18 ± 0·03 | 0·24 ± 0·09 |
| CNR 1728 | 8 | Chromosome | PmrB mutation (G160E) | 0·30 ± 0·08 | 0·27 ± 0·04 | 0·28 ± 0·06 | 0·32 ± 0·09 |
| 41489 | 4 | Plasmid | MCR-1 | 0·92 ± 0·29 | 0·89 ± 0·12 | 0·81 ± 0·13 | 0·83 ± 0·19 |
| J53 + *mcr-1*[*] | 8 | Plasmid | MCR-1 | 2·43 ± 1·10 | 1·61 ± 0·90 | 1·89 ± 0·83 | 2·06 ± 0·52 |
| CNR20140385 | 4 | Plasmid | MCR-1 | 1·88 ± 0·28 | 1.72 ± 0·15 | 1·68 ± 0·32 | 1·55 ± 0·22 |
| S08-056 | 4 | Plasmid | MCR-1 | 3·70 ± 0·40 | 3·10 ± 0·52 | 3·01 ± 0·38 | 2·92 ± 0·61 |
| CNR 117G7 | 4 | Plasmid | MCR-1 | 2·28 ± 0·59 | 2·32 ± 0·32 | 2·23 ± 0·41 | 2·08 ± 0·14 |
| CNR 121G9 | 4 | Plasmid | MCR-1 | 0·71 ± 0·04 | 0·73 ± 0·12 | 0·81 ± 0·10 | 0·64 ± 0·08 |
| CNR 1745 | 4 | Plasmid | MCR-1 | 2·08 ± 0·24 | 1·92 ± 0·29 | 1·98 ± 0·27 | 1·87 ± 0·33 |
| CNR 1604 | 4 | Plasmid | MCR-1 | 3·48 ± 0·25 | 3·00 ± 0·52 | 3·17 ± 0·37 | 2·88 ± 0·90 |
| CNR 1667 | 4 | Plasmid | MCR-1 | 1·67 ± 0·80 | 1·28 ± 0·42 | 1·46 ± 0·63 | 1·32 ± 0·86 |
| CNR 1790 | 4 | Plasmid | MCR-1 | 2·69 ± 0·63 | 2·04 ± 0·47 | 2·13 ± 0·74 | 1·99 ± 0·58 |
| CNR 1859 | 4 | Plasmid | MCR-1 | 2·86 ± 0·37 | 2·73 ± 0·41 | 2·54 ± 0·23 | 2·06 ± 1·02 |
| CNR 1886 | 4 | Plasmid | MCR-1 | 2·61 ± 0·30 | 2·32 ± 0·48 | 2·31 ± 0·25 | 2·01 ± 0·67 |
| 4222 | 4 | Plasmid | MCR-1 | 4·32 ± 0·51 | 4·17 ± 0·63 | 3·99 ± 0·47 | 3·71 ± 0·39 |
| 4070 | 4 | Plasmid | MCR-1 | 1·65 ± 0·28 | 1·55 ± 0·31 | 1·54 ± 0·24 | 1·42 ± 0·38 |
| 979 | 4 | Plasmid | MCR-1 | 1·21 ± 0·14 | 1·25 ± 0·23 | 1·05 ± 0·18 | 1·09 ± 0·36 |
| 1724 | 4 | Plasmid | MCR-1 | 2·07 ± 0·37 | 2·00 ± 0·41 | 1·92 ± 0·32 | 1·85 ± 0·33 |

FIGURE 19 (continued)

| | | | | | | | |
|---|---|---|---|---|---|---|---|
| 6383 | 4 | Plasmid | MCR-1.5 | 2·85 ± 0·76 | 2·66 ± 0·63 | 2·65 ± 0·48 | 2·21 ± 0·77 |
| 1670 | 4 | Plasmid | MCR-1.5 | 0·92 ± 0·18 | 1·06 ± 0·38 | 0·91 ± 0·15 | 0·99 ± 0·30 |
| R12 F5 | 4 | Plasmid | MCR-2 | 3·13 ± 1·05 | 2·23 ± 0·55 | 2·79 ± 0·89 | 2·13 ± 1·17 |
| J53 | 0·5 | - | - | 0·00 ± 0·00 | 0·00 ± 0·00 | 0·00 ± 0·00 | 0·00 ± 0·00 |
| TOP 10 | 0·25 | - | - | 0·00 ± 0·00 | 0·00 ± 0·00 | 0·00 ± 0·00 | 0·00 ± 0·00 |
| DH5α | 0·5 | - | - | 0·00 ± 0·00 | 0·00 ± 0·00 | 0·00 ± 0·00 | 0·00 ± 0·00 |
| 1608071881 | 0·25 | - | - | 0·00 ± 0·00 | 0·00 ± 0·00 | 0·00 ± 0·00 | 0·00 ± 0·00 |
| 1608072264 | 0·25 | - | - | 0·00 ± 0·00 | 0·00 ± 0·00 | 0·00 ± 0·00 | 0·00 ± 0·00 |
| 1608073740 | 0·25 | - | - | 0·00 ± 0·00 | 0·00 ± 0·00 | 0·00 ± 0·00 | 0·00 ± 0·00 |
| 1608070579 | 0·5 | - | - | 0·00 ± 0·00 | 0·00 ± 0·00 | 0·00 ± 0·00 | 0·00 ± 0·00 |
| 1608073561 | 0·25 | - | - | 0·00 ± 0·00 | 0·00 ± 0·00 | 0·00 ± 0·00 | 0·00 ± 0·00 |
| 1608073733 | 0·5 | - | - | 0·00 ± 0·00 | 0·00 ± 0·00 | 0·00 ± 0·00 | 0·00 ± 0·00 |
| 1608073937 | 0·25 | - | - | 0·00 ± 0·00 | 0·00 ± 0·00 | 0·00 ± 0·00 | 0·00 ± 0·00 |
| 1608079010 | 0·25 | - | - | 0·00 ± 0·00 | 0·00 ± 0·00 | 0·00 ± 0·00 | 0·00 ± 0·00 |
| 1608079029 | 0·25 | - | - | 0·00 ± 0·00 | 0·00 ± 0·00 | 0·00 ± 0·00 | 0·00 ± 0·00 |
| 1608079018 | 0·25 | - | - | 0·00 ± 0·00 | 0·00 ± 0·00 | 0·00 ± 0·00 | 0·00 ± 0·00 |
| 1608075385 | 0·12 | - | - | 0·00 ± 0·00 | 0·00 ± 0·00 | 0·00 ± 0·00 | 0·00 ± 0·00 |
| 1608078198 | 0·25 | - | - | 0·00 ± 0·00 | 0·00 ± 0·00 | 0·00 ± 0·00 | 0·00 ± 0·00 |
| 1608073301 | 0·25 | - | - | 0·00 ± 0·00 | 0·00 ± 0·00 | 0·00 ± 0·00 | 0·00 ± 0·00 |
| 1608078105 | 0·25 | - | - | 0·00 ± 0·00 | 0·00 ± 0·00 | 0·00 ± 0·00 | 0·00 ± 0·00 |
| 1608073228 | 0·25 | - | - | 0·00 ± 0·00 | 0·00 ± 0·00 | 0·00 ± 0·00 | 0·00 ± 0·00 |
| 1608078635 | 0·25 | - | - | 0·00 ± 0·00 | 0·00 ± 0·00 | 0·00 ± 0·00 | 0·00 ± 0·00 |
| 1608078858 | 0·25 | - | - | 0·00 ± 0·00 | 0·00 ± 0·00 | 0·00 ± 0·00 | 0·00 ± 0·00 |
| 1608062671 | 0·25 | - | - | 0·00 ± 0·00 | 0·00 ± 0·00 | 0·00 ± 0·00 | 0·00 ± 0·00 |
| 1608064819 | 0·25 | - | - | 0·00 ± 0·00 | 0·00 ± 0·00 | 0·00 ± 0·00 | 0·00 ± 0·00 |

FIGURE 19 (continued)

| | | | | | | | |
|---|---|---|---|---|---|---|---|
| 1608062559 | 0·12 | - | - | 0·00 ± 0·00 | 0·00 ± 0·00 | 0·00 ± 0·00 | 0·00 ± 0·00 |
| 2E6 | 0·25 | - | - | 0·00 ± 0·00 | 0·00 ± 0·00 | 0·00 ± 0·00 | 0·00 ± 0·00 |
| 2E7 | 0·25 | - | - | 0·00 ± 0·00 | 0·00 ± 0·00 | 0·00 ± 0·00 | 0·00 ± 0·00 |
| 2E9 | 0·25 | - | - | 0·00 ± 0·00 | 0·00 ± 0·00 | 0·00 ± 0·00 | 0·00 ± 0·00 |
| 2E10 | 0·25 | - | - | 0·00 ± 0·00 | 0·00 ± 0·00 | 0·00 ± 0·00 | 0·00 ± 0·00 |
| 2H6 | 0·25 | - | - | 0·00 ± 0·00 | 0·00 ± 0·00 | 0·00 ± 0·00 | 0·00 ± 0·00 |
| 2F2 | 0·25 | - | - | 0·00 ± 0·00 | 0·00 ± 0·00 | 0·00 ± 0·00 | 0·00 ± 0·00 |
| 2F3 | 0·25 | - | - | 0·00 ± 0·00 | 0·00 ± 0·00 | 0·00 ± 0·00 | 0·00 ± 0·00 |
| LAN 10.48 | 0·25 | - | - | 0·00 ± 0·00 | 0·00 ± 0·00 | 0·00 ± 0·00 | 0·00 ± 0·00 |
| LEC 11.8 | 0·5 | - | - | 0·00 ± 0·00 | 0·00 ± 0·00 | 0·00 ± 0·00 | 0·00 ± 0·00 |
| VER 9.39 | 0·25 | - | - | 0·00 ± 0·00 | 0·00 ± 0·00 | 0·00 ± 0·00 | 0·00 ± 0·00 |
| NEG 11.87 | 0·25 | - | - | 0·00 ± 0·00 | 0·00 ± 0·00 | 0·00 ± 0·00 | 0·00 ± 0·00 |
| 1F1 | 0·25 | - | - | 0·00 ± 0·00 | 0·00 ± 0·00 | 0·00 ± 0·00 | 0·00 ± 0·00 |
| 1A2 | 0·5 | - | - | 0·00 ± 0·00 | 0·00 ± 0·00 | 0·00 ± 0·00 | 0·00 ± 0·00 |
| 1A6 | 0·25 | - | - | 0·00 ± 0·00 | 0·00 ± 0·00 | 0·00 ± 0·00 | 0·00 ± 0·00 |
| 1A8 | 0·25 | - | - | 0·00 ± 0·00 | 0·00 ± 0·00 | 0·00 ± 0·00 | 0·00 ± 0·00 |
| 1C2 | 0·5 | - | - | 0·00 ± 0·00 | 0·00 ± 0·00 | 0·00 ± 0·00 | 0·00 ± 0·00 |
| 1D10 | 0·25 | - | - | 0·00 ± 0·00 | 0·00 ± 0·00 | 0·00 ± 0·00 | 0·00 ± 0·00 |
| 2A1 | 0·25 | - | - | 0·00 ± 0·00 | 0·00 ± 0·00 | 0·00 ± 0·00 | 0·00 ± 0·00 |
| 2C4 | 0·5 | - | - | 0·00 ± 0·00 | 0·00 ± 0·00 | 0·00 ± 0·00 | 0·00 ± 0·00 |
| 2D5 | 0·25 | - | - | 0·00 ± 0·00 | 0·00 ± 0·00 | 0·00 ± 0·00 | 0·00 ± 0·00 |
| 2D9 | 0·25 | - | - | 0·00 ± 0·00 | 0·00 ± 0·00 | 0·00 ± 0·00 | 0·00 ± 0·00 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012197535 A1
- US 5223409 A, Ladner
- WO 9206204 A

### Non-patent literature cited in the description


- **ARROYO et al.** *Antimicrobial agents and chemotherapy,* 2011, vol. 55 (8), 3743-3751
- **A. TRAN et al.** *Journal of Biological Chemistry,* 2004, vol. 279 (53), 55780-55791
- **PENDELA et al.** *Rapid Communications in Mass Spectrometry,* 2009, vol. 23 (12), 1856-1862
- **PELLETIER et al.** *Antimicrobial Agents and Chemotherapy,* 2013, vol. 57 (10), 4831-4840
- **QURESHI et al.** *Clinical Infectious Diseases,* 2015, vol. 60 (9), 1295-1303
- **PARK et al.** *Clinical Microbiology and Infection,* 2015, vol. 21 (8), 765
- **JULIE D. THOMPSON et al.** CLUSTAL W: Improving the Sensitivity of Progressive Multiple Sequence Alignment Through Sequence Weighting, Position-Specific Gap Penalties and Weight Matrix Choice. *Nucleic Acids Research,* 1994, vol. 22 (22), 4673-4680
- **OSAMU GOTOH.** Significant Improvement in Accuracy of Multiple Protein. Sequence Alignments by Iterative Refinement as Assessed by Reference to Structural Alignments. *J. Mol. Biol.,* 1996, vol. 264 (4), 823-838
- **ERIC DEPIEREUX ; ERNEST FEYTMANS.** Match-Box: A Fundamentally New Algorithm for the Simultaneous Alignment of Several Protein Sequences. *CABIOS,* 1992, vol. 8 (5), 501-509
- **C. E. LAWRENCE et al.** Detecting Subtle Sequence Signals: A Gibbs Sampling Strategy for Multiple Alignment. *Science,* 1993, vol. 262 (5131), 208-214
- **IVO VAN WALLE et al.** Align-M - A New Algorithm for Multiple Alignment of Highly Divergent Sequences. *Bioinformatics,* 2004, vol. 20 (9), 1428-1435
- **ALTSCHUL et al.** *Bull. Math. Bio.,* 1986, vol. 48, 603-16
- **HENIKOFF ; HENIKOFF.** *Proc. Natl. Acad. Sci. USA,* 1992, vol. 89, 10915-19
- **ROBERTSON et al.** *J. Am. Chem. Soc.,* 1991, vol. 113, 2722
- **ELLMAN et al.** *Methods Enzymol.,* 1991, vol. 202, 301
- **CHUNG et al.** *Science,* 1993, vol. 259, 806-9
- **CHUNG et al.** *Proc. Natl. Acad. Sci. USA,* 1993, vol. 90, 10145-9
- **TURCATTI et al.** *J. Biol. Chem.,* 1996, vol. 271, 19991-8
- **KOIDE et al.** *Biochem.,* 1994, vol. 33, 7470-6
- **WYNN ; RICHARDS.** *Protein Sci.,* 1993, vol. 2, 395-403
- **CUNNINGHAM ; WELLS.** *Science,* 1989, vol. 244, 1081-5
- **DE VOS et al.** *Science,* 1992, vol. 255, 306-12
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-7
- **BOWIE ; SAUER.** *Proc. Natl. Acad. Sci. USA,* 1989, vol. 86, 2152-6
- **LOWMAN et al.** *Biochem.,* 1991, vol. 30, 10832-7
- **DERBYSHIRE et al.** *Gene,* 1986, vol. 46, 145
- **NER et al.** *DNA,* 1988, vol. 7, 127
- **SINGLETON et al.** DICTIONARY OF MICROBIOLOGY AND MOLECULAR BIOLOGY. John Wiley and Sons, 1994
- **HALE ; MARHAM.** HARPER COLLINS DICTIONARY OF BIOLOGY. 1991
- **XAVIER BB et al.** *Euro surveillance,* 2016, vol. 21, 27
- **BOROWIAK M et al.** *Journal of Antimicrobial Chemotherapy,* 2017, vol. 72 (12), 3317-3324